# EUROPEAN PATENT SPECIFICATION

(11) **EP 2 802 215 B1**
(45) Date of publication and mention of the grant of the patent: **15.09.2021**
(21) Application number: 13736137.4
(22) Date of filing: 11.01.2013
(51) Int. Cl.: A01P 21/00, A01P 5/00, A01H 3/04, C12N 15/29

(54) **METHOD FOR MODULATING PLANT ROOT ARCHITECTURE**
VERFAHREN ZUR MODULATION DER PFLANZENWURZELARCHITEKTUR
PROCÉDÉ POUR MODULER L'ARCHITECTURE RACINAIRE D'UNE PLANTE

(30) Priority: 11.01.2012 AU 2012900109
(43) Date of publication of application: 19.11.2014
(73) Proprietor: The Australian National University, Acton, ACT 0200 (AU); North Carolina State University, Raleigh NC 27695-8210 (US)
(72) Inventor: DJORDJEVIC, Michael, Kingston, ACT 2604 (AU); IMIN, Nijat, Kaleen, ACT 2617 (AU); BIRD, David McKenzie, Raleigh, NC 27608 (US); DIGENNARO, Peter Michael, Raleigh, NC 27615 (US); RADZMAN, Nadiatul Akmal Mohd, Canberra, ACT 2601 (AU)
(74) Representative: Bösl, Raphael Konrad
(86) International application number: PCT/AU2013/000022
(87) International publication number: WO 2013/104026

(56) References cited:
- EP-A2- 1 033 405
- OELKERS KARSTEN ET AL: "Bioinformatic analysis of the CLE signaling peptide family", BMC PLANT BIOLOGY, BIOMED CENTRAL, LONDON, GB, vol. 8, no. 1, 3 January 2008 (2008-01-03) , page 1, XP021033828, ISSN: 1471-2229
- Anonymous: "At2g35612 - Uncharacterized protein - Arabidopsis thaliana (Mouse-ear cress)", , 8 November 2005 (2005-11-08), XP55218477, Retrieved from the Internet: URL:http://www.uniprot.org/uniprot/Q3EBM6 [retrieved on 2015-10-06]
- Anonymous: "At5g66816 - Uncharacterized protein - Arabidopsis thaliana (Mouse-ear cress)", , 22 July 2008 (2008-07-22), XP55218485, Retrieved from the Internet: URL:http://www.uniprot.org/uniprot/B3H5A9 [retrieved on 2015-10-06]
- TAKAO ARAYA ET AL: "CLE-CLAVATA1 peptide-receptor signaling module regulates the expansion of plant root systems in a nitrogen-dependent manner", PROCEEDINGS OF THE NATIONAL ACADEMY OF SCIENCES, vol. 111, no. 5, 21 January 2014 (2014-01-21), pages 2029-2034, XP55217843, ISSN: 0027-8424, DOI: 10.1073/pnas.1319953111
- EMELINE HUAULT ET AL: "Local and Systemic Regulation of Plant Root System Architecture and Symbiotic Nodulation by a Receptor-Like Kinase", PLOS GENETICS, vol. 10, no. 12, 18 December 2014 (2014-12-18), page e1004891, XP55217833, DOI: 10.1371/journal.pgen.1004891
- T. KONDO ET AL: "A Plant Peptide Encoded by CLV3 Identified by in Situ MALDI-TOF MS Analysis", SCIENCE, vol. 313, no. 5788, 11 August 2006 (2006-08-11), pages 845-848, XP55078270, ISSN: 0036-8075, DOI: 10.1126/science.1128439
- KENTARO OHYAMA ET AL: "Identification of a biologically active, small, secreted peptide in Arabidopsis by in silico gene screening, followed by LC-MS-based structure analysis", THE PLANT JOURNAL, vol. 55, no. 1, 1 July 2008 (2008-07-01), pages 152-160, XP55078268, ISSN: 0960-7412, DOI: 10.1111/j.1365-313X.2008.03464.x
- Y. MATSUZAKI ET AL: "Secreted Peptide Signals Required for Maintenance of Root Stem Cell Niche in Arabidopsis", SCIENCE, vol. 329, no. 5995, 27 August 2010 (2010-08-27), pages 1065-1067, XP55218569, US ISSN: 0036-8075, DOI: 10.1126/science.1191132
- LERON KATSIR ET AL: "Peptide Signaling in Plant Development", CURRENT BIOLOGY, vol. 21, no. 9, 10 May 2011 (2011-05-10), pages R356-R364, XP028384486, ISSN: 0960-9822, DOI: 10.1016/J.CUB.2011.03.012 [retrieved on 2011-04-12]
- K LINDSEY: "Peptides: new signalling molecules in plants", TRENDS IN PLANT SCIENCE, vol. 7, no. 2, 1 February 2002 (2002-02-01), pages 78-83, XP55218282, ISSN: 1360-1385, DOI: 10.1016/S1360-1385(01)02194-X
- I. DE SMET ET AL: "Receptor-Like Kinase ACR4 Restricts Formative Cell Divisions in the Arabidopsis Root", SCIENCE, vol. 322, no. 5901, 24 October 2008 (2008-10-24), pages 594-597, XP55218280, ISSN: 0036-8075, DOI: 10.1126/science.1160158
- BUTENKO M A ET AL: "Plant peptides in signalling: looking for new partners", TRENDS IN PLANT SCIENCE, ELSEVIER SCIENCE, OXFORD, GB, vol. 14, no. 5, 1 May 2009 (2009-05-01), pages 255-263, XP026096935, ISSN: 1360-1385, DOI: 10.1016/J.TPLANTS.2009.02.002 [retrieved on 2009-04-09]
- S. A. CASSON ET AL: "The POLARIS Gene of Arabidopsis Encodes a Predicted Peptide Required for Correct Root Growth and Leaf Vascular Patterning", THE PLANT CELL ONLINE, vol. 14, no. 8, 1 August 2002 (2002-08-01) , pages 1705-1721, XP55218278, DOI: 10.1105/tpc.002618
- Y. MATSUBAYASHI: "Post-Translational Modifications in Secreted Peptide Hormones in Plants", PLANT AND CELL PHYSIOLOGY, vol. 52, no. 1, 11 November 2010 (2010-11-11), pages 5-13, XP55218290, ISSN: 0032-0781, DOI: 10.1093/pcp/pcq169
- OHYAMA K. ET AL.: 'Identification of a biologically active, small, secreted peptide in Arabidopsis by in silico gene screening, followed by LC-MS-based structure analysis' THE PLANT JOURNAL. vol. 55, 2008, pages 152 - 160, XP055078268
- GOVERSE, A. ET AL.: 'The Role of Plant Hormones in Nematode Feeding Cell Formation' GENOMICS AND MOLECULAR GENETICS OF PLANT-NEMATODE INTERACTIONS. 2011, LONDON, pages 325 - 347, XP008174373
- KONDO, T. ET AL.: 'A Plant Peptide Encoded by CLV3 Identified by in Situ MALDI-TOF MS Analysis' SCIENCE. vol. 313, no. 5788, 2006, pages 845 - 848, XP055078270
- OELKERS K. ET AL.: 'Bioinformatic analysis of the CLE signaling peptide family' BMC PLANT BIOLOGY. vol. 8, no. 1, 2008, pages 1 - 15, XP021033828
- REPLOGLE A. ET AL.: 'Nematode CLE signaling in Arabidopsis requires CLAVATA2 and CORYNE' THE PLANT JOURNAL. vol. 65, 2011, pages 430 - 440, XP055078348
- MENG, L. ET AL.: 'CLE-like (CLEL) peptides control the pattern of root growth and lateral root development in Arabidopsis' PROC. NATL. ACAD. SCI. USA. vol. 109, no. 5, 2012, pages 1760 - 1765, XP055078350
- None

### SEQUENCE LISTING

<110> The Australian National University
<120> Method for Modulating Plant Root Architecture
<130> P006236C
<140> PCT filing based on AU 2012900109
   <141> 2013-01-11
<150> 2012900109
   <151> 2012-01-11
<160> 496
<170> PatentIn version 3.2
<210> 1
   <211> 619
   <212> DNA
   <213> Arabidopsis thaliana RAR1 cDNA
<400> 1
   acaccttatg ttctccaatc caacacatat attttctttc acaaaaaaag acatatttta      60
   ccttgttttg ttttacatat tcttatattt tatcgatttg tctttgtccc cggctcatgg     120
   gaatgtcgaa taggtcagtt tctacatcca ttttttccct tgcattggtg gttttgcatg     180
   gaattcagga cacagaagag agacatttga aaactacttc gttagagatt gagggaattt     240
   ataaaaaaac tgaggccgag catcctagca ttgtggtcac atatacacgg cgtggtgtcc     300
   ttcagaagga ggtcattgcc caccccacag actttaggcc aacaaatccc ggaaacagcc     360
   caggcgttgg acactctaac gggcgacatt gattcgatca tatcatacgt cataatctta     420
   tatcatatag aaaattacat gtattttcat tcagacttgt cttctaatgc taaaggggtg     480
   tttggacatc actttatcat ttcaatgttt tgacagtact atgatcatta tgtctttgtc     540
   gtgggtttgt tacactgtca cgtattgtaa gattatataa tgaatgaatt gcttttaaaa     600
   attaatatat acattaatg                                                  619
<210> 2
   <211> 91
   <212> PRT
   <213> Arabidopsis thaliana RAR1
<400> 2
   Met Gly Met Ser Asn Arg Ser Val Ser Thr Ser Ile Phe Phe Leu Ala
   1               5                   10                  15
   Leu Val Val Leu His Gly Ile Gln Asp Thr Glu Glu Arg His Leu Lys
               20                  25                  30
   Thr Thr Ser Leu Glu Ile Glu Gly Ile Tyr Lys Lys Thr Glu Ala Glu
   35 40 45
   His Pro Ser Ile Val Val Thr Tyr Thr Arg Arg Gly Val Leu Gln Lys
   50 55 60
   Glu Val Ile Ala His Pro Thr Asp Phe Arg Pro Thr Asn Pro Gly Asn
   65 70 75 80
   Ser Pro Gly Val Gly His Ser Asn Gly Arg His
   85 90
<210> 3
   <211> 381
   <212> DNA
   <213> Arabidopsis thaliana RAR2 cDNA
<400> 3
   atgaagctat tcattatcac cgtggtgacc attttgacca tctcaagggt atttgacaaa      60
   acaccagcca ccactgaagc aagaaagtcg aaaaagatgg tcggtcatga gcatttcaat     120
   gaatatttgg atcctacttt tgcagggcat acatttggag tagttaaaga agattttctc     180
   gaagtaaaaa agctaaagaa aattggtgat gaaaataatc taaaaaacag atttataaat     240
   gagtttgcgc ctactaatcc agaagatagt ctcggtattg ggcatccaag agttctaaac     300
   aacaaattta caaatgattt tgcgcctact aatccaggag atagtcccgg tatcaggcat     360
   ccaggagttg tgaatgttta a                                               381
<210> 4
   <211> 126
   <212> PRT
   <213> Arabidopsis thaliana RAR2
<400> 4
   Met Lys Leu Phe Ile Ile Thr Val Val Thr Ile Leu Thr Ile Ser Arg
   1 5 10 15
   Val Phe Asp Lys Thr Pro Ala Thr Thr Glu Ala Arg Lys Ser Lys Lys
   20 25 30
   Met Val Gly His Glu His Phe Asn Glu Tyr Leu Asp Pro Thr Phe Ala
   35 40 45
   Gly His Thr Phe Gly Val Val Lys Glu Asp Phe Leu Glu Val Lys Lys
   50 55 60
   Leu Lys Lys Ile Gly Asp Glu Asn Asn Leu Lys Asn Arg Phe Ile Asn
   65 70 75 80
   Glu Phe Ala Pro Thr Asn Pro Glu Asp Ser Leu Gly Ile Gly His Pro
   85 90 95
   Arg Val Leu Asn Asn Lys Phe Thr Asn Asp Phe Ala Pro Thr Asn Pro
   100 105 110
   Gly Asp Ser Pro Gly Ile Arg His Pro Gly Val Val Asn Val
   115 120 125
<210> 5
   <211> 415
   <212> DNA
   <213> Arabidopsis thaliana RAR3 cDNA
<400> 5
   aatacatttt cgccttcgac taaatattat aatttagctt cttttttttt ctaattctcg 60
   tctcggtttt tctagtaatg gcgacgatta atgtttacgt ttttgcattt atctttcttt 120
   tgactattag tgttggttca attgaaggcc gaaaactcac caaattcacc gtaacgacgt 180
   ctgaggaaat cagagctggt ggctctgtat tgtcgtcgtc acctccgact gagccacttg 240
   agtcgccgcc gagccacggg gttgatacct tcagacctac ggaacctggt catagccccg 300
   gtattggaca ttccgtacat aattaacgga gaggaacaat agcatcgtct atgtgattac 360
   atgttgaaaa tatgattggc ctggtgactt tttttttctg aatatgtatt tacgt 415
<210> 6
   <211> 82
   <212> PRT
   <213> Arabidopsis thaliana RAR3
<400> 6
   Met Ala Thr Ile Asn Val Tyr Val Phe Ala Phe Ile Phe Leu Leu Thr
   1               5                   10                  15
   Ile Ser Val Gly Ser Ile Glu Gly Arg Lys Leu Thr Lys Phe Thr Val
               20                  25                  30
   Thr Thr Ser Glu Glu Ile Arg Ala Gly Gly Ser Val Leu Ser Ser Ser
           35                  40                  45
   Pro Pro Thr Glu Pro Leu Glu Ser Pro Pro Ser His Gly Val Asp Thr
       50                  55                  60
   Phe Arg Pro Thr Glu Pro Gly His Ser Pro Gly Ile Gly His Ser Val
   65                  70                  75                  80
   His Asn
<210> 7
   <211> 261
   <212> DNA
   <213> Arabidopsis thaliana RAR4 cDNA
<400> 7
   atggtgtctc gcggttgttc aatcacagtt ttgtttcgct ttcttatagt tcttttggtg      60
   atacaagtac actttgagaa tacaaaagca gctcgacatg caccagttgt ttcgtggtca     120
   ccacctgagc cgcctaagga tgattttgtg tggtaccaca agatcaaccg cttcaaaaac     180
   atagaacaag atgcattccg accaacccac caaggtccta gtcaaggtat tggacacaaa     240
   aaccctccag gtgctcctta a                                               261
<210> 8
   <211> 86
   <212> PRT
   <213> Arabidopsis thaliana RAR4
<400> 8
   Met Val Ser Arg Gly Cys Ser Ile Thr Val Leu Phe Arg Phe Leu Ile
   1               5                   10                  15
   Val Leu Leu Val Ile Gln Val His Phe Glu Asn Thr Lys Ala Ala Arg
               20                  25                  30
   His Ala Pro Val Val Ser Trp Ser Pro Pro Glu Pro Pro Lys Asp Asp
           35                  40                  45
   Phe Val Trp Tyr His Lys Ile Asn Arg Phe Lys Asn Ile Glu Gln Asp
       50                  55                  60
   Ala Phe Arg Pro Thr His Gln Gly Pro Ser Gln Gly Ile Gly His Lys
   65                  70                  75                  80
   Asn Pro Pro Gly Ala Pro
                   85
<210> 9
   <211> 690
   <212> DNA
   <213> Arabidopsis thaliana RAR5 cDNA
<400> 9
   atggtatttt accaaacacc aatcaccact gaagcaagaa gcttgaggaa aacaaacgac      60
   caagatcatt ttaaagctgg atttacagat gatttcgtgc ccacttctcc aggaaacagt     120
   cctggtgtgg gacacaaaaa aggtaatgtg aatgttgaag ggtttcaaga tgacttcaag     180
   cccacggaag gaagaaagtt gctgaaaact aacgttcaag atcatttcaa aaccggatct     240
   acagatgatt ttgcacctac ttcccctgga cacagtcccg ggtgggacac caagaaagga     300
   aatgtcaatg ttgaaagttc cgaagatgac ttcaaacaca aggaaggaag aaagcttcaa     360
   caaacaaacg gtcaaaatca tttcaaaacc ggatctacgg acgattttgc acctacttct     420
   ccgggaaaca gtcctgggat aggtcacaag aaagggcatg caaatgttaa agggtttaaa     480
   gatgacttcg cacccacgga agaaatacga ttgcagaaaa tgaacggtca agatcatttc     540
   aaaaccggat ctaccgatga tttcgcacct acaactccag gaaacagtcc cggtatgggc     600
   cataagaaag gagatgactt caaacccacg acaccaggac atagccccgg ggttggtcat     660
   gctgtcaaga acgatgaacc taaagcttaa                                      690
<210> 10
   <211> 243
   <212> PRT
   <213> Arabidopsis thaliana RAR5
<400> 10
   Met Lys Leu Leu Ser Ile Thr Leu Thr Ser Ile Val Ile Ser Met Val
   1               5                   10                  15
   Phe Tyr Gln Thr Pro Ile Thr Thr Glu Ala Arg Ser Leu Arg Lys Thr
               20                  25                  30
   Asn Asp Gln Asp His Phe Lys Ala Gly Phe Thr Asp Asp Phe Val Pro
           35                  40                  45
   Thr Ser Pro Gly Asn Ser Pro Gly Val Gly His Lys Lys Gly Asn Val
       50                  55                  60
   Asn Val Glu Gly Phe Gln Asp Asp Phe Lys Pro Thr Glu Gly Arg Lys
   65                  70                  75                  80
   Leu Leu Lys Thr Asn Val Gln Asp His Phe Lys Thr Gly Ser Thr Asp
                   85                  90                  95
   Asp Phe Ala Pro Thr Ser Pro Gly His Ser Pro Gly Val Gly His Lys
               100                 105                 110
   Lys Gly Asn Val Asn Val Glu Ser Ser Glu Asp Asp Phe Lys His Lys
           115                 120                 125
   Glu Gly Arg Lys Leu Gln Gln Thr Asn Gly Gln Asn His Phe Lys Thr
       130                 135                 140
   Gly Ser Thr Asp Asp Phe Ala Pro Thr Ser Pro Gly Asn Ser Pro Gly
   145                 150                 155                 160
   Ile Gly His Lys Lys Gly His Ala Asn Val Lys Gly Phe Lys Asp Asp
                   165                 170                 175
   Phe Ala Pro Thr Glu Glu Ile Arg Leu Gln Lys Met Asn Gly Gln Asp
               180                 185                 190
   His Phe Lys Thr Gly Ser Thr Asp Asp Phe Ala Pro Thr Thr Pro Gly
           195                 200                 205
   Asn Ser Pro Gly Met Gly His Lys Lys Gly Asp Asp Phe Lys Pro Thr
       210                 215                 220
   Thr Pro Gly His Ser Pro Gly Val Gly His Ala Val Lys Asn Asp Glu
   225                 230                 235                 240
   Pro Lys Ala
<210> 11
   <211> 501
   <212> DNA
   <213> Arabidopsis thaliana RAR6 cDNA
<400> 11
   acttcacatc accactctca aatattctca agagtcctta acaacgatat ttagtttttt      60
   cccttttttc tgttttcttt ttatctccca tttttcttcc aatataatgg aatcgtttat     120
   gggtcaaaag aaaacattgt acgcgtgtta ttttttaatg ttggtgtttt ttttagggtt     180
   caattgtgtc catggacgaa ccctaaaagt tgatgataag attaatggtg gtcattatga     240
   tagcaagacg atgatggcat tggcaaagca caatgatatg atggttgatg acaaggcaat     300
   gcagttctcg ccgccaccac caccaccacc gccgtcacaa tcgggaggta aagatgctga     360
   agatttcagg cctacaacgc ctggccacag ccctggcatt ggccatagtt tatcccataa     420
   ttgatcattt tcatgcaatt tcacatatgt atatatgtgt tgtgaactta tgattaaata     480
   ttgttcgttt taatttttct t                                               501
<210> 12
   <211> 105
   <212> PRT
   <213> Arabidopsis thaliana RAR6
<400> 12
   Met Glu Ser Phe Met Gly Gln Lys Lys Thr Leu Tyr Ala Cys Tyr Phe
   1               5                   10                  15
   Leu Met Leu Val Phe Phe Leu Gly Phe Asn Cys Val His Gly Arg Thr
               20                  25                  30
   Leu Lys Val Asp Asp Lys Ile Asn Gly Gly His Tyr Asp Ser Lys Thr
           35                  40                  45
   Met Met Ala Leu Ala Lys His Asn Asp Met Met Val Asp Asp Lys Ala
       50                  55                  60
   Met Gln Phe Ser Pro Pro Pro Pro Pro Pro Pro Pro Ser Gln Ser Gly
   65                  70                  75                  80
   Gly Lys Asp Ala Glu Asp Phe Arg Pro Thr Thr Pro Gly His Ser Pro
                   85                  90                  95
   Gly Ile Gly His Ser Leu Ser His Asn
               100                 105
<210> 13
   <211> 306
   <212> DNA
   <213> Arabidopsis thaliana RAR7 cDNA
<400> 13
   atgaaactct cagtttatat cattcttagt attctcttca tttcgacggt attttatgaa     60
   attcagttta cggaggcgag acagttgcga aaaaccgacg atcaagatca tgatgatcat    120
   catttcacag tcgggtacac tgatgatttt gggcctactt ctcctggtaa cagcccgggc    180
   attggtcata agatgaagga gaatgaagaa aatgctggag ggtataaaga tgacttcgaa    240
   cctacgacgc caggacatag tcccggcgtt ggacatgctg tcaagaacaa tgagcctaat    300
   gcttaa                                                               306
<210> 14
   <211> 101
   <212> PRT
   <213> Arabidopsis thaliana RAR7
<400> 14
   Met Lys Leu Ser Val Tyr Ile Ile Leu Ser Ile Leu Phe Ile Ser Thr
   1               5                   10                  15
   Val Phe Tyr Glu Ile Gln Phe Thr Glu Ala Arg Gln Leu Arg Lys Thr
               20                  25                  30
   Asp Asp Gln Asp His Asp Asp His His Phe Thr Val Gly Tyr Thr Asp
           35                  40                  45
   Asp Phe Gly Pro Thr Ser Pro Gly Asn Ser Pro Gly Ile Gly His Lys
       50                  55                  60
   Met Lys Glu Asn Glu Glu Asn Ala Gly Gly Tyr Lys Asp Asp Phe Glu
   65                  70                  75                  80
   Pro Thr Thr Pro Gly His Ser Pro Gly Val Gly His Ala Val Lys Asn
                   85                  90                  95
   Asn Glu Pro Asn Ala
               100
<210> 15
   <211> 589
   <212> DNA
   <213> Medicago truncatula RAR1 cDNA
<400> 15
   atgaaatatt tcgctctttt tcttgcccta attgcatgca actattccct tcaatctcat     60
   gcaaggctca ttaaaccatc gaaccatcac aatgttccaa tttcaacatc agagaagaaa    120
   gttgagtcaa caataaaatc aaacaatgaa gtagctagtt attttggaga ttcaagtgaa    180
   gctcacacaa atgcattcca accaacaaca ccaggaaata gtcctggtgt tggtcataga    240
   tattttaccg atgaagatat cgacgtgaat tcgaaaaaga cggtagctca gagcaaagat    300
   gataataaat atgtgactga ggatactaca aatgagttcc aaaaaacaaa ccctggtcac    360
   agtcctggtg ttggtcattc ttaccaaaac aaaattggaa attgatgtaa atatgcaatt    420
   aataatttat tttattagtt aggtgtatgc tgtataatta atcaatcaat taattattaa    480
   gtgttctcca tagtttcatt ctgcattaca gattgtgaaa ttcttgcata tcccaaacca    540
   tgagcttggg tttaattaat tattggctat tgattgtatc attcaattc                589
<210> 16
   <211> 142
   <212> PRT
   <213> Medicago truncatula RAR1
<400> 16
   Met Ala Tyr Lys Phe Gln Tyr Thr Met Lys Tyr Phe Ala Leu Phe Leu
   1               5                   10                  15
   Ala Leu Ile Ala Cys Asn Tyr Ser Leu Gln Ser His Ala Arg Leu Ile
               20                  25                  30
   Lys Pro Ser Asn His His Asn Val Pro Ile Ser Thr Ser Glu Lys Lys
           35                  40                  45
   Val Glu Ser Thr Ile Lys Ser Asn Asn Glu Val Ala Ser Tyr Phe Gly
       50                  55                  60
   Asp Ser Ser Glu Ala His Thr Asn Ala Phe Gln Pro Thr Thr Pro Gly
   65                  70                  75                  80
   Asn Ser Pro Gly Val Gly His Arg Tyr Phe Thr Asp Glu Asp Ile Asp
                   85                  90                  95
   Val Asn Ser Lys Lys Thr Val Ala Gln Ser Lys Asp Asp Asn Lys Tyr
               100                 105                 110
   Val Thr Glu Asp Thr Thr Asn Glu Phe Gln Lys Thr Asn Pro Gly His
           115                 120                 125
   Ser Pro Gly Val Gly His Ser Tyr Gln Asn Lys Ile Gly Asn
       130                 135                 140
<210> 17
   <211> 249
   <212> DNA
   <213> Medicago truncatula RAR2 cDNA
<400> 17
   atggcacatt tagctcgcat ttgcttgttc tatgtactat tgtttttgtc tcatgaacta    60
   ctactcacta caactgaggg taggagtttg agacaaagca ttcagccacc aaacatagcc   120
   tctaccaaaa tgatgagcac aagccaattg taccaccgta gcaatagaag tttggaggga   180
   gatgttgaag cttttaggcc cacaactcct ggacacagtc ctggcattgg tcattccatt   240
   aataattaa                                                           249
<210> 18
   <211> 82
   <212> PRT
   <213> Medicago truncatula RAR2
<400> 18
   Met Ala His Leu Ala Arg Ile Cys Leu Phe Tyr Val Leu Leu Phe Leu
   1               5                   10                  15
   Ser His Glu Leu Leu Leu Thr Thr Thr Glu Gly Arg Ser Leu Arg Gln
               20                  25                  30
   Ser Ile Gln Pro Pro Asn Ile Ala Ser Thr Lys Met Met Ser Thr Ser
           35                  40                  45
   Gln Leu Tyr His Arg Ser Asn Arg Ser Leu Glu Gly Asp Val Glu Ala
       50                  55                  60
   Phe Arg Pro Thr Thr Pro Gly His Ser Pro Gly Ile Gly His Ser Ile
   65                  70                  75                  80
   Asn Asn
<210> 19
   <211> 640
   <212> DNA
   <213> Medicago truncatula RAR3 cDNA
<400> 19
   ttttaaagta tattggtaca tgtgtaacac gacccaaaat aatttttcta aaaaggaaat      60
   gaaaaaaaga gaaaatgaac atccaaaaca atcaacgttg agctccacca ttttgcagca     120
   gtttatttaa attagtaacc tggctcggca gatcaatcat taataggagg tggagcgctc     180
   atgcgacatt ctacttcttg tgtccagcac ctggactatt tcctggtgtt gtgggacgaa     240
   aatcattcgt ctcaagattc ttgtaacgtt tcaatttgtg agaatggata tcggattgat     300
   cgggagcaaa agtgtgaata tctggaagat gactagcagc tttgtcttga ttaacactaa     360
   cggataatgc aacatgcttg tgatactcac acaccgaaat tatgtctgtc ggggaaatct     420
   tggctttaac taataacgcc ttagtgccgt gacatgacat cagatccctt agagctcgat     480
   tatatgacat taatatagct ttttccgaga ctacagatgg actcaaggat atgaaagcca      540
   atatggccaa caacccagcc aaacaatatt ttggaccata actcatgttt tctagcgcaa     600
   tgcttattaa gtgataatgc aagcactttt atattttttt                           640
<210> 20
   <211> 131
   <212> PRT
   <213> Medicago truncatula RAR3
<400> 20
   Met Ser Tyr Gly Pro Lys Tyr Cys Leu Ala Gly Leu Leu Ala Ile Leu
   1               5                   10                  15
   Ala Phe Ile Ser Leu Ser Pro Ser Val Val Ser Glu Lys Ala Ile Leu
               20                  25                  30
   Met Ser Tyr Asn Arg Ala Leu Arg Asp Leu Met Ser Cys His Gly Thr
           35                  40                  45
   Lys Ala Leu Leu Val Lys Ala Lys Ile Ser Pro Thr Asp Ile Ile Ser
       50                  55                  60
   Val Cys Glu Tyr His Lys His Val Ala Leu Ser Val Ser Val Asn Gln
   65                  70                  75                  80
   Asp Lys Ala Ala Ser His Leu Pro Asp Ile His Thr Phe Ala Pro Asp
                   85                  90                  95
   Gln Ser Asp Ile His Ser His Lys Leu Lys Arg Tyr Lys Asn Leu Glu
               100                 105                 110
   Thr Asn Asp Phe Arg Pro Thr Thr Pro Gly Asn Ser Pro Gly Ala Gly
           115                 120                 125
   His Lys Lys
       130
<210> 21
   <211> 243
   <212> DNA
   <213> Medicago truncatula RAR4 cDNA
<400> 21
   atgggtgaga aaaccatgtt attaacattt ttacttctta ttattatgca acaaaacatt     60
   ggttcaattg aagcatcaag gttgctaaat attaatccac caccaactat tcctaaaagt    120
   ccacaagctc cttcacatga ttattggtat tcgataaacg atgataaggg tggtgacgat    180
   gcttttcgcc ctacaagtcc aggacatagc cctggggtag gacatcaaac accacctcca    240
   tga                                                                  243
<210> 22
   <211> 80
   <212> PRT
   <213> Medicago truncatula RAR4
<400> 22
   Met Gly Glu Lys Thr Met Leu Leu Thr Phe Leu Leu Leu Ile Ile Met
   1               5                   10                  15
   Gln Gln Asn Ile Gly Ser Ile Glu Ala Ser Arg Leu Leu Asn Ile Asn
               20                  25                  30
   Pro Pro Pro Thr Ile Pro Lys Ser Pro Gln Ala Pro Ser His Asp Tyr
           35                  40                  45
   Trp Tyr Ser Ile Asn Asp Asp Lys Gly Gly Asp Asp Ala Phe Arg Pro
       50                  55                  60
   Thr Ser Pro Gly His Ser Pro Gly Val Gly His Gln Thr Pro Pro Pro
   65                  70                  75                  80
<210> 23
   <211> 249
   <212> DNA
   <213> Medicago truncatula RAR5 cDNA
<400> 23
   atggaaaata ctaaaaggct tcaaattatt tgtgttctta ttttgttttt ggttttgcaa 60
   caagaagttg tgattgttca agggaggcat ttgaggtcta aattgtgtag agattgcaca 120
   aagcctcata aaagatccat tgctcatcat ggagggaagt cttcaagacg tgtagggtat 180
   gaagttgatg attttaggcc tacatctcca gggcatagtc caggtgttgg tcattccatc 240
   cataattaa 249
<210> 24
   <211> 85
   <212> PRT
   <213> Medicago truncatula RAR5
<400> 24
   Met Ala His Phe Thr Arg Ser Cys Leu Ile Phe Val Leu Leu Leu Ile
   1 5 10 15
   Ser Cys Glu Leu Leu Ser Ile Glu Gly Arg Ser Leu Arg Lys Ser Ile
   20 25 30
   Gly Ser Pro Lys Ala Ala Ser Val Glu Thr Met Thr Arg Ser Val Val
   35 40 45
   Leu Ser Pro Arg Gln Leu Gln Asn Asn Gly Arg Asn Leu Glu Gly Ser
   50 55 60
   Val Glu Ala Phe Arg Pro Thr Thr Pro Gly His Ser Pro Gly Val Gly
   65 70 75 80
   His Ser Leu Lys Asn
   85
<210> 25
   <211> 249
   <212> DNA
   <213> Medicago truncatula RAR6 cDNA
<400> 25
   atggaaaata ctaaaaggct tcaaattatt tgtgttctta ttttgttttt ggttttgcaa 60
   caagaagttg tgattgttca agggaggcat ttgaggtcta aattgtgtag agattgcaca 120
   aagcctcata aaagatccat tgctcatcat ggagggaagt cttcaagacg tgtagggtat 180
   gaagttgatg attttaggcc tacatctcca gggcatagtc caggtgttgg tcattccatc 240
   cataattaa 249
<210> 26
   <211> 82
   <212> PRT
   <213> Medicago truncatula RAR6
<400> 26
   Met Glu Asn Thr Lys Arg Leu Gln Ile Ile Cys Val Leu Ile Leu Phe
   1               5                   10                  15
   Leu Val Leu Gln Gln Glu Val Val Ile Val Gln Gly Arg His Leu Arg
               20                  25                  30
   Ser Lys Leu Cys Arg Asp Cys Thr Lys Pro His Lys Arg Ser Ile Ala
           35                  40                  45
   His His Gly Gly Lys Ser Ser Arg Arg Val Gly Tyr Glu Val Asp Asp
       50                  55                  60
   Phe Arg Pro Thr Ser Pro Gly His Ser Pro Gly Val Gly His Ser Ile
   65                  70                  75                  80
   His Asn
<210> 27
   <211> 375
   <212> DNA
   <213> Medicago truncatula RAR7 cDNA
<400> 27
   atgggtttgt ttcaagtcac aacaaaatat ttgatcgtta ttctagcact aagtattgta     60
   tacaattcct ttcaaataac tcaagccagg ccaattaaac cattgaatca acaatcttca    120
   ttaaacacac aagactcggg tgcaatccac actaactctt ttcggccgac aacaccagga    180
   agtagtcctg gtgttggcca ccgaaatttt gttgtaggag ataagaacac gagaacaatg    240
   gtggttgttc agagcccgga tgttgaggtt tttgtgacga ataagagatc cgatgatggt    300
   ttcaaaccta caaatcctag tcatagtcct ggagttggcc atggttacca taccaaaatt    360
   agacatttaa attag                                                     375
<210> 28
   <211> 124
   <212> PRT
   <213> Medicago truncatula RAR7
<400> 28
   Met Gly Leu Phe Gln Val Thr Thr Lys Tyr Leu Ile Val Ile Leu Ala
   1               5                   10                  15
   Leu Ser Ile Val Tyr Asn Ser Phe Gln Ile Thr Gln Ala Arg Pro Ile
               20                  25                  30
   Lys Pro Leu Asn Gln Gln Ser Ser Leu Asn Thr Gln Asp Ser Gly Ala
           35                  40                  45
   Ile His Thr Asn Ser Phe Arg Pro Thr Thr Pro Gly Ser Ser Pro Gly
       50                  55                  60
   Val Gly His Arg Asn Phe Val Val Gly Asp Lys Asn Thr Arg Thr Met
   65                  70                  75                  80
   Val Val Val Gln Ser Pro Asp Val Glu Val Phe Val Thr Asn Lys Arg
                   85                  90                  95
   Ser Asp Asp Gly Phe Lys Pro Thr Asn Pro Ser His Ser Pro Gly Val
               100                 105                 110
   Gly His Gly Tyr His Thr Lys Ile Arg His Leu Asn
           115                 120
<210> 29
   <211> 309
   <212> DNA
   <213> Medicago truncatula RAR8 cDNA
<400> 29
   atggcacaaa acaagaccat agttttctct gttatttccc tagcattgat cattttctgc      60
   atgcagtcga tcgaggggcg ccttgtaaaa tacatcgatg aaagtaacct cctgaagaat     120
   gttaaacatg atggaatttc agatgcaaat gaagctactc ttgttaacgt gactccaaca     180
   atattgccac caagtgctgt ggtaggttca aatggggttg cagcacctcc tccaagtcat     240
   gatgtgggtg cttttagacc cacaacccct gggaacagtc ctggtgtagg tcactctatt     300
   cactactag                                                             309
<210> 30
   <211> 102
   <212> PRT
   <213> Medicago truncatula RAR8
<400> 30
   Met Ala Gln Asn Lys Thr Ile Val Phe Ser Val Ile Ser Leu Ala Leu
   1               5                   10                  15
   Ile Ile Phe Cys Met Gln Ser Ile Glu Gly Arg Leu Val Lys Tyr Ile
               20                  25                  30
   Asp Glu Ser Asn Leu Leu Lys Asn Val Lys His Asp Gly Ile Ser Asp
           35                  40                  45
   Ala Asn Glu Ala Thr Leu Val Asn Val Thr Pro Thr Ile Leu Pro Pro
       50                  55                  60
   Ser Ala Val Val Gly Ser Asn Gly Val Ala Ala Pro Pro Pro Ser His
   65                  70                  75                  80
   Asp Val Gly Ala Phe Arg Pro Thr Thr Pro Gly Asn Ser Pro Gly Val
                   85                  90                  95
   Gly His Ser Ile His Tyr
               100
<210> 31
   <211> 456
   <212> DNA
   <213> Medicago truncatula RAR9 cDNA
<400> 31
   atgggtgaat ttcaggccat gcaaaaatat tttgccattt ttcttgtatt agttgcctac 60
   catattttcc ttccaactca agctaggaag ataaaaccat tgattgaaga taatcccaaa 120
   cctaccttca catcccttaa aactgctgta aatattcctt ctccaacatt tgagaagaaa 180
   gttaaccttc ccatgatgcc aaatcatggt gtcgcaagta taggagattc aagcggagat 240
   acaaatgctt tccgacccac aacaccagga agcagtcctg gtgttggtca tcggaagttt 300
   gtaggagagg ttaaagatag tacagttgtt cggagtccga atgttaaagt ttttgtgact 360
   tctgagagat caaaagatgc ttttaaacct acttacccaa atcatagccc aggtgttgga 420
   catgttaacc aaagcacaaa aggacaacta aattag 456
<210> 32
   <211> 151
   <212> PRT
   <213> Medicago truncatula RAR9
<400> 32
   Met Gly Glu Phe Gln Ala Met Gln Lys Tyr Phe Ala Ile Phe Leu Val
   1 5 10 15
   Leu Val Ala Tyr His Ile Phe Leu Pro Thr Gln Ala Arg Lys Ile Lys
   20 25 30
   Pro Leu Ile Glu Asp Asn Pro Lys Pro Thr Phe Thr Ser Leu Lys Thr
   35 40 45
   Ala Val Asn Ile Pro Ser Pro Thr Phe Glu Lys Lys Val Asn Leu Pro
   50 55 60
   Met Met Pro Asn His Gly Val Ala Ser Ile Gly Asp Ser Ser Gly Asp
   65 70 75 80
   Thr Asn Ala Phe Arg Pro Thr Thr Pro Gly Ser Ser Pro Gly Val Gly
   85 90 95
   His Arg Lys Phe Val Gly Glu Val Lys Asp Ser Thr Val Val Arg Ser
   100 105 110
   Pro Asn Val Lys Val Phe Val Thr Ser Glu Arg Ser Lys Asp Ala Phe
   115 120 125
   Lys Pro Thr Tyr Pro Asn His Ser Pro Gly Val Gly His Val Asn Gln
   130 135 140
   Ser Thr Lys Gly Gln Leu Asn
   145 150
<210> 33
   <211> 573
   <212> DNA
   <213> Medicago truncatula RAR10 cDNA
<400> 33
   atggctgaga aaatcatgtt tgtaacctat ttacttatcc ttattattat gcaacaatac      60
   cttggatcaa tggaagcatc aaggtttata aatgataata ataaggatgg tgatgatgct     120
   ttccgtccaa ctccttcagg tcatagtctt ggggtgggac atatattacc accaccatca     180
   agtattatcc ctaaagtctt attgaaaagt caacaacctc cttcatctga ttatttgtat     240
   accataaagg atgataataa ggacggtgat gatcctccat cacatgatta ttggtattcc     300
   ataaatgatg ataataaaga tggtgatgat gctttccgtc caaatcctcc aggtcatagt     360
   cctggagggg gacatacgtt accaccatca ccaccaagtg ttatccctac agtcttattg     420
   gaaaatccac aacctatttc atctgattat ttctataaca taaaggatga taataaggat     480
   ggtgatgatg cttttcgccc aactcctcct ggtcatagcc ctggaggggg acatacatta     540
   ccaccatcac caccaattgt ttttatgaac taa                                  573
<210> 34
   <211> 223
   <212> PRT
   <213> Medicago truncatula RAR10
<400> 34
   Met Ala Glu Lys Ile Met Phe Val Thr Tyr Leu Leu Ile Leu Ile Ile
   1               5                   10                  15
   Met Gln Gln Tyr Leu Gly Ser Met Glu Ala Ser Arg Phe Ile Asn Asp
               20                  25                  30
   Asn Asn Lys Asp Gly Asp Asp Ala Phe Arg Pro Thr Pro Ser Gly His
           35                  40                  45
   Ser Leu Gly Val Gly His Ile Leu Pro Pro Pro Ser Ser Ile Ile Pro
       50                  55                  60
   Lys Val Leu Leu Lys Ser Gln Gln Pro Pro Ser Ser Asp Tyr Leu Tyr
   65                  70                  75                  80
   Thr Ile Lys Asp Asp Asn Lys Asp Gly Asp Asp Ala Phe Arg Pro Thr
                   85                  90                  95
   Pro Pro Gly His Ser Pro Gly Gly Gly His Thr Leu Pro Pro Ser Pro
               100                 105                 110
   Pro Ser Ile Val Pro Ile Ile Ser Leu Lys Ser Leu Gln Pro Pro Ser
           115                 120                 125
   His Asp Tyr Trp Tyr Ser Ile Asn Asp Asp Asn Lys Asp Gly Asp Asp
       130                 135                 140
   Ala Phe Arg Pro Asn Pro Pro Gly His Ser Pro Gly Gly Gly His Thr
   145                 150                 155                 160
   Leu Pro Pro Ser Pro Pro Ser Val Ile Pro Thr Val Leu Leu Glu Asn
                   165                 170                 175
   Pro Gln Pro Ile Ser Ser Asp Tyr Phe Tyr Asn Ile Lys Asp Asp Asn
               180                 185                 190
   Lys Asp Gly Asp Asp Ala Phe Arg Pro Thr Pro Pro Gly His Ser Pro
           195                 200                 205
   Gly Gly Gly His Thr Leu Pro Pro Ser Pro Pro Ile Val Phe Met
       210                 215                 220
<210> 35
   <211> 258
   <212> DNA
   <213> Medicago truncatula RAR11 cDNA
<400> 35
   atggcaaaga aaaccattat gttaagcttt cttgtttttc tcattcttgt gcaaaatttt      60
   ggtttgatgg aagtgctagg gaagaatgtt gaagcaccac caacaattcc aagagttttg     120
   ttgaggagtc cacaagctcc ttccattggc ttttatacca aaaatgatga caaggatagt     180
   caaggtgatg cttttcgtcc aactagtcct ggtcatagtc ctggtgtggg ccatgattcg     240
   ccaccaaatt ttccttaa                                                   258
<210> 36
   <211> 85
   <212> PRT
   <213> Medicago truncatula RAR11
<400> 36
   Met Ala Lys Lys Thr Ile Met Leu Ser Phe Leu Val Phe Leu Ile Leu
   1 5 10 15
   Val Gln Asn Phe Gly Leu Met Glu Val Leu Gly Lys Asn Val Glu Ala
   20 25 30
   Pro Pro Thr Ile Pro Arg Val Leu Leu Arg Ser Pro Gln Ala Pro Ser
   35 40 45
   Ile Gly Phe Tyr Thr Lys Asn Asp Asp Lys Asp Ser Gln Gly Asp Ala
   50 55 60
   Phe Arg Pro Thr Ser Pro Gly His Ser Pro Gly Val Gly His Asp Ser
   65 70 75 80
   Pro Pro Asn Phe Pro
   85
<210> 37
   <211> 105
   <212> PRT
   <213> Arabidopsis lyrata RAR1
<400> 37
   Met Glu Ser Ser Met Gly Gln Lys Lys Thr Leu Tyr Ala Cys Ile Phe
   1 5 10 15
   Leu Met Met Val Phe Phe Leu Gly Phe Asn Cys Gly His Gly Arg Thr
   20 25 30
   Leu Lys Val Asp Asp Lys Ile Asp Gly Gly His Asp Asp Ser Lys Thr
   35 40 45
   Met Met Ala Leu Ala Lys His Asn Val Met Met Val Asp Asp Lys Thr
   50 55 60
   Val Gln Phe Ser Pro Pro Pro Pro Pro Pro Ser Pro Ser Gln Ser Gly
   65 70 75 80
   Gly Lys Glu Ala Glu Asp Phe Arg Pro Thr Thr Pro Gly His Ser Pro
   85 90 95
   Gly Ile Gly His Ser Leu Ser His Asn
   100 105
<210> 38
   <211> 231
   <212> PRT
   <213> Arabidopsis lyrata RAR2
<400> 38
   Met Lys Leu Leu Ser Ile Thr Val Met Thr Ile Val Ile Ser Met Val
   1 5 10 15
   Phe Asp Gln Thr Pro Ile Thr Thr Glu Ala Arg Arg Leu Arg Asn Thr
   20 25 30
   Asn Asp Gln Asp His Phe Lys Ala Gly Ser Thr Asp Asp Phe Ala Pro
   35 40 45
   Thr Ser Pro Gly Asn Ser Pro Gly Val Gly His Arg Lys Gly Lys Val
   50 55 60
   Asn Val Glu Gly Phe Gln Asp Asp Phe Lys Pro Thr Glu Gly Arg Lys
   65 70 75 80
   Leu Leu Lys Thr Asn Gly Gln Asp His Phe Lys Thr Gly Ser Thr Asp
   85 90 95
   Asp Phe Ala Pro Thr Ser Pro Gly His Ser Pro Gly Val Gly His Arg
   100 105 110
   Lys Asp Thr Ala Asn Val Glu Arg Phe Gln Gln Thr Asn Gly Gln Asn
   115 120 125
   His Phe Lys Thr Gly Ser Thr Asp Glu Phe Ala Pro Thr Ser Pro Gly
   130 135 140
   Asn Ser Pro Gly Ile Gly His Lys Lys Gly Asn Ala Asn Val Lys Gly
   145 150 155 160
   Phe Lys Asp Asp Phe Ala Pro Thr Glu Glu Ile Arg Leu Lys Lys Met
   165 170 175
   Asn Gly Lys Asp His Phe Lys Ser Gly Ser Thr Asp Asp Phe Ala Pro
   180 185 190
   Thr Thr Pro Gly Asn Ser Pro Gly Met Gly His Lys Lys Gly Asp Asp
   195 200 205
   Phe Lys Pro Thr Thr Pro Gly His Ser Pro Gly Val Gly His Ala Val
   210 215 220
   Asn Asn Asn Glu Pro Lys Ala
   225 230
<210> 39
   <211> 90
   <212> PRT
   <213> Arabidopsis lyrata RAR3
<400> 39
   Met Gly Met Ser Asn Arg Ser Val Ser Thr Ser Leu Phe Phe Leu Ala
   1 5 10 15
   Leu Val Val Leu His Gly Ile Gln Asp Thr Glu Glu Arg His Leu Lys
   20 25 30
   Thr Thr Ser Leu Glu Val Glu Gly Ile Tyr Lys Lys Thr Glu Ala Glu
   35 40 45
   Asn Pro Ser Ile Val Val Thr Tyr Thr Arg Arg Ser Val Leu Gln Lys
   50 55 60
   Ala Val Ile Ala His Pro Thr Asp Phe Arg Pro Thr Asn Pro Gly Asn
   65 70 75 80
   Ser Pro Gly Val Gly His Ser His Gly Arg
   85 90
<210> 40
   <211> 92
   <212> PRT
   <213> Solanum lycopersicum
<400> 40
   Met Ala Gln Pro Lys Ile Met Tyr Thr Cys Ala Phe Phe Leu Ala Leu
   1 5 10 15
   Ile Phe Phe Ser Tyr Gly Ile Leu Leu Ser Glu Gly Arg Val Leu Phe
   20 25 30
   Lys Lys Glu Lys Asn Asn Asn Thr Ile Phe Ser His His Glu Glu Asn
   35 40 45
   Ser His Thr Lys Val Val Lys Asn Asn Tyr Phe Asn Asn Ile Asp His
   50 55 60
   Asn Asn Met His Asp Asn Ile Asn Ile Ser Glu Glu Gly Gly Pro Gly
   65 70 75 80
   His Ser Pro Gly Val Gly His Gly Gly Gly Pro Pro
   85 90
<210> 41
   <211> 81
   <212> PRT
   <213> Solanum lycopersicum RAR2
<400> 41
   Met Ala Ser Ser Tyr Gln Lys Ser Ile Tyr Met Val Ile Phe Tyr Val
   1 5 10 15
   Phe Leu Phe Leu Phe Leu His Gln Cys Glu Leu Ile Val Ala Ser Arg
   20 25 30
   Val Val Val Met Lys Phe His Gln Pro Met Met Pro Pro Ser Thr Asn
   35 40 45
   Ile Leu Ser Phe Asn Arg Tyr Lys Lys Ser Glu Ile Val Lys Asp Tyr
   50 55 60
   Ser Gly Pro Gly His Ser Pro Gly Met Gly His Asn Asp Pro Pro Gly
   65 70 75 80
   Ala
<210> 42
   <211> 74
   <212> PRT
   <213> Solanum lycopersicum RAR3
<400> 42
   Met Ala Ser Ser Tyr Lys Lys Ser Ile Tyr Met Val Leu Phe Tyr Val
   1 5 10 15
   Phe Val Phe Leu Leu Leu Gln Gln Cys Glu Leu Ile Val Ala Ser Arg
   20 25 30
   Val Val Val Met Lys Phe His Gln Pro Lys Pro Pro Ser Thr Asn Ile
   35 40 45
   Phe Ser Phe Asn Arg Tyr Lys Lys Ser Glu Val Val Lys Asp Tyr Ser
   50 55 60
   Gly Pro Gly His Ser Pro Gly Met Gly His
   65 70
<210> 43
   <211> 82
   <212> PRT
   <213> Lycopersicum esculentum RAR1
<400> 43
   Met Ala Ser Ser Tyr Gln Lys Ser Ile Tyr Met Val Ile Phe Tyr Val
   1               5                   10                  15
   Phe Leu Phe Leu Phe Leu His Gln Cys Glu Leu Ile Val Ala Ser Arg
               20                  25                  30
   Val Val Val Met Lys Phe His Gln Pro Met Met Pro Pro Ser Thr Asn
           35                  40                  45
   Ile Leu Ser Phe Asn Arg Tyr Lys Lys Ser Glu Ile Val Lys Asp Tyr
       50                  55                  60
   Ser Gly Pro Gly His Ser Pro Gly Met Gly His Asn Asp Pro Pro Gly
   65                  70                  75                  80
   Ala Pro
<210> 44
   <211> 120
   <212> PRT
   <213> Lycopersicum esculentum RAR2
<400> 44
   Met Val Ile Val Thr Asn Thr Lys Ile Ile Gln Phe Phe Ala Phe Ile
   1               5                   10                  15
   Leu Val Leu Ile Leu Phe Ser His Glu Ile Leu Cys Val Glu Ala Ile
               20                  25                  30
   Arg His Leu Lys Ser Glu Lys Met Glu Val Val Ser Val Glu Ile Ser
           35                  40                  45
   Val Ser Ser Thr Gln Ile Val Val Thr Ser Glu Thr Phe Asn Lys Ile
       50                  55                  60
   Gly Lys Ile Gln Lys Ser Leu Thr Trp Leu Pro Ser Lys Asp Asp Ile
   65                  70                  75                  80
   His Lys Ser Ile Asn Asp Pro Thr Glu Ala Thr Lys Ser Val Lys Val
                   85                  90                  95
   Val Glu Lys Met Asp Asp Phe Gly Pro Thr Gly Pro Gly His Ser Pro
               100                 105                 110
   Gly Ile Gly His Ser Ile His Ser
           115                 120
<210> 45
   <211> 93
   <212> PRT
   <213> Gossypium hirsutum RAR1
<400> 45
   Leu Phe Leu Phe Gln Pro Leu Lys Trp Pro Lys Pro Thr Ser Leu Tyr
   1               5                   10                  15
   Pro Leu Pro Phe Ser Pro Ser Phe Cys Leu Leu Thr Gly Ser Gln Phe
               20                  25                  30
   Ser Lys Glu Ala Arg Val Leu Lys Ala Asp His Lys Thr His His His
           35                  40                  45
   Ser Ser Leu Asn Val Asn Val Lys Gly Asp Val Leu Pro Asp Gly Ser
       50                  55                  60
   Ala Thr Val Asn Asn Val Gln Lys Ala Ala Tyr Arg Thr Asp Ala Phe
   65                  70                  75                  80
   Arg Ser Thr Thr Pro Gly His Ser Pro Gly Ala Gly His
                   85                  90
<210> 46
   <211> 103
   <212> PRT
   <213> Lactuca sativa RAR1
<400> 46
   Met Val His Phe Gln Ile Tyr Pro Cys Val Phe Phe Leu Leu Ile Ile
   1               5                   10                  15
   Ser Phe His Gly Leu Ile Pro Leu Phe Glu Gly Arg Lys Leu Lys Asp
               20                  25                  30
   Val Thr Ala Phe Arg Pro Thr Thr Pro Gly Asn Ser Pro Gly Ala Gly
           35                  40                  45
   His Ser Phe Thr Glu Asn Arg Pro Tyr Phe Arg Ser Lys Glu Val Glu
       50                  55                  60
   Ser Lys Asp Ser Gly Ile His His Pro Asn Ser Glu Ser Ala Thr Gly
   65                  70                  75                  80
   Phe Arg Pro Thr Lys Pro Gly Asn Ser Pro Gly Ala Gly His Ser Ile
                   85                  90                  95
   His Asn Gln Thr Ala Met Pro
               100
<210> 47
   <211> 210
   <212> PRT
   <213> Euphorbia esula RAR1
<400> 47
   Met Ala Glu Ile Gln Lys Phe Val Ile Phe Leu Leu Ala Ile Val Phe
   1               5                   10                  15
   Tyr Leu Gln Ser Gln Ser Thr Ser Ala Arg Pro Val Lys Phe Val Asn
               20                  25                  30
   Lys Lys Gly Leu Ala Leu Lys Lys Asn Ser Asp Ser Phe Lys Leu His
           35                  40                  45
   Gln Thr Met Lys Lys Glu Gln Met Pro Pro Pro Val Asp Lys Thr Gly
       50                  55                  60
   Phe Phe Gly Asp Phe Ser Asp Lys Ser Thr Asp Asp Phe Arg Pro Thr
   65                  70                  75                  80
   Ser Pro Gly Tyr Ser Pro Gly Val Gly His Pro Lys Ala Val Phe Ala
                   85                  90                  95
   Asn Ser Gln Ser Asp Arg Ile Asp His Ser Thr Ala Arg Lys Glu Glu
               100                 105                 110
   Glu Ser Thr Thr Asp Asp Phe Arg Pro Thr Glu Pro Gly Tyr Ser Pro
           115                 120                 125
   Gly Val Gly His Pro Met Glu Ala Ser Thr Ser Ser Asp Lys Asp Asp
       130                 135                 140
   Tyr Arg Pro Thr Glu Pro Gly His Ser Pro Gly Ala Gly His Pro Lys
   145                 150                 155                 160
   Glu Glu Ser Thr Asp Asp Phe Arg Pro Thr Ala Pro Gly Phe Ser Pro
                   165                 170                 175
   Gly Val Gly His Arg Lys Glu Val Val Thr Val Pro Glu Ala Glu Asn
               180                 185                 190
   Asp Phe Ser Gly Thr Lys Asp Asp Tyr Arg Pro Thr Gln Pro Gly His
           195                 200                 205
   Ser Pro
       210
<210> 48
   <211> 232
   <212> PRT
   <213> Euphorbia esula RAR2
<220>
   <221> misc_feature
   <222> (212) .. (215)
   <223> Xaa can be any naturally occurring amino acid
<220>
   <221> misc_feature
   <222> (218) .. (219)
   <223> Xaa can be any naturally occurring amino acid
<400> 48
   Ile Ser Lys Ser Pro Asn Ala Ile Tyr Pro Pro Ala Thr Ser Ile Ser
   1 5 10 15
   Phe Asp Asp Glu Glu Glu Glu Pro Gln Glu Ala His Val Tyr Ala Phe
   20 25 30
   Arg Pro Thr Ala Pro Gly His Ser Pro Gly Val Gly His Lys Glu Glu
   35 40 45
   Leu Glu Asp Ala His Leu Tyr Ala Phe Arg Pro Thr Ala Pro Gly His
   50 55 60
   Ser Pro Gly Val Gly His Lys Glu Glu Pro Glu Asp Ser Met Asn Ser
   65 70 75 80
   His Val Ile Ile Ser Lys Ser Pro Asn Ala Ile Tyr Pro Pro Thr Thr
   85 90 95
   Ser Ile Ser Phe Asp Glu Glu Glu Glu Pro Gln Glu Ala His Leu Tyr
   100 105 110
   Ala Phe Arg Pro Thr Ala Pro Gly His Ser Pro Gly Val Gly His Lys
   115 120 125
   Glu Glu Leu Glu Asp Ala His Leu Tyr Ala Phe Arg Pro Thr Ala Pro
   130 135 140
   Gly His Ser Pro Gly Val Gly Tyr Lys Glu Glu Pro Glu Asp Ser Met
   145 150 155 160
   Asn Ser His Val Arg Ile Ser Lys Tyr Pro Asn Ala Ile Tyr Pro Pro
   165 170 175
   Thr Thr Ser Ile Ser Phe Asp Glu Glu Glu Glu Glu Pro Gln Glu Ala
   180 185 190
   His Leu Tyr Ala Phe Arg Pro Thr Ala Pro Gly His Ser Pro Gly Val
   195 200 205
   Gly His Lys Xaa Xaa Xaa Xaa Asp Ala Xaa Xaa Tyr Ala Ala Ile Ala
   210 215 220
   Lys Gly Leu Ala Thr Pro Ser Ser
   225 230
<210> 49
   <211> 158
   <212> PRT
   <213> Glycine max RAR1
<400> 49
   Met Ala Lys Phe Gln Val Leu His Glu Tyr Phe Phe Ile Phe Leu Ala
   1               5                   10                  15
   Leu Val Val Cys Asp Gly Ser Leu Leu Thr His Gly Arg Lys Ile Asn
               20                  25                  30
   Ile Lys Pro Leu Asn Gln Leu His Ser Ser Leu Asn Thr Lys Thr Val
           35                  40                  45
   Ala Asn His Pro Asn Pro Thr Ser Leu Pro Ser Leu Lys Thr Lys Val
       50                  55                  60
   Glu Ser Pro Gln His His Glu Glu Ser Ser Lys Leu Glu Asp Ser Gly
   65                  70                  75                  80
   Ala Asp Asn Thr Asn Ala Phe Arg Pro Thr Thr Pro Gly Gly Ser Pro
                   85                  90                  95
   Gly Val Gly His Lys Met Ile Thr Ser Ser Ser Glu Asp Asn Lys Val
               100                 105                 110
   Lys Thr Met Val Val Val His Ser Pro Asp Val Glu Val Phe Lys Thr
           115                 120                 125
   Glu Gly Ser Lys Asp Asp Phe Lys Pro Thr Asp Pro Gly His Ser Pro
       130                 135                 140
   Gly Val Gly His Ala Tyr Lys Asn Lys Ile Gly Asp Glu Asn
   145                 150                 155
<210> 50
   <211> 87
   <212> PRT
   <213> Glycine max RAR2
<400> 50
   Met Ala His Phe Thr Arg Thr Cys Leu Leu Leu Val Leu Leu Phe Leu
   1               5                   10                  15
   Ser Cys Glu Leu Leu Cys Ile Glu Gly Arg Gly Leu Lys Ala Thr Thr
               20                  25                  30
   Lys Ser Pro Lys Ser Val Ser Val Arg Ala Met Ser Thr Thr Lys Gly
           35                  40                  45
   Ala Val Ala Lys Pro Ser Gln Leu Glu Thr Ile Ala Lys Ser Leu Asn
       50                  55                  60
   Gly Phe Val Glu Ala Phe Arg Pro Thr Thr Pro Gly His Ser Pro Gly
   65                  70                  75                  80
   Val Gly His Ser Val Asn Asn
                   85
<210> 51
   <211> 144
   <212> PRT
   <213> Glycine max RAR3
<400> 51
   Met His Lys Tyr Phe Thr Ile Phe Val Ala Leu Phe Ala Cys His Gly
   1               5                   10                  15
   Ser Leu Phe Ala His Gly Arg Gln Ile Lys Pro Leu Asn Gln His Ser
               20                  25                  30
   Ser Leu Asn Thr Asn Pro Ile Leu Ala Pro Leu Ser Arg Thr Ser Ile
           35                  40                  45
   Lys Val Ile Glu Ala Pro Ile Val Pro Lys Phe Lys Phe Ser Asp Val
       50                  55                  60
   Asp Ser Gly Asp Ser Gly Ala Asp His Ala Asn Ala Phe Arg Pro Thr
   65                  70                  75                  80
   Thr Pro Gly Asn Ser Pro Gly Val Gly His Lys Lys Phe Glu Glu Asp
                   85                  90                  95
   Lys Val Met Lys Val Met Gly Ala Leu Val His Ser Pro Asp Val Lys
               100                 105                 110
   Thr Ser Val Ala Glu Gly Ser Phe Glu Asn Asp Phe Lys Pro Thr Asp
           115                 120                 125
   Pro Gly His Ser Pro Gly Val Gly His Pro Arg Gln Asn Lys Arg Asn
       130                 135                 140
<210> 52
   <211> 94
   <212> PRT
   <213> Glycine max RAR4
<400> 52
   Met Ala Gln His Lys Phe Leu Leu Cys Leu Ile Leu Leu Ala Leu Ile
   1               5                   10                  15
   Ile Phe Cys Gln Gly Leu His Ser Ile Glu Gly Arg Tyr Leu Lys Ser
               20                  25                  30
   Asp His Glu Ile Ile Lys His Gln Tyr Gln Met His Ser Gly Ile Ser
           35                  40                  45
   Thr Thr Asn Val Ala Ala Leu Val Ala Asp Val Ser Pro Pro Thr Pro
       50                  55                  60
   Pro Ser Ala Ala Val Pro Gly Arg Asp Asn Asp Asn Phe Arg Pro Thr
   65                  70                  75                  80
   Ala Pro Gly His Ser Pro Gly Val Gly His Ala Ala His Asn
                   85                  90
<210> 53
   <211> 141
   <212> PRT
   <213> Glycine max RAR5
<400> 53
   Met Ala Ala Gln Val Leu His Lys Tyr Phe Phe Ile Phe Leu Ala Leu
   1               5                   10                  15
   Val Val Cys His Gly Ser Leu Val Ala His Gly Arg Lys Ile Asn Val
               20                  25                  30
   Lys Pro Leu Asn Gln Gln His Tyr Ser Leu Asn Thr Lys Thr Val Ala
           35                  40                  45
   Asn Asn Asn Pro Tyr Pro Ser Leu Pro Ser Leu Lys Thr Lys Val Glu
       50                  55                  60
   Ser Pro Gln Tyr Glu Glu Ala Asn Lys Leu Gly Asp Ser Gly Ser Thr
   65                  70                  75                  80
   Gly Val Gly His Lys Ile Ile Thr Ser Ser Glu Asp Asn Lys Met Lys
                   85                  90                  95
   Thr Met Val Val Val Gln Ser Pro Asp Val Glu Val Phe Val Thr Lys
               100                 105                 110
   Gly Ser Lys Asp Asp Phe Lys Pro Thr Asp Pro Gly His Ser Pro Gly
           115                 120                 125
   Val Gly His Val Tyr Gln Asn Lys Ile Gly Gln Ala Asn
       130                 135                 140
<210> 54
   <211> 86
   <212> PRT
   <213> Glycine max RAR6
<400> 54
   Met Glu Asn Ser Ser Leu Arg Asn Ile Ala Phe Val Leu Phe Leu Phe
   1               5                   10                  15
   Leu Ile Leu His His Gln Val Leu Phe Val Gln Gly Arg Asn Leu Lys
               20                  25                  30
   Cys Pro Leu Cys Lys Glu Cys Ser Lys Ser Gln Lys Asn Thr Met Ser
           35                  40                  45
   Val Ala Ser Tyr Glu Val His Gln Glu Gly Leu Arg Arg Val Glu Tyr
       50                  55                  60
   Glu Val Asp Asp Phe Arg Pro Thr Thr Pro Gly His Ser Pro Gly Val
   65                  70                  75                  80
   Gly His Ser Ile Asn Asn
                   85
<210> 55
   <211> 109
   <212> PRT
   <213> Glycine max RAR7
<400> 55
   Met Ala Asn Ser Lys Leu Gly Phe Asn Phe Met Val Ser Ala Ile Phe
   1               5                   10                  15
   Leu Ser Leu Met Thr Phe His Gly Thr Phe Ser Val Gln Gly Arg Pro
               20                  25                  30
   Leu Lys Met Glu Ile Lys Glu Gln Val Thr Thr His Glu Asn Ile Ile
           35                  40                  45
   Asp Glu Ile Ala Lys Ala Ala Glu Tyr Thr Ala Thr Trp His Arg His
       50                  55                  60
   Thr Leu Glu Phe Glu Asp Thr Lys Asn Pro Gln Tyr Asp Gly Val Thr
   65                  70                  75                  80
   Asn Asp Phe Gln Pro Thr Asp Pro Gly His Ser Pro Gly Ala Gly His
                   85                  90                  95
   Ser Ser Pro His Ala Asn Ile Val Ser Ile Ser Lys Pro
               100                 105
<210> 56
   <211> 75
   <212> PRT
   <213> Glycine max RAR8
<220>
   <221> misc_feature
   <222> (75) .. (75)
   <223> Xaa can be any naturally occurring amino acid
<400> 56
   Ile Lys Leu Ile Asp Ala Pro Ile Val Pro Lys Phe Lys Phe Ala Asp
   1               5                   10                  15
   Val Asp Ser Gly Asp Ser Gly Ala Asp His Ala Asn Ala Phe Arg Pro
               20                  25                  30
   Thr Thr Pro Gly Asn Ser Pro Gly Val Gly His Lys Lys Phe Glu Gly
           35                  40                  45
   Glu Asp Lys Asp Ala Gly Ser Phe Glu Asn Asp Phe Arg Pro Thr Asp
       50                  55                  60
   Pro Gly His Ser Pro Gly Val Gly His Pro Xaa
   65                  70                  75
<210> 57
   <211> 87
   <212> PRT
   <213> Glycine max RAR9
<400> 57
   Met Ala Gln Asn Lys Phe Leu Leu Ser Leu Val Leu Leu Ala Leu Ile
   1               5                   10                  15
   Ile Phe Cys Gln Gly Phe His Ser Ile Glu Gly Arg Tyr Leu Lys Ser
               20                  25                  30
   Gly Glu Thr Ile Lys His Gln Met His Ser Gly Ile Ser Thr Thr Asn
           35                  40                  45
   Val Ala Asp Val Ser Pro Pro Thr Pro Pro Ser Ala Ala Val Pro Gly
       50                  55                  60
   Arg Asp Val Asp Asn Phe Arg Pro Thr Ala Pro Gly His Ser Pro Gly
   65                  70                  75                  80
   Val Gly His Thr Val His Asn
                   85
<210> 58
   <211> 108
   <212> PRT
   <213> Glycine max RAR10
<400> 58
   Met Ala Asn Leu Lys Leu Val Phe Thr Met Ser Ser Ile Leu Leu Val
   1 5 10 15
   Leu Val Phe Phe Asn Gly Ile Leu Pro Ala Met Gly Arg Pro Leu Lys
   20 25 30
   Lys Glu His Ile Thr Thr Thr Tyr Glu Asn Ser Val Lys Glu Met Gly
   35 40 45
   Thr Val Glu Asp Asn Asn Ile Leu Leu Trp Arg Arg Ser Ile Ile Glu
   50 55 60
   Asn Asn Ala Ala Asn Asp Gly Gly Val Asp Lys Trp Ile Asp Asp Phe
   65 70 75 80
   Arg Pro Met Asp Pro Gly His Ser Pro Gly Ala Gly His Ser Ser Pro
   85 90 95
   Thr Pro Lys Asp Ala Thr Asn Gly Ala Pro Arg Pro
   100 105
<210> 59
   <211> 81
   <212> PRT
   <213> Glycine max RAR11
<400> 59
   Met Ala Gln Lys Ile Ile Trp Leu Thr Phe Leu Val Phe Leu Ile Leu
   1 5 10 15
   Gln His Asn Phe Gly Thr Met Glu Ala Ser Arg Lys Leu Ile His Thr
   20 25 30
   His Pro Pro Pro Ala Ile Pro Arg Ser Pro Gln Ala Pro Ala Leu Trp
   35 40 45
   Tyr Thr Pro Asn Asp Glu Asp Gly Gly His Asp Ala Phe Arg Pro Thr
   50 55 60
   Cys Arg Gly His Ser Pro Gly Ala Gly His Asp Asn Pro Pro Thr Lys
   65 70 75 80
   Pro
<210> 60
   <211> 81
   <212> PRT
   <213> Glycine max RAR12
<400> 60
   Met Ala Arg Leu Thr His Phe Val Leu Leu Phe Val Leu Leu Phe Leu
   1 5 10 15
   Ser His Glu Leu Leu Gly Ser Glu Gly Arg Asn Leu Arg Gln Ile Thr
   20 25 30
   Ile Gln Ser Pro Asp Ala Thr Lys Ala Met Ser Ile Ala Thr Lys Ser
   35 40 45
   Ala Asn Ala Ile Pro Ser Tyr Arg Ser Ile Arg Ser Leu Ser Gly Asp
   50 55 60
   Val Glu Ala Phe Arg Pro Thr Thr Pro Gly His Ser Pro Gly Val Gly
   65 70 75 80
   His
<210> 61
   <211> 87
   <212> PRT
   <213> Glycine max RAR13
<400> 61
   Met Ala His Phe Thr Arg Thr Cys Leu Leu Leu Val Leu Leu Phe Leu
   1 5 10 15
   Ser Cys Glu Leu Leu Cys Ile Glu Gly Arg Gly Leu Lys Ala Thr Thr
   20 25 30
   Lys Ser Pro Lys Ser Val Ser Val Arg Ala Met Ser Thr Thr Lys Gly
   35 40 45
   Ala Val Ala Lys Pro Ser Gln Leu Glu Thr Ile Ala Lys Ser Leu Asn
   50 55 60
   Gly Phe Val Glu Ala Phe Arg Pro Thr Thr Pro Gly His Ser Pro Gly
   65 70 75 80
   Val Gly His Ser Val Asn Asn
   85
<210> 62
   <211> 163
   <212> PRT
   <213> Glycine max RAR14
<400> 62
   Met Ala Ile Phe Gln Tyr Ala Thr Arg Lys Cys Leu Val Ile Phe Leu
   1               5                   10                  15
   Leu Leu Val Ala Phe Asn Gly Ser Leu Leu Thr His Gly Arg Gln Ile
               20                  25                  30
   Lys Pro Leu Asn Gln Gln His Ser Ser Leu Asn Asn Asp Thr Val Val
           35                  40                  45
   Lys His Ser Val Asn Asn Val Pro Thr His Pro Ser Ser Gly Lys Lys
       50                  55                  60
   Lys Val Val Asp Ser Ser Ser Val Val Pro Lys Tyr Gly Val Glu Ser
   65                  70                  75                  80
   Phe Gly Asp Ser Met Ser Ser Asp Thr Asn Ala Phe Arg Pro Thr Thr
                   85                  90                  95
   Pro Gly Asn Ser Pro Gly Val Gly His Arg Lys Phe Ala Pro Glu Asp
               100                 105                 110
   Lys Asp Val Glu Ala Met Val Ala Ser Val Gln Ser Pro Asp His Val
           115                 120                 125
   Lys Val Tyr Val Thr Glu Gly Thr Gln Asn Gln Asp Gly Phe Lys Pro
       130                 135                 140
   Thr Asn Pro Gly His Ser Pro Gly Val Gly His Ala Gln Gln Asn Lys
   145                 150                 155                 160
   Ile Gly Gln
<210> 63
   <211> 76
   <212> PRT
   <213> Glycine max RAR15
<400> 63
   Glu Ser Pro Gln His His Glu Glu Ser Ser Lys Leu Glu Asp Ser Gly
   1               5                   10                  15
   Ala Asp Asn Thr Asn Ala Phe Arg Pro Thr Thr Pro Gly Gly Ser Pro
               20                  25                  30
   Gly Val Gly His Lys Met Ile Thr Ser Ser Ser Glu Asp Asn Lys Gly
   35 40 45
   Ser Lys Asp Asp Phe Lys Pro Thr Asp Pro Gly His Ser Pro Gly Val
   50 55 60
   Gly His Ala Tyr Lys Asn Lys Ile Gly Asp Gly Asn
   65 70 75
<210> 64
   <211> 108
   <212> PRT
   <213> Glycine max RAR16
<400> 64
   Met Thr Asn Leu Lys Leu Val Phe Thr Ile Ser Ser Ile Leu Leu Ala
   1 5 10 15
   Leu Val Phe Ile Asn Gly Ile Ser Ser Val Met Gly Arg Pro Leu Lys
   20 25 30
   Lys Glu His Ile Ile Thr Thr Thr Tyr Glu Asn Ser Val Lys Glu Met
   35 40 45
   Gly Thr Val Glu Asp Asn Asn Ile Leu Leu Trp Arg Arg Ser Ile Ile
   50 55 60
   Glu Asn Ala Ala Asn Asp Gly Gly Val Asp Lys Trp Ile Asp Asp Phe
   65 70 75 80
   Arg Pro Thr Asp Pro Gly His Ser Pro Gly Ala Gly His Ser Ser Pro
   85 90 95
   Thr Pro Lys Asp Ala Ser Asn Gly Ala Pro Arg Pro
   100 105
<210> 65
   <211> 95
   <212> PRT
   <213> Lotus japonicus RAR1
<400> 65
   Met Thr Asn Ser Lys Lys Leu Val Phe Thr Ile Ser Ser Ile Leu Leu
   1               5                   10                  15
   Leu Thr Leu Met Phe Ser Asn Phe Ile Phe Ser Ala His Gly Arg Pro
               20                  25                  30
   Leu Lys Thr Glu Asn Lys Glu His Val Thr Thr Tyr Glu Asn Asn Ser
           35                  40                  45
   Val Lys Glu Met Ala Thr Gly Glu Asn Asp His Lys Val Gly Lys Leu
       50                  55                  60
   Ile Asn Asp Phe Lys Pro Thr Asp Pro Gly His Ser Pro Gly Val Gly
   65                  70                  75                  80
   His Ser Ser Pro Ile Pro Met Asp Ala Asn Glu Pro Pro Arg Ser
                   85                  90                  95
<210> 66
   <211> 89
   <212> PRT
   <213> Lotus japonica RAR2
<400> 66
   Met Ala Gln Asn Lys Pro Ile Phe Ser Leu Ile Leu Leu Ala Leu Ile
   1               5                   10                  15
   Ile Phe Cys His Gly Phe Gln Ser Ile Glu Gly Arg Tyr Phe Lys Ile
               20                  25                  30
   Gly Glu Gly Thr Gln His Leu Met Lys His Gly Asp Phe Ser Thr Thr
           35                  40                  45
   Asn Gly Val Val Ser Gly Ala Ser Glu Ala Pro Ser Leu Thr Pro Ser
       50                  55                  60
   Arg Asp Val Ser Gly Phe Lys Gln Pro Thr Thr Gly Pro Gly His Ser
   65                  70                  75                  80
   Pro Gly Val Gly His Ser Ile His Asn
                   85
<210> 67
   <211> 99
   <212> PRT
   <213> Lotus japonica RAR3
<400> 67
   Met Ala Lys Thr Asn Leu Lys Phe Val Cys Val Val Phe Leu Leu Leu
   1               5                   10                  15
   Ile Leu His His Gln His Val Cys Val Gln Gly Arg His Leu Arg Ser
               20                  25                  30
   Cys Leu Cys Arg Gly Cys Pro Lys Thr Cys Val Lys Ile Lys Ser Gly
           35                  40                  45
   Val Ala His Gly Val Gly Asp Arg Gly Asn Arg Ala Thr Thr His Asp
       50                  55                  60
   Tyr Asp Thr His Gln Gly Arg Lys Arg Leu Val Glu Tyr Glu Val Glu
   65                  70                  75                  80
   Ala Phe Arg Pro Thr Ser Pro Gly His Ser Pro Gly Val Gly His Ser
                   85                  90                  95
   Ile Asn Asn
<210> 68
   <211> 86
   <212> PRT
   <213> Lotus japonica RAR4
<400> 68
   Met Glu Glu Lys Thr Val Met Leu Thr Leu Leu Val Ile Leu Ile Leu
   1               5                   10                  15
   Gln His Asn Tyr Gly Ser Met Ala Leu Ser Gly Asn Asn Ile His Pro
               20                  25                  30
   Pro Pro Ala Ile Pro Arg Ala Leu Leu Arg Ser Pro Gln Pro Pro Ser
           35                  40                  45
   Pro Gly Trp Tyr Thr Ile Asn Asp Asp Lys Val Gly Glu Gly Asp Ala
       50                  55                  60
   Phe Arg Pro Thr Thr Pro Gly His Ser Pro Gly Val Gly His Asp Ser
   65                  70                  75                  80
   Pro Pro Asn Phe His Ala
                   85
<210> 69
   <211> 91
   <212> PRT
   <213> Lotus japonica RAR5
<400> 69
   Met Ala His Phe Ala Arg Thr Cys Leu Leu Phe Val Leu Leu Phe Val
   1 5 10 15
   Ser Cys Glu Leu Leu Cys Ile Glu Gly Arg Thr Leu Ser Lys Asn Val
   20 25 30
   Leu Asp His Ser Leu Lys Ser Ser Ser Val Lys Ala Met Ser Ile Ala
   35 40 45
   Thr Val Lys Thr Glu Asn Gly Val Val Ala Ser Pro Ser Gln Leu Arg
   50 55 60
   Arg Ser Met Glu Gly Tyr Val Glu Ala Phe Arg Pro Thr Thr Pro Gly
   65 70 75 80
   His Ser Pro Gly Val Gly His Ser Val His Asn
   85 90
<210> 70
   <211> 125
   <212> PRT
   <213> Lotus japonica RAR6
<400> 70
   Met Gly Glu Phe Gln Ala Arg Thr Ile Tyr Phe Leu Val Phe Leu Ala
   1 5 10 15
   Leu Phe Ala Cys Asn Cys Ser Leu Leu Cys His Gly Arg Pro Leu Lys
   20 25 30
   Pro Val Asn Ser Pro Ile Met Pro Asn Gln Asp Val Ala Thr Ser Gly
   35 40 45
   Asp Ala Ala Gly Ala Ser Tyr Thr Asn Ala Phe Glu Pro Thr Thr Pro
   50 55 60
   Gly Asn Ser Pro Gly Val Gly His Arg Ser Phe Ala Gly Glu Asp Asn
   65 70 75 80
   Lys Met Val Ala Ala Gln Ser Pro Asp Val Gly Val Ser Val Thr Gln
   85 90 95
   Gly Ser Glu Ser Asp Phe Lys Pro Thr Asp Pro Gly His Ser Pro Gly
   100 105 110
   Val Gly His Ala Tyr Gln Glu Lys Ile Gly His Leu Asn
   115 120 125
<210> 71
   <211> 210
   <212> PRT
   <213> Populus trichocarpa RAR1
<400> 71
   Met Pro His Pro Ala Val Pro Ser Phe Gly Asn Ser Ala Ala Val Tyr
   1               5                   10                  15
   Lys Asp Asp Phe Arg Pro Thr Thr Pro Gly Val Ser Pro Gly Val Gly
               20                  25                  30
   His Pro Lys Thr Ile Gly Thr Asn Ser Asn Asn Glu His Ser Leu Thr
           35                  40                  45
   Asp Phe Lys Asp Asp Phe Gln Pro Thr Thr Pro Gly His Ser Pro Gly
       50                  55                  60
   Ala Gly His Ala Leu Ala Asn Asp Asp Asp Asn Glu Glu Val Ser Pro
   65                  70                  75                  80
   Lys Ala Pro Gly Pro Ser Ile Glu Arg Ser Gly Thr Ala Phe Lys Pro
                   85                  90                  95
   Thr Thr Pro Gly His Ser Pro Gly Ala Gly His Ala Leu Ala Asn Asp
               100                 105                 110
   Asp Asp Asn Glu Glu Val Ser Pro Lys Ala Pro Gly Ser Ser Ile Glu
           115                 120                 125
   Arg Ser Gly Thr Ala Phe Lys Pro Thr Thr Pro Gly His Ser Pro Gly
       130                 135                 140
   Ile Gly His Leu Phe Ser Glu Asn Asp Thr Asp Lys Asn Glu Ile Thr
   145                 150                 155                 160
   Ala Ser Lys Ala Ser Ser Ile Glu His Ser Val Thr Gly Val Thr Asp
                   165                 170                 175
   Asp Phe Arg Pro Thr Val Pro Gly His Ser Pro Gly Ile Gly His Ala
               180                 185                 190
   Phe Arg Pro Pro Thr Pro Gly His Ser Pro Gly Val Gly His Ser Ile
           195                 200                 205
   His Asn
       210
<210> 72
   <211> 370
   <212> PRT
   <213> Populus trichocarpa RAR2
<400> 72
   Met Ala Glu Thr Cys Lys Cys Ala Phe Leu Ile Leu Ala Phe Val Thr
   1               5                   10                  15
   Cys Phe Gln Ile Leu Phe Ile Glu Gly Arg Ser Ile Lys Gln Thr Asn
               20                  25                  30
   Lys Gln Glu His Val Thr Asn Glu Ile Glu Pro Leu Lys Glu Met Ala
           35                  40                  45
   Asn Gln Ser Thr Asn Thr Asn Leu His His Asn Thr Ala Asn Asn Gln
       50                  55                  60
   Lys Val Ser Leu Pro Ser Pro Pro Val His Ile Pro Thr Val His His
   65                  70                  75                  80
   Ser Lys Ala Gly Arg Lys Glu Met Thr Pro Pro Met Val Pro Ser Phe
                   85                  90                  95
   Ser Gly Ser Pro Gly Val Arg His Pro Lys Thr Pro Gly Ala Asn Ser
               100                 105                 110
   Val Thr Thr Val Lys Asp Asp Phe Lys Pro Ile Thr Ser Gly Gln Ser
           115                 120                 125
   Pro Gly Val Gly His Asn Asn Asp Asn Ser Val Thr Ala Phe Lys Asp
       130                 135                 140
   Asp Phe Gln Pro Thr Thr Pro Gly Asn Ser Pro Gly Val Gly His Ile
   145                 150                 155                 160
   Leu Val Asp Glu Asp Asp Ser Glu Asp Asp Asp Pro Lys Ala Pro Gly
                   165                 170                 175
   Thr Ser Ser Ser Asn Glu Arg Ser Gly Ala Ala Phe Lys Pro Thr Thr
               180                 185                 190
   Pro Gly His Ser Pro Gly Val Gly His Met Ser Ser Val Asp Gln Ser
           195                 200                 205
   Asp Lys Thr Asp Leu Lys Ala Ser Lys Thr Glu Leu Ser Val Thr Thr
       210                 215                 220
   Pro Ala Phe Lys Pro Thr Thr Pro Gly His Ser Pro Gly Val Gly His
   225                 230                 235                 240
   Met Ser Ser Val Asp Gln Ser Asp Lys Ile Asp Ser Lys Ala Ser Glu
                   245                 250                 255
   Ile Glu His Phe Asn Thr Glu His Ser Val Thr Thr Pro Gly His Ser
               260                 265                 270
   Pro Ala Val Gly His Ile Leu Ser Asp Glu Asp Glu Asp Asp Asn Glu
           275                 280                 285
   Asp Val Asp Pro Lys Ala Pro Gly Thr Gly Ser Ser Ile Lys Arg Ser
       290                 295                 300
   Gly Ala Ala Phe Lys Pro Thr Thr Pro Gly His Ser Pro Gly Ile Gly
   305                 310                 315                 320
   His Met Ser Ser Val Asp Gln Ser Asp Lys Thr Asp Arg Lys Ala Thr
                   325                 330                 335
   Asn Ile Glu His Ser Val Ala Arg Val Pro Asp Gly Phe Arg Pro Ala
               340                 345                 350
   Val Pro Ile Gln Gly Pro Gly Val Gly His Val Phe Gln Ala Gln Thr
           355                 360                 365
   Lys Asn
       370
<210> 73
   <211> 158
   <212> PRT
   <213> Populus trichocarpa RAR3
<400> 73
   Met Ile Gln Arg His Pro Val Leu Ala Pro Val Met Lys Arg Ser Gly
   1               5                   10                  15
   Ala Ala Phe Lys Pro Thr Thr Pro Gly His Ser Pro Gly Ile Gly His
               20                  25                  30
   Met Ser Ser Val Asp Gln Thr Phe Lys Pro Thr Thr Pro Gly His Ser
           35                  40                  45
   Pro Gly Ile Gly His Met Ser Ser Val Asp Gln Ser Asp Lys Thr Asp
       50                  55                  60
   Ser Lys Ala Ser Glu Ile Lys His Ser Val Thr Thr Pro Gly His Ser
   65                  70                  75                  80
   Ser Arg Val Gly His Ile Leu Ser Asp Glu Asp Ala Asp Asp Thr Phe
                   85                  90                  95
   Lys Pro Thr Thr Pro Gly His Ser Pro Gly Ile Gly His Met Ser Ser
               100                 105                 110
   Val Asp Gln Ser Asp Lys Thr Asp Arg Lys Ala Thr Asn Ile Glu His
           115                 120                 125
   Ser Val Ala Arg Val Pro Asp Gly Phe Arg Pro Ala Val Pro Ile Gln
       130                 135                 140
   Gly Pro Gly Val Gly His Val Phe Gln Ala Gln Thr Lys Asn
   145                 150                 155
<210> 74
   <211> 90
   <212> PRT
   <213> Populus trichocarpa RAR4
<400> 74
   Met Ala Asp Lys Thr Arg Ser Phe Met Leu Thr Phe Phe Thr Val Val
   1               5                   10                  15
   Leu Leu Leu Leu His Gln His Phe Asp Leu Thr Ala Ala Ser Arg Pro
               20                  25                  30
   Leu Asp Ile His Ser Pro Ala Ile Pro Arg Ser Gly Ser Glu Pro Pro
           35                  40                  45
   Pro Thr Asp Val His Asp Arg Trp Tyr Arg Ile Asn Arg Tyr Lys Asn
       50                  55                  60
   Leu Glu Ser Asp Ala Phe Arg Pro Thr Thr Pro Gly His Ser Pro Gly
   65                  70                  75                  80
   Val Gly His Glu Asn Pro Pro Ala Ala Pro
                   85                  90
<210> 75
   <211> 88
   <212> PRT
   <213> Populus trichocarpa RAR5
<400> 75
   Met Ala Lys Gly Lys Leu Ile Phe Thr Ser Thr Leu Ile Ile Val Leu
   1               5                   10                  15
   Val Leu Cys Tyr Gly Ile Thr Ser Ser Val Gly Arg Leu Leu Lys Thr
               20                  25                  30
   Gly Glu Asn Thr Ser Ser Phe Ser Leu His Arg Asp Leu Leu Val Ser
           35                  40                  45
   Glu Ala Arg Ser Glu Pro Val Thr Pro Gly Pro Asp His Ala Asp Ala
       50                  55                  60
   Asp Ser Asp Asp Phe Lys Pro Thr Thr Pro Gly His Ser Pro Gly Ala
   65                  70                  75                  80
   Gly His Ser Thr Pro Gly His Asn
                   85
<210> 76
   <211> 70
   <212> PRT
   <213> Populus trichocarpa RAR6
<400> 76
   Met Ala Gln Ser Asn Leu Leu Ser Ala Phe Val Phe Leu Val Leu Ile
   1               5                   10                  15
   Phe Ser His Glu Leu Gln Phe Ile Glu Gly Arg Tyr Leu Asn Leu Lys
               20                  25                  30
   Thr Pro Asn Lys Phe Leu Gln Lys Glu Ile Arg Arg Leu Val Glu Ser
           35                  40                  45
   Asn Ser Lys Leu His Val Asn Asp Asn Leu Asp Lys Pro Val Asn Ala
       50                  55                  60
   Thr Lys Val Ala Pro Pro
   65                  70
<210> 77
   <211> 244
   <212> PRT
   <213> Vitis vinifera RAR1
<400> 77
   Met Ala Ile Ile Gln Val Ile His Ala Cys Ser Leu Leu Leu Ala Val
   1               5                   10                  15
   Ile Thr Tyr His Asp Ile Leu Tyr Thr Glu Gly Arg Pro Ile Asn Ser
               20                  25                  30
   Val Thr Lys Gln Glu Phe Ser Ser Thr Asp Phe Glu Pro Gly Asn Glu
           35                  40                  45
   Thr Gly Ser Gln Gly Thr Glu His Lys Glu Asp His Trp Tyr Thr Pro
       50                  55                  60
   Pro Pro Pro Glu Pro Asn Pro Ser Val Lys Asn Ser Val Val Gly Lys
   65                  70                  75                  80
   Asp Ile Leu Pro Pro Ile Thr Pro Asn Tyr Ser Ile Gly Phe Gly Asp
                   85                  90                  95
   Ser Thr Ala Val Tyr Lys Asp Gly Phe Arg Pro Thr Thr Pro Gly Ser
               100                 105                 110
   Ser Pro Gly Ile Gly His Gln Phe Val Pro Thr Lys Glu Asp Ile Gln
           115                 120                 125
   Pro Lys Ala Leu Gly Asn Ser Pro Ser Val Arg His Ser Val Thr Ala
       130                 135                 140
   Tyr Lys Asp Asp Tyr Arg Pro Thr Met Pro Gly His Ser Pro Gly Glu
   145                 150                 155                 160
   Pro Asn Pro Gly Val Lys Asn Ser Val Ala Gly Lys Lys Glu Leu Pro
   165 170 175
   Pro Pro Met Leu Pro Asn Tyr Ser Val Gly Phe Gly Asp Ser Thr Ala
   180 185 190
   Val Ser Lys Asp Asp Phe Arg Pro Thr Thr Pro Gly Ser Ser Pro Gly
   195 200 205
   Val Gly His His Ser Asp Asp Tyr Arg Pro Thr Lys Pro Gly His Ser
   210 215 220
   Pro Gly Val Gly His Ser Leu Gln Lys Thr Asn Ala Glu Pro Asn Ala
   225 230 235 240
   Glu Pro Asn Ala
<210> 78
   <211> 140
   <212> PRT
   <213> Vitis vinifera RAR2
<400> 78
   Met Ala Lys Ile Arg Phe Ile His Ala Tyr Ser Leu Leu Leu Ala Val
   1 5 10 15
   Ile Thr Tyr His His Ile Leu Cys Thr Glu Ala Arg Pro Ile Lys Ser
   20 25 30
   Pro Ser Ser Ile Asp Tyr Glu Pro Gly Lys Glu Thr Gly Ser Gln Gly
   35 40 45
   Thr Glu His Lys Asp Val Trp Ser Gly Pro Pro Pro Pro Glu Pro Asn
   50 55 60
   Pro Ile Val Lys Asn Ser Val Ala Gly Lys Glu Glu Ile Leu Pro Pro
   65 70 75 80
   Met Ile Pro Asn Tyr Ser Val Gly Phe Gly Asp Ser Ala Ala Val His
   85 90 95
   Thr Asp Gly Phe Arg Pro Thr Thr Pro Gly Ser Ser Pro Gly Ile Gly
   100 105 110
   His Ser Ser Ala Pro Thr Lys Glu Asp Ile Glu Pro Lys Ala Pro Gly
   115 120 125
   Asn Ser Pro Lys Thr Phe Arg Gln Gly Lys Gln Gly
   130 135 140
<210> 79
   <211> 140
   <212> PRT
   <213> Vitis vinifera RAR3
<400> 79
   Met Ala Lys Val His Ile Ile His Ala Cys Ser Leu Leu Leu Ala Val
   1               5                   10                  15
   Ile Thr Asn His Asp Ile Leu Tyr Thr Glu Gly Arg Pro Met Lys Ser
               20                  25                  30
   Leu Ser Lys His Glu Phe Ser Ser Ile Asp Ser Gly Pro Gly Thr Glu
           35                  40                  45
   Thr Gly Ser Glu Gly Ile Glu His Lys Asp Asp His Arg Ser Ala Pro
       50                  55                  60
   Pro Pro Pro Glu Pro Asn Pro Gly Val Lys Asn Ser Val Ala Gly Lys
   65                  70                  75                  80
   Lys Glu Leu Pro Pro Pro Met Met Pro Asn Tyr Thr Thr Gly Leu Ala
                   85                  90                  95
   Asp Ser Thr Ala Val Tyr Glu Asp Asp Phe Arg Pro Thr Pro Pro Gly
               100                 105                 110
   Ser Ser Pro Gly Ile Gly His His Phe Asp Phe Arg Pro Thr Thr Pro
           115                 120                 125
   Gly His Ser Pro Gly Val Gly His Ser Leu Gln Asn
       130                 135                 140
<210> 80
   <211> 110
   <212> PRT
   <213> Vitis vinifera RAR4
<400> 80
   Met Ala Lys Val Lys Leu Ile Met Ser Ile Tyr Val Phe Ile Leu Ala
   1               5                   10                  15
   Leu Val Leu Ile Tyr Gly Gly Leu Met Ser Glu Gly Arg Lys Leu Asp
               20                  25                  30
   Ile Glu Lys Asn Ser Lys Cys Glu Met Cys Val Ser Ile Asp Glu Lys
           35                  40                  45
   Ile Ser Val Leu Gly Asn Leu His Arg Ser Ser Lys Ala Asn Ala Arg
       50                  55                  60
   Pro His Ala Pro Ala Arg Gln Ser Pro Gly Ala Asp Arg Leu Phe Thr
   65                  70                  75                  80
   Asp Asp Gly Val Asp Val Gln Ser Thr Thr Pro Gly His Ser Pro Gly
                   85                  90                  95
   Val Gly His Ser Val Gly Pro Ala Ser Asn Asp Pro Asn Pro
               100                 105                 110
<210> 81
   <211> 166
   <212> PRT
   <213> Vitis vinifera RAR5
<400> 81
   Met Asp Arg Gln Asp Ala Ile Leu Asp Ile Gln Trp Leu Lys Lys Ile
   1               5                   10                  15
   Leu Val Arg His Arg Val Ile Thr Asn His Asp Ile Leu Tyr Thr Glu
               20                  25                  30
   Gly Arg Pro Met Lys Ser Leu Ser Lys His Glu Phe Ser Ser Ile Asp
           35                  40                  45
   Ser Gly Pro Gly Thr Glu Thr Gly Ser Glu Gly Ile Glu His Lys Asp
       50                  55                  60
   Asp His Arg Ser Ala Pro Pro Pro Pro Glu Pro Asn Pro Gly Val Lys
   65                  70                  75                  80
   Asn Ser Val Ala Gly Lys Lys Glu Leu Pro Pro Pro Met Met Pro Asn
                   85                  90                  95
   Tyr Thr Thr Gly Leu Ala Asp Ser Thr Ala Val Tyr Glu Asp Asp Phe
               100                 105                 110
   Arg Pro Thr Pro Pro Gly Ser Ser Pro Gly Ile Gly His His Phe Val
           115                 120                 125
   Pro Thr Lys Gly Asp Ile Gln Pro Lys Ala Gln Gly Asn Ser Pro Gly
       130                 135                 140
   Val Gly Gln Ser Val Thr Ala Tyr Lys Asp Asp Tyr Pro Pro Thr Lys
   145                 150                 155                 160
   Pro Ala Arg Ser Gln Pro
                   165
<210> 82
   <211> 173
   <212> PRT
   <213> Vitis vinifera RAR6
<400> 82
   Met Ala Lys Ile Gln Val Ile His Ala Cys Ser Leu Val Leu Ala Val
   1               5                   10                  15
   Ile Thr Tyr His Asp Ile Leu Tyr Thr Glu Gly Arg Pro Ile Lys Ser
               20                  25                  30
   Leu Asp Lys His Glu Phe Ser Ser Ile Asp Ser Glu Pro Gly Thr Glu
           35                  40                  45
   Thr Gly Ser Gln Gly Ile Glu His Lys Asp Asp His Trp Ala Ala Pro
       50                  55                  60
   Pro Gln Glu Pro Asn Pro Gly Val Lys Asn Ser Val Ala Gly Lys Lys
   65                  70                  75                  80
   Glu Leu Pro Pro Pro Met Leu Pro Asn Tyr Ser Val Gly Phe Gly Asp
   85 90 95
   Ser Thr Ala Val Ser Lys Asp Asp Phe Arg Pro Thr Thr Pro Gly Ser
   100 105 110
   Ser Pro Gly Val Gly His His Ser Val Pro Thr Lys Asp Asp Thr Gln
   115 120 125
   Pro Lys Ala Leu Arg Asn Ser Pro Ser Val Arg Gln Ser Val Thr Ala
   130 135 140
   Tyr Lys Asp Asp Tyr Arg Pro Thr Lys Pro Gly His Ser Pro Gly Val
   145 150 155 160
   Gly His Ser Leu Gln Lys Thr Asn Ala Glu Pro Asn Ala
   165 170
<210> 83
   <211> 89
   <212> PRT
   <213> Vitis vinifera RAR7
<400> 83
   Met Ala Asn Thr Arg Phe Leu Gly Ala Cys Ala Val Leu Leu Val Leu
   1 5 10 15
   Leu Leu Cys His Glu Phe Ser Cys Val Lys Gly Arg His Leu Arg Ser
   20 25 30
   Ala Met Cys Lys Lys Cys Ser Arg His Arg Gln Thr Ser Leu Arg Ala
   35 40 45
   Thr Glu Ala Gly Glu Ala Pro Ser Gly Leu Pro Gln Met Ser Thr Ser
   50 55 60
   Lys Met Glu His Ile Glu Asp Phe Arg Pro Thr Ser Pro Gly His Ser
   65 70 75 80
   Pro Gly Val Gly His Ser Ile His Asn
   85
<210> 84
   <211> 267
   <212> PRT
   <213> Ricinus communis RAR1
<400> 84
   Met Arg Lys Gln Leu Glu Ala Phe Gln Lys Glu Leu Ala Lys Arg Gly
   1 5 10 15
   Val Ser Asn Thr Ile Asn Leu His Gln Ser Lys Leu Ala Gly Gln Asp
   20 25 30
   His Gln Glu Gln Thr His Thr Gly Phe Ser Asp Phe Ala Ala Ala Ser
   35 40 45
   Val Asp Ala Phe Arg Pro Thr Pro Pro Gly Asn Ser Pro Gly Val Gly
   50 55 60
   His Pro Lys Ala Val Val Thr Ser Ser Ser Thr Thr Asp Gln His Ser
   65 70 75 80
   Leu Thr Gly Leu Arg His Asp Tyr Ser Asn Leu His Lys Ser Ser His
   85 90 95
   Asn Ile Pro Gly Asn Val Gln Gln Ser Met Ser Gly Lys Glu Glu Thr
   100 105 110
   Ser Pro Thr Ser Leu Asp Val Phe Ala Ala Ala Ser Thr Asp Asp Phe
   115 120 125
   Arg Pro Thr Ser Pro Gly Tyr Ser Pro Gly Val Gly His Pro Lys Ala
   130 135 140
   Val Val Thr Ser Ser Ser Thr Ala Asp Gln His Ser Phe Thr Gly Val
   145 150 155 160
   Lys Asp Tyr Tyr Asn Asn Val His Lys Ser Asn His Ile Gly Val Ala
   165 170 175
   Asp Asn Val Lys Lys Pro Val Ser Gly Lys Gly Glu Met Leu Pro Thr
   180 185 190
   Val Thr Thr Thr Ser Phe Asp Ala Ser Ala Ala Ser Thr Lys Asp Asp
   195 200 205
   Phe Arg Pro Thr Ala Pro Gly Phe Ser Pro Gly Val Gly His Pro Lys
   210 215 220
   Lys Val Val Thr Ser Ser Ser Thr Lys His Ser Ile Thr Gly Phe Lys
   225 230 235 240
   Asp Asp Tyr Arg Pro Thr Gln Pro Gly His Ser Pro Gly Val Gly His
   245 250 255
   Ser Tyr Gln Lys Asn Asn Ala Gly Gln Asp Pro
   260 265
<210> 85
   <211> 101
   <212> PRT
   <213> Ricinus communis RAR2
<400> 85
   Met Ala Ile Ala Ala Ser Ala Ala Ala Thr Asn Leu Met Gly Thr Cys
   1               5                   10                  15
   Thr Cys Leu Leu Val Leu Ile Leu Cys His Glu Ala Ile Tyr Val Val
               20                  25                  30
   Glu Gly Arg His Leu Lys Pro Lys Leu Cys Lys Lys Cys Ser Arg Arg
           35                  40                  45
   Ser Glu Ser Ser Leu Asp Val Ser Lys Asp Gly His His Asn Thr Thr
       50                  55                  60
   Thr His Leu Leu Asn Gly Asp Gln Glu Lys Ile Ser Lys Met Asp Phe
   65                  70                  75                  80
   Val Asp Asp Phe Arg Pro Thr Ala Pro Gly His Ser Pro Gly Val Gly
                   85                  90                  95
   His Ser Ile Gln Asn
               100
<210> 86
   <211> 95
   <212> PRT
   <213> Ricinus communis RAR3
<400> 86
   Met Ala Asn Val Cys Tyr Thr Cys Leu Phe Phe Leu Val Met Leu Leu
   1               5                   10                  15
   Ser Tyr Asp Leu Val Cys Ile Glu Ala Arg Gln Leu Lys Leu Arg Glu
               20                  25                  30
   Asn Met Lys Cys Val Lys Cys Leu Ser Ala Pro Asp Ser Lys Glu Ser
           35                  40                  45
   Ile Thr Arg Asn Pro Arg Gly Asp Asn Ala Met Ser Ser Ser Gln Asp
       50                  55                  60
   Gly Ile Glu Pro Lys Asp Gly Ser Asn Asn Phe Asp Ala Phe Arg Pro
   65                  70                  75                  80
   Thr Asn Pro Gly His Ser Pro Gly Val Gly His Ser Ile Gln His
                   85                  90                  95
<210> 87
   <211> 86
   <212> PRT
   <213> Ricinus communis RAR4
<400> 87
   Met Ala Arg Val Lys Leu Asn Phe Ser Ile Val Leu Val Ile Ala Leu
   1               5                   10                  15
   Val Val Ser Tyr Gly Ile Thr Ser Thr Glu Glu Arg Gln Leu Arg Met
               20                  25                  30
   Gln Val Arg Ala Ala Gly Met Glu Lys Gly Thr Gly Asn Leu Tyr Phe
           35                  40                  45
   Gly Arg Ser Leu Leu Val Asp Asn Asp Gly Asp Ser Asp Asp Phe Arg
       50                  55                  60
   Pro Thr Asn Pro Gly His Ser Pro Gly Ala Gly His Ser Thr Gly Pro
   65                  70                  75                  80
   Ser Ser Lys Asn Ala His
                   85
<210> 88
   <211> 123
   <212> PRT
   <213> Ricinus communis RAR5
<400> 88
   Met Ala Gln Thr Asn Leu Leu Phe Gly Cys Ile Phe Ile Met Leu Ile
   1               5                   10                  15
   Phe Phe Gln Glu Leu Gln Ser Ile Ser Gly Arg His Leu Asn Leu Glu
               20                  25                  30
   Thr Asn His Lys Phe Ser Lys Ile Gln Val Ser Tyr Ile Asn Phe Glu
           35                  40                  45
   Arg Gln His Arg Gln Phe Ile Gly His Asn Val Asp Ile Glu His Asn
       50                  55                  60
   Asp Leu Asn Lys Asp Val Phe Ala Ala Asn Lys Met Ser Pro Ala Ala
   65                  70                  75                  80
   Pro Val Ala Ala Ala Gly Gly Ile Gly Glu Ala Glu Ser Pro Pro Pro
                   85                  90                  95
   Pro Pro Ala Ser Gly His Val Asp Asp Phe Arg Pro Thr Ala Pro Gly
               100                 105                 110
   His Ser Pro Gly Val Gly His Ser Ile Gln Asn
           115                 120
<210> 89
   <211> 89
   <212> PRT
   <213> Ricinus communis RAR6
<400> 89
   Met Ala Asn Arg Ala Phe Leu Leu Thr Leu Phe Ala Ile Ser Phe Leu
   1               5                   10                  15
   Leu Leu His Gln His Leu Asp Ser Ala Val Ala Ser Arg Pro Leu His
               20                  25                  30
   Met His Pro Pro Ala Ile Ile Ser Gln Gly Ser Leu Lys Arg Pro Leu
           35                  40                  45
   Pro Pro Ser Thr Ala Leu Leu Tyr Ser Ile Asn Arg His Lys Phe Thr
       50                  55                  60
   Glu Thr Glu Ala Phe Arg Pro Thr Ala Pro Gly His Ser Ser Gly Val
   65                  70                  75                  80
   Gly His Gly Asn Pro Pro Ala Ala Pro
                   85
<210> 90
   <211> 267
   <212> PRT
   <213> Ricinus communis RAR7
<400> 90
   Met Arg Lys Gln Leu Glu Ala Phe Gln Lys Glu Leu Ala Lys Arg Gly
   1               5                   10                  15
   Val Ser Asn Thr Ile Asn Leu His Gln Ser Lys Leu Ala Gly Gln Asp
               20                  25                  30
   His Gln Glu Gln Thr His Thr Gly Phe Ser Asp Phe Ala Ala Ala Ser
           35                  40                  45
   Val Asp Ala Phe Arg Pro Thr Pro Pro Gly Asn Ser Pro Gly Val Gly
       50                  55                  60
   His Pro Lys Ala Val Val Thr Ser Ser Ser Thr Thr Asp Gln His Ser
   65                  70                  75                  80
   Leu Thr Gly Leu Arg His Asp Tyr Ser Asn Leu His Lys Ser Ser His
                   85                  90                  95
   Asn Ile Pro Gly Asn Val Gln Gln Ser Met Ser Gly Lys Glu Glu Thr
               100                 105                 110
   Ser Pro Thr Ser Leu Asp Val Phe Ala Ala Ala Ser Thr Asp Asp Phe
           115                 120                 125
   Arg Pro Thr Ser Pro Gly Tyr Ser Pro Gly Val Gly His Pro Lys Ala
       130                 135                 140
   Val Val Thr Ser Ser Ser Thr Ala Asp Gln His Ser Phe Thr Gly Val
   145                 150                 155                 160
   Lys Asp Tyr Tyr Asn Asn Val His Lys Ser Asn His Ile Gly Val Ala
                   165                 170                 175
   Asp Asn Val Lys Lys Pro Val Ser Gly Lys Gly Glu Met Leu Pro Thr
               180                 185                 190
   Val Thr Thr Thr Ser Phe Asp Ala Ser Ala Ala Ser Thr Lys Asp Asp
           195                 200                 205
   Phe Arg Pro Thr Ala Pro Gly Phe Ser Pro Gly Val Gly His Pro Lys
       210                 215                 220
   Lys Val Val Thr Ser Ser Ser Thr Lys His Ser Ile Thr Gly Phe Lys
   225                 230                 235                 240
   Asp Asp Tyr Arg Pro Thr Gln Pro Gly His Ser Pro Gly Val Gly His
                   245                 250                 255
   Ser Tyr Gln Lys Asn Asn Ala Gly Gln Asp Pro
               260                 265
<210> 91
   <211> 61
   <212> PRT
   <213> Ricinus communis RAR8
<400> 91
   Met Lys Cys Val Lys Cys Leu Ser Ala Pro Asp Ser Lys Glu Ser Ile
   1 5 10 15
   Thr Arg Asn Pro Arg Gly Asp Asn Ala Met Ser Ser Ser Gln Asp Gly
   20 25 30
   Ile Glu Pro Lys Asp Gly Ser Asn Asn Phe Asp Ala Phe Arg Pro Thr
   35 40 45
   Asn Pro Gly His Ser Pro Gly Gly His Ser Ile Gln His
   50 55 60
<210> 92
   <211> 70
   <212> PRT
   <213> Ricinus communis RAR9
<400> 92
   Met Ala Gln Ser Asn Leu Leu Ser Ala Phe Val Phe Leu Val Leu Ile
   1 5 10 15
   Phe Ser His Glu Leu Gln Phe Ile Glu Gly Arg Tyr Leu Asn Leu Lys
   20 25 30
   Thr Pro Asn Lys Phe Leu Gln Lys Glu Ile Arg Arg Leu Val Glu Ser
   35 40 45
   Asn Ser Lys Leu His Val Asn Asp Asn Leu Asp Lys Pro Val Asn Ala
   50 55 60
   Thr Lys Val Ala Pro Pro
   65 70
<210> 93
   <211> 84
   <212> PRT
   <213> Casuarina glauca RAR1
<400> 93
   Met Ala His Arg Thr Leu Met Leu Thr Leu Ser Leu Val Ile Leu Leu
   1 5 10 15
   Leu Gln Gln Thr Ile Val Ser Val Thr Ala Ser Arg Pro Val Ser Ile
   20 25 30
   His Pro Pro Asp Val Leu Arg Gly Ser Leu Ser Ile Pro Lys Pro Pro
   35 40 45
   Ser Thr Glu Trp Phe Thr Val Asn Arg Tyr Lys Lys Leu Glu Asp Ala
   50 55 60
   Phe Arg Pro Thr Ser Pro Gly His Ser Pro Gly Val Gly His Gly Thr
   65 70 75 80
   Pro Pro Ala Ala
<210> 94
   <211> 124
   <212> PRT
   <213> Cotton RAR
<400> 94
   Met Glu Phe Arg Arg Met His Thr Phe Ala Val Phe Leu Leu Ile Ala
   1               5                   10                  15
   Cys Tyr Leu Val Leu Ser Val Glu Gly Arg Phe Leu Lys Ser Leu Ser
               20                  25                  30
   Lys Asn Asn Ser Lys Gln Val Leu Pro Pro Pro Thr Pro Thr Lys Ala
           35                  40                  45
   Ser Asp Phe Gly Asp Ser Ile Glu Gly Tyr Lys Glu Asp Phe Arg Pro
       50                  55                  60
   Thr Thr Pro Gly Asn Ser Pro Gly Val Gly His Ser Phe Ala Asp Val
   65                  70                  75                  80
   Val Glu Asp Ile Val Glu Gln Asn Pro Ala Ser Ile Ser Val Gln Gly
                   85                  90                  95
   Asn Gly Lys Arg Ser Ile Ala Val His Ser Pro Gly Val Gly His Ser
               100                 105                 110
   Phe Ala Asp Val Val Glu Asp Ile Val Glu Gln Asn
           115                 120
<210> 95
   <211> 360
   <212> PRT
   <213> Jatropha curcas RAR2
<400> 95
   Met Ala Glu Thr Leu Val Ser Tyr Lys Trp Thr Leu Phe Leu Leu Ala
   1               5                   10                  15
   Leu Ile Ser Trp Leu Gln Ile Leu Phe Ser Gln Ala Arg Pro Ile Lys
               20                  25                  30
   Ser Thr Asp Ile His Gln Ser Ser Asn Asp Asn Phe Leu Pro Lys Ala
           35                  40                  45
   Pro Ala Gly Phe Thr Ser Pro Lys Gly Ala Asn Pro Val Thr Ser Ser
       50                  55                  60
   Ser Ala Asp Asp Phe Arg Pro Thr Thr Gly Gly His Ser Pro Gly Ala
   65                  70                  75                  80
   Gly His Pro Lys Lys Met Val Thr Ser Ser Asp Val Glu His Ser Val
                   85                  90                  95
   Thr Lys Pro Glu Ala Asp Gly Arg Thr Val Lys Leu His Gln Asn Lys
   100 105 110
   Leu Thr Gly Thr Thr Thr Ala Ser Thr Ala Asn Asp Phe Arg Pro Thr
   115 120 125
   Lys Pro Gly Tyr Ser Pro Gly Val Gly His Pro Lys Gln Ile Val Thr
   130 135 140
   Ser Ser Asn Ile Glu His Ser Ile Thr Gly Phe Lys Ala Thr Lys Pro
   145 150 155 160
   Val Leu Gly Ser Asp Thr Tyr Asn Leu His Gln Asn Lys Leu Thr Gly
   165 170 175
   Thr Thr Met Ala Ser Thr Thr Asn Asp Phe Arg Pro Thr Ser Pro Gly
   180 185 190
   Tyr Ser Pro Gly Val Gly His Pro Lys Lys Ile Asp Ala Ser Ser Asn
   195 200 205
   Val Glu His Ser Val Thr Gly Phe Lys Ala Asn Ile Ala Val Gly Gly
   210 215 220
   Thr Asp Asn Leu His Gln Asn Lys Leu Thr Gly Thr Thr Ile Ala Ser
   225 230 235 240
   Thr Thr Asn Asp Phe Arg Pro Thr Ser Pro Gly Tyr Ser Pro Gly Val
   245 250 255
   Gly His Pro Lys Lys Val Asp Ala Ser Ser Asn Val Glu His Ser Val
   260 265 270
   Thr Gly Phe Lys Ala Asn Ile Ala Val Gly Gly Thr Asp Asn Leu His
   275 280 285
   Gln Asn Lys Leu Thr Gly Thr Ala Thr Ala Ser Thr Thr Asn Asp Phe
   290 295 300
   Arg Pro Thr Ala Pro Gly Tyr Ser Pro Gly Val Gly His Pro Lys Ala
   305 310 315 320
   Val Leu Val Pro Ser Ser Thr Asn Ser Asn Val Asp Asp Tyr Arg Pro
   325 330 335
   Thr Gln Pro Gly His Ser Pro Gly Val Gly His Lys Lys Ser Ser Asp
   340 345 350
   Leu Val Pro Asn Pro Glu Thr Gly
   355 360
<210> 96
   <211> 107
   <212> PRT
   <213> Theobroma cacao RAR1
<400> 96
   Met Ala Lys Thr Asn Leu Ile Val Leu Ala Gly Ala Leu Leu Leu Val
   1               5                   10                  15
   Leu Leu Phe Ser Tyr Gly Ile Thr Phe Thr Glu Glu Arg Val Leu Lys
               20                  25                  30
   Thr Asp Lys Asp Val Lys Pro Ala Gly Asn Tyr Val Thr Asn Val Met
           35                  40                  45
   Thr Ser Ser His Lys Thr Asn Leu Asn Arg Asp Ile Leu Glu Asp Gly
       50                  55                  60
   Thr Val Asp Val Pro Thr Ser Ser Ser Gly Asn Gly Thr Ala Phe Asp
   65                  70                  75                  80
   Ala Asp Asp Phe Arg Pro Thr Thr Pro Gly His Ser Pro Gly Ala Gly
                   85                  90                  95
   His Ser Thr Gly Pro Ala Ser Asn Asp Lys Asn
               100                 105
<210> 97
   <211> 107
   <212> PRT
   <213> Theobroma cacao RAR2
<400> 97
   Met Ala Lys Thr Asn Leu Val Val Leu Ala Gly Ala Leu Leu Leu Val
   1               5                   10                  15
   Leu Leu Phe Ser Tyr Gly Ile Thr Phe Thr Glu Glu Arg Val Leu Lys
               20                  25                  30
   Thr Asp Lys Asp Val Lys Pro Ala Gly Asn Ser Val Thr Asn Val Met
           35                  40                  45
   Thr Ser Ser Arg Lys Thr Asn Leu Asn Arg Asp Asn Leu Glu Asp Gly
       50                  55                  60
   Thr Asp Asp Val Pro Thr Ala Ser Ser Gly Asn Asp Thr Ala Phe Asp
   65                  70                  75                  80
   Ala Asp Asp Phe Arg Pro Thr Pro Pro Gly His Ser Pro Gly Ala Gly
                   85                  90                  95
   His Ser Thr Gly Pro Ala Ser Ser Asp Lys Asn
               100                 105
<210> 98
   <211> 107
   <212> PRT
   <213> Theobroma cacao RAR3
<400> 98
   Met Ala Lys Thr Asn Leu Ile Val Leu Ala Gly Ala Leu Leu Leu Val
   1 5 10 15
   Leu Leu Phe Ser Tyr Gly Ile Thr Phe Thr Glu Glu Arg Val Leu Lys
   20 25 30
   Thr Asp Lys Asp Val Lys Pro Ala Gly Asn Tyr Val Thr Asn Val Met
   35 40 45
   Thr Ser Ser His Lys Thr Asn Leu Asn Arg Asp Ile Leu Glu Asp Gly
   50 55 60
   Thr Val Asp Val Pro Thr Ser Ser Ser Gly Asn Gly Thr Ala Phe Asp
   65 70 75 80
   Ala Asn Asp Phe Arg Pro Thr Thr Pro Gly His Ser Pro Gly Ala Gly
   85 90 95
   His Ser Thr Gly Pro Ala Ser Asn Asp Lys Asn
   100 105
<210> 99
   <211> 121
   <212> PRT
   <213> Theobroma cacao RAR4
<400> 99
   Ala Glu Gly Val Leu Gly Thr Ala Lys Asp Val Asn Pro Gly Gly Lys
   1 5 10 15
   Phe Val Thr Asn Val Ala Ala Gly Arg His Lys Thr Asn Leu Ile Arg
   20 25 30
   Ala Phe Leu Glu Asp Gly Thr Val Asp Val Pro Thr Ser Ser Ser Gly
   35 40 45
   Asn Gly Thr Ala Phe Gly Ala Asn Asp Phe Arg Pro Pro Thr Pro Gly
   50 55 60
   His Gly Pro Gly Ala Gly His Ser Thr Gly Pro Ala Ser Asn Asp Lys
   65 70 75 80
   Asn Trp Ile Pro Leu Pro Ala Arg Thr Ile Ile Phe Pro Leu Pro Trp
   85 90 95
   Val Ala Thr Phe Thr Gln Ser Leu Val Gly Tyr Ile Ser Tyr Asp Phe
   100 105 110
   Val Leu Ala Leu Pro Lys Ala Leu Lys
   115 120
<210> 100
   <211> 96
   <212> PRT
   <213> Malus domeStica
<400> 100
   Met Ala Asn Gly Lys Leu Ser Phe Leu Leu Leu Val Leu Ile Ser Ser
   1               5                   10                  15
   Tyr Gly Ile Ile Ser Thr Glu Glu Arg Phe Leu Lys Thr Asp His Thr
               20                  25                  30
   Asn Gly Gly Ser Thr Ser Met Ile Ser His Asp Asn Tyr Leu Asn Ser
           35                  40                  45
   Arg Arg Asn Val Phe Glu Asn Glu Leu Ser Asp Ser Val Pro Pro Val
       50                  55                  60
   Pro Gly Tyr His Ser Ala Ser Asp Tyr Arg Pro Thr Thr Pro Gly His
   65                  70                  75                  80
   Ser Pro Gly Ala Gly His Ser Val Gly Pro Gln Val Glu Pro Asn Gln
                   85                  90                  95
<210> 101
   <211> 153
   <212> PRT
   <213> Carica Papaya RAR1
<400> 101
   Met Glu Tyr Gln Thr Ile Val Arg Cys Gly Ile Leu Leu Ala Leu Leu
   1 5 10 15
   Phe Ala Ser Leu Met Ile Thr Glu Ala Arg Lys Ile Arg Glu Leu Ile
   20 25 30
   Thr Gly Asn Asn Gly Asp Phe Asp Asp Ser Phe Ala Ala His Asp Thr
   35 40 45
   Ala Gly Phe Arg Pro Thr Thr Pro Gly Ile Ser Pro Gly Val Gly His
   50 55 60
   Ser Phe Gln Asn Gly Asn Lys Asp Met Ser Gly Ser Lys Ala Ala His
   65 70 75 80
   Phe Lys Pro Pro Ser Ser Asp Tyr Gln Lys Glu Thr Ser Pro Pro Arg
   85 90 95
   Ala Pro Lys Ala Pro Gly Asn Ser Pro Gly Gly Ile Gly Asp Ser Phe
   100 105 110
   Ala Asp Val Asn Ser Gln Gly Trp Ser Asn Lys Asp Asp Phe Gln Val
   115 120 125
   Thr Val Gln Ala Thr Ser Pro Gly His Ser Gly Gly Val Gly His Gly
   130 135 140
   Asp Asn Asp Asp Glu Pro Asn Ala Arg
   145 150
<210> 102
   <211> 70
   <212> PRT
   <213> Carica papaya RAR2
<400> 102
   Met Ala Asn Val Ala Cys Ser Cys Leu Phe Leu Val Val Met Ile Leu
   1 5 10 15
   Cys Ser His Cys Leu His Gly Thr Gln Gly Arg Asn Leu Lys Asn Thr
   20 25 30
   Pro Ser Ser Ser Lys Asn Met Asn Phe Pro Lys Pro Ser Ser Val Lys
   35 40 45
   Ser Thr Glu Ala Ile Val Glu Ala Phe Arg Pro Thr Thr Pro Gly His
   50 55 60
   Ser Pro Gly Val Gly His
   65 70
<210> 103
   <211> 114
   <212> PRT
   <213> Carica papaya RAR3
<400> 103
   Met Ala Arg Thr Gly Leu Met Gly Val Cys Val Leu Phe Leu Val Leu
   1               5                   10                  15
   Leu Val Cys Gln Glu Ile Val Phe Val Asn Ala Arg His Leu Arg Asp
               20                  25                  30
   Arg Ile Leu Cys Glu Lys Cys Ser Thr Thr His His His Arg His His
           35                  40                  45
   His His His His His His His His His Leu Asp Lys Ile Arg Leu Ser
       50                  55                  60
   Val Ala Pro Ala Asn Gly Ala Gly Pro Val His Val Asn Asp Gly Ala
   65                  70                  75                  80
   Gly Ser Glu Gln Gln Arg Trp Ser Thr Lys Asp Glu Tyr Val Asp Asp
                   85                  90                  95
   Phe Arg Pro Thr Thr Pro Gly His Ser Pro Gly Val Gly His Ser Ile
               100                 105                 110
   Gly Asn
<210> 104
   <211> 151
   <212> PRT
   <213> Fragaria vesca RAR1
<400> 104
   Met Glu Ala Lys Cys Ala Val Val Phe Ala Leu Ile Ala Cys Leu Asp
   1               5                   10                  15
   Ile Ala Ser Val Glu Gly Ile Arg Pro Phe Trp Ser Glu Thr Lys Ser
   20 25 30
   Thr Glu Thr Ile Leu Ile Asp Ser Ile Glu Ala Asn Tyr Lys Arg Glu
   35 40 45
   Leu Gly Glu Gln Ser Gly Gln His Asn Asn Leu Lys Gly Glu Phe Lys
   50 55 60
   Ser Ala Val Val Lys Asn Gln Gly His Phe Ala Lys Leu Gly Ala Pro
   65 70 75 80
   Ala Tyr Asn Asp Glu Glu Asp Phe Arg Pro Thr Thr Pro Gly Asn Ser
   85 90 95
   Pro Gly Ala Gly His Lys Ser Leu Gln Val Ser Glu Pro Lys Thr Val
   100 105 110
   Val Val Ala Gly Arg Asn Tyr Phe Thr Ala Gly Thr Lys Glu Asp Tyr
   115 120 125
   Arg Pro Thr Gln Pro Gly His Ser Pro Gly Val Gly His Ala Leu Gln
   130 135 140
   Glu Asn Val Lys Pro Met Pro
   145 150
<210> 105
   <211> 101
   <212> PRT
   <213> Fragaria vesca RAR2
<400> 105
   Met Ala Asn Ala Thr Tyr Thr Cys Leu Phe Phe Leu Leu Val Ile Phe
   1 5 10 15
   Ser His Glu Leu Ile Ser Cys Thr Glu Gly Arg Asn Leu Lys Val Thr
   20 25 30
   Ser Lys Lys Leu Lys Cys Gly Lys Cys Leu Ser Pro Asp Ile Asp Ala
   35 40 45
   Lys Ser Ile Ala Gly Asp Gln Gly Ser Gly Gly Ser Ser Ser Ser Asn
   50 55 60
   Gln Ile Gln Ser Pro Pro Val Val Pro Leu Pro Ala Ser Pro Gly Arg
   65 70 75 80
   Val Glu Ala Phe Arg Pro Thr Thr Pro Gly His Ser Pro Gly Val Gly
   85 90 95
   His Ser Val His Asn
   100
<210> 106
   <211> 103
   <212> PRT
   <213> Fragaria vesca RAR3
<400> 106
   Met Ala Lys Arg Arg Gln Thr Arg Leu Ile Ala Thr Thr Thr Thr Cys
   1               5                   10                  15
   Thr Leu Leu Ile Val Leu Ile Cys Cys His Glu Ile Thr Leu Val Asp
               20                  25                  30
   Gly Arg His Leu Lys Pro Gly Asp Cys Lys Lys Cys Ser Arg Arg His
           35                  40                  45
   Arg Glu Leu Asn Thr Leu Ser Ala Ala Lys Val Gly Asp His Asn Arg
       50                  55                  60
   Ser Ala Arg Leu Val Arg Ala Ala Glu Thr Lys Thr Ser Lys Ala Glu
   65                  70                  75                  80
   His Val Thr Asp Asp Phe Arg Pro Thr Thr Pro Gly His Ser Pro Gly
                   85                  90                  95
   Val Gly His Ser Ile Asn Asn
               100
<210> 107
   <211> 87
   <212> PRT
   <213> Prunus persica RAR1
<400> 107
   Met Val Ser Phe Phe Met Val Ala Leu Leu Leu Gly Gln Asn Ser Asp
   1               5                   10                  15
   Leu Val Ala Ala Ser Arg Pro Leu His Leu His Thr His Pro Pro Ala
               20                  25                  30
   Ile His Ile Gly Ser Leu Asn Lys Pro Ile Pro Pro Ser Ile Gly Arg
           35                  40                  45
   Phe Thr Ile Asn Arg Tyr Lys Met Thr Glu Ser Ser Ser Gly Ala Asp
       50                  55                  60
   Ala Phe Arg Pro Thr Ser Pro Gly His Ser Pro Gly Val Gly His Gln
   65                  70                  75                  80
   Asp Pro Pro Gly Ala Leu Leu
                   85
<210> 108
   <211> 91
   <212> PRT
   <213> Cucumis sativus RAR1
<400> 108
   Met Ala His Arg Ser Leu His Leu Asn Ser Phe Phe Pro Leu Val Ala
   1               5                   10                  15
   Leu Leu Leu Leu Leu Leu Leu Leu His Ser Leu Phe Val Thr Ser Ser
               20                  25                  30
   Arg Pro Leu His Gly Ile His Pro His Asn Pro His Ala Ile Thr Pro
           35                  40                  45
   Pro Ala Pro Val Ser Leu Glu Thr Ser Phe Ser Ile Asn Arg Tyr Lys
       50                  55                  60
   Tyr Val Glu Thr Asp Ala Phe Arg Pro Thr Ser Pro Gly His Ser Pro
   65                  70                  75                  80
   Gly Val Gly His Asn Glu Pro Pro Gly Lys Pro
                   85                  90
<210> 109
   <211> 155
   <212> PRT
   <213> Manihot esculenta RAR1
<400> 109
   Leu His Cys Phe Cys Cys Arg Cys Phe Arg Pro Thr Asp Pro Gly Asn
   1 5 10 15
   Ser Pro Gly Val Gly His His Leu Ser Gln Glu Glu Ser Asp Glu Glu
   20 25 30
   Thr Asp Pro Lys Pro Pro Arg Lys Asp Tyr Gly Pro Lys Pro Gly His
   35 40 45
   Ser Gln Pro Val Gly Arg Asp Ile Ile Phe Ser Asn Pro Ser Asn Thr
   50 55 60
   Lys Gly Ser Gln Pro Ala Ser Ser Ser His Asn Pro Val Asn Ala Val
   65 70 75 80
   Pro Leu Thr Pro Thr Ala Phe Asp Ala Ser Ala Ala Ser Ser Met Glu
   85 90 95
   Gly Phe Arg Pro Thr Thr Pro Gly Tyr Ser Pro Gly Val Gly His Pro
   100 105 110
   Asn Ala Glu Ile Ser Ser Ser Asn Val Glu Thr Ser Val Thr Arg Phe
   115 120 125
   Glu Asp Asp His Arg Pro Thr Gln Pro Gly His Ser Pro Gly Val Gly
   130 135 140
   His Ala Tyr Leu Glu Asn Asn Ala Glu Pro Asn
   145 150 155
<210> 110
   <211> 80
   <212> PRT
   <213> Citrus sinensis RAR1
<400> 110
   Met Leu Thr Leu Leu Val Val Leu Leu Leu Ser Lys Ser Phe Asp Leu
   1 5 10 15
   Ile Ser Ala Ser Arg Pro Pro His Ile His Pro Pro Thr Ile Pro Arg
   20 25 30
   Gly Ser Leu Leu Asn Lys Val Lys Pro Pro Ser Phe His Ala Tyr Thr
   35 40 45
   Ala Asn Arg Tyr Lys Leu Thr Glu Ser Glu Ala Phe Arg Pro Thr Ser
   50 55 60
   Pro Gly His Ser Pro Gly Val Gly His Lys Gly Pro Pro Gly Ser Asp
   65 70 75 80
<210> 111
   <211> 80
   <212> PRT
   <213> Citrus clementina RAR1
<400> 111
   Met Leu Thr Leu Leu Val Val Leu Leu Leu Ser Lys Ser Phe Asp Leu
   1               5                   10                  15
   Ile Ser Ala Ser Arg Pro Pro His Ile His Pro Pro Thr Ile Pro Arg
               20                  25                  30
   Gly Ser Leu Leu Asn Lys Val Lys Pro Pro Ser Phe His Ala Tyr Thr
           35                  40                  45
   Ala Asn Arg Tyr Lys Leu Thr Glu Ser Glu Ala Phe Arg Pro Thr Ser
       50                  55                  60
   Pro Gly His Ser Pro Gly Val Gly His Lys Gly Pro Pro Gly Ser Asp
   65                  70                  75                  80
<210> 112
   <211> 81
   <212> PRT
   <213> Catharanthus roseus RAR1
<400> 112
   Met Ala Asn Ile Ser Cys Lys Cys Leu Phe Met Ile Phe Leu Leu Ile
   1               5                   10                  15
   Leu Val Ser Ile Glu Gln Val Pro Ile Ser Val Glu Gly Arg Asn Leu
               20                  25                  30
   Arg Gly Glu Lys Val Lys Val Arg Ile Leu Gly Gln Glu Thr Arg Asn
           35                  40                  45
   Arg Ala Glu Lys Ser Arg Arg Val Leu Gln Gly Glu Val Asp Ser Phe
       50                  55                  60
   Arg Pro Thr Asn Pro Gly Arg Ser Pro Gly Ile Gly His Ser Thr His
   65                  70                  75                  80
   Asp
<210> 113
   <211> 81
   <212> PRT
   <213> Solanum tuberosum RAR1
<400> 113
   Met Ala Ser Ser Tyr Lys Lys Ser Ile Tyr Met Val Leu Phe Tyr Val
   1 5 10 15
   Phe Val Phe Leu Leu Leu Gln Gln Cys Glu Leu Ile Val Ala Ser Arg
   20 25 30
   Val Val Val Met Lys Phe His Gln Pro Lys Pro Pro Ser Thr Asn Ile
   35 40 45
   Phe Ser Phe Asn Arg Tyr Lys Lys Ser Glu Val Val Lys Asp Tyr Ser
   50 55 60
   Gly Pro Gly His Ser Pro Gly Met Gly His Asp Asn Pro Pro Gly Ala
   65 70 75 80
   Ser
<210> 114
   <211> 94
   <212> PRT
   <213> Solanum tuberosum RAR2
<400> 114
   Met Ala Ile Leu Ser Tyr His Lys Val Ile Cys Met Phe Ile Leu Tyr
   1 5 10 15
   Ile Phe Ile Ile Ser Ile Ala Leu Gln Gln Phe Val Leu Val Asp Ala
   20 25 30
   Ser Arg Ser Phe Ser Arg Tyr Pro Pro Pro Pro Pro Pro Val Glu Ile
   35 40 45
   Thr His Gly Glu Val Lys Ser Leu Ser Ser Asp Asn Phe Ser Phe Asn
   50 55 60
   Gly Ser Lys Ser Lys Tyr Glu Lys Lys Asp Ile Pro Tyr Val Thr Pro
   65 70 75 80
   Gly His Ser Pro Gly Met Gly His Asp Thr Pro Pro Ser Ser
   85 90
<210> 115
   <211> 99
   <212> PRT
   <213> Brassica napus RAR1
<400> 115
   Met Gly Gln Lys Lys Thr Leu Phe Val Cys Val Phe Phe Val Met Val
   1 5 10 15
   Leu Phe Asn Gly Phe Asn Cys Val His Gly Arg Thr Leu Arg Asn Met
   20 25 30
   Lys Val Asp Asp Lys Met Asn Val Gly His Asp Asp Ser Lys Thr Met
   35 40 45
   Lys Ala Met Asn Asn Asp Leu Ile Val Asp Glu Lys Ala Val Gln Leu
   50 55 60
   Ser Gln Pro Pro Pro Ser Pro Pro Pro Glu Ser Lys Asp Ala Glu Asp
   65 70 75 80
   Phe Arg Pro Thr Thr Pro Gly His Ser Pro Gly Ile Gly His Ser Leu
   85 90 95
   Ser His Asn
<210> 116
   <211> 56
   <212> PRT
   <213> Brassica napus RAR2
<400> 116
   Asn Val Asp Gly His Lys Glu Gly Ile Glu Val Phe Gln Ala Lys Ile
   1 5 10 15
   Leu Lys Asn Ile Tyr Ala Tyr Ala Pro Thr Asp Pro Gly Asn Ser Pro
   20 25 30
   Gly Ile Gly His His Lys Met Asp Val His Val Ser Asn Asp Phe Lys
   35 40 45
   Val Val Arg Lys Leu Lys Lys Asn
   50 55
<210> 117
   <211> 188
   <212> PRT
   <213> Brassica napus RAR3
<400> 117
   Met Met Thr Ile Met Ala Ile Ser Ile Val Phe Val Gln Val Pro Ser
   1 5 10 15
   Thr Thr Glu Ala Arg Pro Leu Glu Ile Thr Glu Asn Lys Asn His Phe
   20 25 30
   Lys Val Thr Ser Leu Asn Asn Phe Val Ser Thr Ile Pro Val Gly His
   35 40 45
   Asn Val Asp Gly His Lys Glu Gly Ile Glu Leu Phe Gln Glu Lys Ile
       50                  55                  60
   Leu Lys Asn Ile Tyr Ala Tyr Ala Pro Thr Asp Pro Gly Asn Ser Pro
   65                  70                  75                  80
   Gly Ile Gly His His Lys Met Asp Val His Ala Pro Glu Leu Ser Asn
                   85                  90                  95
   Asp Phe Lys Val Val Arg Pro Leu Glu Ile Thr Glu Asn Lys Asn His
               100                 105                 110
   Phe Lys Val Met Ser Leu Asn Asn Phe Val Ser Thr Val Pro Glu Gly
           115                 120                 125
   His Asn Val Asp Gly His Lys Glu Gly Ile Glu Val Phe Gln Ala Lys
       130                 135                 140
   Ile Leu Lys Asn Ile Tyr Ala Tyr Ala Pro Thr Asp Pro Gly Asn Ser
   145                 150                 155                 160
   Pro Gly Ile Gly His His Lys Met Asp Val His Ala Pro Ala Arg Ser
                   165                 170                 175
   Asn Asp Phe Lys Val Val Arg Lys Leu Lys Lys Asn
               180                 185
<210> 118
   <211> 154
   <212> PRT
   <213> Eucalyptus grandis RAR1
<400> 118
   Met Leu Ser Arg His Leu Leu Ser Arg Val Leu Ser Leu Ser Leu Ser
   1               5                   10                  15
   Leu Ser Leu Ser Pro Pro Pro Leu Pro Pro Thr Val Pro Ala Met Ala
               20                  25                  30
   Pro Asn Lys Val Leu Tyr Ala Phe Ala Phe Leu Leu Leu Ala Leu Ser
           35                  40                  45
   Leu Glu Leu Gln Ser Thr Gln Ala Arg Gln Leu Lys Leu Thr Met Gln
       50                  55                  60
   Lys Gln Lys Ser Phe Pro Asn Lys Leu Pro Asn Val His Lys Leu Leu
   65                  70                  75                  80
   Glu Lys Glu Leu Arg Lys Thr Ile Ala Glu Gln Ser Arg Asn Leu His
                   85                  90                  95
   Gly Glu Ile Leu Asn Lys Ala Thr Asn Ala Ala Val Ser Thr Thr Pro
               100                 105                 110
   Ala Pro Pro Pro Ser Ser Thr Ile Val Ala Ala Thr Thr Pro Pro Pro
           115                 120                 125
   Ser Pro Gly Arg Ser Leu Asp Asp Phe Arg Pro Thr Gln Pro Gly His
       130                 135                 140
   Ser Pro Gly Val Gly His Ser Leu Gln Asn
   145                 150
<210> 119
   <211> 83
   <212> PRT
   <213> Mimulus guttatus RAR1
<400> 119
   Met Ala Ala Lys Val Phe Ala Cys Leu Val Phe Ile Phe Ala Ile Leu
   1               5                   10                  15
   Ser Asn Gln Val Phe His Met Glu Gly Arg Asn Leu Val Val Arg Glu
               20                  25                  30
   Asn Ala Ser Asn Ala Glu Ala Arg Gly Glu Asn Ile Lys Ser Pro Asn
           35                  40                  45
   Lys Glu Ile Gly Ser His Arg Phe Arg Phe Asp Glu Gly Tyr Met Asp
       50                  55                  60
   Ser Tyr Arg Pro Thr Thr Pro Gly His Ser Pro Gly Ile Gly His Ser
   65                  70                  75                  80
   Lys His Asp
<210> 120
   <211> 92
   <212> PRT
   <213> Aquilegia coerulea RAR1
<400> 120
   Met Ala Lys Asn Lys Leu Ile Cys Thr Cys Thr Leu Leu Leu Val Leu
   1               5                   10                  15
   Val Leu Ser His Glu Met Ile His Thr Glu Gly Arg His Leu Lys Ile
               20                  25                  30
   Lys Lys Arg Thr Ala Cys Val Lys Cys Ser Ser Ser Asn Thr Val Arg
           35                  40                  45
   Gly Lys Lys Glu Ser Asp Gly Gln Lys Thr Ser Asp Val His His Lys
       50                  55                  60
   Ile Thr Pro Met Ala Gly Phe Val Glu Ala Phe Arg Pro Thr Thr Pro
   65                  70                  75                  80
   Gly His Ser Pro Gly Ile Gly His Ser Ile Gln His
                   85                  90
<210> 121
   <211> 190
   <212> PRT
   <213> Oryza sativa RAR1
<400> 121
   Met Lys Gly Lys Arg Thr Phe Leu Ser Ser Leu Asn Lys Glu His Ile
   1               5                   10                  15
   Lys Lys Phe Tyr Val Leu Glu Arg Val Val Ala Gln Phe Tyr Leu Phe
               20                  25                  30
   Ser Ser Gln Gly Arg Pro Leu Pro Asp Asp Asp Gly Ile Thr Ser Glu
           35                  40                  45
   Met Gln Ile Arg Arg Tyr Leu Leu Ser His Gly Asn Gly Val Val Glu
       50                  55                  60
   Gly Ala Val Ser Pro Ser Ser Glu Ile Gly Gly Pro Met Val Gly Ala
   65                  70                  75                  80
   Ser Gly Gly Val Arg Pro Thr Asn Pro Gly His Ser Pro Gly Ile Gly
                   85                  90                  95
   His His Val Ala Ile Asn Gly Asp Val Asp Asp Asp Asp Val Arg Pro
               100                 105                 110
   Thr Asn Pro Gly His Ser Pro Gly Ile Gly His His Ala Ile Val Asn
           115                 120                 125
   Gly Ala Asp Asp Ala Asp Asp Val Arg Pro Thr Asn Pro Gly His Ser
       130                 135                 140
   Pro Gly Ile Gly His Ala Val Val Asn Ser Ala Asp Asp Asp Ala Asp
   145                 150                 155                 160
   Asp Val Arg Pro Thr Asn Pro Gly His Ser Pro Gly Ile Gly His Ala
                   165                 170                 175
   Phe Val Asn Lys Ile Asp Gly Pro Ala Gly Lys Lys Lys Leu
               180                 185                 190
<210> 122
   <211> 176
   <212> PRT
   <213> Oryza sativa RAR2
<400> 122
   Met Ala Asn Ile Cys Thr Met Leu Ala Ile Leu Val Phe Ser Leu Gln
   1 5 10 15
   Leu Phe Ser Ser Gln Gly Arg Pro Leu Pro Asp Asp Asp Gly Ile Thr
   20 25 30
   Ser Glu Met Gln Ile Arg Arg Tyr Leu Leu Ser His Gly Asn Gly Val
   35 40 45
   Val Glu Gly Ala Val Ser Pro Ser Ser Glu Ile Gly Gly Pro Met Val
   50 55 60
   Gly Ala Ser Gly Gly Val Arg Pro Thr Asn Pro Gly His Ser Pro Gly
   65 70 75 80
   Ile Gly His His Val Val Ile Asn Gly Asp Val Asp Asp Asp Asp Val
   85 90 95
   Arg Pro Thr Asn Pro Gly His Ser Pro Gly Ile Gly His His Ala Ile
   100 105 110
   Val Asn Gly Ala Asp Asp Ala Asp Asp Val Arg Pro Thr Asn Pro Gly
   115 120 125
   His Ser Pro Gly Ile Gly His Ala Val Val Asn Gly Ala Asp Asp Asn
   130 135 140
   Ala Asp Asp Val Arg Pro Thr Asn Pro Gly His Ser Pro Gly Ile Gly
   145 150 155 160
   His Ala Phe Val Asn Lys Ile Asp Gly Pro Ala Gly Lys Lys Lys Leu
   165 170 175
<210> 123
   <211> 102
   <212> PRT
   <213> Oryza sativa RAR3
<400> 123
   Met Ala Leu Asn Lys Ser Ser Asn Ser Ile Ser Lys Ala Phe Phe Leu
   1               5                   10                  15
   Val Leu Ile Ile Leu Ala Ser Gln Val Met Leu Ser His Gly Ile Pro
               20                  25                  30
   Leu Glu Met His Arg Arg Tyr Leu Leu Ser His Ala Ala Asp Ala Thr
           35                  40                  45
   Lys Gly Val Met Glu Gly Thr Ile Thr Pro Thr Glu Gly Glu Gly Phe
       50                  55                  60
   Ala Gly Ala Asn Asp Asp Val Arg Pro Thr Asn Pro Gly His Ser Pro
   65                  70                  75                  80
   Gly Ile Gly His Ala Phe Thr Asn Asn Lys Ile Gly Arg Lys Leu Leu
                   85                  90                  95
   Leu Ala Ala Asp Asp Val
               100
<210> 124
   <211> 137
   <212> PRT
   <213> Oryza sativa RAR4
<400> 124
   Met Ser Ser Ser Lys Leu Asn Leu Ile Phe Val Leu Gly Ile Ile Phe
   1               5                   10                  15
   Phe Leu Ser Ser Asp Met Ile Ile Val Cys Ser Gln Gly Arg Pro Leu
               20                  25                  30
   Ile Ala Glu Ala Ala Ala Ala Ala Ala Ala Gln Gln Gln Arg His Leu
   35 40 45
   Leu Ser Ser Ser Ser Ser Ala Pro Arg Ser Gly Gly Asp Val Glu Glu
   50 55 60
   Ala Ala Ala Gly Gly Gly Lys Gly Thr Thr Thr Ala Met Thr Gln Gly
   65 70 75 80
   Thr Leu Ser Pro Asp Ala Ala Glu Ser Gly Gly Gly Gly Gly Gly Gly
   85 90 95
   Val Gly Ile Val Glu Asp Ala Arg Pro Thr Ala Pro Gly His Ser Pro
   100 105 110
   Gly Ala Gly His Ala Phe Thr Asn Lys Asn Gly Val Gly Arg Arg Leu
   115 120 125
   Leu Val Val Thr Ile Ser Thr Leu Ile
   130 135
<210> 125
   <211> 102
   <212> PRT
   <213> Oryza sativa RAR5
<400> 125
   Met Ala Leu Asn Lys Ser Ser Asn Ser Ile Ser Ile Ala Phe Phe Leu
   1 5 10 15
   Val Leu Ile Ile Leu Ala Ser Gln Val Met Leu Ser His Gly Ile Pro
   20 25 30
   Leu Glu Met His Arg Arg Tyr Leu Leu Ser His Ala Ala Asp Ala Thr
   35 40 45
   Lys Gly Val Met Glu Gly Thr Ile Thr Pro Thr Glu Gly Glu Gly Phe
   50 55 60
   Ala Gly Ala Asn Asp Asp Val Arg Pro Thr Asn Pro Gly His Ser Pro
   65 70 75 80
   Gly Ile Gly His Ala Phe Thr Asn Asn Lys Ile Gly Arg Lys Leu Leu
   85 90 95
   Leu Ala Ala Asp Asp Val
   100
<210> 126
   <211> 182
   <212> PRT
   <213> Oryza barthii RAR1
<400> 126
   Met Ala Leu Asn Lys Asn Val Ser Asn Ile Cys Thr Met Leu Ala Ile
   1               5                   10                  15
   Leu Val Phe Ser Leu Gln Leu Phe Ser Ser Gln Gly Arg Pro Leu Pro
               20                  25                  30
   Asp Asp Asp Gly Ile Thr Ser Glu Met Gln Ile Arg Arg Tyr Leu Leu
           35                  40                  45
   Ser His Gly Asn Arg Val Val Glu Gly Ala Val Ser Pro Ser Ser Glu
       50                  55                  60
   Ile Gly Gly Pro Met Val Gly Ala Ser Gly Gly Val Arg Pro Thr Asn
   65                  70                  75                  80
   Pro Gly His Ser Pro Gly Ile Gly His His Val Val Ile Asn Gly Asp
                   85                  90                  95
   Ile Asp Asp Asp Asp Val Arg Pro Thr Asn Pro Gly His Ser Pro Gly
               100                 105                 110
   Ile Gly His His Ala Ile Val Asn Gly Ala Asp Asp Ala Asp Asp Val
           115                 120                 125
   Arg Pro Thr Asn Pro Gly His Ser Pro Gly Ile Gly His Ala Val Val
       130                 135                 140
   Asn Gly Ala Asp Asp Asp Ala Asp Asp Val Arg Pro Thr Asn Pro Gly
   145                 150                 155                 160
   His Ser Pro Gly Ile Gly His Ala Phe Val Asn Lys Ile Asp Gly Pro
                   165                 170                 175
   Ala Gly Lys Lys Lys Leu
               180
<210> 127
   <211> 113
   <212> PRT
   <213> Sorghum bicolor RAR1
<400> 127
   Met Ala Leu Asn Lys Lys Asn Thr Asn Asn Thr Cys Thr Ser Val Leu
   1 5 10 15
   Leu Leu Leu Ser Leu Val Ile Phe Ser Gln Phe Leu Ala Ser His Gly
   20 25 30
   Arg Pro Leu Pro Thr Gly Ser Tyr Ile Thr Thr Ala Ala Ala Val His
   35 40 45
   Gly Arg Asn Leu Leu Ser His Gly Ser Gly Ser Val Pro Lys Gly Met
   50 55 60
   Leu Glu Gly Thr Val Ser Pro Ser Ser Glu Ile His Gly Asp Asn Gly
   65 70 75 80
   Ser Met Val Gly Ala Asp Asp Val Arg Pro Ser Asn Pro Gly His Ser
   85 90 95
   Pro Gly Ile Gly His Ala Phe Ile Asn Glu Lys Gly Thr Gly Arg Lys
   100 105 110
   Leu
<210> 128
   <211> 96
   <212> PRT
   <213> Sorghum bicolor RAR2
<400> 128
   Met Ala Ser Ser Lys Val Val Cys Ala Cys Ile Leu Ile Ile Leu Val
   1 5 10 15
   Ile Ser Ser Gln Ala Asp Ala Arg Arg Leu Val Thr Ala Thr Cys Asn
   20 25 30
   Gly Lys Glu Gly Ala Cys Lys Gly Gly Val Val Val Val Glu Gly Tyr
   35 40 45
   Gly Gly Phe Ser Ala Lys Gln Lys Met Ala Thr Ala Thr Ser Ser Glu
   50 55 60
   Gln Val Gly Glu Gly Met Pro Ala Thr Thr Thr Asp Ser Arg Pro Thr
   65 70 75 80
   Ala Pro Gly Asn Ser Pro Gly Ile Gly Asn Arg Gly Lys Thr Asn Asn
   85 90 95
<210> 129
   <211> 93
   <212> PRT
   <213> Triticum aestivum RAR1
<400> 129
   Met Ala Gly Ser Lys His Ala Ser Ser Cys Thr Cys Ile Leu Ile Ile
   1 5 10 15
   Leu Val Val Ser Ser His Leu Ala Pro Cys Glu Ala Arg Arg Leu Met
   20 25 30
   Val Ala Ser Ala Lys Ile Thr Gly Asp Glu Ala Cys Lys Ser Ser Gly
   35 40 45
   Cys Arg Ala Val Gln Gly Thr Ala Ser Gly Ala Ala Ala Thr Ser Lys
   50 55 60
   Met Ala Thr Thr Asp Gly Arg Gly Thr Gly Pro Gly His Ser Pro Gly
   65 70 75 80
   Ile Gly Asn Lys Leu His Ala Ala Gly Asn Asp Arg Arg
   85 90
<210> 130
   <211> 99
   <212> PRT
   <213> Triticum aestivum RAR2
<400> 130
   Met Ala Gly Ser Lys His Val Ser Ser Cys Thr Cys Ile Leu Ile Met
   1 5 10 15
   Leu Val Val Ser Ser His Leu Ala Ser Cys Pro Cys Glu Ala Arg Arg
   20 25 30
   Leu Met Ala Ala Ser Ala Lys Ile Asn Gly Asp Glu Ala Cys Met Ser
   35 40 45
   Ala Gly Cys Arg Ala Val Gln Gly Thr Ala Ser Gly Thr Ala Glu Ala
   50 55 60
   Thr Trp Lys Met Ala Thr Thr Asp Ser Arg Gly Thr Ala Pro Gly His
   65 70 75 80
   Ser Pro Gly Ile Gly Asn Lys Leu His Ala Ala Gly Thr Val Thr Val
   85 90 95
   Lys Arg Asn
<210> 131
   <211> 99
   <212> PRT
   <213> Triticum aestivum RAR3
<400> 131
   Met Ala Arg Ser Lys Val Leu Cys Thr Cys Ile Leu Ile Ile Ile Leu
   1 5 10 15
   Ser Ser Ile Gln Ala Glu Ala Arg Arg Leu Thr Thr Ala Thr Ala Val
   20 25 30
   Thr Val Ala Ser Lys Gly Lys Glu Pro Trp Cys Ala Leu Glu Ser Asn
   35 40 45
   Ser Arg Ser Leu Arg Ala Thr Ser Ser Glu Thr Ser Ile Ala Gly Ala
   50 55 60
   Gln Gly Leu Asn Gly Gly Ala Met Ser Thr Ala Thr Thr Val Glu Ser
   65 70 75 80
   Arg Gly Thr Ala Pro Gly Asn Ser Pro Gly Ile Gly Asn Lys Gly Lys
   85 90 95
   Ile Asn Asn
<210> 132
   <211> 88
   <212> PRT
   <213> Hordeum vulgare RAR1
<400> 132
   Phe Arg Pro Gly Ala Pro Ala Thr Gly Gly Arg Arg Arg Arg Arg Arg
   1 5 10 15
   Trp Ser Gly Gly Ser Ser Arg Arg Thr Ser Thr Arg Trp Ala Ala Ala
   20 25 30
   Trp Cys Cys Ala Arg Arg Thr Ser Arg Gly Arg Arg Cys Ser Trp Arg
   35 40 45
   Pro Thr Thr Pro Gly Thr Ser Pro Gly Ile Thr Ser Trp Trp Thr Ala
   50 55 60
   Gly Ser Pro Trp Arg Ser Arg Ser Thr Cys Arg Arg Ala Glu Asp Gly
   65 70 75 80
   Gly Glu Gln Pro Glu Glu Asn Glu
   85
<210> 133
   <211> 71
   <212> PRT
   <213> Hordeum vulgare RAR1 #2
<400> 133
   Arg His Glu Ala Ser Arg Arg Thr Ser Thr Arg Trp Ala Ala Ala Trp
   1               5                   10                  15
   Cys Cys Ala Arg Arg Thr Ser Arg Gly Arg Arg Cys Ser Trp Arg Pro
               20                  25                  30
   Thr Thr Pro Gly Thr Ser Pro Gly Ile Thr Ser Trp Trp Thr Ala Gly
           35                  40                  45
   Ser Pro Trp Arg Ser Arg Ser Thr Cys Arg Arg Ala Glu Asp Gly Gly
       50                  55                  60
   Glu Gln Pro Glu Glu Asn Glu
   65                  70
<210> 134
   <211> 75
   <212> PRT
   <213> Hordeum vulgare RAR2
<400> 134
   Met Ala Ile Ser Ser Lys Ile Ala Val Val Phe Met Leu Leu Leu Ser
   1               5                   10                  15
   Thr Thr Phe Met Gln Leu Pro Val Pro Ala Asp Ala Arg Arg Leu Glu
               20                  25                  30
   Val Lys Ala Pro Ile Leu Asn Val His Arg Pro Cys Thr Gly Arg Ser
           35                  40                  45
   Thr Leu Glu Thr Pro Pro Glu Gln Val Glu Ser Thr Thr Pro Gly His
       50                  55                  60
   Ser Pro Ser Ile Gly His Asn Ser Pro Pro Asn
   65                  70                  75
<210> 135
   <211> 96
   <212> PRT
   <213> Saccharum officinarum RAR1
<400> 135
   Met Ala Ser Ser Lys Val Val Cys Ala Cys Ile Leu Ile Ile Leu Val
   1               5                   10                  15
   Ile Ser Ser Arg Ala Asp Ala Arg Arg Leu Val Ala Ala Thr Cys Asn
               20                  25                  30
   Gly Lys Glu Gly Ala Cys Lys Gly Gly Ile Ile Val Val Glu Gly Tyr
           35                  40                  45
   Gly Gly Phe Ser Ala Lys Gln Lys Met Ala Thr Ala Arg Ser Thr Glu
       50                  55                  60
   Glu Val Ser Glu Gly Met Pro Ala Thr Thr Met Asp Ser Arg Pro Thr
   65                  70                  75                  80
   Tyr Pro Gly Asn Ser Pro Gly Ile Gly Asn Lys Gly Gln Ile Asn Asn
                   85                  90                  95
<210> 136
   <211> 34
   <212> PRT
   <213> Saccharum officinarum RAR2
<400> 136
   Thr Met Asp Ser Arg Pro Thr Tyr Pro Gly Asn Ser Pro Gly Ile Gly
   1               5                   10                  15
   Asn Lys Gly Glu Asn Gln Gln Leu Ala Gly Arg Val Leu Ile Cys Val
               20                  25                  30
   Leu Ile
<210> 137
   <211> 60
   <212> PRT
   <213> Zea mays RAR1
<400> 137
   Ser Ser His Ala Asp Ala Arg Arg Leu Val Ala Thr Thr Cys Asn Gly
   1               5                   10                  15
   Thr Glu Gly Gly Ala Cys Lys Gly Gly Ile Phe Val Gln Gly Tyr Ala
               20                  25                  30
   Gly Leu Ser Ala Arg Gln Lys Met Ala Ala Thr Ala Thr Ser Thr Glu
           35                  40                  45
   Gln Val Val Gly Gly Gly Gly Glu Gly Met Pro Ala
       50                  55                  60
<210> 138
   <211> 112
   <212> PRT
   <213> Zea mays RAR2
<400> 138
   Met Gln Gln Pro Tyr Glu His Ser Gly Ser Ser Gly Ser Ser Pro Ala
   1               5                   10                  15
   Cys Arg Ser Arg Gln Ile Ala Pro Ala His Cys Thr Ala Pro Thr Thr
               20                  25                  30
   Val Ala Ser Thr Pro Arg Ser Arg Tyr Leu Leu Val Val Gln Ser Ser
           35                  40                  45
   Ser Ala Thr Thr Ala Thr Ala Tyr Ser Thr Thr Lys Ala Gly Met Ile
       50                  55                  60
   Glu Gly Thr Val Thr Pro Ser Glu Gly Gly Ala Pro Gly Ala Thr Glu
   65                  70                  75                  80
   Asp Val Arg Pro Thr Asn Pro Ser His Ser Pro Gly Ile Gly His Ala
                   85                  90                  95
   Phe Thr His Asn Lys Ile Gly Arg Lys Leu Leu Ala Ala Ile Ser Gln
               100                 105                 110
<210> 139
   <211> 90
   <212> PRT
   <213> Zea mays RAR3
<400> 139
   Ala Ala Gly Leu Val Val Lys Pro Ser Trp Ala Cys Ile Val Ile Ile
   1               5                   10                  15
   Val Leu Ile Val Thr Leu Ser Ser Gly Ala Ala Ser Gly Glu Ala Arg
               20                  25                  30
   Arg Leu Leu Met Ala Glu Lys His Ala Ala Glu Gly Ala Cys Ala Gly
           35                  40                  45
   Gly Cys Ser Pro Pro Val Gln Gly Leu Thr Ala Thr Thr Thr Ser Lys
       50                  55                  60
   Met Ala Thr Thr Asp Gly Arg Pro Thr Ala Pro Gly His Ser Pro Gly
   65                  70                  75                  80
   Ile Gly Asn Lys Ile Ala Gly Asn Thr Arg
   85 90
<210> 140
   <211> 92
   <212> PRT
   <213> Zea mays RAR4
<400> 140
   Met His Ser His Arg Ser Arg His Leu Lys Pro Arg Gln Arg Glu Ala
   1 5 10 15
   Ala Gly Gly Val Phe Phe Asn Gly Gly Thr Glu Gly Gly Ala Cys Lys
   20 25 30
   Gly Gly Ile Phe Val Gln Gly Tyr Ala Gly Leu Ser Ala Arg Gln Lys
   35 40 45
   Met Ala Ala Thr Ala Thr Ser Thr Glu Gln Val Val Val Val Gly Glu
   50 55 60
   Gly Met Pro Ala Thr Thr Thr Asp Ser Arg Pro Thr Ala Pro Gly Asn
   65 70 75 80
   Ser Pro Gly Ile Gly Asn Lys Gly Lys Ile Asn Asn
   85 90
<210> 141
   <211> 113
   <212> PRT
   <213> Brachypodium distachyon RAR1
<400> 141
   Met Ala Pro Ser Ile Ser Lys Asn Thr Asn Thr Cys Thr Cys Ala Leu
   1 5 10 15
   Leu Leu Ile Phe Val Val Leu Phe Ser Gln Leu Val Glu Ser Gln Ser
   20 25 30
   Arg Ser Leu Pro His Gly Ser Leu Ile Ser Thr Met His Arg Arg Tyr
   35 40 45
   Leu Leu Ser His Val Asn Gly Ala Ser Pro Asn Gly Leu Ala Glu Gly
   50 55 60
   Ala Val Ser Pro Pro Ser Glu Ile His Gly Gly Asp Gly Pro Leu Val
   65 70 75 80
   Asp Val Arg Asp Gly Val Arg Pro Ser Asn Pro Gly His Ser Pro Gly
   85 90 95
   Ile Gly His Ser Phe Val Asn Arg Asn Gly Pro Ala Gly Asn Asn Lys
   100 105 110
   Leu
<210> 142
   <211> 109
   <212> PRT
   <213> Setaria italica RAR1
<400> 142
   Met Ala Leu Asn Lys Asn Pro Ser Thr Cys Thr Ser Ala Leu Leu Leu
   1 5 10 15
   Leu Ala Leu Leu Val Thr Phe Ser Gln Leu Leu Ala Ser Gln Gly Arg
   20 25 30
   Pro Phe Pro Thr Val Ser Tyr Ile Thr Thr Met His Gly Arg Thr Leu
   35 40 45
   Leu Ser His Gly Ser Asp Ser Val Pro Lys Gly Met Val Glu Gly Thr
   50 55 60
   Val Ser Pro Ser Ser Glu Ile His Gly Asp Lys Gly Ser Met Val Asp
   65 70 75 80
   Ala Asp Asp Val Arg Pro Ser Thr Pro Arg His Ser Pro Gly Ile Gly
   85 90 95
   His Ala Phe Ile Asn Lys Asn Gly Leu Gly Arg Lys Leu
   100 105
<210> 143
   <211> 135
   <212> PRT
   <213> Phoenix dactilifera RAR1
<400> 143
   Met Ala Gly Lys Lys Gln Phe Tyr Ser Cys Ile Leu Val Ile Val Leu
   1               5                   10                  15
   Ile Leu Ala Asn Asp Tyr Leu Ser Ser Glu Gly Arg His Leu Lys Glu
               20                  25                  30
   Glu Lys Phe Lys Ser Arg Gly Cys Arg Glu Cys Pro Glu Arg Gly Asp
           35                  40                  45
   Ser Lys Ile Glu Arg Arg Thr Ser Ser Met Val Ser Asn Thr Ile Glu
       50                  55                  60
   Gly His Asp Asn Arg Val Leu Met Val Ala Met Asp Ala Arg Pro Thr
   65                  70                  75                  80
   Ala Gly Asp Ser Asn Ile Glu Arg Gly Thr Ser Ser Met Thr Ser Lys
                   85                  90                  95
   Thr Ile Glu Gly His Asp Ala Arg Val Leu Thr Ala Ala Ile Asp Ser
               100                 105                 110
   Arg Pro Thr Ala Pro Gly His Ser Pro Gly Val Gly His Ser Ile Asn
           115                 120                 125
   Ser Arg Gly Gly Asp Lys Asn
       130                 135
<210> 144
   <211> 92
   <212> PRT
   <213> Phoenix dactylifera RAR2
<400> 144
   Met Val Gly Ile Lys Pro Val His Ile Ser Ala Leu Phe Val Val Leu
   1               5                   10                  15
   Ile Leu Ala Arg Lys Phe Ala Leu Thr Glu Glu Arg His Phe Ile Leu
               20                  25                  30
   Val Lys Thr Lys Ile Ser Glu Lys Cys Pro Lys Gln Gly Asp Thr Arg
           35                  40                  45
   Ile Gly Arg Met Asn Arg Gly Ile Asn His Gly Asp Ala Val Leu Ala
       50                  55                  60
   Phe Ala Asp Gly Asp Arg Pro Ser Val Pro Gly His Ser Pro Gly Val
   65                  70                  75                  80
   Gly His Ser His Glu Ser Lys Asp Gly Gly Lys Asn
                   85                  90
<210> 145
   <211> 115
   <212> PRT
   <213> Phoenix dactylifera RAR3
<400> 145
   Met Pro Asn Leu Pro Leu Ser Leu Ser Leu Ser Leu Ser Leu Met Ala
   1               5                   10                  15
   Gly Lys Lys His Phe Tyr Ala Cys Ala Leu Val Ile Val Leu Ile Leu
               20                  25                  30
   Val Asn Glu Cys Leu Ser Ser Glu Gly Arg His Leu Met Ala Gly Lys
           35                  40                  45
   Phe Lys Ala Lys Gly Cys Glu Glu Cys Leu Ala Arg Gly Gly Asn Asn
       50                  55                  60
   Ile Glu Gly Thr Thr Ser Ser Leu Val Ser His Thr Ile Glu Gly His
   65                  70                  75                  80
   Asp Asp Arg Val Leu Ile Val Thr Thr Glu Asp Ala Arg Pro Thr Thr
                   85                  90                  95
   Pro Gly His Ser Pro Gly Val Gly His Gly Ile Lys Ser Ser Gly Gly
               100                 105                 110
   Asp Lys Asn
           115
<210> 146
   <211> 99
   <212> PRT
   <213> Phoenix dactylifera RAR4
<400> 146
   Met Ala Ala Asn Lys Arg Phe Tyr Pro Cys Ala Leu Leu Ile Ile Met
   1 5 10 15
   Val Leu Ala Ser Glu Thr Phe Thr Ser Glu Gly Arg Thr Leu Met Glu
   20 25 30
   Asp Lys Ala Arg Val Cys Arg Arg Cys Leu Val Glu Asn Thr Ser Phe
   35 40 45
   Lys Gly Leu Val Glu Gly Pro Thr Val Pro Pro Ala Val Asp Gly Asp
   50 55 60
   Asn Ala Leu Met Ala Asp Thr Glu Asp Ala Arg Pro Thr Thr Pro Gly
   65 70 75 80
   His Ser Pro Gly Val Gly His Ser Phe Asn Gly Lys Asp Val Ile Asn
   85 90 95
   Lys Asp Val
<210> 147
   <211> 100
   <212> PRT
   <213> Phoenix dactylifera RAR5
<400> 147
   Met Ala Ala Asn Lys Pro Phe Tyr Thr Tyr Ala Leu Leu Ile Leu Met
   1 5 10 15
   Ile Leu Ala Phe Glu Thr Phe Thr Ser Val Gly Arg Thr Leu Val Glu
   20 25 30
   Asp Lys Thr Lys Val Cys Arg Arg Cys Leu Val Gln Asp Ala Gly Ala
   35 40 45
   Lys Gly Met Val Glu Gly Pro Ile Ser Pro Pro Ala Ile His Gly Asp
   50 55 60
   Asp Ala Leu Met Val Gly Ile Ser Asp Ala Arg Pro Thr Thr Pro Gly
   65 70 75 80
   His Ser Pro Gly Val Gly His Ser Phe Asn Tyr Lys Asn Val Val Ile
   85 90 95
   Asn Lys Asn Val
   100
<210> 148
   <211> 15
   <212> PRT
   <213> Arabidopsis thaliana RAR1 domain
<400> 148
   Asp Phe Arg Pro Thr Asn Pro Gly Asn Ser Pro Gly Val Gly His
   1 5 10 15
<210> 149
   <211> 15
   <212> PRT
   <213> At RAR2 domain
<400> 149
   Asp Phe Ala Pro Thr Asn Pro Gly Asp Ser Pro Gly Ile Arg His
   1 5 10 15
<210> 150
   <211> 15
   <212> PRT
   <213> At RAR3 domain
<400> 150
   Thr Phe Arg Pro Thr Glu Pro Gly His Ser Pro Gly Ile Gly His
   1 5 10 15
<210> 151
   <211> 15
   <212> PRT
   <213> At RAR4 domain
<400> 151
   Ala Phe Arg Pro Thr His Gln Gly Pro Ser Gln Gly Ile Gly His
   1 5 10 15
<210> 152
   <211> 15
   <212> PRT
   <213> At RAR5 domain #1
<400> 152
   Asp Phe Val Pro Thr Ser Pro Gly Asn Ser Pro Gly Val Gly His
   1 5 10 15
<210> 153
   <211> 15
   <212> PRT
   <213> At RAR5 domain #2
<400> 153
   Asp Phe Ala Pro Thr Ser Pro Gly His Ser Pro Gly Val Gly His
   1 5 10 15
<210> 154
   <211> 15
   <212> PRT
   <213> At RAR5 domain #3
<400> 154
   Asp Phe Ala Pro Thr Ser Pro Gly Asn Ser Pro Gly Ile Gly His
   1 5 10 15
<210> 155
   <211> 15
   <212> PRT
   <213> At RAR5 domain #4
   <,400> 155
<210> 156
   <211> 15
   <212> PRT
   <213> At RAR6 domain
<400> 156
   Asp Phe Arg Pro Thr Thr Pro Gly His Ser Pro Gly Ile Gly His
   1 5 10 15
<210> 157
   <211> 15
   <212> PRT
   <213> At RAR7 domain #1
<400> 157
   Asp Phe Gly Pro Thr Ser Pro Gly Asn Ser Pro Gly Ile Gly His
   1 5 10 15
<210> 158
   <211> 15
   <212> PRT
   <213> At RAR7 domain #2
<400> 158
   Asp Phe Glu Pro Thr Thr Pro Gly His Ser Pro Gly Val Gly His
   1 5 10 15
<210> 159
   <211> 15
   <212> PRT
   <213> A lyrata RAR1 domain
<400> 159
   Asp Phe Arg Pro Thr Thr Pro Gly His Ser Pro Gly Ile Gly His
   1 5 10 15
<210> 160
   <211> 15
   <212> PRT
   <213> A lyrata RAR2 domain
<400> 160
   Thr Phe Arg Pro Thr Ala Pro Gly His Ser Pro Gly Ile Gly His
   1 5 10 15
<210> 161
   <211> 15
   <212> PRT
   <213> A lyrata RAR3 domain #1
<400> 161
   Asp Phe Ala Pro Thr Ser Pro Gly Asn Ser Pro Gly Val Gly His
   1 5 10 15
<210> 162
   <211> 15
   <212> PRT
   <213> A lyrata RAR3 domain #2
<400> 162
   Asp Phe Ala Pro Thr Ser Pro Gly His Ser Pro Gly Val Gly His
   1 5 10 15
<210> 163
   <211> 15
   <212> PRT
   <213> A lyrata RAR3 domain #3
<400> 163
   Glu Phe Ala Pro Thr Ser Pro Gly Asn Ser Pro Gly Ile Gly His
   1 5 10 15
<210> 164
   <211> 15
   <212> PRT
   <213> A lyrata RAR3 domain #4
<400> 164
   Asp Phe Ala Pro Thr Thr Pro Gly Asn Ser Pro Gly Met Gly His
   1               5                   10                  15
<210> 165
   <211> 15
   <212> PRT
   <213> A lyrata RAR3 domain #5
<400> 165
   Asp Phe Lys Pro Thr Thr Pro Gly His Ser Pro Gly Val Gly His
   1               5                   10                  15
<210> 166
   <211> 15
   <212> PRT
   <213> M. truncatula RAR1 domain #1
<400> 166
   Ala Phe Gln Pro Thr Thr Pro Gly Asn Ser Pro Gly Val Gly His
   1               5                   10                  15
<210> 167
   <211> 15
   <212> PRT
   <213> M. truncatula RAR1 domain #2
<400> 167
   Glu Phe Gln Lys Thr Asn Pro Gly His Ser Pro Gly Val Gly His
   1               5                   10                  15
<210> 168
   <211> 15
   <212> PRT
   <213> M. truncatula RAR2 domain
<400> 168
   Ala Phe Arg Pro Thr Thr Pro Gly His Ser Pro Gly Ile Gly His
   1               5                   10                  15
<210> 169
   <211> 15
   <212> PRT
   <213> M. truncatula RAR3 domain
<400> 169
   Asp Phe Arg Pro Thr Thr Pro Gly Asn Ser Pro Gly Ala Gly His
   1               5                   10                  15
<210> 170
   <211> 15
   <212> PRT
   <213> M. truncatula RAR4 domain
<400> 170
   Ala Phe Arg Pro Thr Ser Pro Gly His Ser Pro Gly Val Gly His
   1               5                   10                  15
<210> 171
   <211> 15
   <212> PRT
   <213> M. truncatula RAR5 domain
<400> 171
   Ala Phe Arg Pro Thr Thr Pro Gly His Ser Pro Gly Val Gly His
   1               5                   10                  15
<210> 172
   <211> 15
   <212> PRT
   <213> M. truncatula RAR6 domain
<400> 172
   Asp Phe Arg Pro Thr Ser Pro Gly His Ser Pro Gly Val Gly His
   1               5                   10                  15
<210> 173
   <211> 15
   <212> PRT
   <213> M. truncatula RAR7 domain #1
<400> 173
   Ser Phe Arg Pro Thr Thr Pro Gly Ser Ser Pro Gly Val Gly His
   1               5                   10                  15
<210> 174
   <211> 15
   <212> PRT
   <213> M. truncatula RAR7 domain #2
<400> 174
   Gly Phe Lys Pro Thr Asn Pro Ser His Ser Pro Gly Val Gly His
   1 5 10 15
<210> 175
   <211> 15
   <212> PRT
   <213> M. truncatula RAR8 domain
<400> 175
   Ala Phe Arg Pro Thr Thr Pro Gly Asn Ser Pro Gly Val Gly His
   1 5 10 15
<210> 176
   <211> 15
   <212> PRT
   <213> M. truncatula RAR9 domain #1
<400> 176
   Ala Phe Arg Pro Thr Thr Pro Gly Ser Ser Pro Gly Val Gly His
   1 5 10 15
< 210> 177
   <211> 15
   <212> PRT
   <213> M. truncatula RAR9 domain #2
<400> 177
   Ala Phe Lys Pro Thr Tyr Pro Asn His Ser Pro Gly Val Gly His
   1 5 10 15
<210> 178
   <211> 15
   <212> PRT
   <213> M. truncatula RAR10 domain #1
<400> 178
   Ala Phe Arg Pro Thr Pro Ser Gly His Ser Leu Gly Val Gly His
   1 5 10 15
<210> 179
   <211> 15
   <212> PRT
   <213> M. truncatula RAR10 domain #2
<400> 179
   Ala Phe Arg Pro Thr Pro Pro Gly His Ser Pro Gly Gly Gly His
   1 5 10 15
<210> 180,
   <211> 15
   <212> PRT
   <213> M. truncatula RAR10 domain #3
<400> 180
   Ala Phe Arg Pro Asn Pro Pro Gly His Ser Pro Gly Gly Gly His
   1 5 10 15
<210> 181
   <211> 15
   <212> PRT
   <213> M. truncatula RAR10 domain #4
<400> 181
   Ala Phe Arg Pro Thr Pro Pro Gly His Ser Pro Gly Gly Gly His
   1 5 10 15
<210> 182
   <211> 15
   <212> PRT
   <213> M. truncatula RAR11 domain
<400> 182
   Ala Phe Arg Pro Thr Ser Pro Gly His Ser Pro Gly Val Gly His
   1 5 10 15
<210> 183
   <211> 15
   <212> PRT
   <213> S. lycopersicum RAR1 domain
<400> 183
   Ile Ser Glu Glu Gly Gly Pro Gly His Ser Pro Gly Val Gly His
   1 5 10 15
<210> 184
   <211> 15
   <212> PRT
   <213> S. lycopersicum RAR2 domain
<400> 184
   Val Lys Asp Tyr Ser Gly Pro Gly His Ser Pro Gly Met Gly His
   1 5 10 15
<210> 185
   <211> 15
   <212> PRT
   <213> S. lycopersicum RAR3 domain
<400> 185
   Val Lys Asp Tyr Ser Gly Pro Gly His Ser Pro Gly Met Gly His
   1 5 10 15
<210> 186
   <211> 15
   <212> PRT
   <213> S. lycopersicum RAR4 domain
<400> 186
   Asp Phe Gly Pro Thr Gly Pro Gly His Ser Pro Gly Ile Gly His
   1 5 10 15
<210> 187
   <211> 15
   <212> PRT
   <213> G. hirsutum RAR1 domain
<400> 187
   Ala Phe Arg Ser Thr Thr Pro Gly His Ser Pro Gly Ala Gly His
   1 5 10 15
<210> 188
   <211> 15
   <212> PRT
   <213> L. sativa RAR1 domain #1
<400> 188
   Ala Phe Arg Pro Thr Thr Pro Gly Asn Ser Pro Gly Ala Gly His
   1               5                   10                  15
<210> 189
   <211> 15
   <212> PRT
   <213> L. sativa RAR1 domain #2
<400> 189
   Gly Phe Arg Pro Thr Lys Pro Gly Asn Ser Pro Gly Ala Gly His
   1               5                   10                  15
<210> 190
   <211> 15
   <212> PRT
   <213> E. esula RAR1 domain #1
<400> 190
   Asp Phe Arg Pro Thr Ser Pro Gly Tyr Ser Pro Gly Val Gly His
   1               5                   10                  15
<210> 191
   <211> 15
   <212> PRT
   <213> E. esula RAR1 domain #2
<400> 191
   Asp Phe Arg Pro Thr Glu Pro Gly Tyr Ser Pro Gly Val Gly His
   1               5                   10                  15
<210> 192
   <211> 15
   <212> PRT
   <213> E. esula RAR1 domain #3
<400> 192
   Asp Tyr Arg Pro Thr Glu Pro Gly His Ser Pro Gly Ala Gly His
   1               5                   10                  15
<210> 193
   <211> 15
   <212> PRT
   <213> E. esula RAR1 domain #4
<400> 193
   Asp Phe Arg Pro Thr Ala Pro Gly Phe Ser Pro Gly Val Gly His
   1 5 10 15
<210> 194
   <211> 15
   <212> PRT
   <213> E. esula RAR2 domain #1
<400> 194
   Ala Phe Arg Pro Thr Ala Pro Gly His Ser Pro Gly Val Gly His
   1 5 10 15
<210> 195
   <211> 15
   <212> PRT
   <213> E. esula RAR2 domain #2
<400> 195
   Ala Phe Arg Pro Thr Ala Pro Gly His Ser Pro Gly Val Gly His
   1 5 10 15
<210> 196
   <211> 15
   <212> PRT
   <213> E. esula RAR2 domain #3
<400> 196
   Ala Phe Arg Pro Thr Ala Pro Gly His Ser Pro Gly Val Gly His
   1 5 10 15
<210> 197
   <211> 15
   <212> PRT
   <213> E. esula RAR2 domain #4
<400> 197
   Ala Phe Arg Pro Thr Ala Pro Gly His Ser Pro Gly Val Gly Tyr
   1 5 10 15
<210> 198
   <211> 15
   <212> PRT
   <213> E. esula RAR2 domain #5
<400> 198
   Ala Phe Arg Pro Thr Ala Pro Gly His Ser Pro Gly Val Gly His
   1 5 10 15
<210> 199
   <211> 15
   <212> PRT
   <213> G. max RAR1 domain #1
<400> 199
   Ala Phe Arg Pro Thr Thr Pro Gly Gly Ser Pro Gly Val Gly His
   1 5 10 15
<210> 200
   <211> 15
   <212> PRT
   <213> G. max RAR1 domain #2
<400> 200
   Asp Phe Lys Pro Thr Asp Pro Gly His Ser Pro Gly Val Gly His
   1 5 10 15
<210> 201
   <211> 15
   <212> PRT
   <213> G. max RAR2 domain
<400> 201
   Ala Phe Arg Pro Thr Thr Pro Gly His Ser Pro Gly Val Gly His
   1 5 10 15
<210> 202
   <211> 15
   <212> PRT
   <213> G. max RAR3 domain
<400> 202
   Ala Phe Arg Pro Thr Thr Pro Gly Asn Ser Pro Gly Val Gly His
   1 5 10 15
<210> 203
   <211> 15
   <212> PRT
   <213> G. max RAR4 domain
<400> 203
   Asn Phe Arg Pro Thr Ala Pro Gly His Ser Pro Gly Val Gly His
   1 5 10 15
<210> 204
   <211> 15
   <212> PRT
   <213> G. max RAR5 domain
<400> 204
   Asp Phe Lys Pro Thr Asp Pro Gly His Ser Pro Gly Val Gly His
   1 5 10 15
<210> 205
   <211> 15
   <212> PRT
   <213> G. max RAR6 domain
<400> 205
   Asp Phe Arg Pro Thr Thr Pro Gly His Ser Pro Gly Val Gly His
   1 5 10 15
<210> 206
   <211> 15
   <212> PRT
   <213> G. max RAR7 domain
<400> 206
   Asp Phe Gln Pro Thr Asp Pro Gly His Ser Pro Gly Ala Gly His
   1 5 10 15
<210> 207
   <211> 15
   <212> PRT
   <213> G. max RAR8 domain #1
<400> 207
   Ala Phe Arg Pro Thr Thr Pro Gly Asn Ser Pro Gly Val Gly His
   1 5 10 15
<210> 208
   <211> 15
   <212> PRT
   <213> G. max RAR8 domain #2
<400> 208
   Asp Phe Arg Pro Thr Asp Pro Gly His Ser Pro Gly Val Gly His
   1 5 10 15
<210> 209
   <211> 15
   <212> PRT
   <213> G. max RAR9 domain
<400> 209
   Asn Phe Arg Pro Thr Ala Pro Gly His Ser Pro Gly Val Gly His
   1 5 10 15
<210> 210
   <211> 15
   <212> PRT
   <213> G. max RAR10 domain
<400> 210
   Asp Phe Arg Pro Met Asp Pro Gly His Ser Pro Gly Ala Gly His
   1 5 10 15
<210> 211
   <211> 15
   <212> PRT
   <213> G. max RAR11 domain
<400> 211
   Ala Phe Arg Pro Thr Cys Arg Gly His Ser Pro Gly Ala Gly His
   1 5 10 15
<210> 212
   <211> 15
   <212> PRT
   <213> G. max RAR12 domain
<400> 212
   Ala Phe Arg Pro Thr Thr Pro Gly His Ser Pro Gly Val Gly His
   1 5 10 15
<210> 213
   <211> 15
   <212> PRT
   <213> G. max RAR13 domain
<400> 213
   Gly Phe Lys Pro Thr Asn Pro Gly His Ser Pro Gly Val Gly His
   1 5 10 15
<210> 214
   <211> 15
   <212> PRT
   <213> G. max RAR14 domain
<400> 214
   Ala Phe Arg Pro Thr Thr Pro Gly Asn Ser Pro Gly Val Gly His
   1 5 10 15
<210> 215
   <211> 15
   <212> PRT
   <213> G. max RAR15 domain #1
<400> 215
   Ala Phe Arg Pro Thr Thr Pro Gly Gly Ser Pro Gly Val Gly His
   1 5 10 15
<210> 216
   <211> 15
   <212> PRT
   <213> G. max RAR15 domain #2
<400> 216
   Asp Phe Lys Pro Thr Asp Pro Gly His Ser Pro Gly Val Gly His
   1 5 10 15
<210> 217
   <211> 15
   <212> PRT
   <213> G. max RAR16 domain
<400> 217
   Asp Phe Arg Pro Thr Asp Pro Gly His Ser Pro Gly Ala Gly His
   1 5 10 15
<210> 218
   <211> 15
   <212> PRT
   <213> L. japonicus RAR1 domain
<400> 218
   Asp Phe Lys Pro Thr Asp Pro Gly His Ser Pro Gly Val Gly His
   1 5 10 15
<210> 219
   <211> 15
   <212> PRT
   <213> L. japonicus RAR2 domain
<400> 219
   Lys Gln Pro Thr Thr Gly Pro Gly His Ser Pro Gly Val Gly His
   1 5 10 15
<210> 220
   <211> 15
   <212> PRT
   <213> L. japonicus RAR3 domain
<400> 220
   Ala Phe Arg Pro Thr Ser Pro Gly His Ser Pro Gly Val Gly His
   1 5 10 15
<210> 221
   <211> 15
   <212> PRT
   <213> L. japonicus RAR4 domain
<400> 221
   Ala Phe Arg Pro Thr Thr Pro Gly His Ser Pro Gly Val Gly His
   1 5 10 15
<210> 222
   <211> 15
   <212> PRT
   <213> L. japonicus RAR5 domain
<400> 222
   Ala Phe Arg Pro Thr Thr Pro Gly His Ser Pro Gly Val Gly His
   1 5 10 15
<210> 223
   <211> 15
   <212> PRT
   <213> L. japonicus RAR6 domain #1
<400> 223
   Ala Phe Glu Pro Thr Thr Pro Gly Asn Ser Pro Gly Val Gly His
   1 5 10 15
<210> 224
   <211> 15
   <212> PRT
   <213> L. japonicus RAR6 domain #2
<400> 224
   Asp Phe Lys Pro Thr Asp Pro Gly His Ser Pro Gly Val Gly His
   1 5 10 15
<210> 225
   <211> 15
   <212> PRT
   <213> P. trichocarpa RAR1 domain #1
<400> 225
   Asp Phe Arg Pro Thr Thr Pro Gly Val Ser Pro Gly Val Gly His
   1 5 10 15
<210> 226
   <211> 15
   <212> PRT
   <213> P. trichocarpa RAR1 domain #2
<400> 226
   Asp Phe Gln Pro Thr Thr Pro Gly His Ser Pro Gly Ala Gly His
   1 5 10 15
<210> 227
   <211> 15
   <212> PRT
   <213> P. trichocarpa RAR1 domain #3
<400> 227
   Ala Phe Lys Pro Thr Thr Pro Gly His Ser Pro Gly Ala Gly His
   1 5 10 15
<210> 228
   <211> 15
   <212> PRT
   <213> P. trichocarpa RAR1 domain #4
<400> 228
   Ala Phe Lys Pro Thr Thr Pro Gly His Ser Pro Gly Ile Gly His
   1 5 10 15
<210> 229
   <211> 15
   <212> PRT
   <213> P. trichocarpa RAR1 domain #5
<400> 229
   Asp Phe Arg Pro Thr Val Pro Gly His Ser Pro Gly Ile Gly His
   1 5 10 15
<210> 230
   <211> 15
   <212> PRT
   <213> P. trichocarpa RAR1 domain #6
<400> 230
   Ala Phe Arg Pro Pro Thr Pro Gly His Ser Pro Gly Val Gly His
   1 5 10 15
<210> 231
   <211> 15
   <212> PRT
   <213> P. trichocarpa RAR2 domain #1
<400> 231
   Asp Phe Lys Pro Ile Thr Ser Gly Gln Ser Pro Gly Val Gly His
   1 5 10 15
<210> 232
   <211> 15
   <212> PRT
   <213> P. trichocarpa RAR2 domain #2
<400> 232
   Asp Phe Gln Pro Thr Thr Pro Gly Asn Ser Pro Gly Val Gly His
   1 5 10 15
<210> 233
   <211> 15
   <212> PRT
   <213> P. trichocarpa RAR2 domain #3
<400> 233
   Ala Phe Lys Pro Thr Thr Pro Gly His Ser Pro Gly Val Gly His
   1 5 10 15
<210> 234
   <211> 15
   <212> PRT
   <213> P. trichocarpa RAR2 domain #4
<400> 234
   Ala Phe Lys Pro Thr Thr Pro Gly His Ser Pro Gly Val Gly His
   1 5 10 15
<210> 235
   <211> 15
   <212> PRT
   <213> P. trichocarpa RAR2 domain #5
<400> 235
   Glu His Ser Val Thr Thr Pro Gly His Ser Pro Ala Val Gly His
   1 5 10 15
<210> 236
   <211> 15
   <212> PRT
   <213> P. trichocarpa RAR2 domain #6
<400> 236
   Ala Phe Lys Pro Thr Thr Pro Gly His Ser Pro Gly Ile Gly His
   1 5 10 15
<210> 237
   <211> 15
   <212> PRT
   <213> P. trichocarpa RAR2 domain #7
<400> 237
   Gly Phe Arg Pro Ala Val Pro Ile Gln Gly Pro Gly Val Gly His
   1 5 10 15
<210> 238
   <211> 15
   <212> PRT
   <213> P. trichocarpa RAR3 domain #1
<400> 238
   Ala Phe Lys Pro Thr Thr Pro Gly His Ser Pro Gly Ile Gly His
   1 5 10 15
<210> 239
   <211> 15
   <212> PRT
   <213> P. trichocarpa RAR3 domain #2
<400> 239
   Thr Phe Lys Pro Thr Thr Pro Gly His Ser Pro Gly Ile Gly His
   1 5 10 15
<210> 240
   <211> 15
   <212> PRT
   <213> P. trichocarpa RAR3 domain #3
<400> 240
   Lys His Ser Val Thr Thr Pro Gly His Ser Ser Arg Val Gly His
   1 5 10 15
<210> 241
   <211> 15
   <212> PRT
   <213> P. trichocarpa RAR3 domain #4
<400> 241
   Thr Phe Lys Pro Thr Thr Pro Gly His Ser Pro Gly Ile Gly His
   1 5 10 15
<210> 242
   <211> 15
   <212> PRT
   <213> P. trichocarpa RAR4 domain
<400> 242
   Ala Phe Arg Pro Thr Thr Pro Gly His Ser Pro Gly Val Gly His
   1 5 10 15
<210> 243
   <211> 15
   <212> PRT
   <213> P. trichocarpa RAR5 domain
<400> 243
   Asp Phe Lys Pro Thr Thr Pro Gly His Ser Pro Gly Ala Gly His
   1 5 10 15
<210> 244
   <211> 15
   <212> PRT
   <213> P. trichocarpa RAR6 domain
<400> 244
   Asp Phe Arg Pro Thr Ala Pro Gly His Ser Pro Gly Val Gly His
   1 5 10 15
<210> 245
   <211> 15
   <212> PRT
   <213> V. vinifera RAR1 domain #1
<400> 245
   Gly Phe Arg Pro Thr Thr Pro Gly Ser Ser Pro Gly Ile Gly His
   1 5 10 15
<210> 246
   <211> 15
   <212> PRT
   <213> V. vinifera RAR1 domain #2
<400> 246
   Asp Phe Arg Pro Thr Thr Pro Gly Ser Ser Pro Gly Val Gly His
   1 5 10 15
<210> 247
   <211> 15
   <212> PRT
   <213> V. vinifera RAR1 domain #3
<400> 247
   Asp Tyr Arg Pro Thr Lys Pro Gly His Ser Pro Gly Val Gly His
   1 5 10 15
<210> 248
   <211> 15
   <212> PRT
   <213> V. vinifera RAR2 domain
<400> 248
   Gly Phe Arg Pro Thr Thr Pro Gly Ser Ser Pro Gly Ile Gly His
   1 5 10 15
<210> 249
   <211> 15
   <212> PRT
   <213> V. vinifera RAR3 domain #1
<400> 249
   Asp Phe Arg Pro Thr Pro Pro Gly Ser Ser Pro Gly Ile Gly His
   1 5 10 15
<210> 250
   <211> 15
   <212> PRT
   <213> V. vinifera RAR3 domain #2
<400> 250
   Asp Phe Arg Pro Thr Thr Pro Gly His Ser Pro Gly Val Gly His
   1 5 10 15
<210> 251
   <211> 15
   <212> PRT
   <213> V. vinifera RAR4 domain
<400> 251
   Asp Val Gln Ser Thr Thr Pro Gly His Ser Pro Gly Val Gly His
   1 5 10 15
<210> 252
   <211> 15
   <212> PRT
   <213> V. vinifera RAR5 domain
<400> 252
   Asp Phe Arg Pro Thr Pro Pro Gly Ser Ser Pro Gly Ile Gly His
   1 5 10 15
<210> 253
   <211> 15
   <212> PRT
   <213> V. vinifera RAR6 domain #1
<400> 253
   Asp Phe Arg Pro Thr Thr Pro Gly Ser Ser Pro Gly Val Gly His
   1 5 10 15
<210> 254
   <211> 15
   <212> PRT
   <213> V. vinifera RAR6 domain #2
<400> 254
   Asp Tyr Arg Pro Thr Lys Pro Gly His Ser Pro Gly Val Gly His
   1 5 10 15
<210> 255
   <211> 15
   <212> PRT
   <213> V. vinifera RAR7 domain
<400> 255
   Asp Phe Arg Pro Thr Ser Pro Gly His Ser Pro Gly Val Gly His
   1 5 10 15
<210> 256
   <211> 15
   <212> PRT
   <213> R. communis RAR1 domain #1
<400> 256
   Ala Phe Arg Pro Thr Pro Pro Gly Asn Ser Pro Gly Val Gly His
   1 5 10 15
<210> 257
   <211> 15
   <212> PRT
   <213> R. communis RAR1 domain #2
<400> 257
   Asp Phe Arg Pro Thr Ser Pro Gly Tyr Ser Pro Gly Val Gly His
   1 5 10 15
<210> 258
   <211> 15
   <212> PRT
   <213> R. communis RAR1 domain #3
<400> 258
   Asp Phe Arg Pro Thr Ala Pro Gly Phe Ser Pro Gly Val Gly His
   1 5 10 15
<210> 259
   <211> 15
   <212> PRT
   <213> R. communis RAR1 domain #4
<400> 259
   Asp Tyr Arg Pro Thr Gln Pro Gly His Ser Pro Gly Val Gly His
   1 5 10 15
<210> 260
   <211> 15
   <212> PRT
   <213> R. communis RAR2 domain
<400> 260
   Asp Phe Arg Pro Thr Ala Pro Gly His Ser Pro Gly Val Gly His
   1 5 10 15
<210> 261
   <211> 15
   <212> PRT
   <213> R. communis RAR3 domain
<400> 261
   Ala Phe Arg Pro Thr Asn Pro Gly His Ser Pro Gly Val Gly His
   1 5 10 15
<210> 262
   <211> 15
   <212> PRT
   <213> R. communis RAR4 domain
<400> 262
   Asp Phe Arg Pro Thr Asn Pro Gly His Ser Pro Gly Ala Gly His
   1 5 10 15
<210> 263
   <211> 15
   <212> PRT
   <213> R. communis RAR5 domain
<400> 263
   Asp Phe Arg Pro Thr Ala Pro Gly His Ser Pro Gly Val Gly His
   1 5 10 15
<210> 264
   <211> 15
   <212> PRT
   <213> R. communis RAR6 domain
<400> 264
   Ala Phe Arg Pro Thr Ala Pro Gly His Ser Ser Gly Val Gly His
   1 5 10 15
<210> 265
   <211> 15
   <212> PRT
   <213> R. communis RAR7 domain
<400> 265
   Ala Phe Arg Pro Thr Asn Pro Gly His Ser Pro Gly Val Gly His
   1 5 10 15
<210> 266
   <211> 15
   <212> PRT
   <213> C. glauca RAR1 domain
<400> 266
   Ala Phe Arg Pro Thr Ser Pro Gly His Ser Pro Gly Val Gly His
   1 5 10 15
<210> 267
   <211> 15
   <212> PRT
   <213> J. curcas RAR1 domain
<400> 267
   Ala Phe Arg Pro Thr Asn Pro Gly His Ser Pro Gly Val Gly His
   1 5 10 15
<210> 268
   <211> 15
   <212> PRT
   <213> J. curcas RAR2 domain #1
<400> 268
   Asp Phe Arg Pro Thr Thr Gly Gly His Ser Pro Gly Ala Gly His
   1 5 10 15
<210> 269
   <211> 15
   <212> PRT
   <213> J. curcas RAR2 domain #2
<400> 269
   Asp Phe Arg Pro Thr Lys Pro Gly Tyr Ser Pro Gly Val Gly His
   1 5 10 15
<210> 270
   <211> 15
   <212> PRT
   <213> J. curcas RAR2 domain #3
<400> 270
   Asp Phe Arg Pro Thr Ser Pro Gly Tyr Ser Pro Gly Val Gly His
   1 5 10 15
<210> 271
   <211> 15
   <212> PRT
   <213> J. curcas RAR2 domain #4
<400> 271
   Asp Phe Arg Pro Thr Ser Pro Gly Tyr Ser Pro Gly Val Gly His
   1 5 10 15
<210> 272
   <211> 15
   <212> PRT
   <213> J. curcas RAR2 domain #5
<400> 272
   Asp Phe Arg Pro Thr Ala Pro Gly Tyr Ser Pro Gly Val Gly His
   1 5 10 15
<210> 273
   <211> 15
   <212> PRT
   <213> T. cacao RAR1 domain
<400> 273
   Asp Phe Arg Pro Thr Thr Pro Gly His Ser Pro Gly Ala Gly His
   1 5 10 15
<210> 274
   <211> 15
   <212> PRT
   <213> T. cacao RAR2 domain
<400> 274
   Asp Phe Arg Pro Thr Pro Pro Gly His Ser Pro Gly Ala Gly His
   1 5 10 15
<210> 275
   <211> 15
   <212> PRT
   <213> T. cacao RAR4 domain
<400> 275
   Asp Phe Arg Pro Pro Thr Pro Gly His Gly Pro Gly Ala Gly His
   1 5 10 15
<210> 276
   <211> 15
   <212> PRT
   <213> M. domestica RAR1 domain
<400> 276
   Asp Tyr Arg Pro Thr Thr Pro Gly His Ser Pro Gly Ala Gly His
   1 5 10 15
<210> 277
   <211> 15
   <212> PRT
   <213> C. papaya RAR1 domain
<400> 277
   Gly Phe Arg Pro Thr Thr Pro Gly Ile Ser Pro Gly Val Gly His
   1 5 10 15
<210> 278
   <211> 15
   <212> PRT
   <213> C. papaya RAR2 domain
<400> 278
   Ala Phe Arg Pro Thr Thr Pro Gly His Ser Pro Gly Val Gly His
   1 5 10 15
<210> 279
   <211> 15
   <212> PRT
   <213> C. papaya RAR3 domain
<400> 279
   Asp Phe Arg Pro Thr Thr Pro Gly His Ser Pro Gly Val Gly His
   1 5 10 15
<210> 280
   <211> 15
   <212> PRT
   <213> F. vesca RAR1 domain #1
<400> 280
   Asp Phe Arg Pro Thr Thr Pro Gly Asn Ser Pro Gly Ala Gly His
   1 5 10 15
<210> 281
   <211> 15
   <212> PRT
   <213> F. vesca RAR1 domain #2
<400> 281
   Asp Tyr Arg Pro Thr Gln Pro Gly His Ser Pro Gly Val Gly His
   1 5 10 15
<210> 282
   <211> 15
   <212> PRT
   <213> F. vesca RAR2 domain
<400> 282
   Ala Phe Arg Pro Thr Thr Pro Gly His Ser Pro Gly Val Gly His
   1 5 10 15
<210> 283
   <211> 15
   <212> PRT
   <213> F. vesca RAR3 domain
<400> 283
   Asp Phe Arg Pro Thr Thr Pro Gly His Ser Pro Gly Val Gly His
   1 5 10 15
<210> 284
   <211> 15
   <212> PRT
   <213> P. persica RAR1 domain
<400> 284
   Ala Phe Arg Pro Thr Ser Pro Gly His Ser Pro Gly Val Gly His
   1 5 10 15
<210> 285
   <211> 15
   <212> PRT
   <213> C. sativus RAR1 domain #1
<400> 285
   Ala Phe Arg Pro Thr Ser Pro Gly His Ser Pro Gly Val Gly His
   1 5 10 15
<210> 286
   <211> 15
   <212> PRT
   <213> M. esculenta RAR1 domain #1
<400> 286
   Cys Phe Arg Pro Thr Asp Pro Gly Asn Ser Pro Gly Val Gly His
   1 5 10 15
<210> 287
   <211> 15
   <212> PRT
   <213> M. esculenta RAR1 domain #2
<400> 287
   Gly Phe Arg Pro Thr Thr Pro Gly Tyr Ser Pro Gly Val Gly His
   1 5 10 15
<210> 288
   <211> 15
   <212> PRT
   <213> M. esculenta RAR1 domain #3
<400> 288
   Asp His Arg Pro Thr Gln Pro Gly His Ser Pro Gly Val Gly His
   1               5                   10                  15
<210> 289
   <211> 15
   <212> PRT
   <213> C. sinensis RAR1 domain
<400> 289
   Ala Phe Arg Pro Thr Ser Pro Gly His Ser Pro Gly Val Gly His
   1               5                   10                  15
<210> 290
   <211> 15
   <212> PRT
   <213> C. clementina RAR1 domain
<400> 290
   Ala Phe Arg Pro Thr Ser Pro Gly His Ser Pro Gly Val Gly His
   1               5                   10                  15
<210> 291
   <211> 15
   <212> PRT
   <213> C. roseus RAR1 domain
<400> 291
   Ser Phe Arg Pro Thr Asn Pro Gly Arg Ser Pro Gly Ile Gly His
   1               5                   10                  15
<210> 292
   <211> 15
   <212> PRT
   <213> S. tuberosum RAR1, domain
<400> 292
   Val Lys Asp Tyr Ser Gly Pro Gly His Ser Pro Gly Met Gly His
   1               5                   10                  15
<210> 293
   <211> 15
   <212> PRT
   <213> S. tuberosum RAR2 domain
<400> 293
   Asp Ile Pro Tyr Val Thr Pro Gly His Ser Pro Gly Met Gly His
   1 5 10 15
<210> 294
   <211> 15
   <212> PRT
   <213> B. napus RAR1 domain
<400> 294
   Asp Phe Arg Pro Thr Thr Pro Gly His Ser Pro Gly Ile Gly His
   1 5 10 15
<210> 295
   <211> 15
   <212> PRT
   <213> B. napus RAR2 domain
<400> 295
   Ala Tyr Ala Pro Thr Asp Pro Gly Asn Ser Pro Gly Ile Gly His
   1 5 10 15
<210> 296
   <211> 15
   <212> PRT
   <213> B. napus RAR3 domain #1
<400> 296
   Ala Tyr Ala Pro Thr Asp Pro Gly Asn Ser Pro Gly Ile Gly His
   1 5 10 15
<210> 297
   <211> 15
   <212> PRT
   <213> B. napus RAR3 domain #2
<400> 297
   Ala Tyr Ala Pro Thr Asp Pro Gly Asn Ser Pro Gly Ile Gly His
   1 5 10 15
<210> 298
   <211> 15
   <212> PRT
   <213> E. grandis RAR1 domain
<400> 298
   Asp Phe Arg Pro Thr Gln Pro Gly His Ser Pro Gly Val Gly His
   1 5 10 15
<210> 299
   <211> 15
   <212> PRT
   <213> E. grandis RAR2 domain
<400> 299
   Ser Tyr Arg Pro Thr Thr Pro Gly His Ser Pro Gly Ile Gly His
   1 5 10 15
<210> 300
   <211> 15
   <212> PRT
   <213> A. coerulea RAR1 domain
<400> 300
   Ala Phe Arg Pro Thr Thr Pro Gly His Ser Pro Gly Ile Gly His
   1 5 10 15
<210> 301
   <211> 15
   <212> PRT
   <213> O. sativa RAR1 domain #1
<400> 301
   Gly Val Arg Pro Thr Asn Pro Gly His Ser Pro Gly Ile Gly His
   1 5 10 15
<210> 302
   <211> 15
   <212> PRT
   <213> O. sativa RAR1 domain #2
<400> 302
   Asp Val Arg Pro Thr Asn Pro Gly His Ser Pro Gly Ile Gly His
   1 5 10 15
<210> 303
   <211> 15
   <212> PRT
   <213> O. sativa RAR1 domain #3
<400> 303
   Asp Val Arg Pro Thr Asn Pro Gly His Ser Pro Gly Ile Gly His
   1 5 10 15
<210> 304
   <211> 15
   <212> PRT
   <213> O. sativa RAR1 domain #4
<400> 304
   Asp Val Arg Pro Thr Asn Pro Gly His Ser Pro Gly Ile Gly His
   1 5 10 15
<210> 305
   <211> 15
   <212> PRT
   <213> O. sativa RAR2 domain #1
<400> 305
   Gly Val Arg Pro Thr Asn Pro Gly His Ser Pro Gly Ile Gly His
   1 5 10 15
<210> 306
   <211> 15
   <212> PRT
   <213> O. sativa RAR2 domain #2
<400> 306
   Asp Val Arg Pro Thr Asn Pro Gly His Ser Pro Gly Ile Gly His
   1 5 10 15
<210> 307
   <211> 15
   <212> PRT
   <213> O. sativa RAR2 domain #3
<400> 307
   Asp Val Arg Pro Thr Asn Pro Gly His Ser Pro Gly Ile Gly His
   1 5 10 15
<210> 308
   <211> 15
   <212> PRT
   <213> O. sativa RAR2 domain #4
<400> 308
   Asp Val Arg Pro Thr Asn Pro Gly His Ser Pro Gly Ile Gly His
   1               5                   10                  15
<210> 309
   <211> 15
   <212> PRT
   <213> O. sativa RAR3 domain
<400> 309
   Asp Val Arg Pro Thr Asn Pro Gly His Ser Pro Gly Ile Gly His
   1               5                   10                  15
<210> 310
   <211> 15
   <212> PRT
   <213> O. sativa RAR4 domain
<400> 310
   Asp Ala Arg Pro Thr Ala Pro Gly His Ser Pro Gly Ala Gly His
   1               5                   10                  15
<210> 311
   <211> 15
   <212> PRT
   <213> O. sativa RAR5 domain
<400> 311
   Asp Val Arg Pro Thr Asn Pro Gly His Ser Pro Gly Ile Gly His
   1               5                   10                  15
<210> 312
   <211> 15
   <212> PRT
   <213> O. barthii RAR1 domain #1
<400> 312
   Gly Val Arg Pro Thr Asn Pro Gly His Ser Pro Gly Ile Gly His
   1               5                   10                  15
<210> 313
   <211> 15
   <212> PRT
   <213> O. barthii RAR1 domain #2
<400> 313
   Asp Val Arg Pro Thr Asn Pro Gly His Ser Pro Gly Ile Gly His
   1 5 10 15
<210> 314
   <211> 15
   <212> PRT
   <213> O. barthii RAR1 domain #3
<400> 314
   Asp Val Arg Pro Thr Asn Pro Gly His Ser Pro Gly Ile Gly His
   1 5 10 15
<210> 315
   <211> 15
   <212> PRT
   <213> S. bicolor RAR1 domain
<400> 315
   Asp Val Arg Pro Ser Asn Pro Gly His Ser Pro Gly Ile Gly His
   1 5 10 15
<210> 316
   <211> 15
   <212> PRT
   <213> S. bicolor RAR2 domain
<400> 316
   Asp Ser Arg Pro Thr Ala Pro Gly Asn Ser Pro Gly Ile Gly Asn
   1 5 10 15
<210> 317
   <211> 15
   <212> PRT
   <213> T. aestivum RAR1 domain
<400> 317
   Asp Gly Arg Gly Thr Gly Pro Gly His Ser Pro Gly Ile Gly Asn
   1 5 10 15
<210> 318
   <211> 15
   <212> PRT
   <213> T. aestivum RAR2 domain #1
<400> 318
   Asp Ser Arg Gly Thr Ala Pro Gly His Ser Pro Gly Ile Gly Asn
   1 5 10 15
<210> 319
   <211> 15
   <212> PRT
   <213> T. aestivum RAR2 domain #2
<400> 319
   Asp Ser Arg Gly Thr Ala Pro Gly His Ser Pro Gly Ile Gly Asn
   1 5 10 15
<210> 320
   <211> 15
   <212> PRT
   <213> T. aestivum RAR3 domain
<400> 320
   Glu Ser Arg Gly Thr Ala Pro Gly Asn Ser Pro Gly Ile Gly Asn
   1 5 10 15
<210> 321
   <211> 15
   <212> PRT
   <213> H. vulgare RAR1 domain
<400> 321
   Ser Trp Arg Pro Thr Thr Pro Gly Thr Ser Pro Gly Ile Thr Ser
   1 5 10 15
<210> 322
   <211> 15
   <212> PRT
   <213> H. vulgare RAR2 domain
<400> 322
   Ser Trp Arg Pro Thr Thr Pro Gly Thr Ser Pro Gly Ile Thr Ser
   1 5 10 15
<210> 323
   <211> 15
   <212> PRT
   <213> H. vulgare RAR3 domain
<400> 323
   Gln Val Glu Ser Thr Thr Pro Gly His Ser Pro Ser Ile Gly His
   1               5                   10                  15
<210> 324
   <211> 15
   <212> PRT
   <213> S. officinarum RAR1 domain
<400> 324
   Asp Ser Arg Pro Thr Tyr Pro Gly Asn Ser Pro Gly Ile Gly Asn
   1               5                   10                  15
<210> 325
   <211> 15
   <212> PRT
   <213> S. officinarum RAR2 domain
<400> 325
   Asp Ser Arg Pro Thr Tyr Pro Gly Asn Ser Pro Gly Ile Gly Asn
   1               5                   10                  15
<210> 326
   <211> 15
   <212> PRT
   <213> Z. mays RAR1 domain
<400> 326
   Asp Ser Arg Pro Thr Ala Pro Gly Asn Ser Pro Gly Ile Gly Asn
   1               5                   10                  15
<210> 327
   <211> 15
   <212> PRT
   <213> Z. mays RAR2 domain
<400> 327
   Asp Val Arg Pro Thr Asn Pro Ser His Ser Pro Gly Ile Gly His
   1               5                   10                  15
<210> 328
   <211> 15
   <212> PRT
   <213> Z. mays RAR3 domain
<400> 328
   Asp Gly Arg Pro Thr Ala Pro Gly His Ser Pro Gly Ile Gly Asn
   1 5 10 15
<210> 329
   <211> 15
   <212> PRT
   <213> Z. mays RAR4 domain
<400> 329
   Asp Ser Arg Pro Thr Ala Pro Gly Asn Ser Pro Gly Ile Gly Asn
   1 5 10 15
<210> 330
   <211> 15
   <212> PRT
   <213> B. distachyon RAR1 domain
<400> 330
   Gly Val Arg Pro Ser Asn Pro Gly His Ser Pro Gly Ile Gly His
   1 5 10 15
<210> 331
   <211> 15
   <212> PRT
   <213> S. italica RAR1 domain
<400> 331
   Asp Val Arg Pro Ser Thr Pro Arg His Ser Pro Gly Ile Gly His
   1 5 10 15
<210> 332
   <211> 15
   <212> PRT
   <213> P. dactylifera RAR1 domain
<400> 332
   Asp Ser Arg Pro Thr Ala Pro Gly His Ser Pro Gly Val Gly His
   1 5 10 15
<210> 333
   <211> 15
   <212> PRT
   <213> P. dactylifera RAR2 domain
<400> 333
   Gly Asp Arg Pro Ser Val Pro Gly His Ser Pro Gly Val Gly His
   1               5                   10                  15
<210> 334
   <211> 15
   <212> PRT
   <213> P. dactylifera RAR3 domain
<400> 334
   Asp Ala Arg Pro Thr Thr Pro Gly His Ser Pro Gly Val Gly His
   1               5                   10                  15
<210> 335
   <211> 15
   <212> PRT
   <213> P. dactylifera RAR4 domain
<400> 335
   Asp Ala Arg Pro Thr Thr Pro Gly His Ser Pro Gly Val Gly His
   1               5                   10                  15
<210> 336
   <211> 15
   <212> PRT
   <213> P. dactylifera RAR5 domain
<400> 336
   Asp Ala Arg Pro Thr Thr Pro Gly His Ser Pro Gly Val Gly His
   1               5                   10                  15
<210> 337
   <211> 2187
   <212> DNA
   <213> Medicago truncatula RAR1 gene
<400> 337
   cttcaacttt gacgatttag attcgatatt ttgacacacc aacaataaag aatacaaaac     60
   tatcatcata gatataaaac attagagcta gtaatgatgc attcaaaacc caatcaaatt    120
   gtttatacat gcagtgtagt acaaaatttt gctacaagtc aattcaatat ttcaatttga    180
   gagaagaaac ttatacaatt cattattttt tcttcaactt tgagcatgcg gtgcacccaa    240
   tttgataatt agcctactat aaagtttaaa caagagagga acccagaaaa agctgccttt    300
   cacttcaact tgtccatcaa aataaaatgc tgaaattaaa tagagaccat aaactgctta    360
   agtagtgctt aattatacta taatcaatga aattaattaa actataccac tgttagtgga    420
   agggaaggta caagagaatc tcatatatac atatctttct tcatcagggg tccattctaa    480
   attaatggtg cgccagtcct tcaactattc tttattggtt catcataact tcaaatctta    540
   ccagttttcc aaaggaacaa aattacaact acatatctca actacttgct aaatgtccct    600
   ttctcaacct tcatattaac ctataccaag cacatatatt aacctatact catcggatta    660
   gacgtgtctt actgttggat acatattgtg tctgacatca tcacatatgt ttttttaacg     720
   gaaaaattta ttaaaaatca agtccctaaa gagagaccga caagttagaa acaacataat    780
   aaattattat tgacacatat gattacatta aattgtataa atttttcaaa taattatcga    840
   tgttgacgtt ctgtattgtg ttccgtgtcc gtgtcttagt ccatacttca tacataataa    900
   ctattacaat gtatggccat gcaaaattga caaaaaataa ttggccaact acattggaga    960
   ttcgtattga ttgaacttaa ttttgtatgg aatagagata cgattcacat gtattatagg 1020
   atgctgataa taatcatgtt ttggacataa atagaatgga atagagaaaa caagagagca 1080
   gaataaagca aaacttccat tagaggccac aattatttat attaaaaaat aataattaat 1140
   tatgcttcaa catacattgt gatagattct catcatttct gaaaagaatt ggaagatttc 1200
   aacactaatt ttgagagtat atagtctaaa aaatttatac ggtttacgat tcagttatgg 1260
   ttaagtatta tttaaaaaac taacagaaat atgttttttt gtttaatttt gttgaagata 1320
   agtaaggata gattaaaata caactaatta tttttgtttc ttgtagtttt tgaaatcact 1380
   tttaaatgag tttttgacaa aactgtgaca atgtaacaaa atacataaaa ataatggaaa 1440
   aatcacaagt ttccgaaaat ttgcaaaaca aacattaaag ttcgatagaa aacatagaat 1500
   actgaaccgt aagcaattga tccattgctc ggcccataat ctaaatatat aaaataaatg 1560
   tcaccatttt aaaaaatgta tatatttata caccaaaaac ttaagtaatt tgagctcacc 1620
   atttcttata ttattgagca tatctaagcg taattgtgac tgttccactt aaaatcaaga 1680
   tatttagatc ttaaatcata tataataagg ttgatatgca atgcaacata agaagagagg 1740
   tgaatatcaa aagagacata tatcatattc ataataatgc aaatgactaa aaagagaaag 1800
   atttttatat atgcaccccc aaaagagcat gttcgcccac gtgagatagt gatgtgaccc 1860
   atagaattaa aaaatatact aatttgacat ataaaattaa aagctagcta tttttgacac 1920
   ataaacggtc caaatttgca gatcataaca ctccacaaat taagtcaaat gaaataatta 1980
   gcactaagaa tcttataaga gagcactgtc acactcacac acattctaca ttataaatac 2040
   ccctcaagat cccataacat ttcatatcat atatcttcta atttgaacta taacaagctt 2100
   aaactttcaa tacatatagt tcattcattc tctctactct accttctcta tttgttcgtg 2160
   ttaatggctt ataaatttca atacaca 2187
<210> 338
   <211> 140
   <212> PRT
   <213> Picea glauca RAR-like 1
<400> 338
   Met Lys Gly Tyr Ala Met Ile Val Leu Leu Leu Leu Ala Ser Arg Leu
   1               5                   10                  15
   Gly Glu Ala Ala Arg Ile Phe Gly Phe Lys Pro Phe Tyr Leu Asn Ser
               20                  25                  30
   Asp Ser Gln Val Arg Ala Ala Pro Ala Ser Ser Tyr Pro Ser Val Glu
           35                  40                  45
   Glu Lys Thr Pro Glu Lys Ala Val Leu Ala Leu Asn Glu Arg Leu Arg
       50                  55                  60
   Arg Arg Ala Ser Val Glu Lys His Pro Gly Ser Glu Thr Asn Leu Lys
   65                  70                  75                  80
   Pro Asn Leu Ser Ala Lys Ser Lys Ala Ser Asn Gln Arg Ser Asn Gly
                   85                  90                  95
   Leu Leu Arg Thr Thr Phe Pro Ser Val Lys Phe Asp Val Val Glu Ala
               100                 105                 110
   Asp Met Glu Lys Thr Val Ala Tyr Pro Glu Leu Leu Gly Lys Ser Pro
           115                 120                 125
   Gly Val Gly His Asp Ile Gln Pro Gly Ser Arg His
       130                 135                 140
<210> 339
   <211> 140
   <212> PRT
   <213> Picea glauca RAR-like 2
<400> 339
   Met Lys Gly Tyr Ala Met Ile Val Leu Leu Leu Leu Ala Ser Arg Leu
   1 5 10 15
   Gly Glu Ala Ala Arg Ile Phe Gly Phe Lys Pro Phe Tyr Leu Asn Ser
   20 25 30
   Asp Ser Gln Val Arg Ala Ala Leu Ala Ser Ser Tyr Pro Ser Val Glu
   35 40 45
   Glu Lys Thr Pro Glu Lys Ala Val Leu Ala Leu Asn Glu Arg Leu Arg
   50 55 60
   Arg Arg Ala Ser Val Glu Lys His Pro Gly Ser Glu Ser Asn Leu Lys
   65 70 75 80
   Pro Asn Leu Ser Ala Lys Ser Lys Ala Ser Asn Gln Arg Ser Asn Glu
   85 90 95
   Leu Leu Arg Thr Ile Leu Pro Ser Val Lys Phe Asp Ala Val Glu Pro
   100 105 110
   Asp Met Glu Lys Thr Val Ala Pro Phe Glu Pro Leu Gly His Ser Pro
   115 120 125
   Gly Ile Gly His Asp Asp Pro Pro Arg Ser Arg His
   130 135 140
<210> 340
   <211> 141
   <212> PRT
   <213> Picea glauca RAR-like 3
<400> 340
   Met Lys Gly Cys Ala Met Ile Val Leu Leu Phe Leu Ala Ala Pro Leu
   1 5 10 15
   Gly Glu Ala Ala Arg Ile Leu Cys Phe Lys Leu Phe Leu Met Asn Ser
               20                  25                  30
   Asp Ser Gln Val Lys Ala Ala Pro Ala Arg Ser Tyr Ala Leu Val Gln
           35                  40                  45
   Glu Arg Ala Pro Gly Lys Ala Val Leu Glu Leu Lys Glu Arg Leu Ser
       50                  55                  60
   Arg Lys Ala Ser Arg Glu Lys Tyr His Gly Ser Glu Ala Asn Met Asn
   65                  70                  75                  80
   Pro Asn Ile Ser Ala Asn Ser Thr Ala Ser His Gln His Ser Asn Gly
                   85                  90                  95
   Leu Leu Gln Lys Ile His Pro Ser Leu Lys Phe Asp Val Val Glu Pro
               100                 105                 110
   Glu Arg Glu Lys Ser Phe Thr Pro Phe Leu Pro Leu Leu Gly His Ser
           115                 120                 125
   Pro Gly Val Gly His Asn Asn Pro Pro Gly Phe Arg His
       130                 135                 140
<210> 341
   <211> 142
   <212> PRT
   <213> Picea glauca RAR-like 4
<400> 341
   Met Met Lys Gly Cys Ala Met Ile Val Leu Leu Phe Leu Ala Ala Pro
   1               5                   10                  15
   Leu Gly Glu Ala Ser Arg Ile Leu Cys Phe Lys Leu Phe Leu Met Asn
               20                  25                  30
   Ser Asp Ser Gln Val Lys Ala Ala Pro Ala Arg Ser Tyr Ala Leu Val
           35                  40                  45
   Gln Glu Arg Ala Pro Gly Lys Ala Val Leu Glu Leu Lys Glu Arg Leu
       50                  55                  60
   Ser Arg Lys Ala Ser Arg Glu Lys Tyr His Gly Ser Glu Ala Asn Met
   65                  70                  75                  80
   Asn Pro Asn Ile Ser Ala Asn Ser Thr Ala Ser His Gln His Ser Asn
                   85                  90                  95
   Gly Leu Leu Gln Lys Ile His Pro Ser Leu Lys Phe Asp Val Val Glu
               100                 105                 110
   Pro Glu Arg Glu Lys Ser Phe Thr Pro Phe Leu Pro Leu Leu Gly His
           115                 120                 125
   Ser Pro Gly Val Gly His Asn Asn Pro Pro Gly Phe Arg His
       130                 135                 140
<210> 342
   <211> 142
   <212> PRT
   <213> Picea glauca RAR-like 5
<400> 342
   Met Met Lys Gly Cys Ala Met Ile Val Leu Leu Phe Leu Ala Ala Pro
   1               5                   10                  15
   Leu Gly Glu Ala Ser Arg Ile Leu Cys Phe Lys Leu Phe Leu Met Asn
               20                  25                  30
   Ser Asp Ser Gln Val Lys Ala Ala Pro Ala Arg Ser Tyr Ala Leu Val
           35                  40                  45
   Gln Glu Arg Ala Pro Gly Lys Ala Val Leu Glu Leu Lys Glu Arg Leu
       50                  55                  60
   Ser Arg Lys Ala Ser Arg Glu Lys Tyr His Gly Ser Glu Ala Asn Met
   65                  70                  75                      80
   Asn Pro Asn Ile Ser Ala Asn Ser Thr Ala Ser His Gln His Ser Asn
                   85                  90                  95
   Gly Leu Leu Gln Lys Ile His Pro Ser Leu Lys Phe Asp Val Val Glu
               100                 105                 110
   Pro Glu Arg Glu Lys Ser Phe Thr Pro Phe Leu Pro Leu Leu Gly His
           115                 120                 125
   Ser Pro Gly Ile Gly His Asn Asn Pro Pro Gly Phe Ser His
       130                 135                 140
<210> 343
   <211> 142
   <212> PRT
   <213> Picea glauca RAR-like 6
<400> 343
   Met Met Lys Gly Cys Ala Met Ile Val Leu Leu Phe Leu Ala Ala Pro
   1               5                   10                  15
   Leu Arg Glu Ala Ser Arg Ile Leu Cys Phe Lys Leu Phe Leu Met Asn
               20                  25                  30
   Ser Asp Ser Gln Val Lys Ala Ala Pro Ala Arg Ser Tyr Ala Leu Val
           35                  40                  45
   Gln Glu Arg Ala Pro Gly Lys Ala Val Leu Glu Leu Lys Glu Arg Leu
       50                  55                  60
   Ser Arg Lys Ala Ser Arg Glu Lys Tyr His Gly Ser Glu Ala Asn Met
   65                  70                  75                      80
   Asn Pro Asn Ile Ser Ala Asn Ser Thr Ala Ser His Gln His Ser Asn
                   85                  90                  95
   Gly Leu Leu Gln Lys Ile His Pro Ser Leu Lys Phe Asp Val Val Glu
   100 105 110
   Pro Glu Arg Glu Lys Ser Phe Thr Pro Phe Leu Pro Leu Leu Gly His
   115 120 125
   Ser Pro Gly Val Gly His Asn Asn Pro Pro Gly Phe Arg His
   130 135 140
<210> 344
   <211> 136
   <212> PRT
   <213> Picea glauca RAR-like 7
<400> 344
   Met Met Lys Ile Cys Ala Val Ile Ile Leu Leu Leu Phe Ala Ala Pro
   1 5 10 15
   Leu Gly Glu Ala Ser Arg Ile Phe Gly Phe Lys Pro Phe Ser Leu Lys
   20 25 30
   Asn Asp Ser Gln Val Lys Ala Thr Thr Ala Val Glu Glu Ser Thr Ala
   35 40 45
   Glu Lys Val Val Leu Glu Met Asn Glu Cys Phe Ser Lys Arg Ala Asn
   50 55 60
   Leu Glu Lys His Pro Gly Ser Glu Ala Ser Leu Lys Pro Asn Val Ser
   65 70 75 80
   Ala Lys Ser Lys Ala Ser Asp Gln Arg Ser Asp Glu Leu Pro Gln Thr
   85 90 95
   Ile Leu Leu Ser Leu Lys Phe Asn Ala Val Gln His Glu Glu Lys Lys
   100 105 110
   Ser Val Pro Pro Phe Gln Pro Leu Gly His Ser Pro Gly Ile Gly His
   115 120 125
   Glu Asn Pro Pro Gly Leu Arg Gln
   130 135
<210> 345
   <211> 155
   <212> PRT
   <213> Picea glauca RAR-like 8
<400> 345
   Met Lys Leu Gly Leu Trp Ser Val Trp Gly Ala Leu Met Leu Ser Cys
   1               5                   10                  15
   Val Leu Ser Tyr Ser Thr Ala Lys Ala Arg Leu Met Gly Phe Asn Pro
               20                  25                  30
   Asn Ala Ile Gln Pro Pro Arg Pro Pro Ala Leu Tyr Lys Ala Asn Glu
           35                  40                  45
   Val Gly Asn Ile Phe Arg Asp Thr Pro Met Gly Arg Ser Ser Thr Ile
       50                  55                  60
   Glu Lys Lys Gln Ile Ser Ile Ala Pro Ala Glu Thr Lys Leu Pro Ser
   65                  70                  75                  80
   Thr Leu Lys Val Thr Val Gln Gly Ser Leu Gly His Asn Asp Ala His
                   85                  90                  95
   Gly Ile Lys Glu Ala Glu Thr Val Ala Gly Gly Thr Gln Ile Phe Ser
               100                 105                 110
   Lys Arg Pro Ser Glu Ser Asn Asn Asp Ser Ala Arg Met Lys Lys Val
           115                 120                 125
   Asp Ala Val Met Ala Phe Arg Pro Ser Ser Ser Gly His Ser Pro Gly
       130                 135                 140
   Ile Gly His Asp Asp Pro Pro Gly Pro Met Leu
   145                 150                 155
<210> 346
   <211> 73
   <212> PRT
   <213> Pinus contorta RAR-like 1
<400> 346
   Ala Asp Leu Glu Glu His Pro Gly Ser Asp Ala Asn Phe Lys Pro Ser
   1               5                   10                  15
   Val Phe Val Lys Ser Asn Ala Ser Asp Gln Arg Ser Asp Gly His Val
               20                  25                  30
   Glu Glu Leu Val Pro Ser Leu Lys Phe Glu Val Val Gln His Asp Val
           35                  40                  45
   Gln Lys Thr Ile Ser Pro Phe Lys Pro Leu Gly His Ser Pro Gly Ile
       50                  55                  60
   Gly His Asp Asp Pro Pro Gly Ser Lys
   65                  70
<210> 347
   <211> 74
   <212> PRT
   <213> Pinus contorta RAR-like 2
<400> 347
   Ala Asp Leu Glu Glu His Pro Gly Ser Asp Ala Asn Phe Lys Pro Ser
   1               5                   10                  15
   Val Phe Val Lys Ser Asn Ala Ser Asp Gln Arg Phe Asp Gly His Val
               20                  25                  30
   Glu Glu Leu Val Pro Ser Leu Lys Phe Glu Val Val Gln His Asp Val
           35                  40                  45
   Gln Lys Thr Ile Ser Pro Phe Lys Pro Leu Gly His Ser Pro Gly Ile
       50                  55                  60
   Gly His Asp Asp Pro Pro Gly Ser Lys His
   65                  70
<210> 348
   <211> 143
   <212> PRT
   <213> Pinus contorta RAR-like 3
<400> 348
   Met Lys Thr Ser Val Leu Ile Met Leu Met Phe Leu Ala Ala Pro Leu
   1               5                   10                  15
   Val Glu Ala Ala Arg Ile Ile Gly Phe Lys Pro Phe Ser Leu Asn Arg
               20                  25                  30
   Asp Ser Gln Val Lys Ala Thr Pro Ala Thr Ser Tyr Pro Leu Val Glu
           35                  40                  45
   Glu Arg Ala Pro Ala Lys Val Phe Val Glu Leu Lys Glu Pro Phe Gly
       50                  55                  60
   Arg Arg Ala Asp Leu Thr Asp Leu Glu Glu His Pro Gly Ser Asp Ala
   65                  70                  75                  80
   Asn Phe Lys Pro Ser Val Phe Val Lys Ser Asn Ala Ser Asp Gln Arg
                   85                  90                  95
   Ser Asp Gly His Val Glu Glu Leu Val Pro Ser Leu Lys Phe Glu Val
               100                 105                 110
   Val Gln His Asp Val Gln Lys Thr Ile Ser Pro Phe Lys Pro Leu Gly
           115                 120                 125
   His Ser Pro Gly Ile Gly His Asp Asp Pro Pro Gly Ser Lys His
       130                 135                 140
<210> 349
   <211> 137
   <212> PRT
   <213> Pinus contorta RAR-like 4
<400> 349
   Ile Met Leu Met Phe Leu Ala Ala Pro Leu Val Glu Ala Ala Arg Ile
   1               5                   10                  15
   Ile Gly Phe Lys Pro Phe Ser Leu Asn Arg Asp Ser Gln Val Lys Ala
               20                  25                  30
   Thr Pro Ala Thr Ser Tyr Pro Leu Val Glu Glu Arg Ala Pro Ala Lys
           35                  40                  45
   Val Phe Val Glu Leu Lys Glu Pro Phe Gly Arg Arg Ala Asp Leu Thr
       50                  55                  60
   Asp Leu Glu Glu His Pro Gly Ser Asp Ala Asn Phe Lys Pro Ser Val
   65 70 75 80
   Phe Val Lys Ser Asn Ala Ser Asp Gln Arg Phe Asp Gly His Val Glu
   85 90 95
   Glu Leu Val Pro Ser Leu Lys Phe Glu Val Val Gln His Asp Val Gln
   100 105 110
   Lys Thr Ile Ser Pro Phe Lys Pro Leu Gly His Ser Pro Gly Ile Gly
   115 120 125
   His Asp Asp Pro Pro Gly Ser Lys His
   130 135
<210> 350
   <211> 112
   <212> PRT
   <213> Pinus engelmannii x RAR-like 1
<400> 350
   Met Asn Ser Asp Ser Gln Val Lys Ala Thr Pro Ala Arg Ser Tyr Ala
   1 5 10 15
   Leu Val Gln Glu Arg Ala Pro Gly Lys Ala Val Leu Glu Leu Lys Glu
   20 25 30
   Arg Leu Ser Arg Lys Ala Ser Arg Glu Lys His His Gly Ser Glu Ala
   35 40 45
   Asn Met Asn Pro Asn Ile Ser Ala Asn Ser Thr Ala Ser His Gln Arg
   50 55 60
   Ser Asn Gly Ile Leu Gln Lys Ile His Ser Ser Leu Lys Phe Asp Val
   65 70 75 80
   Val Glu Pro Glu Arg Glu Lys Ser Phe Thr Pro Phe Leu Pro Leu Leu
   85 90 95
   Gly His Ser Pro Gly Val Gly His Asn Asn Pro Pro Gly Phe Lys His
   100 105 110
<210> 351
   <211> 15
   <212> PRT
   <213> P. glauca RAR-like 1 domain
<400> 351
   Val Ala Tyr Pro Glu Leu Leu Gly Lys Ser Pro Gly Val Gly His
   1 5 10 15
<210> 352
   <211> 15
   <212> PRT
   <213> P. glauca RAR-like 2 domain
<400> 352
   Val Ala Pro Phe Glu Pro Leu Gly His Ser Pro Gly Ile Gly His
   1 5 10 15
<210> 353
   <211> 15
   <212> PRT
   <213> P. glauca RAR-like 3 domain
<400> 353
   Thr Pro Phe Leu Pro Leu Leu Gly His Ser Pro Gly Val Gly His
   1 5 10 15
<210> 354
   <211> 15
   <212> PRT
   <213> P. glauca RAR-like 4 domain
<400> 354
   Thr Pro Phe Leu Pro Leu Leu Gly His Ser Pro Gly Val Gly His
   1 5 10 15
<210> 355
   <211> 15
   <212> PRT
   <213> P. glauca RAR-like 5 domain
<400> 355
   Thr Pro Phe Leu Pro Leu Leu Gly His Ser Pro Gly Ile Gly His
   1 5 10 15
<210> 356
   <211> 15
   <212> PRT
   <213> P. glauca RAR-like 6 domain
<400> 356
   Thr Pro Phe Leu Pro Leu Leu Gly His Ser Pro Gly Val Gly His
   1 5 10 15
<210> 357
   <211> 15
   <212> PRT
   <213> P. glauca RAR-like 7 domain
<400> 357
   Val Pro Pro Phe Gln Pro Leu Gly His Ser Pro Gly Ile Gly His
   1 5 10 15
<210> 358
   <211> 15
   <212> PRT
   <213> P. glauca RAR-like 8 domain
<400> 358
   Ala Phe Arg Pro Ser Ser Ser Gly His Ser Pro Gly Ile Gly His
   1 5 10 15
<210> 359
   <211> 15
   <212> PRT
   <213> P. contorta RAR-like 1 domain
<400> 359
   Ile Ser Pro Phe Lys Pro Leu Gly His Ser Pro Gly Ile Gly His
   1 5 10 15
<210> 360
   <211> 15
   <212> PRT
   <213> P. contorta RAR-like 2 domain
<400> 360
   Ile Ser Pro Phe Lys Pro Leu Gly His Ser Pro Gly Ile Gly His
   1 5 10 15
<210> 361
   <211> 15
   <212> PRT
   <213> P. contorta RAR-like 3 domain
<400> 361
   Ile Ser Pro Phe Lys Pro Leu Gly His Ser Pro Gly Ile Gly His
   1 5 10 15
<210> 362
   <211> 15
   <212> PRT
   <213> P. contorta RAR-like 4 domain
<400> 362
   Ile Ser Pro Phe Lys Pro Leu Gly His Ser Pro Gly Ile Gly His
   1 5 10 15
<210> 363
   <211> 15
   <212> PRT
   <213> P. engelmannii x RAR-like 1 domain
<400> 363
   Thr Pro Phe Leu Pro Leu Leu Gly His Ser Pro Gly Val Gly His
   1 5 10 15
<210> 364
   <211> 201
   <212> DNA
   <213> Meloidogyne hapla RAR1 cDNA
<400> 364
   atgattaata ttaattcaat tagatttttt attattttta taattaattt tatgatttat 60
   caagtaatgg ctgttaataa ttcagctaat gacttccgac caacaaaccc aggccattca 120
   ccaggaattg gacattgtaa tttaatttta tattttattg gcaaaataat atatcaaaaa 180
   ataagattag tcagaaaata a 201
<210> 365
   <211> 66
   <212> PRT
   <213> Meloidogyne hapla RAR1
<400> 365
   Met Ile Asn Ile Asn Ser Ile Arg Phe Phe Ile Ile Phe Ile Ile Asn
   1 5 10 15
   Phe Met Ile Tyr Gln Val Met Ala Val Asn Asn Ser Ala Asn Asp Phe
   20 25 30
   Arg Pro Thr Asn Pro Gly His Ser Pro Gly Ile Gly His Cys Asn Leu
   35 40 45
   Ile Leu Tyr Phe Ile Gly Lys Ile Ile Tyr Gln Lys Ile Arg Leu Val
   50 55 60
   Arg Lys
   65
<210> 366
   <211> 183
   <212> DNA
   <213> Meloidogyne hapla RAR2 cDNA
<400> 366
   atgattaaaa ttaattctat tatatttttt attattttta taattaattt tatgatttat     60
   caaataatgg ctgctaataa ttcagttgat gccttccgac caacagcccc aggccattca    120
   cccggagttg gacattgtaa tttaatttta aattttatat acaaaattaa atataaaaaa    180
   taa                                                                  183
<210> 367
   <211> 60
   <212> PRT
   <213> Meloidogyne hapla RAR2
<400> 367
   Met Ile Lys Ile Asn Ser Ile Ile Phe Phe Ile Ile Phe Ile Ile Asn
   1               5                   10                  15
   Phe Met Ile Tyr Gln Ile Met Ala Ala Asn Asn Ser Val Asp Ala Phe
               20                  25                  30
   Arg Pro Thr Ala Pro Gly His Ser Pro Gly Val Gly His Cys Asn Leu
           35                  40                  45
   Ile Leu Asn Phe Ile Tyr Lys Ile Lys Tyr Lys Lys
       50                  55                  60
<210> 368
   <211> 183
   <212> DNA
   <213> Meloidogyne hapla RAR3 cDNA
<400> 368
   atgattaata ttaattttat tatatttttt attattttta ttattaattt tatgatttat     60
   ttcacaatgg ctggttacca accaacaaac ccaggccatt cacccggaat tggccattgt    120
   aatgaattat ctcaaaaaag attagggagt aataatttat cagatctgat tagggttttt    180
   tag                                                                  183
<210> 369
   <211> 60
   <212> PRT
   <213> Meloidogyne hapla RAR3
<400> 369
   Met Ile Asn Ile Asn Phe Ile Ile Phe Phe Ile Ile Phe Ile Ile Asn
   1 5 10 15
   Phe Met Ile Tyr Phe Thr Met Ala Gly Tyr Gln Pro Thr Asn Pro Gly
   20 25 30
   His Ser Pro Gly Ile Gly His Cys Asn Glu Leu Ser Gln Lys Arg Leu
   35 40 45
   Gly Ser Asn Asn Leu Ser Asp Leu Ile Arg Val Phe
   50 55 60
<210> 370
   <211> 213
   <212> DNA
   <213> Meloidogyne hapla RAR4 cDNA
<400> 370
   atgactaaaa ttaattctat tatatttttt attattttta ttattaattt tatgatttat      60
   tacaatttgg ctgataatga taaaccagcc aaaattccac ctttcaaaac agtcccaggc     120
   cagagttcac ctggagtagg gcatggaatt ccaaatggag gtccacctgg agttggacat     180
   tgtgatttaa ttaaatttga tttacaaaat taa                                  213
<210> 371
   <211> 70
   <212> PRT
   <213> Meloidogyne hapla RAR4
<400> 371
   Met Thr Lys Ile Asn Ser Ile Ile Phe Phe Ile Ile Phe Ile Ile Asn
   1 5 10 15
   Phe Met Ile Tyr Tyr Asn Leu Ala Asp Asn Asp Lys Pro Ala Lys Ile
   20 25 30
   Pro Pro Phe Lys Thr Val Pro Gly Gln Ser Ser Pro Gly Val Gly His
   35 40 45
   Gly Ile Pro Asn Gly Gly Pro Pro Gly Val Gly His Cys Asp Leu Ile
   50 55 60
   Lys Phe Asp Leu Gln Asn
   65 70
<210> 372
   <211> 133
   <212> DNA
   <213> Meloidogyne hapla RAR5 cDNA
<400> 372
   atgactaaaa ttaattctat tatatttttt attcttttta taattaattt tatgatttat 60
   cacataatgg cagataatgt tattaaacca gcatgcattg gtaattcacc tggagttgga 120
   cattgtaatt gaa 133
<210> 373
   <211> 43
   <212> PRT
   <213> Meloidogyne hapla RAR5
<400> 373
   Met Thr Lys Ile Asn Ser Ile Ile Phe Phe Ile Leu Phe Ile Ile Asn
   1 5 10 15
   Phe Met Ile Tyr His Ile Met Ala Asp Asn Val Ile Lys Pro Ala Cys
   20 25 30
   Ile Gly Asn Ser Pro Gly Val Gly His Cys Asn
   35 40
<210> 374
   <211> 171
   <212> DNA
   <213> Meloidogyne hapla RAR6 cDNA
<400> 374
   atgattaata ttaattctat tttatttttt atttttttta taattaattt tatgatttat 60
   ttcactatgg ctgccttccg accaacaaat ccaggccctt cacccgcaat tggacatgga 120
   attccaaatg gagttccaca acctccaccc gtaaatggac attgtaatta a 171
<210> 375
   <211> 56
   <212> PRT
   <213> Meloidogyne hapla RAR6
<400> 375
   Met Ile Asn Ile Asn Ser Ile Leu Phe Phe Ile Phe Phe Ile Ile Asn
   1 5 10 15
   Phe Met Ile Tyr Phe Thr Met Ala Ala Phe Arg Pro Thr Asn Pro Gly
   20 25 30
   Pro Ser Pro Ala Ile Gly His Gly Ile Pro Asn Gly Val Pro Gln Pro
   35 40 45
   Pro Pro Val Asn Gly His Cys Asn
   50 55
<210> 376
   <211> 153
   <212> DNA
   <213> Meloidogyne hapla RAR7 cDNA
<400> 376
   atgcctaaaa ttaattctat tttatttttt attcttttta ttattaattt tatgatttat     60
   ttcacaatgg ctggattccg accaacaaat ccaggcaatt cacccggagc tggacatgga    120
   gctccaaatg gaccccaaag tctccacccg taa                                 153
<210> 377
   <211> 50
   <212> PRT
   <213> Meloidogyne hapla RAR7
<400> 377
<210> 374
   <211> 171
   <212> DNA
   <213> Meloidogyne hapla RAR6 cDNA
<400> 374
   atgattaata ttaattctat tttatttttt atttttttta taattaattt tatgatttat      60
   ttcactatgg ctgccttccg accaacaaat ccaggccctt cacccgcaat tggacatgga    120
   attccaaatg gagttccaca acctccaccc gtaaatggac attgtaatta a             171
<210> 375
   <211> 56
   <212> PRT
   <213> Meloidogyne hapla RAR6
<400> 375
   Met Ile Asn Ile Asn Ser Ile Leu Phe Phe Ile Phe Phe Ile Ile Asn
   1               5                   10                  15
   Phe Met Ile Tyr Phe Thr Met Ala Ala Phe Arg Pro Thr Asn Pro Gly
               20                  25                  30
   Pro Ser Pro Ala Ile Gly His Gly Ile Pro Asn Gly Val Pro Gln Pro
           35                  40                  45
   Pro Pro Val Asn Gly His Cys Asn
       50                  55
<210> 376
   <211> 153
   <212> DNA
   <213> Meloidogyne hapla RAR7 cDNA
<400> 376
   atgcctaaaa ttaattctat tttatttttt attcttttta ttattaattt tatgatttat      60
   ttcacaatgg ctggattccg accaacaaat ccaggcaatt cacccggagc tggacatgga     120
   gctccaaatg gaccccaaag tctccacccg taa                                  153
<210> 377
   <211> 50
   <212> PRT
   <213> Meloidogyne hapla RAR7
<400> 377
   Met Pro Lys Ile Asn Ser Ile Leu Phe Phe Ile Leu Phe Ile Ile Asn
   1               5                   10                  15
   Phe Met Ile Tyr Phe Thr Met Ala Gly Phe Arg Pro Thr Asn Pro Gly
               20                  25                  30
   Asn Ser Pro Gly Ala Gly His Gly Ala Pro Asn Gly Pro Gln Ser Leu
           35                  40                  45
   His Pro
       50
<210> 378
   <211> 168
   <212> DNA
   <213> Meloidogyne hapla RAR8 cDNA
<400> 378
   atgactaaaa ttaattctat tatatttttt attattttta taattaattt tatgatttat 60
   caaataatgg ccgctaataa gtcatgtaat accttccgac ccacagctcc gggccattca 120
   cccggaattg gaaattgtag tttaattaaa ttttatttac aaaattaa 168
<210> 379
   <211> 55
   <212> PRT
   <213> Meloidogyne hapla RAR8
<400> 379
   Met Thr Lys Ile Asn Ser Ile Ile Phe Phe Ile Ile Phe Ile Ile Asn
   1 5 10 15
   Phe Met Ile Tyr Gln Ile Met Ala Ala Asn Lys Ser Cys Asn Thr Phe
   20 25 30
   Arg Pro Thr Ala Pro Gly His Ser Pro Gly Ile Gly Asn Cys Ser Leu
   35 40 45
   Ile Lys Phe Tyr Leu Gln Asn
   50 55
<210> 380
   <211> 177
   <212> DNA
   <213> Meloidogyne hapla RAR9 cDNA
<400> 380
   atgactaaaa ttaattctat tatatttttt attattttta ttattaattt tatgatttat 60
   caaataatag cacctcaacc tcctttctgc acaggaccag gccattcacc tggagttgga 120
   catggaattc caaatggact tccatgtaag ccaccagtaa atggacaatg taattaa 177
<210> 381
   <211> 58
   <212> PRT
   <213> Meloidogyne hapla RAR9
<400> 381
   Met Thr Lys Ile Asn Ser Ile Ile Phe Phe Ile Ile Phe Ile Ile Asn
   1 5 10 15
   Phe Met Ile Tyr Gln Ile Ile Ala Pro Gln Pro Pro Phe Cys Thr Gly
   20 25 30
   Pro Gly His Ser Pro Gly Val Gly His Gly Ile Pro Asn Gly Leu Pro
   35 40 45
   Cys Lys Pro Pro Val Asn Gly Gln Cys Asn
   50 55
<210> 382
   <211> 212
   <212> DNA
   <213> Meloidogyne hapla RAR10 cDNA
<400> 382
   atgactaaaa ttaactttct atttatattt ttttatttat ttttgattat taattttatg 60
   atttatcaaa taatagcacc tcaacctcct ttctgcacag gatcaggcca ttcacccgga 120
   gttggacatg gaattccaaa tggacttcca tgtaagccac cagtaaatgg acattgtaat 180
   ttaattaaat tttatttgca aaattatata at 212
<210> 383
   <211> 70
   <212> PRT
   <213> Meloidogyne hapla RAR10
<400> 383
   Met Thr Lys Ile Asn Phe Leu Phe Ile Phe Phe Tyr Leu Phe Leu Ile
   1 5 10 15
   Ile Asn Phe Met Ile Tyr Gln Ile Ile Ala Pro Gln Pro Pro Phe Cys
   20 25 30
   Thr Gly Ser Gly His Ser Pro Gly Val Gly His Gly Ile Pro Asn Gly
   35 40 45
   Leu Pro Cys Lys Pro Pro Val Asn Gly His Cys Asn Leu Ile Lys Phe
   50 55 60
   Tyr Leu Gln Asn Tyr Ile
   65 70
<210> 384
   <211> 183
   <212> DNA
   <213> Meloidogyne hapla RAR11 cDNA
<400> 384
   atgactaaaa ttaattctat tatattttta ttatttttta taattaattt tatgatttat      60
   caaataatgg ctgttaataa ttcagttgat gccttccgac caacagcccc aggccattca     120
   cccggagttg gacattgtaa tttaatttta aaatttattg ccaaaattaa atctctcaaa     180
   taa                                                                   183
<210> 385
   <211> 60
   <212> PRT
   <213> Meloidogyne hapla RAR11
<400> 385
   Met Thr Lys Ile Asn Ser Ile Ile Phe Leu Leu Phe Phe Ile Ile Asn
   1               5                   10                  15
   Phe Met Ile Tyr Gln Ile Met Ala Val Asn Asn Ser Val Asp Ala Phe
               20                  25                  30
   Arg Pro Thr Ala Pro Gly His Ser Pro Gly Val Gly His Cys Asn Leu
           35                  40                  45
   Ile Leu Lys Phe Ile Ala Lys Ile Lys Ser Leu Lys
       50                  55                  60
<210> 386
   <211> 135
   <212> DNA
   <213> Meloidogyne hapla RAR12 cDNA
<400> 386
   atgattaata ttaattctat tatatttttt attattttaa taattaattt tatgatttat      60
   ttgacaatgg caggcaatcc acctttccat actggcactg gccgttcacc cggagctggc     120
   catcattgta tttaa                                                      135
<210> 387
   <211> 44
   <212> PRT
   <213> Meloidogyne hapla RAR12
<400> 387
   Met Ile Asn Ile Asn Ser Ile Ile Phe Phe Ile Ile Leu Ile Ile Asn
   1               5                   10                  15
   Phe Met Ile Tyr Leu Thr Met Ala Gly Asn Pro Pro Phe His Thr Gly
               20                  25                  30
   Thr Gly Arg Ser Pro Gly Ala Gly His His Cys Ile
           35                  40
<210> 388
   <211> 39
   <212> PRT
   <213> Meloidogyne incognita RAR1
<400> 388
   Met Thr Lys Ile Asn Ser Ile Ile Phe Leu Ile Phe Leu Ile Ile Asn
   1               5                   10                  15
   Phe Met Asn Tyr Tyr Ile Ile Ala Asp Val His Pro Asn Asn Pro Gly
               20                  25                  30
   His Ser Pro Gly Ile Gly His
           35
<210> 389
   <211> 39
   <212> PRT
   <213> Meloidogyne incognita RAR2
<400> 389
   Met Thr Lys Ile Asn Ser Ile Ile Phe Leu Ile Phe Leu Ile Ile Asn
   1               5                   10                  15
   Phe Met Asn Tyr Tyr Ile Met Ala Ser Arg Pro Thr Gly Pro Gly His
               20                  25                  30
   Ser Pro Gly Val Gly Asn Ser
           35
<210> 390
   <211> 39
   <212> PRT
   <213> Meloidogyne incognita RAR3
<400> 390
   Met Thr Lys Ile Asn Ser Ile Ile Phe Leu Ile Phe Leu Ile Ile Asn
   1               5                   10                  15
   Phe Met Asn Tyr Tyr Ile Met Ala Ala Phe Arg Pro Thr Asn Pro Gly
               20                  25                  30
   His Ser Pro Gly Val Gly His
           35
<210> 391
   <211> 39
   <212> PRT
   <213> Meloidogyne incognita RAR4
<400> 391
   Met Thr Lys Ile Asn Ser Ile Ile Phe Leu Ile Phe Leu Ile Ile Asn
   1               5                   10                  15
   Phe Met Asn Tyr Tyr Ile Ile Ala Glu Val His Pro Asn Asn Pro Gly
               20                  25                  30
   His Ser Pro Gly Ile Gly His
           35
<210> 392
   <211> 39
   <212> PRT
   <213> Meloidogyne incognita RAR5
<400> 392
   Met Thr Lys Ile Asn Ser Ile Ile Phe Leu Ile Phe Leu Ile Ile Asn
   1               5                   10                  15
   Phe Met Asn Tyr Tyr Ile Ile Ala Ser Arg Pro Thr Gln Pro Gly His
               20                  25                  30
   Ser Pro Gly Val Gly Asn Gly
           35
<210> 393
   <211> 57
   <212> PRT
   <213> Meloidogyne incognita RAR6
<400> 393
   Met Thr Lys Ile Asn Ser Ile Ile Phe Leu Ile Ile Leu Ile Ile Asn
   1 5 10 15
   Phe Met Asn Tyr Tyr Ile Val Ala Gly Thr Arg Ala Thr Glu Pro Gly
   20 25 30
   His Ser Pro Gly Ala Gly His Asp Ala Pro Asn Val Ala Ala His Gly
   35 40 45
   Ala His Gly His Gly Gly Pro Gly Lys
   50 55
<210> 394
   <211> 57
   <212> PRT
   <213> Meloidogyne incognita RAR7
<400> 394
   Met Thr Lys Ile Asn Ser Ile Ile Phe Leu Ile Phe Leu Ile Ile Asn
   1 5 10 15
   Phe Met Asn Tyr Tyr Ile Met Ala Gly Thr Arg Pro Thr Glu Pro Gly
   20 25 30
   His Ser Pro Gly Ala Gly His Asp Ala Pro Asn Val Ala Ala Tyr Gly
   35 40 45
   Ala His Gly His Gly Pro Glu Asn Lys
   50 55
<210> 395
   <211> 43
   <212> PRT
   <213> Meloidogyne incognita RAR8
<400> 395
   Met Ile Lys Ile Asn Ser Lys Ile Ile Phe Leu Leu Phe Leu Leu Ile
   1 5 10 15
   Ile Phe Met Ile Tyr Tyr Thr Met Ala Pro Ala Pro Pro Gly Arg Asn
   20 25 30
   Thr Ala Pro Gly His Ser Pro Gly Ile Gly His
   35 40
<210> 396
   <211> 15
   <212> PRT
   <213> M. hapla RAR1 domain
<400> 396
   Asp Phe Arg Pro Thr Asn Pro Gly His Ser Pro Gly Ile Gly His
   1 5 10 15
<210> 397
   <211> 15
   <212> PRT
   <213> M. hapla RAR2 domain
<400> 397
   Ala Phe Arg Pro Thr Ala Pro Gly His Ser Pro Gly Val Gly His
   1 5 10 15
<210> 398
   <211> 15
   <212> PRT
   <213> M. hapla RAR3 domain
<400> 398
   Gly Tyr Gln Pro Thr Asn Pro Gly His Ser Pro Gly Ile Gly His
   1 5 10 15
<210> 399
   <211> 15
   <212> PRT
   <213> M. hapla RAR4 domain
<400> 399
   Pro Phe Lys Thr Val Pro Gly Gln Ser Ser Pro Gly Val Gly His
   1 5 10 15
<210> 400
   <211> 15
   <212> PRT
   <213> M. hapla RAR5 domain
<400> 400
   Val Ile Lys Pro Ala Cys Ile Gly Asn Ser Pro Gly Val Gly His
   1 5 10 15
<210> 401
   <211> 15
   <212> PRT
   <213> M. hapla RAR6 domain
<400> 401
   Ala Phe Arg Pro Thr Asn Pro Gly Pro Ser Pro Ala Ile Gly His
   1 5 10 15
<210> 402
   <211> 15
   <212> PRT
   <213> M. hapla RAR7 domain
<400> 402
   Gly Phe Arg Pro Thr Asn Pro Gly Asn Ser Pro Gly Ala Gly His
   1 5 10 15
<210> 403
   <211> 15
   <212> PRT
   <213> M. hapla RAR8 domain
<400> 403
   Thr Phe Arg Pro Thr Ala Pro Gly His Ser Pro Gly Ile Gly Asn
   1 5 10 15
<210> 404
   <211> 15
   <212> PRT
   <213> M. hapla RAR9 domain
<400> 404
   Pro Pro Phe Cys Thr Gly Pro Gly His Ser Pro Gly Val Gly His
   1 5 10 15
<210> 405
   <211> 15
   <212> PRT
   <213> M. hapla RAR10 domain
<400> 405
   Pro Pro Phe Cys Thr Gly Ser Gly His Ser Pro Gly Val Gly His
   1 5 10 15
<210> 406
   <211> 15
   <212> PRT
   <213> M. hapla RAR11 domain
<400> 406
   Ala Phe Arg Pro Thr Ala Pro Gly His Ser Pro Gly Val Gly His
   1 5 10 15
<210> 407
   <211> 15
   <212> PRT
   <213> M. hapla RAR12 domain
<400> 407
   Pro Pro Phe His Thr Gly Thr Gly Arg Ser Pro Gly Ala Gly His
   1 5 10 15
<210> 408
   <211> 15
   <212> PRT
   <213> M. incognita RAR1 domain
<400> 408
   Asp Val His Pro Asn Asn Pro Gly His Ser Pro Gly Ile Gly His
   1 5 10 15
<210> 409
   <211> 15
   <212> PRT
   <213> M. incognita RAR2 domain
<400> 409
   Ala Ser Arg Pro Thr Gly Pro Gly His Ser Pro Gly Val Gly Asn
   1 5 10 15
<210> 410
   <211> 15
   <212> PRT
   <213> M. incognita RAR3 domain
<400> 410
   Ala Phe Arg Pro Thr Asn Pro Gly His Ser Pro Gly Val Gly His
   1 5 10 15
<210> 411
   <211> 15
   <212> PRT
   <213> M. incognita RAR4 domain
<400> 411
   Glu Val His Pro Asn Asn Pro Gly His Ser Pro Gly Ile Gly His
   1               5                   10                  15
<210> 412
   <211> 15
   <212> PRT
   <213> M. incognita RAR5 domain
<400> 412
   Ser Arg Pro Thr Gln Pro Gly His Ser Pro Gly Val Gly Asn Gly
   1               5                   10                  15
<210> 413
   <211> 15
   <212> PRT
   <213> M. incognita RAR6 domain
<400> 413
   Gly Thr Arg Ala Thr Glu Pro Gly His Ser Pro Gly Ala Gly His
   1               5                   10                  15
<210> 414
   <211> 15
   <212> PRT
   <213> M. incognita RAR7 domain
<400> 414
   Gly Thr Arg Pro Thr Glu Pro Gly His Ser Pro Gly Ala Gly His
   1               5                   10                  15
<210> 415
   <211> 15
   <212> PRT
   <213> M. incognita RAR8 domain
<400> 415
   Pro Gly Arg Asn Thr Ala Pro Gly His Ser Pro Gly Ile Gly His
   1               5                   10                  15
<210> 416
   <211> 53
   <212> PRT
   <213> M. hapla putative RGF-like peptide
<400> 416
   Met Lys Lys Met Ile Lys Phe Ile Asn Asp Lys Lys Ala Lys Tyr Phe
   1               5                   10                  15
   Ser Leu Asn Glu Asp Tyr Thr Gly Pro Lys His His Gly Pro Arg Asn
               20                  25                  30
   Asn Asp Lys Asp Thr Ser Ser His Lys Asn Val Asn Lys Gly Lys Leu
           35                  40                  45
   Gln Thr Lys Lys Pro
       50
<210> 417
   <211> 68
   <212> PRT
   <213> M. incognita putative RGF-like peptide
<400> 417
   Met Lys Ser Thr Thr Phe Gln Phe Ile Phe Leu Leu Ile Ile Gly Ile
   1               5                   10                  15
   Gln Leu Ile Tyr Asn Leu Asn Asn Val Asp Gly Ala Asn Glu Gln Glu
               20                  25                  30
   Ile Asn Val Asp Lys Ile Lys Gly Asn Asn Asn Gly Asp Gly Gly Ser
           35                  40                  45
   Leu Leu Asn Glu Glu Asp Asp Tyr Ser Ala Pro Asn Lys Lys Pro Pro
       50                  55                  60
   Ile His Asn Pro
   65
<210> 418
   <211> 65
   <212> PRT
   <213> M. hapla putative IDA-like peptide
<400> 418
   Met Phe Lys Leu Ile Ser Leu Ile Ile Ile Ala Phe Leu Phe Leu Ala
   1               5                   10                  15
   Gly Ser Ile Ile Cys Pro Lys Gly Val His Ile Pro Pro Ser Gly Pro
               20                  25                  30
   Ser Lys Asp Ile Pro Cys His Val Asp Pro Tyr Gly Lys Glu Cys Lys
           35                  40                  45
   Asp Phe Cys Lys Asn Asn Arg Asn Asp Lys Leu Cys Lys Lys Gly Gly
       50                  55                  60
   Arg
   65
<210> 419
   <211> 681
   <212> DNA
   <213> M. hapla signal peptidase I cDNA
<400> 419
   atgaagatcc ctgttgccgc ctggctccgc gagaaccgga gcttcctgct gttcgtggct 60
   ggtttcctgt tcttccgcac ggcgatcgcg gattggaatc cggtgcccag cggctcgatg 120
   cggccgacga tcctggaagg cgacgtcgtc ttcgtcgatc ggaccgccta tgactggaaa 180
   gtgccgctga ccgatctgtc gctgcgccgc gtgggcgagc cccggcgcgg ggatgtggtg 240
   accttcagct cgccgcgcga cggcacgcgg ctgatcaagc gcgtggtggc ggtgcccggc 300
   gacgtggtcg agctgcgtgg cgaccgactg atcctcaacg gcgaggcggc gacgtacgcg 360
   ccggtggagg aggtcgcgga ggcactggtc cccggggtcg aggtgcgcgg ggtgcgcgag 420
   gtcgagcaat ggggcggtca ggagcgcatc gtgcagttcc tgcccgcgct gaacgcgccg 480
   cgcgatttcg gtccggtggt ggtgcccgcc gatgcgttct tcatgatggg cgacaaccgg 540
   gacaacagcc aggactcgcg ctacatcggg ctggtgccgc gccggctgat catcgggcag 600
   gcgcatcggg tgctggtgtc ggcggacatc aaggggcgct ggacgccgag atgggatcgt 660
   ttcgccgcgc ccttgaagtg a 681
<210> 420
   <211> 226
   <212> PRT
   <213> M. hapla signal peptidase I
<400> 420
   Met Lys Ile Pro Val Ala Ala Trp Leu Arg Glu Asn Arg Ser Phe Leu
   1 5 10 15
   Leu Phe Val Ala Gly Phe Leu Phe Phe Arg Thr Ala Ile Ala Asp Trp
   20 25 30
   Asn Pro Val Pro Ser Gly Ser Met Arg Pro Thr Ile Leu Glu Gly Asp
   35 40 45
   Val Val Phe Val Asp Arg Thr Ala Tyr Asp Trp Lys Val Pro Leu Thr
   50 55 60
   Asp Leu Ser Leu Arg Arg Val Gly Glu Pro Arg Arg Gly Asp Val Val
   65 70 75 80
   Thr Phe Ser Ser Pro Arg Asp Gly Thr Arg Leu Ile Lys Arg Val Val
   85 90 95
   Ala Val Pro Gly Asp Val Val Glu Leu Arg Gly Asp Arg Leu Ile Leu
   100 105 110
   Asn Gly Glu Ala Ala Thr Tyr Ala Pro Val Glu Glu Val Ala Glu Ala
   115 120 125
   Leu Val Pro Gly Val Glu Val Arg Gly Val Arg Glu Val Glu Gln Trp
   130 135 140
   Gly Gly Gln Glu Arg Ile Val Gln Phe Leu Pro Ala Leu Asn Ala Pro
   145 150 155 160
   Arg Asp Phe Gly Pro Val Val Val Pro Ala Asp Ala Phe Phe Met Met
   165 170 175
   Gly Asp Asn Arg Asp Asn Ser Gln Asp Ser Arg Tyr Ile Gly Leu Val
   180 185 190
   Pro Arg Arg Leu Ile Ile Gly Gln Ala His Arg Val Leu Val Ser Ala
   195 200 205
   Asp Ile Lys Gly Arg Trp Thr Pro Arg Trp Asp Arg Phe Ala Ala Pro
   210 215 220
   Leu Lys
   225
<210> 421
   <211> 15
   <212> PRT
   <213> Artificial
<220>
   <223> Nematode RAR searching sequence
<220>
   <221> misc_feature
   <222> (1)..(1)
   <223> Xaa can be any naturally occurring amino acid
<220>
   <221> misc_feature
   <222> (6)..(6)
   <223> Xaa can be any naturally occurring amino acid
<220>
   <221> misc_feature
   <222> (9)..(9)
   <223> Xaa can be any naturally occurring amino acid
<220>
   <221> misc_feature
   <222> (13)..(13)
   <223> Xaa can be any naturally occurring amino acid
<220>
   <221> misc_feature
   <222> (15)..(15)
   <223> Xaa can be any naturally occurring amino acid
<400> 421
   Xaa Phe Arg Pro Thr Xaa Pro Gly Xaa Ser Pro Gly Xaa Gly Xaa
   1               5                   10                  15
<210> 422
   <211> 32
   <212> DNA
   <213> M. truncatula RAR1 forward primer
<400> 422
   caccatggct tataaatttc aatacacaat ga 32
<210> 423
   <211> 23
   <212> DNA
   <213> M. truncatula RAR1 reverse primer
<400> 423
   tcaatttcca attttgtttt ggt 23
<210> 424
   <211> 22
   <212> DNA
   <213> M. truncatula RAR1 forward PCR primer
<400> 424
   ccgatgaaga tatcgacgtg aa 22
<210> 425
   <211> 29
   <212> DNA
   <213> M. truncatula RAR1 reverse PCR primer
<400> 425
   gaactcattt gtagtatcct cagtcacat 29
<210> 426
   <211> 20
   <212> DNA
   <213> M. truncatula RAR2 forward PCR primer
<400> 426
   tagctcgcat ttgcttgttc 20
<210> 427
   <211> 20
   <212> DNA
   <213> M. truncatula RAR2 reverse PCR primer
<400> 427
   ggctgaatgc tttgtctcaa 20
<210> 428
   <211> 20
   <212> DNA
   <213> M. truncatula RAR3 forward PCR primer
<400> 428
   acgttgagct ccaccatttt 20
<210> 429
   <211> 20
   <212> DNA
   <213> M. truncatula RAR3 reverse PCR primer
<400> 429
   gagcgctcca cctcctatta 20
<210> 430
   <211> 20
   <212> DNA
   <213> M. truncatula RAR4 forward PCR primer
<400> 430
   catggaggtg gtgtttgatg 20
<210> 431
   <211> 20
   <212> DNA
   <213> M. truncatula RAR4 reverse PCR primer
<400> 431
   ttttcgccct acaagtccag 20
<210> 432
   <211> 20
   <212> DNA
   <213> M. truncatula RAR5 forward PCR primer
<400> 432
   gtgttgtttt gagcccaagg 20
<210> 433
   <211> 20
   <212> DNA
   <213> M. truncatula RAR5 reverse PCR primer
<400> 433
   tgttggtcga aaagcttcaa 20
<210> 434
   <211> 20
   <212> DNA
   <213>M. truncatula RAR6 forward PCR primer
<400> 434
   gctcatcatg gagggaagtc 20
<210> 435
   <211> 20
   <212> DNA
   <213> M. truncatula RAR6 reverse PCR primer
<400> 435
   tatgccctgg agatgtaggc 20
<210> 436
   <211> 20
   <212> DNA
   <213> M. truncatula RAR7 forward PCR primer
<400> 436
   ccggatgttg aggtttttgt 20
<210> 437
   <211> 20
   <212> DNA
   <213> M. truncatula RAR7 reverse PCR primer
<400> 437
   ggccaactcc aggactatga 20
<210> 438
   <211> 20
   <212> DNA
   <213> M. truncatula RAR8 forward PCR primer
<400> 438
   tccaacaata ttgccaccaa 20
<210> 439
   <211> 20
   <212> DNA
   <213> M. truncatula RAR8 reverse PCR primer
<400> 439
   gggttgtggg tctaaaagca 20
<210> 440
   <211> 20
   <212> DNA
   <213> M. truncatula RAR9 forward PCR primer
<400> 440
   tgatgccaaa tcatggtgtc 20
<210> 441
   <211> 20
   <212> DNA
   <213> M. truncatula RAR9 reverse PCR primer
<400> 441
   ggactgcttc ctggtgttgt 20
<210> 442
   <211> 21
   <212> DNA
   <213> M. truncatula RAR10 forward PCR primer
<400> 442
   tcaatggaag catcaaggtt t 21
<210> 443
   <211> 20
   <212> DNA
   <213> M. truncatula RAR10 reverse PCR primer
<400> 443
   tatatgtccc accccaagac 20
<210> 444
   <211> 20
   <212> DNA
   <213> M. truncatula RAR11 forward PCR primer
<400> 444
   agctccttcc attggctttt 20
<210> 445
   <211> 19
   <212> DNA
   <213> M. truncatula RAR11 reverse PCR primer
<400> 445
   ccccaccagg actatgacc 19
<210> 446
   <211> 21
   <212> DNA
   <213> M. truncatula NRT2.5 forward PCR primer
<400> 446
   ggagaaggag aaagggtctc a 21
<210> 447
   <211> 21
   <212> DNA
   <213> M. truncatula NRT2.5 reverse PCR primer
<400> 447
   tcagaaggcc tagttgaaat g 21
<210> 448
   <211> 19
   <212> DNA
   <213> M. truncatula AGL1 forward PCR primer
<400> 448
   gaaccgaagg gaagcataa 19
<210> 449
   <211> 20
   <212> DNA
   <213> M. truncatula AGL1 reverse PCR primer
<400> 449
   tgtcgtgcca tacacctttt 20
<210> 450
   <211> 20
   <212> DNA
   <213> M. truncatula LBD38 forward PCR primer
<400> 450
   gccacgctac tgttttcgta 20
<210> 451
   <211> 20
   <212> DNA
   <213> M. truncatula LBD38 reverse PCR primer
<400> 451
   gagctggtct ctgtggttca 20
<210>452
   <211> 22
   <212> DNA
   <213> M. hapla Rar10 forward PCR primer
<400> 452
   gcacctcaac ctcctttctg ca 22
<210> 453
   <211> 26
   <212> DNA
   <213> M. hapla Rar10 reverse PCR primer
<400> 453
   tgtccattta ctggtggctt acatgg 26
<210> 454
   <211> 21
   <212> DNA
   <213> M. truncatula UBQ10 forward PCR primer
<400> 454
   aacttgttgc atgggtcttg a 21
<210> 455
   <211> 30
   <212> DNA
   <213> M. truncatula UBQ10 reverse PCR primer
<400> 455
   cattaagttt gacaaagaga aagagacaga 30
<210> 456
   <211> 15
   <212> PRT
   <213> M. truncatula RAR1 conserved domain #1
<220>
   <221> hydroxyproline
   <222> (4)..(4)
<220>
   <221> hydroxyproline
   <222> (11)..(11)
<400> 456
   Ala Phe Gln Pro Thr Thr Pro Gly Asn Ser Pro Gly Val Gly His
   1               5                   10                  15
<210> 457
   <211> 15
   <212> PRT
   <213> M. truncatula RAR1 conserved domain #2
<220>
   <221> hydroxyproline
   <222> (11)..(11)
<400> 457
   Glu Phe Gln Lys Thr Asn Pro Gly His Ser Pro Gly Val Gly His
   1               5                   10                  15
<210> 458
   <211> 19
   <212> PRT
   <213> M. hapla RAR2 conserved domain #1
<220>
   <221> hydroxyproline
   <222> (4)..(4)
<220>
   <221> hydroxyproline
   <222>(11)..(11)
<400> 458
   Ala Phe Arg His Tyr Pro Thr Ala Pro Gly His Ser His Tyr Pro Gly
   1               5                   10                  15
   Val Gly His
<210> 459
   <211> 14
   <212> PRT
   <213> RKN and plant RAR peptide consensus sequence
<220>
   <221> D, A, P, G, S, V, E, P, T, Q, I, N, K, C,
   <222> (1)..(1)
   <223> N-terminal additional amino acid
<220>
   <221> F, V, T, S, A, K, Y, R, G, I, Q, H, D, G, W
   <222> (1)..(1)
<220>
   <221> misc_feature
   <222> (1)..(5)
   <223> Xaa can be any naturally occurring amino acid
<220>
   <221> R, A, Q, G, E, V, D, K, P, S, H, Q
   <222> (2)..(2)
<220>
   <221> P, G, S, T, A, N, C, H, V, E, Y, K
   <222> (3).. (3)
<220>
   <221> T, S, A, P, G, V, N, M, I,
   <222> (4)..(4)
<220>
   <221> T, N, A, G, P, D, K, S, V, Y, Q, E, C, H
   <222> (5).. (5)
<220>
   <221> H, N, S, Y, P, R, T, G, V, F, Q, D,
   <222> (8)..(8)
<220>
   <221> misc_feature
   <222> (8)..(8)
   <223> Xaa can be any naturally occurring amino acid
<220>
   <221> I, V, A
   <222> (12)..(12)
<220>
   <221> misc_feature
   <222> (12)..(12)
   <223> Xaa can be any naturally occurring amino acid
<220>
   <221> H, N
   <222> (14)..(14)
<220>
   <221> misc_feature
   <222> (14)..(14)
   <223> Xaa can be any naturally occurring amino acid
<400> 459
   Xaa Xaa Xaa Xaa Xaa Pro Gly Xaa Ser Pro Gly Xaa Gly Xaa
   1               5                   10
<210> 460
   <211> 13
   <212> PRT
   <213> M. hapla RGF peptide domain
<400> 460
   Asp Tyr Thr Gly Pro Lys His His Gly Pro Arg Asn Asn
   1               5                   10
<210> 461
   <211> 14
   <212> PRT
   <213> M. incognita RGF peptide domain
<400> 461
   Asp Tyr Ser Ala Pro Asn Lys Lys Pro Pro Ile His Asn Pro
   1               5                   10
<210> 462
   <211> 13
   <212> PRT
   <213> Selaginella moellendorffii RGF peptide domain
<400> 462
   Asp Tyr Thr Pro Val His Arg Lys Pro Pro Ile Asn Asn
   1               5                   10
<210> 463
   <211> 14
   <212> PRT
   <213> Gymnosperm RGF peptide domain
<400> 463
   Asp Tyr Ser Thr Pro Gly Asp Glu Pro Asp Arg Gln Lys Asn
   1 5 10
<210> 464
   <211> 116
   <212> PRT
   <213> Arabidopsis thaliana RGF1
<400> 464
   Met Val Ser Ile Arg Val Ile Cys Tyr Leu Leu Val Phe Ser Val Leu
   1 5 10 15
   Gln Val His Ala Lys Val Ser Asn Ala Asn Phe Asn Ser Gln Ala Pro
   20 25 30
   Gln Met Lys Asn Ser Glu Gly Leu Gly Ala Ser Asn Gly Thr Gln Ile
   35 40 45
   Ala Lys Lys His Ala Glu Asp Val Ile Glu Asn Arg Lys Thr Leu Lys
   50 55 60
   His Val Asn Val Lys Val Glu Ala Asn Glu Lys Asn Gly Leu Glu Ile
   65 70 75 80
   Glu Ser Lys Glu Met Val Lys Lys Arg Lys Asn Lys Lys Arg Leu Thr
   85 90 95
   Lys Thr Glu Ser Leu Thr Ala Asp Tyr Ser Asn Pro Gly His His Pro
   100 105 110
   Pro Arg His Asn
   115
<210> 465
   <211> 109
   <212> PRT
   <213> Arabidopsis thaliana RGF2
<400> 465
   Met Thr Asn Ile Thr Ser Ser Phe Leu Cys Leu Leu Ile Leu Leu Leu
   1 5 10 15
   Phe Cys Leu Ser Phe Gly Tyr Ser Leu His Gly Asp Lys Asp Glu Val
   20 25 30
   Leu Ser Val Asp Val Gly Ser Asn Ala Lys Val Met Lys His Leu Asp
   35 40 45
   Gly Asp Asp Ala Met Lys Lys Ala Gln Val Arg Gly Arg Ser Gly Gln
   50 55 60
   Glu Phe Ser Lys Glu Thr Thr Lys Met Met Met Lys Lys Thr Thr Lys
   65 70 75 80
   Lys Glu Thr Asn Val Glu Glu Glu Asp Asp Leu Val Ala Tyr Thr Ala
   85 90 95
   Asp Tyr Trp Lys Pro Arg His His Pro Pro Lys Asn Asn
   100 105
<210> 466
   <211> 110
   <212> PRT
   <213> Arabidopsis thaliana RGF3
<400> 466
   Met Thr Thr Leu Ser Lys Ile Leu Cys Val Leu Ile Ile Leu Leu Leu
   1 5 10 15
   Cys Ser Phe Phe Arg Tyr Ser Leu His Glu Asp Gly Asn Gln Gln Ser
   20 25 30
   Ser Arg Asp Phe Val Ser Thr Ala Lys Ala Ile Lys Tyr Gly Asp Val
   35 40 45
   Met Lys Lys Met Ile Arg Gly Arg Lys Leu Met Met Ala Ser Gly Glu
   50 55 60
   Lys Glu Glu Ala Glu Thr Lys Met Lys Arg Gly Asn Arg Glu Thr Glu
   65 70 75 80
   Arg Asn Ser Ser Lys Ser Val Glu Glu Asp Gly Leu Val Ala Tyr Thr
   85 90 95
   Ala Asp Tyr Trp Arg Ala Lys His His Pro Pro Lys Asn Asn
   100 105 110
<210> 467
   <211> 163
   <212> PRT
   <213> Arabidopsis thaliana RGF4
<400> 467
   Met Met Arg Phe Thr Ile Ile Val Ile Ala Phe Leu Leu Ile Ile Gln
   1 5 10 15
   Ser Leu Glu Glu Glu His Ile Leu Val Tyr Ala His Glu Gly Gly Glu
   20 25 30
   Ala Gly His Lys Ser Leu Asp Tyr Gln Gly Asp Gln Asp Ser Ser Thr
   35 40 45
   Leu His Pro Lys Glu Leu Phe Asp Ala Pro Arg Lys Val Arg Phe Gly
   50 55 60
   Arg Thr Thr Arg Ala Glu Lys Glu Gln Val Thr Ala Met Asn Asn Asp
   65 70 75 80
   Ser Trp Ser Phe Lys Ile Ser Gly Glu His Lys Gln Thr Asn Ile Leu
   85 90 95
   Ala Asp His Asp Thr Thr Lys Asn Thr Phe Cys Lys Lys Met Met Ile
   100 105 110
   Ile Val Asn Asp Leu Thr Ser Leu Pro Thr Leu Glu Pro Ser Thr Ser
   115 120 125
   Thr Asn Asp Met Glu Lys Leu Ala Arg Leu Leu Arg Asp Asp Tyr Pro
   130 135 140
   Ile Tyr Ser Lys Pro Arg Arg Lys Pro Pro Val Asn Asn Arg Ala Pro
   145 150 155 160
   Asp Lys Phe
<210> 468
   <211> 88
   <212> PRT
   <213> Arabidopsis thaliana RGF5
<400> 468
   Met Ser Ser Ile His Val Ala Ser Met Ile Leu Leu Leu Phe Leu Phe
   1               5                   10                  15
   Leu His His Ser Asp Ser Arg His Leu Asp Asn Val His Ile Thr Ala
               20                  25                  30
   Ser Arg Phe Ser Leu Val Lys Asp Gln Asn Val Val Ser Ser Ser Thr
           35                  40                  45
   Ser Lys Glu Pro Val Lys Val Ser Arg Phe Val Pro Gly Pro Leu Lys
       50                  55                  60
   His His His Arg Arg Pro Pro Leu Leu Phe Ala Asp Tyr Pro Lys Pro
   65                  70                  75                  80
   Ser Thr Arg Pro Pro Arg His Asn
                   85
<210> 469
   <211> 86
   <212> PRT
   <213> Arabidopsis thaliana RGF6
<400> 469
   Met Ser Cys Ser Leu Arg Ser Gly Leu Val Ile Val Phe Cys Phe Ile
   1               5                   10                  15
   Leu Leu Leu Leu Ser Ser Asn Val Gly Cys Ala Ser Ala Ala Arg Arg
               20                  25                  30
   Leu Arg Ser His Lys His His His His Lys Val Ala Ser Leu Asp Val
           35                  40                  45
   Phe Asn Gly Gly Glu Arg Arg Arg Ala Leu Gly Gly Val Glu Thr Gly
       50                  55                  60
   Glu Glu Val Val Val Met Asp Tyr Pro Gln Pro His Arg Lys Pro Pro
   65                  70                  75                  80
   Ile His Asn Glu Lys Ser
                   85
<210> 470
   <211> 102
   <212> PRT
   <213> Arabidopsis thaliana RGF7
<400> 470
   Met Glu Met Lys Lys Trp Ser Tyr Ala Asn Leu Ile Thr Leu Ala Leu
   1 5 10 15
   Leu Phe Leu Phe Phe Ile Ile Leu Leu Leu Ala Phe Gln Gly Gly Ser
   20 25 30
   Arg Asp Asp Asp His Gln His Val His Val Ala Ile Arg Thr Lys Asp
   35 40 45
   Ile Ser Met Gly Arg Lys Leu Lys Ser Leu Lys Pro Ile Asn Pro Thr
   50 55 60
   Lys Lys Asn Gly Phe Glu Tyr Pro Asp Gln Gly Ser His Asp Val Gln
   65 70 75 80
   Glu Arg Glu Val Tyr Val Glu Leu Arg Asp Tyr Gly Gln Arg Lys Tyr
   85 90 95
   Lys Pro Pro Val His Asn
   100
<210> 471
   <211> 123
   <212> PRT
   <213> Arabidopsis thaliana RGF8
<400> 471
   Met Lys Leu Ile Arg Val Thr Leu Phe Leu Cys Ala Leu Ala Ile Leu
   1 5 10 15
   Leu Leu Val Thr Pro Thr Ser Ser Leu Gln Leu Lys His Pro Tyr Ser
   20 25 30
   Ser Pro Ser Gln Gly Leu Ser Lys Lys Ile Val Thr Lys Met Ala Thr
   35 40 45
   Arg Lys Leu Met Ile Ile Ser Ser Glu Tyr Val Met Thr Ser Thr Ser
   50 55 60
   His Glu Gly Ser Ser Glu Gln Leu Arg Val Thr Ser Ser Gly Lys Ser
   65 70 75 80
   Lys Asp Glu Glu Lys Lys Leu Ser Glu Glu Glu Glu Glu Lys Lys Ala
   85 90 95
   Leu Ala Lys Tyr Leu Ser Met Asp Tyr Arg Thr Phe Arg Arg Arg Arg
   100 105 110
   Pro Val His Asn Lys Ala Leu Pro Leu Asp Pro
   115 120
<210> 472
   <211> 79
   <212> PRT
   <213> Arabidopsis thaliana RGF9
<400> 472
   Met Ala Ile Arg Val Ser His Lys Ser Phe Leu Val Ala Leu Leu Leu
   1               5                   10                  15
   Ile Leu Phe Ile Ser Ser Pro Thr Gln Ala Arg Ser Leu Arg Glu Val
               20                  25                  30
   Val Arg Asn Arg Thr Leu Leu Val Val Glu Lys Ser Gln Glu Ser Arg
           35                  40                  45
   Lys Ile Arg His Glu Gly Gly Gly Ser Asp Val Asp Gly Leu Met Asp
       50                  55                  60
   Met Asp Tyr Asn Ser Ala Asn Lys Lys Arg Pro Ile His Asn Arg
   65                  70                  75
<210> 473
   <211> 53
   <212> PRT
   <213> Meloidogyne hapla RGF1
<400> 473
   Met Lys Lys Met Ile Lys Phe Ile Asn Asp Lys Lys Ala Lys Tyr Phe
   1               5                   10                  15
   Ser Leu Asn Glu Asp Tyr Thr Gly Pro Lys His His Gly Pro Arg Asn
               20                  25                  30
   Asn Asp Lys Asp Thr Ser Ser His Lys Asn Val Asn Lys Gly Lys Leu
           35                  40                  45
   Gln Thr Lys Lys Pro
       50
<210> 474
   <211> 68
   <212> PRT
   <213> Meloidogyne incognita RGF1
<400> 474
   Met Lys Ser Thr Thr Phe Gln Phe Ile Phe Leu Leu Ile Ile Gly Ile
   1               5                   10                  15
   Gln Leu Ile Tyr Asn Leu Asn Asn Val Asp Gly Ala Asn Glu Gln Glu
               20                  25                  30
   Ile Asn Val Asp Lys Ile Lys Gly Asn Asn Asn Gly Asp Gly Gly Ser
           35                  40                  45
   Leu Leu Asn Glu Glu Asp Asp Tyr Ser Ala Pro Asn Lys Lys Pro Pro
       50                  55                  60
   Ile His Asn Pro
   65
<210> 475
   <211> 13
   <212> PRT
   <213> Arabidopsis thaliana RGF1 domain
<400> 475
   Asp Tyr Ser Asn Pro Gly His His Pro Pro Arg His Asn
   1 5 10
<210> 476
   <211> 13
   <212> PRT
   <213> Arabidopsis thaliana RGF2 domain
<400> 476
   Asp Tyr Trp Lys Pro Arg His His Pro Pro Lys Asn Asn
   1 5 10
<210> 477
   <211> 13
   <212> PRT
   <213> Arabidopsis thaliana RGF3 domain
<400> 477
   Asp Tyr Trp Arg Ala Lys His His Pro Pro Lys Asn Asn
   1 5 10
<210> 478
   <211> 15
   <212> PRT
   <213> Arabidopsis thaliana RGF4 domain
<400> 478
   Asp Tyr Pro Ile Tyr Ser Lys Pro Arg Arg Lys Pro Pro Val Asn
   1 5 10 15
<210> 479
   <211> 13
   <212> PRT
   <213> Arabidopsis thaliana RGF5 domain
<400> 479
   Asp Tyr Pro Lys Pro Ser Thr Arg Pro Pro Arg His Asn
   1 5 10
<210> 480
   <211> 13
   <212> PRT
   <213> Arabidopsis thaliana RGF6 domain
<400> 480
   Asp Tyr Pro Gln Pro His Arg Lys Pro Pro Ile His Asn
   1 5 10
<210> 481
   <211> 13
   <212> PRT
   <213> Arabidopsis thaliana RGF7 domain
<400> 481
   Asp Tyr Gly Gln Arg Lys Tyr Lys Pro Pro Val His Asn
   1 5 10
<210> 482
   <211> 13
   <212> PRT
   <213> Arabidopsis thaliana RGF8 domain
<400> 482
   Asp Tyr Arg Thr Phe Arg Arg Arg Arg Pro Val His Asn
   1 5 10
<210> 483
   <211> 13
   <212> PRT
   <213> Arabidopsis thaliana RGF9 domain
<400> 483
   Asp Tyr Asn Ser Ala Asn Lys Lys Arg Pro Ile His Asn
   1 5 10
<210> 484
   <211> 77
   <212> PRT
   <213> Arabidopsis thaliana IDA
<400> 484
   Met Ala Pro Cys Arg Thr Met Met Val Leu Leu Cys Phe Val Leu Phe
   1               5                   10                  15
   Leu Ala Ala Ser Ser Ser Cys Val Ala Ala Ala Arg Ile Gly Ala Thr
               20                  25                  30
   Met Glu Met Lys Lys Asn Ile Lys Arg Leu Thr Phe Lys Asn Ser His
           35                  40                  45
   Ile Phe Gly Tyr Leu Pro Lys Gly Val Pro Ile Pro Pro Ser Ala Pro
       50                  55                  60
   Ser Lys Arg His Asn Ser Phe Val Asn Ser Leu Pro His
   65                  70                  75
<210> 485
   <211> 86
   <212> PRT
   <213> Arabidopsis thaliana IDL1
<400> 485
   Met Asn Leu Ser His Lys Thr Met Phe Met Thr Leu Tyr Ile Val Phe
   1               5                   10                  15
   Leu Leu Ile Phe Gly Ser Tyr Asn Ala Thr Ala Arg Ile Gly Pro Ile
               20                  25                  30
   Lys Leu Ser Glu Thr Glu Ile Val Gln Thr Arg Ser Arg Gln Glu Ile
           35                  40                  45
   Ile Gly Gly Phe Thr Phe Lys Gly Arg Val Phe His Ser Phe Ser Lys
       50                  55                  60
   Arg Val Leu Val Pro Pro Ser Gly Pro Ser Met Arg His Asn Ser Val
   65                  70                  75                  80
   Val Asn Asn Leu Lys His
                   85
<210> 486
   <211> 95
   <212> PRT
   <213> Arabidopsis thaliana IDL2
<400> 486
   Met Ser Ser Arg Asn Gln Arg Ser Arg Ile Thr Ser Ser Phe Phe Val
   1 5 10 15
   Ser Phe Phe Thr Arg Thr Ile Leu Leu Leu Leu Ile Leu Leu Leu Gly
   20 25 30
   Phe Cys Asn Gly Ala Arg Thr Asn Thr Asn Val Phe Asn Ser Lys Pro
   35 40 45
   His Lys Lys His Asn Asp Ala Val Ser Ser Ser Thr Lys Gln Phe Leu
   50 55 60
   Gly Phe Leu Pro Arg His Phe Pro Val Pro Ala Ser Gly Pro Ser Arg
   65 70 75 80
   Lys His Asn Asp Ile Gly Leu Leu Ser Trp His Arg Ser Ser Pro
   85 90 95
<210> 487
   <211> 99
   <212> PRT
   <213> Arabidopsis thaliana IDL3
<400> 487
   Met Ser Ser Arg Ser His Arg Ser Arg Lys Tyr Gln Leu Thr Arg Thr
   1 5 10 15
   Ile Pro Ile Leu Val Leu Leu Leu Val Leu Leu Ser Cys Cys Asn Gly
   20 25 30
   Ala Arg Thr Thr Asn Val Phe Asn Thr Ser Ser Pro Pro Lys Gln Lys
   35 40 45
   Asp Val Val Ser Pro Pro His Asp His Val His His Gln Val Gln Asp
   50 55 60
   His Lys Ser Val Gln Phe Leu Gly Ser Leu Pro Arg Gln Phe Pro Val
   65 70 75 80
   Pro Thr Ser Gly Pro Ser Arg Lys His Asn Glu Ile Gly Leu Ser Ser
   85 90 95
   Thr Lys Thr
<210> 488
   <211> 93
   <212> PRT
   <213> Arabidopsis thaliana IDL4
<400> 488
   Met Tyr Pro Thr Arg Pro His Tyr Trp Arg Arg Arg Leu Ser Ile Asn
   1 5 10 15
   Arg Pro Gln Ala Phe Leu Leu Leu Ile Leu Cys Leu Phe Phe Ile His
   20 25 30
   His Cys Asp Ala Ser Arg Phe Ser Ser Ser Ser Val Phe Tyr Arg Asn
   35 40 45
   Pro Asn Tyr Asp His Ser Asn Asn Thr Val Arg Arg Gly His Phe Leu
   50 55 60
   Gly Phe Leu Pro Arg His Leu Pro Val Pro Ala Ser Ala Pro Ser Arg
   65 70 75 80
   Lys His Asn Asp Ile Gly Ile Gln Ala Leu Leu Ser Pro
   85 90
<210> 489
   <211> 111
   <212> PRT
   <213> Arabidopsis thaliana IDL5
<400> 489
   Met Gly Asn Lys Arg Ile Lys Ala Met Met Ile Leu Val Val Met Ile
   1 5 10 15
   Met Met Val Phe Ser Trp Arg Ile Cys Glu Ala Asp Ser Leu Arg Arg
   20 25 30
   Tyr Ser Ser Ser Ser Arg Pro Gln Arg Phe Phe Lys Val Arg Arg Pro
   35 40 45
   Asn Pro Arg Asn His His His Gln Asn Gln Gly Phe Asn Gly Asp Asp
   50 55 60
   Tyr Pro Pro Glu Ser Phe Ser Gly Phe Leu Pro Lys Thr Leu Pro Ile
   65 70 75 80
   Pro His Ser Ala Pro Ser Arg Lys His Asn Val Tyr Gly Leu Gln Arg
   85 90 95
   Thr Asn Ser Arg Ser Ser Thr Phe Trp Thr His Ala His Leu Ser
   100 105 110
<210> 490
   <211> 20
   <212> PRT
   <213> A. thaliana IDA domain
<400> 490
   Phe Gly Tyr Leu Pro Lys Gly Val Pro Ile Pro Pro Ser Ala Pro Ser
   1 5 10 15
   Lys Arg His Asn
   20
<210> 491
   <211> 21
   <212> PRT
   <213> A. thaliana IDL1 domain
<400> 491
   Val Phe His Ser Phe Ser Lys Arg Val Leu Val Pro Pro Ser Gly Pro
   1 5 10 15
   Ser Met Arg His Asn
   20
<210> 492
   <211> 21
   <212> PRT
   <213> A. thaliana IDL2 domain
<400> 492
   Phe Leu Gly Phe Leu Pro Arg His Phe Pro Val Pro Ala Ser Gly Pro
   1 5 10 15
   Ser Arg Lys His Asn
   20
<210> 493
   <211> 21
   <212> PRT
   <213> A. thaliana IDL3 domain
<400> 493
   Phe Leu Gly Ser Leu Pro Arg Gln Phe Pro Val Pro Thr Ser Gly Pro
   1 5 10 15
   Ser Arg Lys His Asn
   20
<210> 494
   <211> 21
   <212> PRT
   <213> A. thaliana IDL4 domain
<400> 494
   Phe Leu Gly Phe Leu Pro Arg His Leu Pro Val Pro Ala Ser Ala Pro
   1 5 10 15
   Ser Arg Lys His Asn
   20
<210> 495
   <211> 21
   <212> PRT
   <213> A. thaliana IDL5 domain
<400> 495
   Phe Ser Gly Phe Leu Pro Lys Thr Leu Pro Ile Pro His Ser Ala Pro
   1 5 10 15
   Ser Arg Lys His Asn
   20
<210> 496
   <211> 17
   <212> PRT
   <213> M. hapla IDL1 domain
<400> 496
   Cys Pro Lys Gly Val His Ile Pro Pro Ser Gly Pro Ser Lys Asp Ile
   1 5 10 15
   Pro

## Description

### Field of the Invention

The present invention relates to methods for modulating the architecture of plant roots and thereby modulating the ability of plants to take up nutrients and/or water from soil - as well as to plants obtainable from such methods as defined in the claims. Further contemplated are methods and materials for treating or preventing root knot nematode attack of plant roots.

### Background to the Invention

Root architecture is an important agronomic trait governed by a poorly understood integration of nascent lateral organ development programs with nutritional and environmental parameters. To effectively adapt to variations in the soil environment, root systems evolved two important features: developmental plasticity and a wide range of molecular systems to sense and respond to various soil parameters. The availability of nitrogen is a major determinant of root architecture. A complex but poorly understood interplay occurs between environmental cues and the innate root development pathways. Collectively this interplay dictates the optimization of root architecture and influences plant productivity and fitness. Exemplary peptides in the art are disclosed in Oelkers et al, BMC Plant Biology, 8(1), p.1, on bioinformatic analysis of the CLE signaling peptide family, in EP1033405, as well as via http://www.uniprot.org/uniprot/Q3EBM6 and http://www.uniprot.org/uniprot/B3H5A9, which relate to Arabidopsis thaliana proteins At2g35612 and At5g66816, respectively.

There is therefore a need for a better understanding of root architecture and factors involved in this to develop plants with root architecture allowing for improved or more efficient nutrient uptake, or for otherwise better adapting plants to the environment they are to be grown in.

### Summary of the Invention

The present investigations have shown that the root architecture of plants, including nodule formation and competency thereof, can be modulated by a family of peptides herein referred to as root architecture regulatory (RAR) peptides. It has also been found that root knot nematodes (RKNs) also express and secrete peptides of this family, thereby affecting the architecture of affected roots. This knowledge may provide a route for controlling the effects of root knot nematode attack on plant roots. Furthermore, in the course of these studies it has also been found that root knot nematodes also express and secrete other plant-like regulatory peptides, including root growth factors (RGFs), CLV3/ESR-related peptides (CLEs) and Inflorescence Deficient in Abscission peptides (IDAs).

Generally, the present invention provides the subject-matter as defined in the claims.

According to an aspect of the invention, the present invention provides a method for reducing lateral root development by a plant relative to an untreated or wild-type plant, comprising contacting the root zone of said plant with at least one RAR or introducing at least one exogenous RAR-encoding nucleic acid into one or more plant cells which results in increased RAR expression by cells of a plant regenerated from or comprising said one or more plant cells, wherein said RAR is not an *Arabidopsis thaliana* CEP1 peptide and said RAR-encoding nucleic acid is not an *Arabidopsis* CEP 1-encoding sequence, and wherein said RAR comprises an RAR domain comprising an amino acid sequence (X₁)ₙX₂X₃X₄X₅X₆PGX₉SPGX₁₃GX₁₅ (SEQ ID NO: 459), wherein n may be 1 or 0.

According to another aspect, the present invention provides a method for promoting lateral root growth and development of a plant relative to an untreated or wild-type plant, comprising introducing at least one mutation in (a) one or more RAR-encoding genes or upstream sequences thereof; or (b) one or more RAR receptor-encoding genes or upstream sequences; into one or more plant cells which at least one mutation results in:
(i) decreased expression of one or more RARs, decreased expression of one or more RAR receptors, or decreased expression of one or more RARs and one or more RAR receptors by cells of a plant regenerated from or comprising said one or more plant cells, wherein said decreased expression of said RAR(s) or RAR receptor(s) occurs under conditions which would otherwise promote expression of said RAR(s) or RAR receptor(s); or
(ii) reduced affinity of one or more RARs for their respective RAR receptors, which reduced affinity arises through modifications in the RAR(s), RAR receptor(s) or in both expressed RAR(s) and RAR receptor(s) expressed by cells of a plant regenerated from or comprising said one or more plant cells;
and wherein said RAR comprises an RAR domain comprising an amino acid sequence (X₁)ₙX₂X₃X₄X₅X₆PGX₉SPGX₁₃GX₁₅ (SEQ ID NO: 459), wherein n may be 1 or 0.

Further described is a method for inhibiting modulation of the root architecture of a plant by the action of a root knot nematode, comprising: contacting the root zone of a plant with a root knot nematode RAR signaling antagonist or binding agent; introducing into one or more plant cells at least one exogenous nucleic acid which suppresses, or the product of which suppresses expression of a root knot nematode RAR-encoding nucleic acid (such as a microRNA or siRNA or a microRNA-encoding or siRNA-encoding sequence); introducing into one or more plant cells at least one mutation or exogenous nucleic acid which results in modulated affinity of one or more root knot nematode RARs for plant RAR receptors, which modulated affinity arises through modifications in the plant RAR receptor(s); or introducing into one or more plant cells one or more exogenous nucleic acids which results in expression by said cell(s) of one or more root knot nematode RAR binding agents or antagonists.

Further described is a method for inhibiting modulation of the root architecture of a plant by the action of a root knot nematode, comprising: contacting the root zone of a plant with a root knot nematode RGF signaling antagonist or binding agent; introducing into one or more plant cells at least one exogenous nucleic acid which suppresses, or the product of which suppresses expression of a root knot nematode RGF-encoding nucleic acid (such as a microRNA or siRNA or a microRNA-encoding or siRNA-encoding sequence); introducing into one or more plant cells at least one mutation or exogenous nucleic acid which results in modulated affinity of one or more root knot nematode RGFs for plant RGF receptors, which modulated affinity arises through modifications in the plant RGF receptor(s); or introducing into one or more plant cells one or more exogenous nucleic acids which results in expression by said cell(s) of one or more root knot nematode RGF binding agents or antagonists.

In view of the discovery that RKNs express a number of plant signaling molecules, including RARs, RGFs, IDAs and CLEs, and appear to deploy them to subvert normal plant root growth to their needs, it is postulated that another method for controlling damage by RKNs to plants may comprise inhibiting expression of peptidases or proteases responsible for cleaving signal peptides from the translated pre-secretion precursors of the regulatory proteins/peptides secreted by RKNs.

There is therefore further contemplated a method for inhibiting modulation of the root architecture of a plant by the action of a root knot nematode, said method comprising introducing at least one exogenous nucleic acid into one or more plant cells which suppresses, or the product of which suppresses expression of a component of a root knot nematode signal peptidase complex.

Plants as defined in the claims obtained by the methods outlined above, and respective plant parts (including leaves, stems, roots, tubers, flowers, fruit, seeds and parts thereof) are also provided by the invention. Accordingly, in another aspect, the invention provides a plant with altered horizontal or vertical root system growth pattern relative to a wild-type plant, or a part of said plant, wherein said plant or part thereof comprises at least one exogenous RAR-encoding nucleic acid, and wherein said plant or part thereof is obtainable by the methods as defined in the claims.

Further described is an isolated root architecture regulator peptide (RAR) or an analogue thereof, with the proviso that said RAR is not an *Arabidopsis* CEP1 peptide and said RAR-encoding nucleic acid is not an *Arabidopsis* CEP1 encoding sequence. The RAR may be a root knot nematode RAR

Further disclosed is an isolated polynucleotide comprising a nucleotide sequence coding for a RAR, with the proviso that said polynucleotide is not the *Arabidopsis* CEP1 encoding sequence, or a portion thereof. The polynucleotide may also comprise a secretion signal peptide sequence. The RAR-encoding sequence may be a root knot nematode RAR-encoding sequence.

Further disclosed is an isolated polynucleotide comprising a nucleotide sequence coding for a root knot nematode RGF, or a portion thereof.

Also provided, used or disclosed, respectively, are vectors, transgenic cells, including plant cells, and transgenic plants, and seeds thereof comprising polynucleotides in context with the invention.

Further described are methods for screening plants or root not nematodes for RARs, RAR receptor(s) and encoding sequences, and isolation of such peptides and encoding sequences. Further contemplated are methods for screening root knot nematodes for RGFs and encoding sequences, and isolation of such peptides and encoding sequences. Such screening methods may identify additional RARs, RGFs, as well as RAR or RGF receptors, encoding sequences and associated regulatory regions with altered properties (including altered affinities or altered nutrient responses).

Screening methods may comprise the use of polyclonal or monoclonal antibodies raised against one or more of the RARs or RGFs disclosed herein, labeled RAR(s) and RGF(s), or oligonucleotide probes or primer pairs based on one or more of the RAR-encoding or RGF-encoding sequences disclosed herein.

### Brief Description of the Drawings

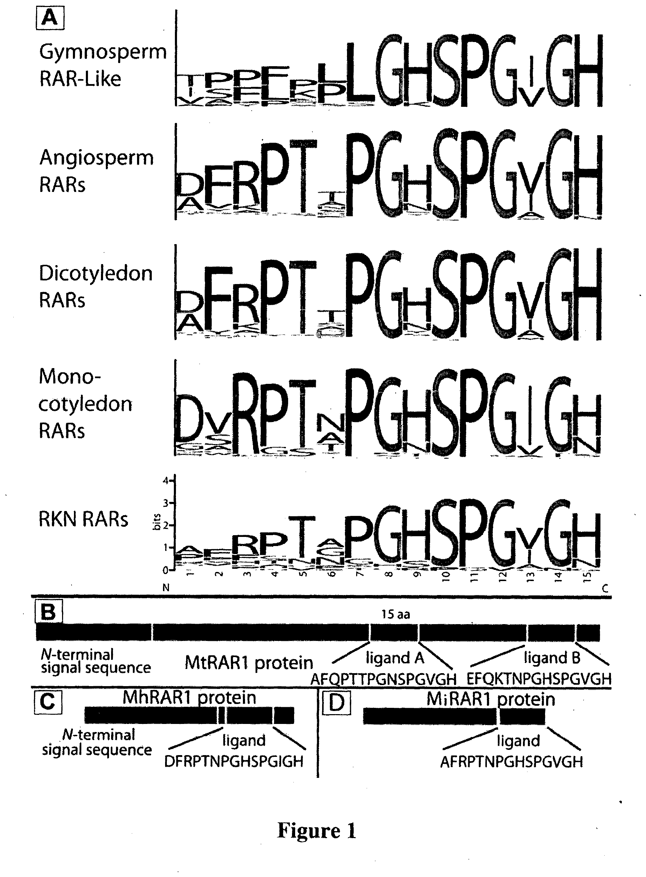

**Figure** 1 - RAR peptide ligands occur in higher plants and RKNs. (A) Weblogo plots show the 15 AA RAR peptides each with particularly strong C-terminal conservation. Angiosperm and RKN RAR peptides show strong similarity. Gymnosperm RAR-like peptides exhibit divergence at the amino-terminus with a highly conserved leucine instead of proline at position 7. Unlike dicots, monocot RARs do not contain F at position 2. (B) Putative MtRAR1 protein sequence. The amino-terminal signal sequence is blue and the conserved RAR peptides are red. Non-conserved sequences are green. (C, D) Putative MhRAR1 and MiRAR1 proteins. Two forms of RARs exist: the first has sequences flanking the RAR peptide (e.g. MhRARl, C) and the second has no flanking sequences (e.g. MiRARl; D).

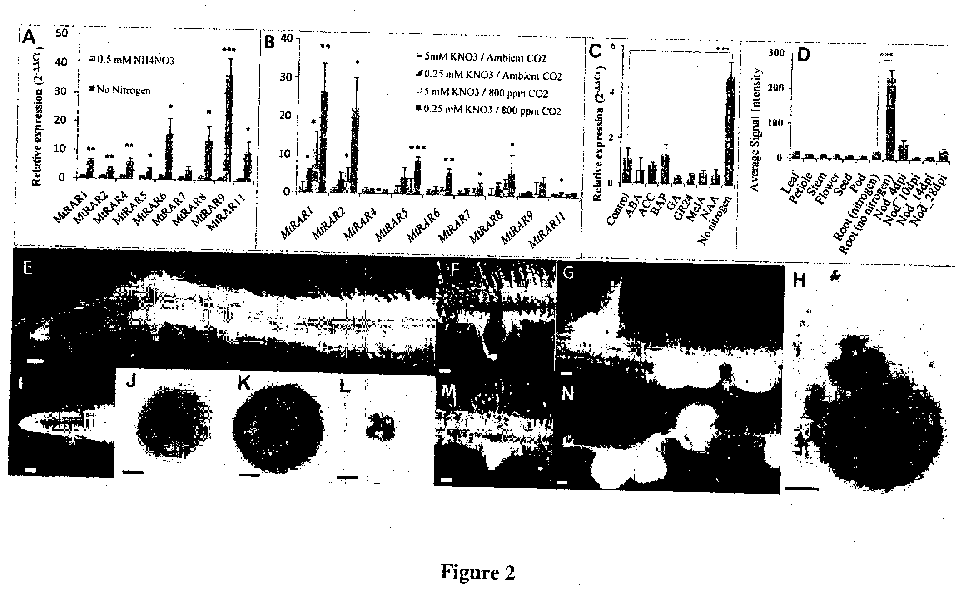

**Figure 2** - *MtRAR* gene expression is up-regulated by low N (nitrogen) and high CO₂ levels and is developmentally regulated during lateral organogenesis in *M*. *truncatula.* (A) Eight of 11 RAR genes were significantly up-regulated after four days growth on a N-free medium compared to plants grown with 0.5 mM NH₄NO₃. The expression of *MtRAR3* and *MtRAR10* was not included; they were not detected by qRT-PCR. N≥12; student's t-test *: P<0.05; **: P<0.01; ***: P<0.001. Bars indicate standard deviations. (B) Relative expression of *MtRAR* genes in response to high and low levels of nitrate and atmospheric CO₂. *MtRAR1-2, 5-8* and *11* are significantly up-regulated in the roots after 14 days growth under low nitrate (0.25 mM KNO₃) and high CO₂ (800 ppm) compared to the roots of similar plants grown under high nitrate (5mM KNO₃) and ambient CO₂. Plants grown at 0.25 mM KNO3 were supplemented with 4.75 mM KCl to standardize potassium levels. *MtRAR1* and *MtRAR11* are up-regulated by low nitrate and *MtRAR1* and *MtRAR2* are up-regulated by high CO₂ even in high the presence of high nitrate suggesting independent regulation of RAR by CO₂ and nitrate. N>12. (C). *MtRAR1* expression is not affected by 24h hormone treatments. (D) Microarray analysis of the *MtRAR1* in different tissues. *MtRAR1* is expressed in N-deprived roots and suppressed during all stages of nodule development (http://mtgea.noble.org/v2). Root (no nitrogen): grown under no nitrogen condition for four days; Nod: nodules; dpi: days post-inoculation with *S. meliloti.* (E-N) *ProMtRAR1:GUS* expression in the transgenic roots. (E and F) Nitrogen starvation for 4 days. (I and M) grown on 5 mM KNO₃. (J-L) Transverse sections at the root tip of N-starved plants; indicative locations of the sections are shown by the red lines in E. (G and N) *MtRAR1:GUS* roots inoculated with S. *meliloti* for one (G) or two weeks (N). (H) A cross-section of nodule induced by Sm 1021 at two weeks. Scale bars: 100 µM in E-G, I, M and N; 50 µM in H and J-L.

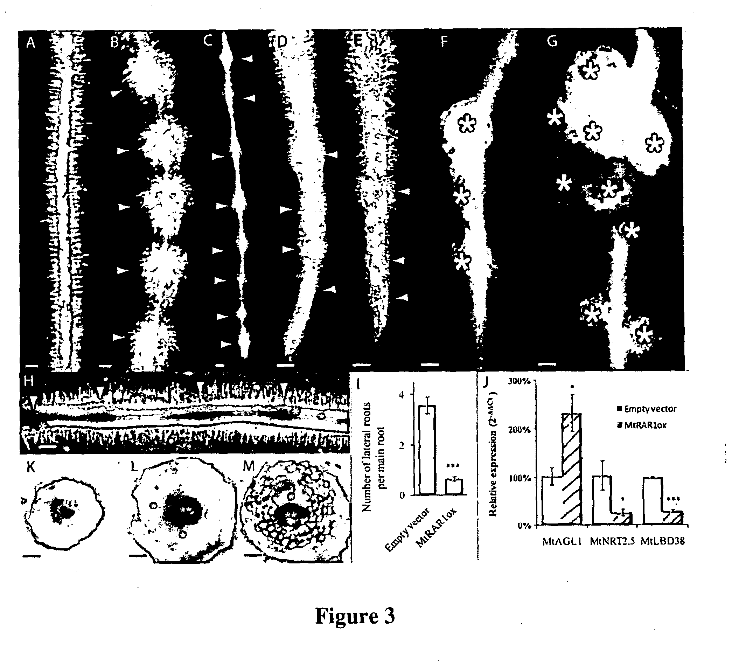

**Figure 3** - Multiple root architecture phenotypes in *M. truncatula* caused by *MtRAR1* over-expression or synthetic (putative) ligand addition. (A) Control root showing normal root hair development. (B-C) periodic bumps (arrows) caused by *MtRAR1* over-expression. (D-E) Periodic bumps (arrows) caused by synthetic MtRAR1 or MhRAR2 peptide respectively. (F-G) Root nodule formation on transgenic roots with the empty vector or *MtRAR1* over-expression respectively. GFP is visible in C, F and G. (H) *MtRAR1* over-expression caused periodic bumps in the *GH3:GUS* reporter construct line. *GH3:GUS* expresses strongly at bump sites and arrested lateral roots can be seen at two of the four sites (asterisk). A section of the control *GH3:GUS* root is shown in K and sections of two bump sites bearing arrested lateral roots are in L and M. Bumps are typified by circumferential cell proliferation ("CCP") and this term is used interchangeably throughout. Asterisk, arrested lateral root; double asterisk, strong vascular *GH3:GUS* expression (I) Lateral root number is repressed six-fold by *MtRAR1* over-expression. Histogram showing a comparison of lateral root numbers formed on the transgenic roots containing *MtRAR1ox* to an empty vector control. N≥319; ***: P<0.001. Bars indicate standard deviations. (J) The relative expression of nitrogen-signaling related genes *MtAGL1, MtNRT2.5* and *MtLBD38* are modulated by *MtRAR1* over-expression in the transgenic roots. N≥12; *: P<0.05; ***: P<0.001. Scale bars: 100 µM in A-H; 50 µM in L-M.

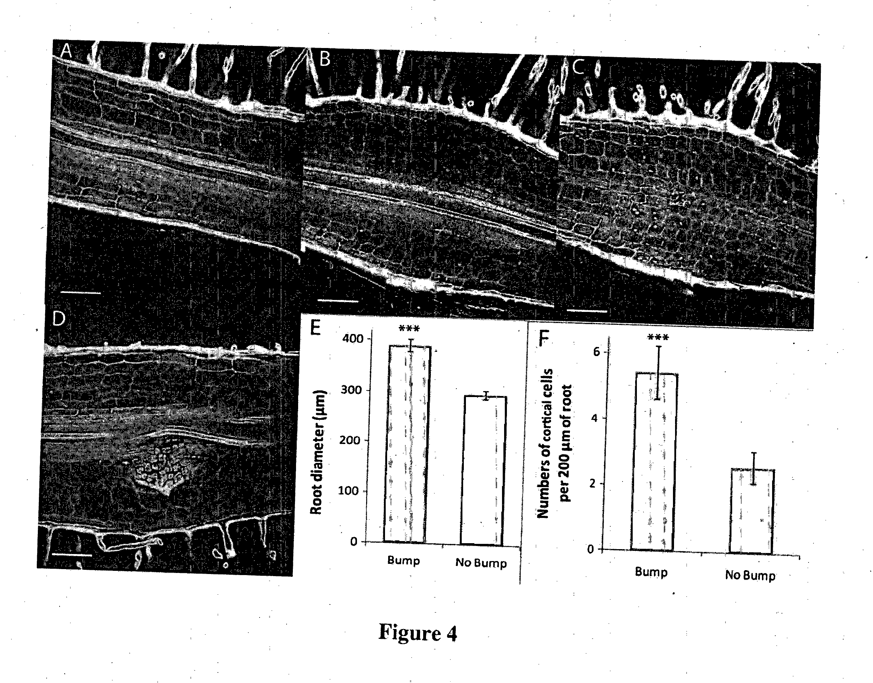

**Figure 4** - Periodic bumps are sites of localized cell division or contain arrested lateral roots. (A) Confocal imaging of a non-bump site on transgenic roots over-expressing *MtRAR1.* (B-C) CCP sites showing altered cell divisions occurring in epidermal, cortical and pericycle cells. Central nuclei are seen in C. (D) Bump site with arrested lateral root showing divided but un-differentiated cells. (E-F) Root diameter and the numbers of cortical cells are significantly increased at bump (CCP) sites (P< 0.05). Scale bars: 50 µM.

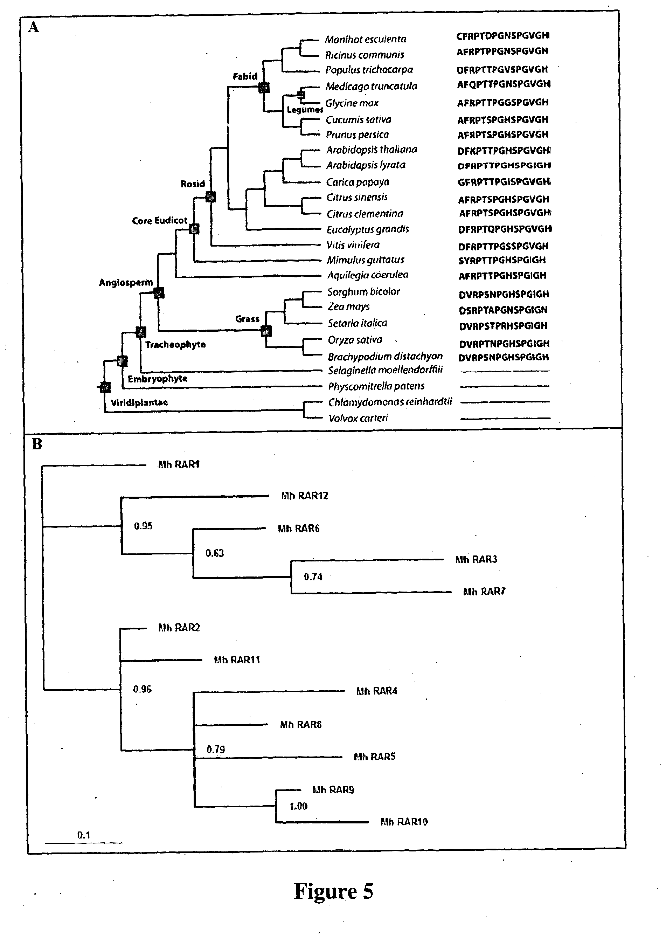

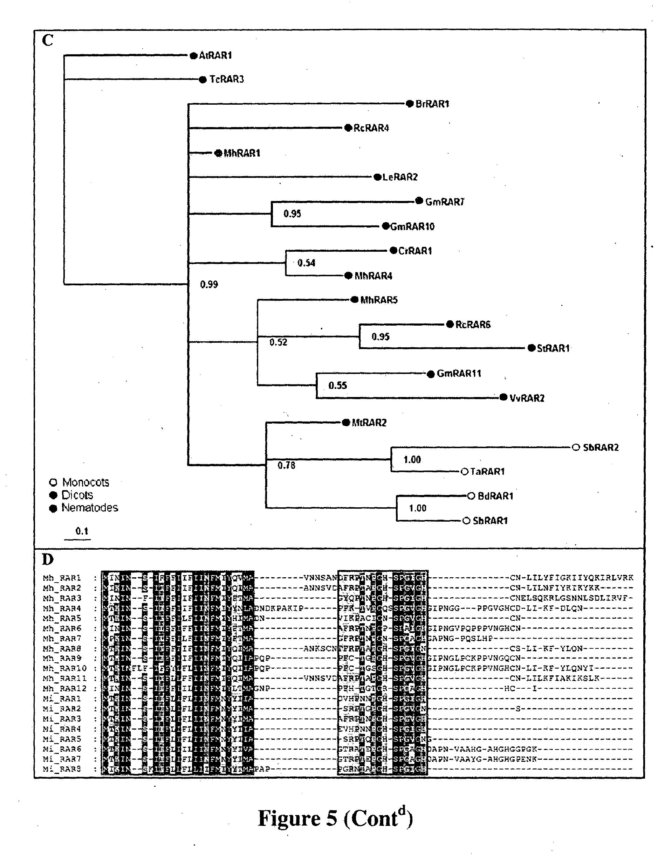

**Figure 5** - Shows a sequence analysis of RAR domains in higher plants and the RKNs. (A) Cladistic representation of RAR genes in plants. The RAR domains are shown for each evolutionarily significant clade only. (B) Phylogenetic analysis of RAR domains in Meloidogyne hapla. (C) Phylogenetic analysis of RAR domains in higher plants and RKNs. (D) Alignment of RKN RARs. Blue box indicates signal sequence. Red box indicates RAR domain. For (B) and (C) sequence alignment of pro- RAR and C-terminal domains was done by a combination of ClustalW and manual adjustment. Phylogenic tree construction was done by MrBayes. Numbers report the posterior probabilities of the 50% majority consensus tree.)

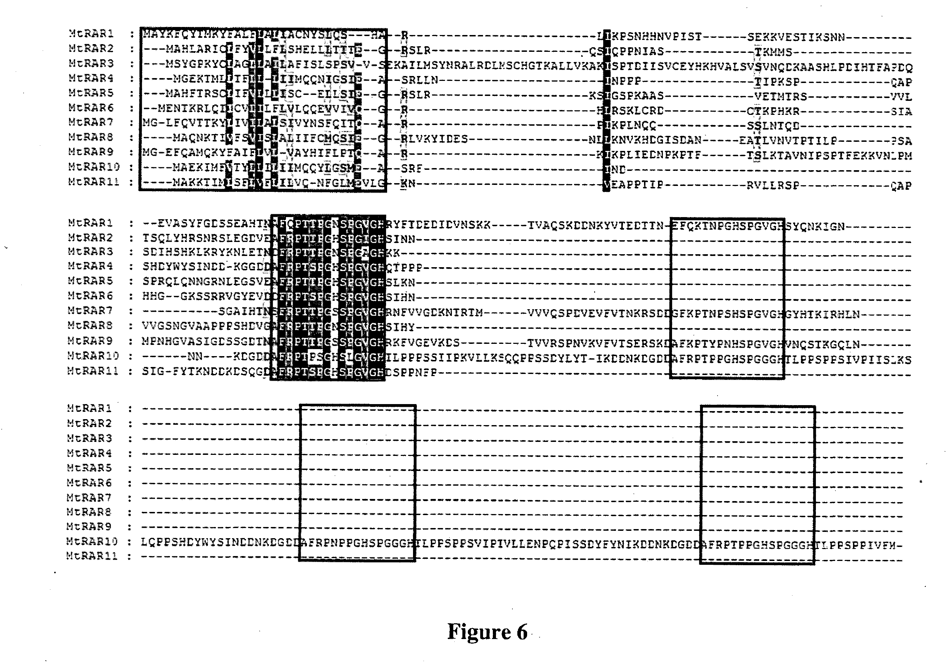

**Figure 6** - Amino acid sequence alignment of 11 putative RAR-coding genes in *M. truncatula.* All 11 sequences have predicted signal peptides at the N-terminus (boxed in blue), an intervening variable region of little or no sequence conservation and 15 amino acid long conserved region(s) close to the C-terminus end. RAR domains are boxed in red. Note some RARs have more than one RAR domain (e.g. MtRAR10 has four RAR peptide motifs whereas MtRAR 7, 9 and 1 each have two).

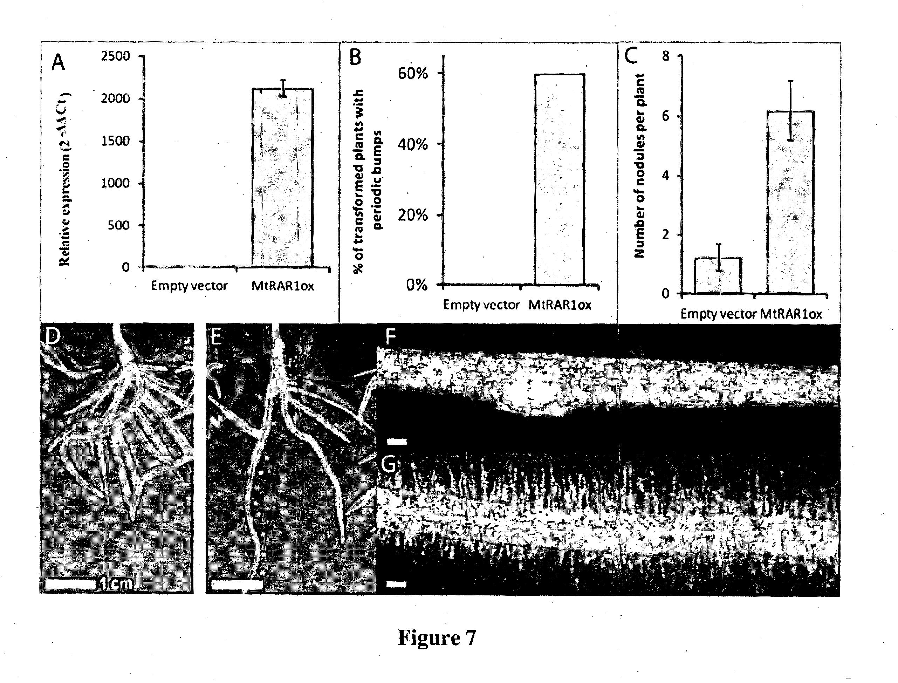

**Figure 7** - Phenotypic changes caused by MtRAR1 over-expression in *M*. *truncatula* transgenic roots. (A) The qRT-PCR results show that the expression of MtRAR1 is significantly elevated in transgenic roots containing the MtRAR1ox construct compared to transgenic roots containing the empty vector (control n ≥).9 ; student's t -test ***: P<0.0001. Error bars indicate standard deviations. (B) Quantification of periodic CCP site formation in MtRAR1ox transgenic roots. The results of double blind scoring for the presence of periodic CCP site formation are shown. N≥ 300, Student's t-test ***: P<0.001. (C) MtRARlox increases number of nodules when inoculated with *S. meliloti.* N≥20; student's t-test *: P<0.05. Error bars indicate standard deviations. (D-E) MtRARlox reduces lateral root numbers. Compared to transgenic roots containing the empty vector control (D), fewer lateral roots are formed in transgenic roots bearing MtRARlox (E). Periodic CCP on MtRARlox roots are indicated by asterisk. Scale bar = lcm. (F-G) MtRARlox reduces root hair length and numbers when grown under agar. Fewer and shorter root hairs are formed in transgenic roots bearing MtRARlox (F) compared to transgenic roots containing the empty vector control (G). Scale bar = 0.1 cm.

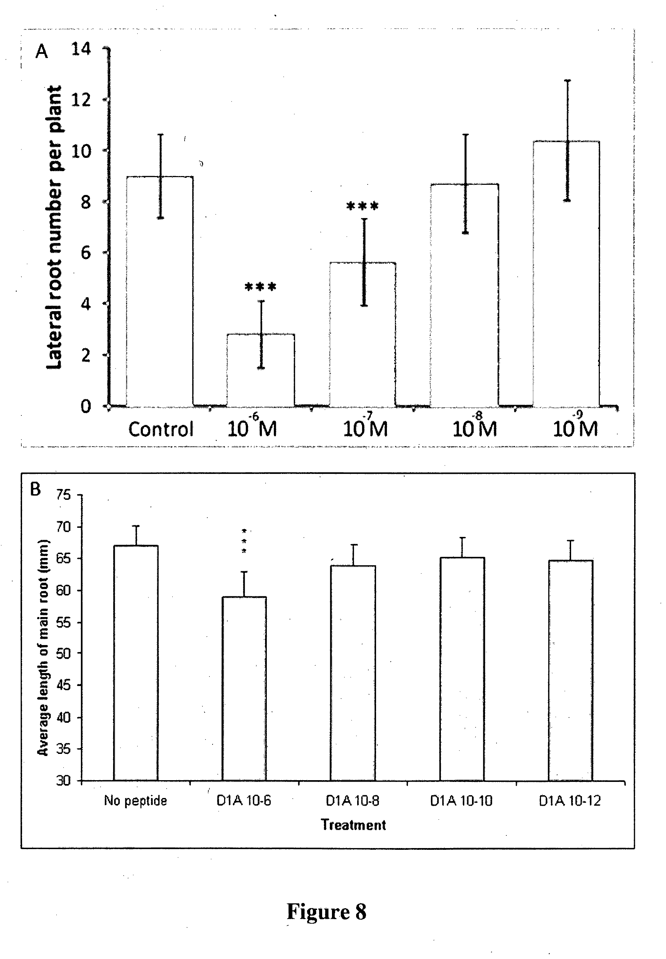

**Figure 8** - Effect of a concentration gradient of MtRAR1 synthetic peptide on lateral root formation and root growth in *M. truncatula.* (A). Lateral root formation is significantly reduced on A17 plants grown on F medium containing 25 mM KNO₃ and 10⁻⁶M D1A peptide (MtRARl domain one peptide). (B) Main root growth is significantly inhibited at 10⁻⁶M D1A peptide. t-test ***: P<0.001. Error bars indicate standard deviations.

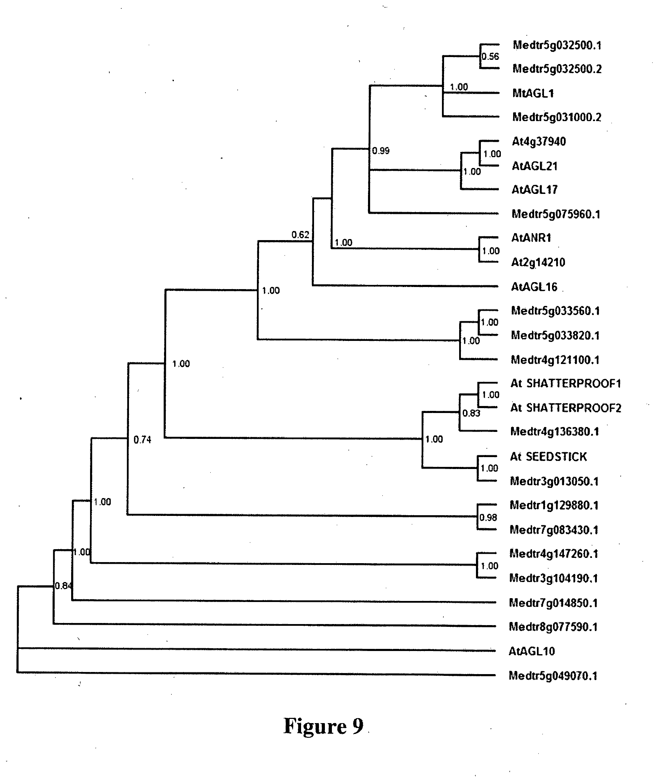

**Figure 9** - Phylogenetic tree of MADS-box homologues in *Arabidopsis* and *M*. *truncatula.* Sequence alignment was done by CLUSTAL and adjusted by hand. Phylogeny was created using MrBayes, with two chains running for 500000 generations, sampled every 500. A mixed amino acid model was used. Burn in was 250 data points. The above phylogeny represents the 50% majority consensus tree. Numbers are posterior probabilities.

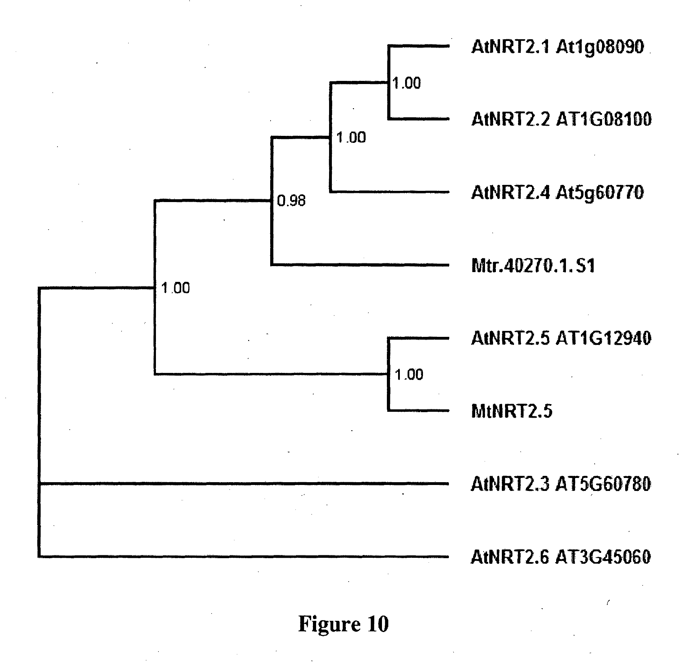

**Figure 10** - Phylogenetic tree of NRT homologues in *Arabidopsis* and *M*. *truncatula.* Sequence alignment was done by CLUSTAL and adjusted by hand. Phylogeny was created using MrBayes, with two chains running for 500000 generations, sampled every 500. A mixed amino acid model was used. Burn in was 250 data points. The above phylogeny represents the 50% majority consensus tree. Numbers are posterior probabilities.

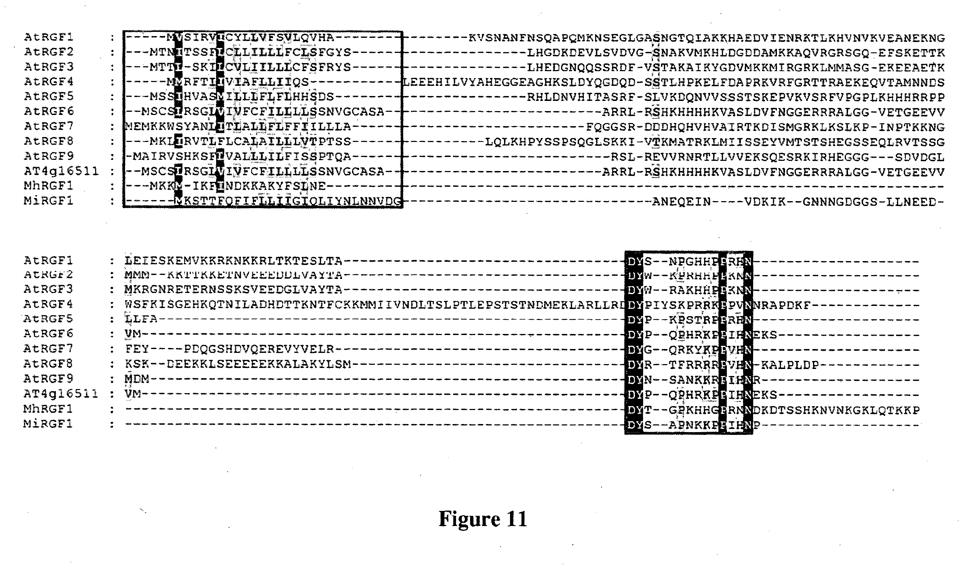

**Figure 11** - Alignment of the *M. hapla* putative RGF with RGFs from *A. thaliana.* The active domain region (boxed in red) is well conserved between the species.

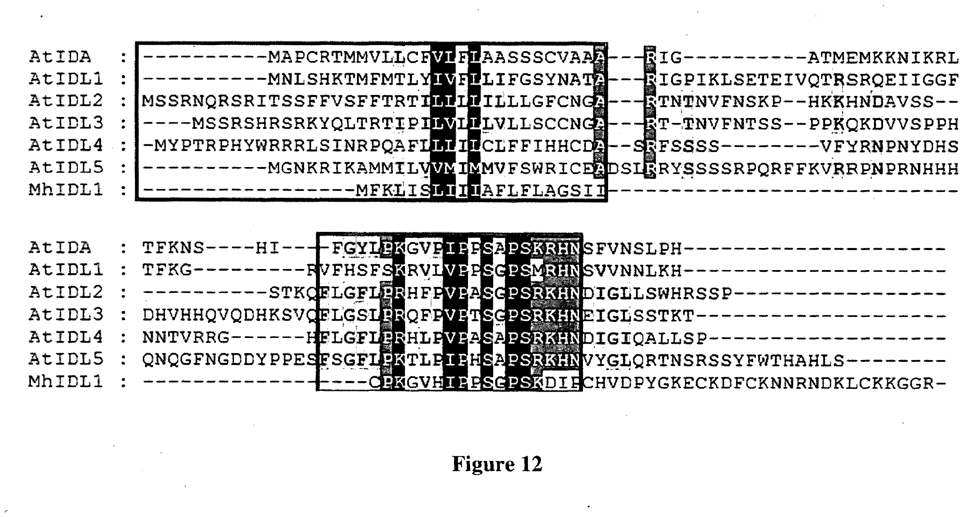

**Figure 12** - Putative IDA of *M. hapla* aligned with IDA and IDA-Like proteins from *A. thaliana.* In *M. hapla,* the signal sequence (blue box) is immediately followed by an IDA-Like domain (red box).

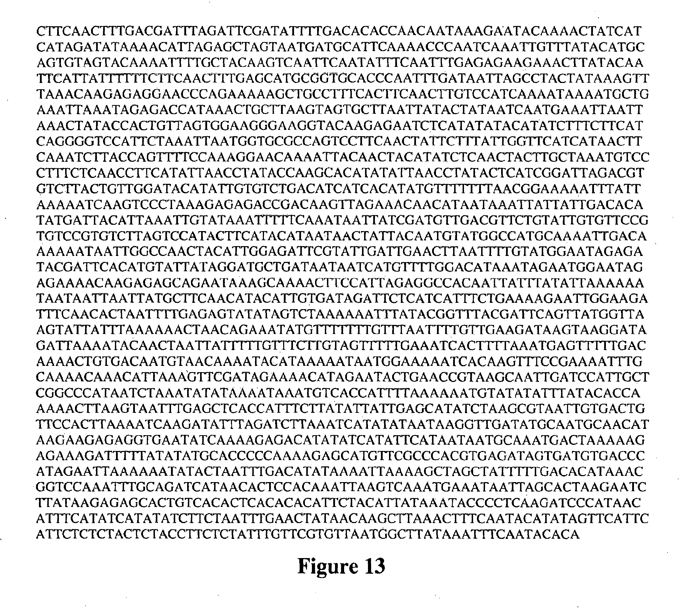

**Figure 13** - Shows the 5-prime upstream region of the *M. truncatula RAR1* gene, including the promoter region and the first 86 nucleotides of the RAR1-encoding region.

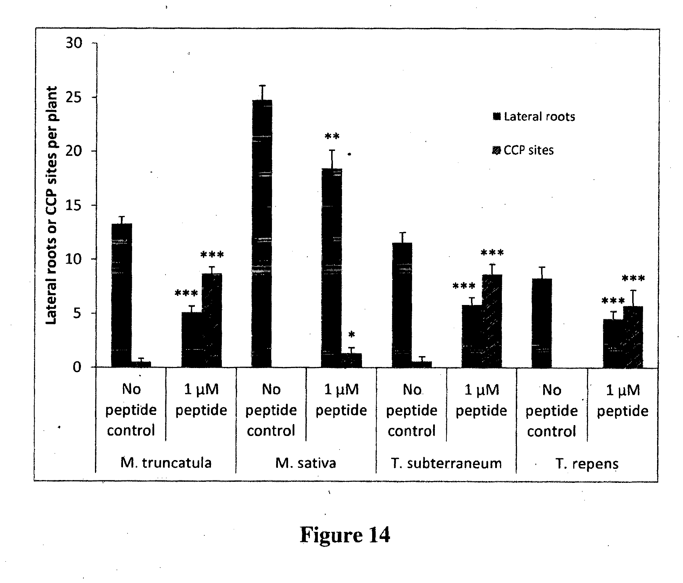

**Figure 14** - Shows the effect of RAR peptide application on lateral root formation of the plants grown in jars. *M. truncatula, M. sativa,* Subterranean Clover Cultivar *Woogenellup* and white clover *(Trifolium repens)* plants were grown in 0.4% agar-containing magenta jars supplemented with F media with or without peptides for two weeks. The media contained 5 mM KNO₃. MtRAR1 peptide: FQ(P)TTPGNS(P)GVGH where (P) is hydroxyproline. Students' t-test *: P < 0.05; **: P < 0.01; ***: P < 0.001; N>12. Error bars represent SE.

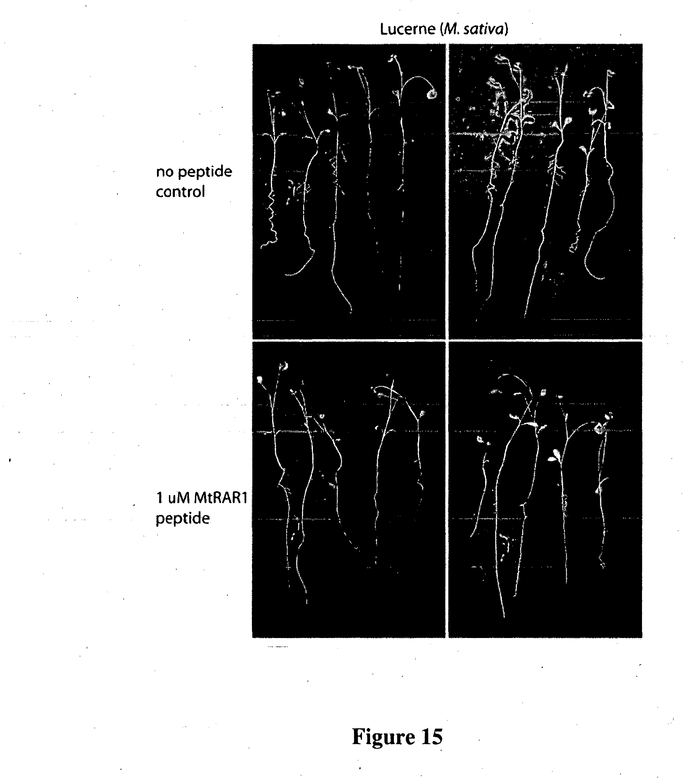

**Figure 15** - Shows the effect of RAR peptide application on lateral root formation of *M. sativa* plants. The plants were grown in 0.4% agar-containing magenta jars supplemented with F media with or without peptides for two weeks. The medium contained 5 mM KNO₃. MtRAR1 peptide: FQ(P)TTPGNS(P)GVGH where (P) is hydroxyproline.

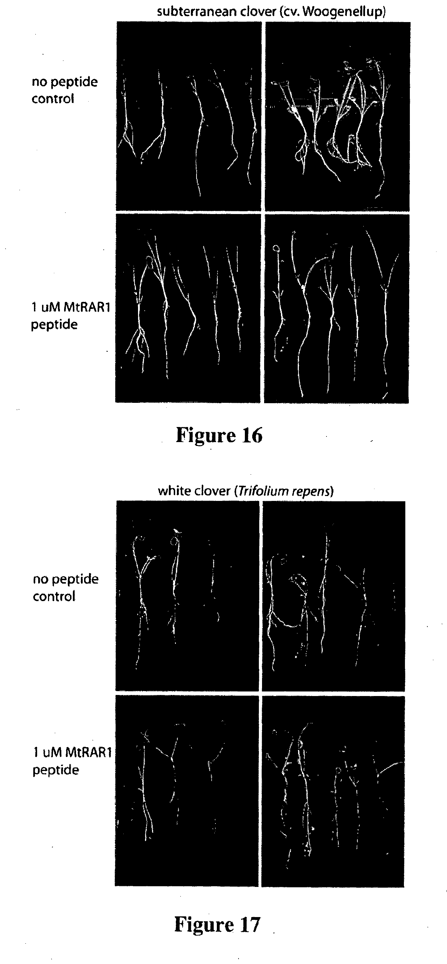

**Figure 16** - Shows the effect of RAR peptide application on lateral root formation of subterranean clover cultivar *Woogenellup* plants. The plants were grown in 0.4% agar-containing magenta jars supplemented with F media with or without peptides for two weeks. The media contained 5 mM KNO₃. MtRAR1 peptide: FQ(P)TTPGNS(P)GVGH where (P) is hydroxyproline.
**Figure 17** - Shows the effect of RAR peptide application on lateral root formation of white clover *(Trifolium repens)* plants. The plants were grown in 0.4% agar-containing magenta jars supplemented with F medium with or without peptides for two weeks. The media contained 5 mM KNO₃. MtRAR1 peptide: FQ(P)TTPGNS(P)GVGH where (P) is hydroxyproline.

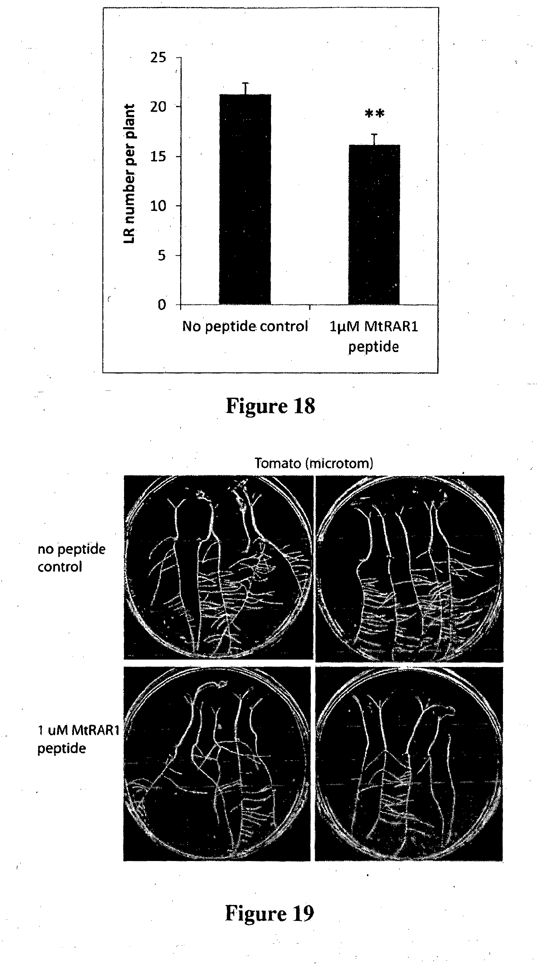

**Figure 18** - Shows the effect of RAR peptide application on lateral root formation on microtom tomatoes. The plants were grown in 0.8% agar-containing plates supplemented with F media with or without peptides for two weeks. The media contained 5 mM KNO₃. MtRAR1 peptide: FQ(P)TTPGNS(P)GVGH where (P) is hydroxyproline. Students' t-test, **: P < 0.01; N>12. Error bars represent SE.
**Figure 19** - Shows the effect of RAR peptide application on lateral root formation on microtom tomatoes. The plants were grown in 0.8% agar-containing plates supplemented with F media with or without peptides for two weeks. The medium contained 5 mM KNO₃. MtRAR1 peptide: FQ(P)TTPGNS(P)GVGH where (P) is hydroxyproline.

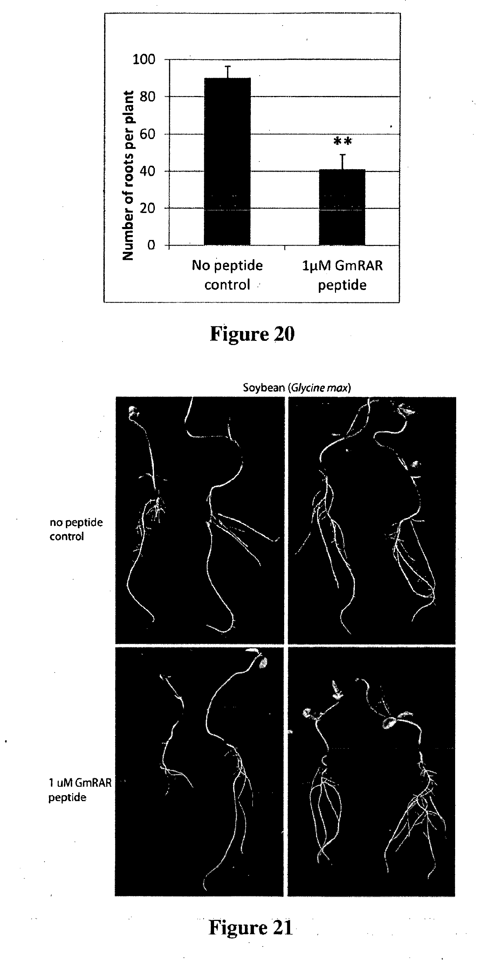

**Figure 20** - Shows the effect of RAR peptide application on root formation of soybean. The plants were grown in liquid supplemented with F medium with or without peptides for two weeks. The medium contained 5 mM KNO₃. GmRAR peptide: NFR(P)TAPGHS(P)GVGH where (P) is hydroxyproline. Students' t-test, **: P < 0.01; N>6. Error bars represent SE.
**Figure 21** - Shows the effect of RAR peptide application on root formation of soybean. The plants were grown in liquid supplemented with F medium with or without peptides for two weeks. The media contained 5 mM KNO₃. GmRAR peptide: NFR(P)TAPGHS(P)GVGH where (P) is hydroxyproline.

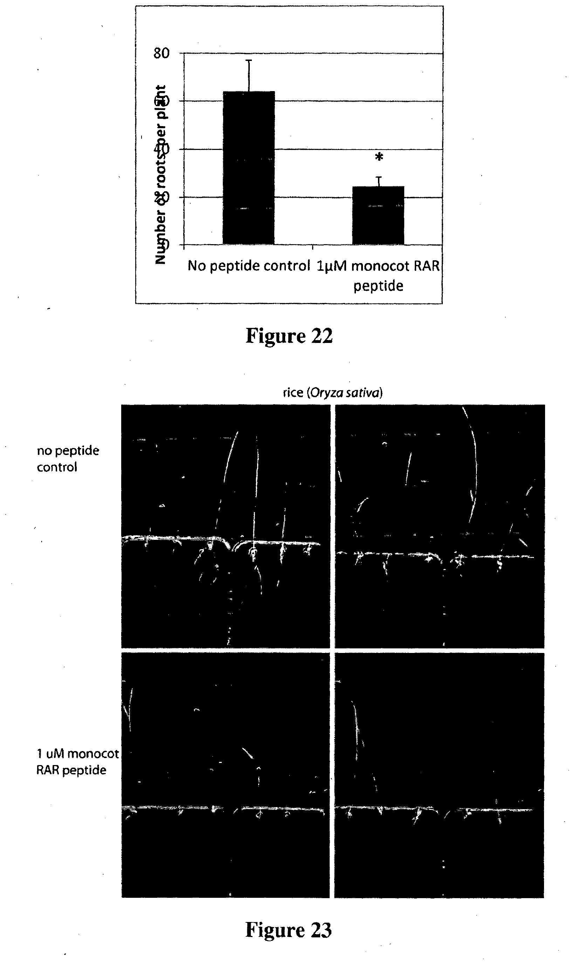

**Figure 22** - Shows the effect of RAR peptide application on root formation of rice plants. The plants were grown in liquid media containing half strength MS media with or without peptides for two weeks. Monocot RAR peptide: DTRATDPGHS(P)GAGH where (P) is hydroxyproline. Students' t-test, *: P < 0.05; N>6. Error bars represent SE.
**Figure 23** - Shows the effect of RAR peptide application on root formation of rice plants. The plants were grown in liquid media containing half strength MS media with or without peptides for two weeks. Monocot RAR peptide: DTRATDPGHS(P)GAGH where (P) is hydroxyproline.

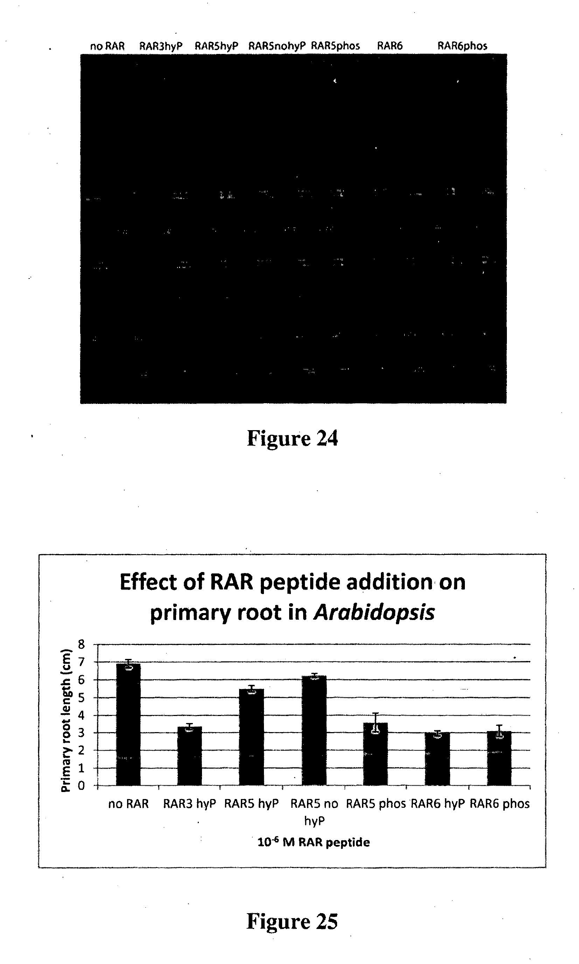

**Figure 24** - Shows the phenotypes of 12 day old WT *Arabidopsis* plants treated with 1 µM of RAR peptide.
**Figure 25** - Shows the effect of RAR peptide addition on primary root in *Arabidopsis.*

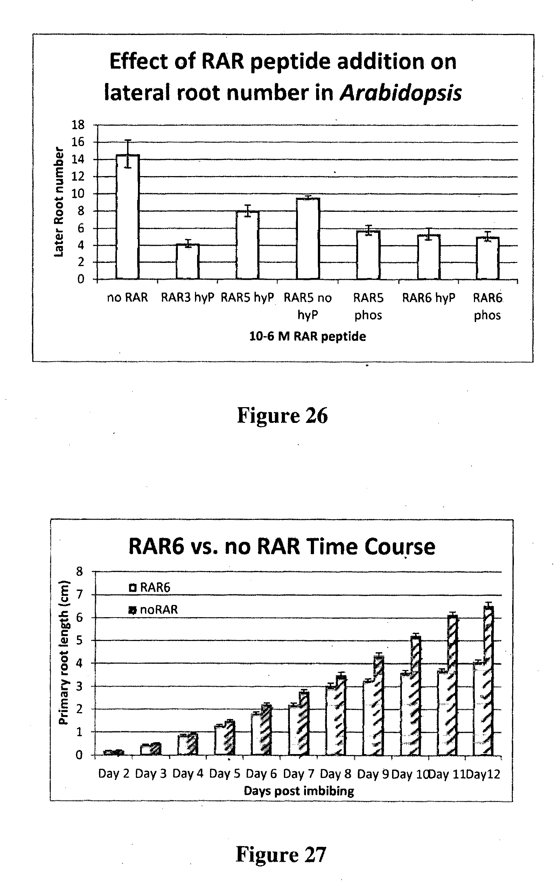

**Figure 26** - Shows the effect of RAR peptide addition on lateral root number in *Arabidopsis.*
**Figure 27** - Shows the RAR time course showing primary root length.

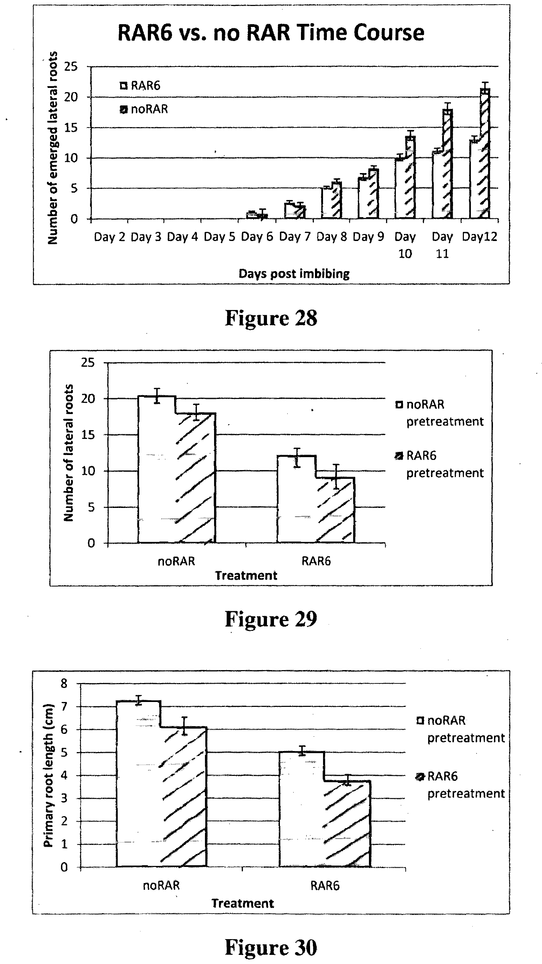

**Figure 28** - Shows the RAR time course showing lateral root number.
**Figure 29** - Shows the effect of RAR on the number of lateral roots can be released by removing RAR exposure.
**Figure** 30 - Shows the effect of RAR on primary root length can be released.

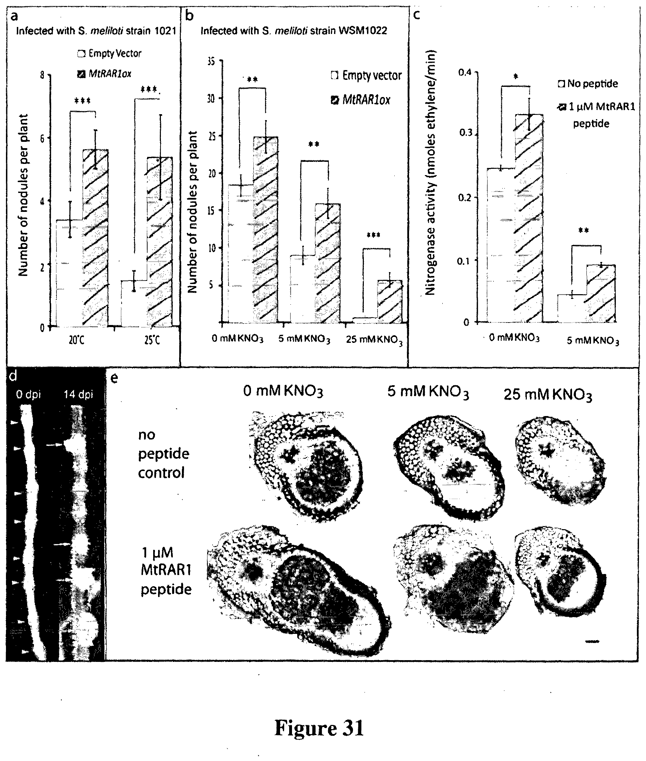

**Figure 31** - *MtRAR1* over-expression increases nodulation and promotes nodule development at different nitrate concentrations: **(a)** Comparison of nodulation between *MtRAR1* over-expressing plants and control plants at different temperatures when inoculated with *S. meliloti* strain 1021; **(b)** Partial nitrate tolerance of nodule development induced by WSM1022 on *MtRAR1* over-expressing plants; **(c)** Acetylene reduction assay results; **(d)** Inoculation of plants with preformed CCP sites led to root nodule formation at the CCP sites at 25 mM KNO₃; **(e)** Nodule sections from RAR1 peptide-treated and untreated plants grown at either 0, 5 mM or 25 mM KNO₃ for three weeks.

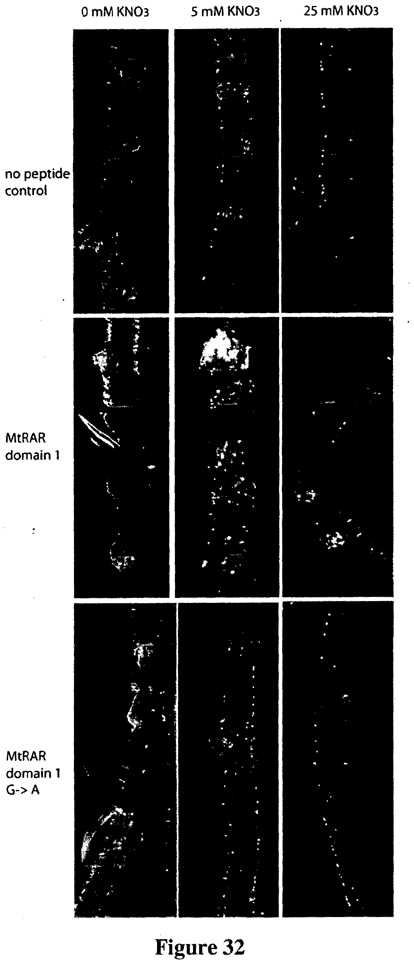

**Figure 32** - *M. truncatula* plants grown in 0.8% agar-containing plates supplemented with F media with or without peptides.

### Definitions

As used herein, the term "comprising" means "including principally, but not necessarily solely". Variations of the word "comprising", such as "comprise" and "comprises", have correspondingly similar meanings.

As used herein the term "gene", refers to a defined region that is located within a genome and that may comprise regulatory, nucleic acid sequences responsible for the control of expression, i.e., transcription and translation of the coding portion. A gene may also comprise other 5' and 3' untranslated sequences and termination sequences. Further elements that may be present are, for example, introns and coding sequences.

As used herein, the term "analogue" in the context of a peptide or protein means an artificial or natural substance that resembles the peptide or protein in function. For example, an RAR analogue will bind an RAR receptor and thereby bring about the same or similar result as if a natural RAR had bound to the receptor. Such analogues may also resemble the RAR peptide in structure. Analogues may include fully or partially peptidomimetic compounds as well as peptides or proteins resembling a subject peptide in activity but comprising addition, deletion, or substitution of one or more amino acids compared to the subject peptide or protein. The term "analogue" as used herein with reference to nucleotide sequences encompasses sequences comprising addition, deletion, or substitution (including conservative amino acid substitutions) of one or more bases relative to a subject nucleotide sequence, wherein the encoded polypeptide resembles the polypeptide encoded by the subject nucleic acid molecule in function.

As used herein, the term "homologue" in the context of proteins means proteins having substantially the same functions and similar properties in different species, and which, within at least regions, share at least 50% amino acid identity. Such homologous proteins may share, over their entire amino acid sequences, at least about 30% amino acid identity, at least about 40% amino acid identity, at least about 50% amino acid identity, at least about 60% amino acid identity, at least about 70% amino acid identity, at least about 80% amino acid identity, at least about 90% amino acid identity or at least about 95% identity. Similarly, homologues of nucleic acid molecules are nucleic acid molecules that encode proteins having substantially the same functions and similar properties in different species, wherein the encoded proteins share, within at least regions, at least 50% amino acid identity (such nucleic acid homologues may share significantly less than 50% identity due to degeneracy in the genetic code, and differences in preferred codon usage amongst different genuses and species), and may share at least about 30% amino acid identity, at least about 40% amino acid identity, at least about 50% amino acid identity, at least about 60% amino acid identity, at least about 70% amino acid identity, at least about 80% amino acid identity, at least about 90% amino acid identity or at least about 95% identity over the whole encoded amino acid sequences.

"Conservative amino acid substitutions" refer to the interchangeability of residues having similar side chains. For example, a group of amino acids having aliphatic side chains includes glycine, alanine, valine, leucine, and isoleucine; a group of amino acids having aliphatic-hydroxyl side chains includes serine and threonine; a group of amino acids having amide-containing side chains includes asparagine and glutamine; a group of amino acids having aromatic side chains includes phenylalanine, tyrosine, and tryptophan; a group of amino acids having basic side chains includes lysine, arginine, and histidine; and a group of amino acids having sulfur-containing side chains includes cysteine and methionine. Typically, conservative amino acids substitution groups are: valine-leucine-isoleucine, phenylalanine-tyrosine, lysine-arginine, alanine-valine, and asparagine-glutamine. Typically, conservative amino acid substitution(s) will result in a protein or polypeptide retaining at least some of the biological activity of the protein or polypeptide without such a conservative amino acid substitution. More typically, conservative amino acid substitution(s) will result in a protein or polypeptide having substantially the same, or at least comparable biological activity as the protein or polypeptide without such a conservative amino acid substitution. Conservative amino acid substitution(s) may result in proteins or polypeptides having greater biological activity than the protein or polypeptide without such a conservative amino acid substitution.

The term "antibody" refers to an immunoglobulin molecule able to bind to a specific epitope on an antigen, and which may be comprised of a polyclonal mixture, or be monoclonal in nature. Antibodies may be entire immunoglobulins derived from natural sources, or from recombinant sources. An antibody may exist in a variety of forms including, for example, whole antibody, an antibody fragment, or another immunologically active fragment thereof, such as a complementarity determining region. Similarly, the antibody may be an antibody fragment having functional antigen-binding domains, that is, heavy and light chain variable domains. The antibody fragment may also exist in a form selected from the group consisting of: Fv, F_{ab}, F(ab)₂, scFv (single chain Fv), dAb (single domain antibody), bi-specific antibodies, diabodies and triabodies.

The term "isolated" indicates that the material in question has been removed from its naturally existing environment, and associated impurities reduced or eliminated. Essentially, the 'isolated' material is enriched with respect to other materials extracted from the same source (ie., on a molar basis it is more abundant than any other of the individual species extracted from a given source), and preferably a substantially purified fraction is a composition wherein the 'isolated' material comprises at least about 30 percent (on a molar basis) of all macromolecular species present. Generally, a substantially pure composition of the material will comprise more than about 80 to 90 percent of the total of macromolecular species present in the composition. Most preferably, the 'isolated' material is purified to essential homogeneity (contaminant species cannot be detected in the composition by conventional detection methods) wherein the composition consists essentially of the subject macromolecular species.

As used herein, the term "agonist" in the context of a peptide, polypeptide or protein refers to a molecule that binds with a receptor for that peptide, polypeptide or protein to trigger a physiological response usually triggered by the peptide, polypeptide or protein when it binds to said receptor. For example, a RAR agonist is a molecule that binds to a RAR receptor to trigger a root architectural response.

As used herein, the term "antagonist" in the context of a peptide, polypeptide or protein refers to a substance that interferes with the physiological response usually triggered by the peptide, polypeptide or protein when it binds to said receptor, or which interferes with binding of said peptide, polypeptide or protein to its receptor. For example, a RAR antagonist may be a substance that binds to a RAR or a RAR receptor to inhibit interaction between the RAR and the RAR receptor, which interaction would trigger a root architectural response. RAR antagonists may include antibodies to RARs or RAR receptors.

As used herein, the term "mutation" means any change in a polypeptide or nucleic acid molecule relative to a wild-type polypeptide or nucleic acid molecule from which the 'mutant' is derived and may, for example, comprise single or multiple amino acid or nucleotide changes, or both nucleotide and amino acid changes, including point mutations, null mutations, frame-shift mutations, and may comprise deletions, or insertions, or substitutions of one or more nucleic acids or amino acids, which may comprise naturally or non-naturally occurring nucleotides or amino acids or analogues thereof.

A "nucleic acid", as referred to herein, refers to deoxyribonucleotides or ribonucleotides and polymers thereof in either single-, double-stranded or triplexed form. The term may encompass nucleic acids containing known analogues of natural nucleotides having similar binding properties as the reference nucleic acid. A particular nucleic acid sequence may also implicitly encompass conservatively modified variants thereof (e.g. degenerate codon substitutions) and complementary sequences. The terms "nucleic acid", "nucleic acid sequence" or "polynucleotide" may also be used interchangeably with gene, cDNA, and mRNA encoded by a gene.

The terms "polypeptide", "peptide" and "protein" may be used interchangeably herein to refer to a polymer of amino acid residues. Included within the scope of these terms are polymers in which one or more amino acid residues may comprise artificial chemical analogue(s) of corresponding naturally occurring amino acid(s), as well as, or instead of naturally occurring amino acid polymers. The terms "polypeptide", "peptide" and "protein" may also include polymers including modifications, including post-translational modifications, such as, but not limited to, glycosylation, lipid attachment, sulfation, phosphorylation, gamma-carboxylation of glutamic acid residues, hydroxylation and ADP-ribosylation.

The term "primer" as used herein means a single-stranded oligonucleotide capable of acting as a point of initiation of template-directed DNA synthesis. An "oligonucleotide" is a short nucleic acid, typically ranging in length from 2 to about 500 bases. The precise length of a primer will vary according to the particular application, but typically ranges from 15 to 30 nucleotides. A primer need not reflect the exact sequence of the template but must be sufficiently complementary to hybridize to the template.

Within the scope of the terms "protein", "polypeptide", "polynucleotide" and "nucleic acid" as used herein are fragments and variants thereof, including but not limited to reverse compliment and antisense forms of polynucleotides and nucleic acids.

### Detailed Description of the Invention

Cell-to-cell communication mechanisms coordinate cellular proliferation and differentiation in plants. Recently, new signal molecules have emerged that preside over the positional information required to co-ordinate plant growth. Amongst these are growth regulating peptides that act primarily as extracellular signals. Growth regulating plant peptides regulate all aspects of plant growth and development. The CLE (CLAVATA3/EMBRYO SURROUNDING REGION-related) peptides are well understood: different classes of plant CLEs regulate the differentiation and renewal of stem cell and control the developmental competency of legume roots for root nodule formation. Similarly, root growth factors (RGFs) are regulatory peptides that maintain the stem cell niche and transit cell proliferation.

An *Arabidopsis* gene, *AtCEP1* (*C*-terminal encoded peptide), encoding a 14 or 15 amino acid secreted ligand has been previously described and reported to influence primary root growth (Ohyama K, Ogawa M, and Matsubayashi Y (2008), "Identification of a biologically active, small, secreted peptide in Arabidopsis by in silico gene screening, followed by LC-MS-based structure analysis", The Plant Journal 55(1): 152-160). *AtCEP1* corresponds to *AtRAR1* according to the nomenclature used herein.

In the present studies, a number of RAR peptides have been identified across a broad range of plant families (angiosperms and gymnosperms), and some of these characterised. Phylogenetic and genetic tools were used to examine the distribution and function of this multigene family, and analyses indicate that this family of genes is unique to higher plants and, surprisingly, occur in root knot nematode (RKN) genomes. Generally, these genes encode secreted peptides that contain 14-15 amino acid long conserved domains. It has been found that in the legume *Medicago truncatula* (barrel medic) these genes are regulated by N-limited conditions and elevated CO₂ and control the expression of genes (*MtAGL1, MtNRT2.5* and *MtLBD38*) likely to coordinate N-status and uptake. Over-expression studies were used to demonstrate that one member of this gene family, designated as *MtRAR1*, which encodes two 15 amino acid peptides of this family, affects multiple aspects of root architecture and development including lateral root, nodule and root hair development, as well as shoot to root ratio. Multiple periodic bumps (CCP sites) form on roots at the expense of lateral root emergence and confocal imaging corroborates that these bumps share features with RKN-induced galls. Results also revealed RAR ligand mimicry by RKNs suggesting a role for these nematode peptides in gall/knot formation, including hyperplasia of cortical, epidermal and pericycle cells. Our results also revealed RAR ligand mimicry by RKNs suggesting a role for nematode RAR peptides in gall formation.

As the present studies place *M. truncatula* RARs at the crossroads of root development and responses to nutritional cues, we have renamed CEPs to RARs (Root Architecture Regulators) to more accurately reflect this function, and will be referred to as such from hereon.

The present invention therefore generally relates to methods for modulating the root architecture of plants, to create plants more suited to their environments, with increased or decreased lateral root development. Further contemplated are plants with increased nodulation (which in turn should lead to increased nitrogen assimilation).

Nematodes are represented by a wide number of species and constitute a major proportion of the diversity in the entire animal kingdom. A subset of the nematodes is plant parasitic and these have significant negative impacts on plant growth and yield. Of these, root knot nematodes (RKNs) (and not other nematodes) possess and express the RAR family of genes. This was surprising and intriguing. RKNs are obligate parasites infecting a broad spectrum of (probably all) vascular plants, causing crop yield losses estimated at more than USD50 billion per annum. Crop losses of 10-20% are common. Effective RKN control strategies are limited; current strategies are toxic and non-specific as they also kill the much larger and diverse population of benign and ecologically important nematodes in the soil. Central to the RKN-plant interaction is the *de novo* induction of a root organ (called a knot) in which vascular parenchyma cells develop into multinucleate giant cells that provide a feeding site for the RKN and surrounding cells exhibit various degrees of hypertrophy and hyperplasia. The proliferation of surrounding cells is called a gall. It has long been postulated that factors secreted by RKNs are required to subvert plant development pathways for knot formation. Remarkably, gall formation and symbiotic root nodule formation, a developmental program that provides a reliable N-supply to legumes, share mechanistic features.

During the course of the present studies it has been found that RKNs possess genetic sequences coding for homologues of the RARs, as well as other types of plant growth regulating peptides involved in root development and morphology, including the root growth factors (RGFs), and inflorescence deficient in abscission (IDA) families. This finding supports the view that RKNs subvert plant root development pathways, and that this may be done through secretion of one or more of these plant peptide homologues. Means that limit nematodes from secreting these peptides may enable strategies for at least controlling or limiting RKN damage to plant roots. An RKN-specific virulence target would be welcome, since the vast majority of other soil nematodes are benign to plant growth and serve important ecological functions.

Thus, the present description not only discusses methods of the present invention for modulating the root architecture of plants as defined in the claims, but also discloses methods for at least limiting damage to plant roots by RKNs. Such methods may involve: contacting the root zone of a plant with a RAR or a homologue or analogue of a RAR, or a RAR signaling agonist; contacting the root zone of a plant with a RAR and/or a RGF signaling antagonist; introducing one or more mutations or exogenous nucleic acids into one or more plant cells which mutation or introduced exogenous nucleic acid results in modulated RAR or RGF expression by root cells of a plant or by a RKN; introducing one or more mutations or exogenous nucleic acids into one or more plant cells which mutation or introduced exogenous nucleic acid results in modulated RAR or RGF receptor expression by root cells of a plant; introducing one or more mutations or exogenous nucleic acids into one or more plant cells which results in modulated affinity of one or more RARs or RGFs for their respective receptors, which modulated affinity arises through modifications in the RAR(s), RGF(s), their respective receptor(s) or in both expressed RAR(s)/RGF(s) and their respective receptor(s); or introducing one or more exogenous nucleic acids into one or more plant cells which results in expression of one or more root knot nematode RAR or RGF binding agents or antagonists by said cell(s).

Further described are isolated RKN RAR genes, RKN RAR-encoding nucleotide sequences and vectors and transformed cells comprising them, and isolated RKN RARs. Further described are isolated RKN RGF-encoding nucleotide sequences and vectors and transformed cells comprising them, and isolated RKN RGFs. Also contemplated are RKN IDA-encoding nucleotide sequences and vectors and transformed cells comprising them and isolated RKN IDAs, as well as at least methods for inhibiting RKN damage to plants relating to such materials.

### RARs and encoding nucleic acids and genes

Herein described and provided are RARs from a wide range of plants, including gymnosperms and angiosperms, as well as root knot nematodes (RKNs), as well as their encoding nucleotides. Previously described *Arabidopsis thaliana* CEP1 and its encoding nucleotide sequence is excluded from the RAR used in the method for reducing lateral root development by a plant relative to an untreated or wild-type plant as defined in the claims, but may be used in other methods of the invention for modulation of plant root architecture.

The RARs may broadly have features as shown in Figure 1A and, according to an embodiment, the RAR may comprise an amino acid sequence selected from SEQ ID NOs: 4, 6, 8, 10, 12, 14, 16, 18, 20, 22, 24, 26, 28, 30, 32, 34, 36 to 147, 338-350, 365, 367, 369, 371, 373, 375, 377, 379, 381, 383, 385, 387-395), or an RAR domain having an amino acid sequence selected from SEQ ID Nos: 148-336, 351-363 or 396-415, or may comprise an amino acid sequence sharing at least 60% identity, at least 70% identity, at least 80% identity, at least 85% identity, at least 90% identity, at least 95% identity, or at least 99% identity with said amino acid sequences.

RAR peptides used in the invention may include modifications to one or more of the amino acids. Such modifications may include natural modifications, such as post-translational modifications, including, for example, phosphorylation, hydroxylation, sulphonation and glycosylation. Such modifications may also be artificially created or instigated. According to an embodiment, an RAR peptide may comprise such modifications may comprise phosphorylation of one or more threonine or serine residues, where present, hydroxylation of one or more proline residues, where present, and sulphonation of the tyrosine at position 2, or at any other position when present, especially when preceded by aspartic acid.

According to another embodiment, the RAR-encoding nucleic acid may comprise a nucleotide sequence selected from SEQ ID NOs 3, 5, 7, 9, 11, 13, 15, 17, 19, 21, 23, 25, 27, 29, 31, 33, 35, 364, 366, 368, 370, 372, 374, 376, 378, 380, 382, 384 or 386, or may comprise a nucleotide sequence sharing at least 60% identity, at least 70% identity, at least 80% identity, at least 85% identity, at least 90% identity, at least 95% identity, or at least 99% identity with a nucleotide sequence selected from SEQ ID NOs 3, 5, 7, 9, 11, 13, 15, 17, 19, 21, 23, 25, 27, 29, 31, 33, 35, 364, 366, 368, 370, 372, 374, 376, 378, 380, 382, 384 or 386.

According to another embodiment, the RAR is a root knot nematode RAR and may have the RKN RAR features shown in Figure 1A. According to a further embodiment, the RKN RAR may comprise an amino acid sequence selected from SEQ ID NOs 365, 367, 369, 371, 373, 375, 377, 379, 381, 383, 385, 387-395 or comprise an RAR domain having an amino acid sequence selected from SEQ ID Nos: 396-415, or may comprise an amino acid sequence sharing at least 60% identity, at least 70% identity, at least 80% identity, at least 85% identity, at least 90% identity, at least 95% identity, or at least 99% identity with an amino acid sequence selected from SEQ ID NOs 365, 367, 369, 371, 373, 375, 377, 379, 381, 383, 385, 387-415.

According to another embodiment, the RKN RAR-encoding nucleic acid may comprise a nucleotide sequence selected from SEQ ID NOs 364, 366, 368, 370, 372, 374, 376, 378, 380, 382, 384 or 386, or may comprise a nucleotide sequence sharing at least 60% identity, at least 70% identity, at least 80% identity, at least 85% identity, at least 90% identity, at least 95% identity, or at least 99% identity with a nucleotide sequence selected from SEQ ID NOs 364, 366, 368, 370, 372, 374, 376, 378, 380, 382, 384 or 386.

During the course of the studies leading to the present invention, the whole gene coding for the *Medicago truncatula* (MtRARl) was identified, and the promoter found to be regulated by nutrient levels, especially available nitrogen. It is contemplated that such a promoter may be beneficial for expressing RAR peptides during periods of nitrogen limitation. Thus, according to a further embodiment, there is provided a RAR-encoding gene comprising the nucleotide sequence as shown in SEQ ID NO. 101, or comprising a nucleotide sequence sharing at least 60% identity, at least 70% identity, at least 80% identity, at least 85% identity, at least 90% identity, at least 95% identity, or at least 99% identity with the nucleotide sequence as shown in SEQ ID NO. 101. According to a yet further embodiment, there is provided a promoter sequence comprising the nucleotide sequence as shown in SEQ ID NO. 337, or comprising a nucleotide sequence sharing at least 60% identity, at least 70% identity, at least 80% identity, at least 85% identity, at least 90% identity, at least 95% identity, or at least 99% identity with the nucleotide sequence as shown in SEQ ID NO. 337.

Nucleic acid molecules for identifying other RAR-encoding sequences (and thereby the encoded peptides), or for suppressing the expression of RAR-encoding sequences (plant or RKN) are also contemplated. Suitable nucleic acid molecules may be any appropriate sequence which is designed based on any one of the RAR-encoding sequences as disclosed herein (SEQ ID NOs: 3, 5, 7, 9, 11, 13, 15, 17, 19, 21, 23, 25, 27, 29, 31, 33, 35, 364, 366, 368, 370, 372, 374, 376, 378, 380, 382, 384 or 386).

The nucleotide sequence of said nucleic acid molecule may be identical to, or be complementary to at least a portion of any one of SEQ ID NOs: 3, 5, 7, 9, 11, 13, 15, 17, 19, 21, 23, 25, 27, 29, 31, 33, 35, 364, 366, 368, 370, 372, 374, 376, 378, 380, 382, 384 or 386, and may comprise the full sequence, or complement thereof or, may comprise an oligonucleotide from about 10 nucleotides in length to about 100 nucleotides in length, such as from about 10 to about 50 nucleotides in length, about 15 to about 100 nucleotides in length, about 15 to about 50 nucleotides in length, about 10 to about 30 nucleotides in length, or about 15 to about 30 nucleotides in length.

Alternatively, a nucleic acid molecule in context with the invention may comprise a nucleotide sequence designed based on the amino acid sequence of one of the RARs disclosed herein (SEQ ID NOs: 4, 6, 8, 10, 12, 14, 16, 18, 20, 22, 24, 26, 28, 30, 32, 34, 36 to 147, 338-350, 365, 367, 369, 371, 373, 375, 377, 379, 381, 383, 385, 387-395), or an RAR domain having an amino acid sequence selected from SEQ ID Nos: 148-336, 351-363 or 396-415, using degeneracy of the genetic code, and optionally preferred codon usage information. Suitable nucleic acid molecule sizes are as already discussed immediately above.

Nucleotide sequences in context with the invention as described above which may be employed as, or which may be comprised in primers, probes, antisense molecules, microRNA molecules or strands in double-stranded RNAi molecules may comprise one or more modifications as known in the art for stabilising the molecule(s) (for example, against enzymic degradation by ribonucleases), or for increasing the strength of hybridization with complementary molecule(s).

### RGFs and IDAs and encoding nucleic acids and genes

Also herein described and provided are novel root growth factor (RGF) and putative Inflorescence Deficient for Abscission (IDA) peptide sequences from root knot nematodes, as well as their encoding nucleotide sequences. RGFs and IDAs have been previously described in plants, but not in RKNs. It is contemplated that such RGFs or IDAs, their encoding nucleotide sequences, or both may be used to modulate plant root architecture. It is also contemplated that RKN RGFs or IDAs, or RKN RGF- or IDA-specific binding agents or their encoding nucleotide sequences (or fragments thereof), may be used to control the effects or infestation of plants by RKNs.

The RKN RGF may be any RGF identified from RKNs. An RKN RGF may comprise an amino acid sequence as shown in SEQ ID NOs: 416 or 417 or comprise an RGF domain comprising an amino acid sequence as shown in SEQ ID NO: 460 or 461, or may comprise an amino acid sequence sharing at least 60% identity, at least 70% identity, at least 80% identity, at least 85% identity, at least 90% identity, at least 95% identity, or at least 99% identity with said amino acid sequences.

According to another embodiment, the RKN RGF-encoding nucleic acid may comprise a nucleotide sequence designed based on an amino acid sequence as shown in SEQ ID NOs: 416, 417, 460 or 461 using degeneracy of the genetic code, and optionally preferred codon usage information. Suitable nucleic acid molecule sizes are as already discussed immediately above.

The RKN IDAs may be any IDA identified from RKNs. An RKN IDA may comprise an amino acid sequence as shown in SEQ ID NO: 418 or may comprise an IDA domain comprising an amino acid sequence as shown in SEQ ID NO: 496, or may comprise an amino acid sequence sharing at least 60% identity, at least 70% identity, at least 80% identity, at least 85% identity, at least 90% identity, at least 95% identity, or at least 99% identity with said amino acid sequences.

Nucleic acid molecules for identifying other RKN RGF- or IDA-encoding sequences (and thereby the encoded peptides), or for suppressing the expression of RKN RGF-encoding sequences are also contemplated. Suitable nucleic acid molecules may be any appropriate sequence which is designed based on an amino acid sequence as shown in SEQ ID NOs: 416, 417, 460 or 461 (for RGF-encoding sequences) or SEQ ID NOs: 418 or 496 (for IDA-encoding sequences).

The nucleotide sequence of nucleic acid molecules encoding RGFs or IDAs as described above may comprise the full sequence, or complement thereof or, may comprise an oligonucleotide of from about 10 nucleotides in length to about 100 nucleotides in length, such as from about 10 to about 50 nucleotides in length, about 15 to about 100 nucleotides in length, about 15 to about 50 nucleotides in length, about 10 to about 30 nucleotides in length, or about 15 to about 30 nucleotides in length.

Alternatively, a nucleic acid molecule in context with the invention may comprise a nucleotide sequence designed based on the amino acid sequence of SEQ ID NOs: 416, 417, 460 or 461 (RGFs) or SEQ ID NOs: 418 or 496 (IDAs) using degeneracy of the genetic code, and optionally preferred codon usage information. Suitable nucleic acid molecule sizes are as already discussed immediately above.

Nucleotide sequences in context with the invention as described above which may be employed as, or which may be comprised in, for example, primers, probes, antisense molecules, microRNA molecules, or strands in double-stranded RNAi molecules may comprise one or more modifications as known in the art for stabilising the molecule(s) (for example, against enzymic degradation by ribonucleases), or for increasing the strength of hybridization with complementary molecule(s).

### RAR, RGF or IDA receptors and encoding sequences

Receptors for RARs, RGFs or IDAs and their encoding sequences may be identified, isolated and sequenced by methods well known and understood in the art using RAR, RGF or IDA sequences as disclosed herein. Methods for identifying and characterising plant receptors through knowledge of their ligands are well established and have been described in, for example, Shinya T et al (2010), Plant Cell Physiol 51(2): 262-270, which describes a use of affinity cross-linking with biotinylated ligands to isolate receptors.

### Methods for modulating root architecture

In agriculture it would generally be desirable to be able to create plants which are better suited to the soil environment they are to be grown in, physically or chemically. Such adaptations may include greater nutrient uptake efficiency, a root system having nitrogen-fixing nodules, or a root system having greater extent of nodulation, or a root system with altered horizontal or vertical growth patterns (ie. reduced or greater lateral root development).

One manner of achieving such adaptation(s) may be through increased or decreased expression of RAR or RGF genes (and possibly IDA genes), use of the expressed peptides, binding agents, their receptors and modulation of RAR or RGF signaling.

Methods of the present invention or disclosure, respectively, for modulating root architecture may include:
(a) contacting the root zone of said plant with a root architecture regulator peptide (RAR), an analogue thereof or an RAR signaling agonist;
(b) contacting the root zone of said plant with a RAR signaling antagonist;
(c) introducing at least one mutation or exogenous nucleic acid into one or more plant cells which results in modulated RAR expression by root cells of a plant regenerated from or comprising said one or more plant cells or in a root knot nematode;
(d) introducing at least one mutation or exogenous nucleic acid into one or more plant cells which results in modulated RAR receptor expression by root cells of a plant regenerated from or comprising said one or more plant cells;
(e) introducing at least one mutation or exogenous nucleic acid into one or more plant cells which results in modulated affinity of one or more RARs for their respective RAR receptors, which modulated affinity arises through modifications in the RAR(s), RAR receptor(s) or in both expressed RAR(s) and RAR receptor(s) expressed by root cells of a plant regenerated from or comprising said one or more plant cells; or
(f) introducing at least one exogenous nucleic acid into one or more plant cells which results in expression of one or more root knot nematode RAR binding agents or antagonists and secretion thereof into the root zone of said plant.

Similar techniques could be applied in the context of RGFs and possibly IDAs.

Of great interest to agriculture is the prospect of increasing nutrient uptake from soils (which may be achieved by increasing or reducing lateral root development, depending on the circumstances), developing nitrogen-fixation in non-legumes, and increased nodulation in legumes.

Methods for decreasing lateral root development by a plant relative to an untreated or wild-type plant, may comprise contacting the root zone of said plant with at least one RAR, an RAR analogue, or an RAR signaling agonist. Methods for decreasing lateral root development by a plant relative to an untreated or wild-type plant, may also comprise treating seeds of plants with at least one RAR, an RAR analogue, or an RAR signaling agonist prior to sowing.

RARs for use in such methods may be any RAR as mentioned above. As defined in the claims, according to certain embodiments, the RAR is not an *Arabidopsis thaliana* CEP1 peptide. According to other embodiments, the RAR is an RAR comprising an amino acid sequence as shown in SEQ ID NOs: 4, 6, 8, 10, 12, 14, 16, 18, 20, 22, 24, 26, 28, 30, 32, 34, 36 to 147, 338-350, 365, 367, 369, 371, 373, 375, 377, 379, 381, 383, 385, 387-395 or comprises an RAR domain having an amino acid sequence selected from SEQ ID Nos: 148-336, 351-363 or 396-415. According to another embodiment, the RAR is an RKN RAR and, according to a further embodiment, comprises an amino acid sequence as shown in SEQ ID NOs 365, 367, 369, 371, 373, 375, 377, 379, 381, 383, 385, 387-395 or comprises an RAR domain having an amino acid sequence selected from SEQ ID Nos: 396-415 or is encoded by a nucleic acid comprising a nucleotide sequence selected from SEQ ID NOs 364, 366, 368, 370, 372, 374, 376, 378, 380, 382, 384 or 386.

An RAR analogue contemplated herein may be any artificial or natural substance that resembles the protein in function. For example, an RAR analogue may bind an RAR receptor and thereby bring about the same or similar result as if a natural RAR had bound to the receptor. Such analogues may also resemble the protein in structure. Analogues contemplated herein include fully or partially peptidomimetic compounds based on the structures of the RARs disclosed herein. Peptidomimetic compounds (compounds designed to mimic biologically active peptides, but comprising structural differences - to provide advantages, especially in terms of stability, but also interaction with ligands/binding partners or substrates - and comprising unnatural amino acids or other unusual compounds) and their design is well-studied and is described in, for example, Floris M. et al (2011), Nucleic Acids Research 39(18): W261-269. Alternatively, an RAR analogue may be a peptide that resembles an RAR in function and activity, but comprise one or more amino acid substitutions, deletions or insertions compared to the subject RAR, and may share at least about 50% amino acid identity, at least about 60% identity, at least about 70% identity, at least about 80% identity, at least about 90% identity, at least about 95% identity, or at least about 99% identity with the amino acid sequence of the subject RAR

An RAR signaling agonist for use in a method of the present invention may be any molecule that binds with a RAR receptor to trigger a physiological response usually triggered by a RAR peptide when it binds to the receptor. For example, a RAR agonist may be a molecule that binds to a RAR receptor to trigger a root architectural response.

Methods for decreasing lateral root development by a plant relative to an untreated or wild-type plant, may also comprise introducing into one or more plant cells at least one exogenous RAR-encoding nucleic acid into one or more plant cells. Plants with increased RAR expression by root cells of the plants may be regenerated from, or comprise such transformed plant cells.

Transgenic plants with an introduced RAR-encoding sequence may be generated using standard plant transformation methods known to those skilled in the art including, for example, *Agrobacterium-mediated* transformation, cation or polyethylene glycol treatment of protoplasts, calcium phosphate precipitation, electroporation, microinjection, viral infection, protoplast fusion, microparticle bombardment, agitation of cell suspensions in solution with microbeads or microparticles coated with the transforming DNA, direct DNA uptake, liposome-mediated DNA uptake, and the like, as also described in a wide range of publicly available texts, such as: "Methods for Plant Molecular Biology" (Weissbach & Weissbach, eds., 1988); Clough, S.J. and Bent, A.F. (1998) "Floral dip: a simplified method for Agrobacterium-mediated transformation of Arabidopsis thaliana" Plant J. 16, 735-743; "Methods in Plant Molecular Biology" (Schuler & Zielinski, eds., 1989); "Plant Molecular Biology Manual" (Gelvin, Schilperoort, Verma, eds., 1993); and "Methods in Plant Molecular Biology-A Laboratory Manual" (Maliga, Klessig, Cashmore, Gruissem & Varner, eds., 1994). See also Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000).

The coding region may also be operably linked to an appropriate 3' regulatory sequence. For example, the nopaline synthetase (NOS) polyadenylation region or the octopine synthetase (OCS) polyadenylation region may be used.

The preferred method of transformation may depend upon the plant to be transformed. *Agrobacterium* vectors are often used to transform dicot species. For transformation of monocot species, biolistic bombardment with particles coated with transforming DNA and silicon fibers coated with transforming DNA are often useful for nuclear transformation. However, *Agrobacterium-mediated* transformation of monocotyledonous species, including wheat, are now known (see, for example, International patent publications WO 97/48814; see also Hiei, Y. et al (1994), Plant J. 6(2):271-282 and international patent publication WO 92/06205).

An RAR-encoding sequence can be comprised in a vector. Representative vectors include plasmids, cosmids, and viral vectors. Vectors can also comprise nucleic acids including expression control elements, such as transcription/translation control signals, origins of replication, polyadenylation signals, internal ribosome entry sites, promoters, enhancers, etc., wherein the control elements are operatively associated with a nucleic acid encoding a gene product. Selection of these and other common vector elements are conventional and many such sequences can be derived from commercially available vectors. See, for example, Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000).

According to an embodiment, the vector is an expression vector capable of directing the transcription of an RAR-encoding sequence into RNA.

DNA constructs for transforming a selected plant may comprise a coding sequence of interest operably linked to appropriate 5' regulatory sequences (e.g., promoters and translational regulatory sequences) and 3' regulatory sequences (e.g., terminators). In a preferred embodiment, the coding region is placed under a powerful constitutive promoter, such as the Cauliflower Mosaic Virus (CaMV) 35S promoter or the figwort mosaic virus 35S promoter. Other constitutive promoters contemplated for use in the present invention include, but are not limited to: T-DNA mannopine synthetase, nopaline synthase (NOS) and octopine synthase (OCS) promoters.

Using an *Agrobacterium* binary vector system for transformation, the selected coding region, under control of a constitutive or inducible promoter as described above, may be linked to a nuclear drug resistance marker, such as kanamycin resistance. Other useful selectable marker systems include, but are not limited to: other genes that confer antibiotic resistances (e.g., resistance to hygromycin or bialaphos) or herbicide resistance (e.g., resistance to sulfonylurea, phosphinothricin, or glyphosate).

According to an embodiment, the RAR-encoding sequence is operably linked to a promoter which is constitutive or inducible. An inducible promoter, for the purposes of the present invention, may be inducible by any appropriate stimulus. According to certain embodiments, an inducible promoter for use according to the present invention may be inducible by nutrient, drought, or other abiotic stress. According to an embodiment, an inducible promoter for use according to the present invention is inducible by nutrient status, such as by nitrogen starvation or by high carbon dioxide.

According to another embodiment, the RAR-encoding sequence is operably linked to a promoter which is root-specific.

According to another embodiment, the RAR-encoding sequence comprises a secretion signal sequence.

According to an embodiment, the RAR-encoding sequence is operably linked to a promoter comprising the nucleotide sequence as shown in SEQ ID NO: 337 or a homologue thereof sharing at least 60% identity with SEQ ID NO: 337. Alternatively, the promoter may be a root-specific glutamine synthetase gene promoter.

According to an embodiment, a method of the invention for modulating the root architecture of a plant, relative to a wild-type plant, comprises introducing into one or more plant cells the *Medicago truncatula* RAR1 gene, including the promoter sequence, disclosed herein as SEQ ID NO: 337, and the RAR-encoding sequence, disclosed herein as SEQ ID NO: 15.

The coding region may also be operably linked to an appropriate 3' regulatory sequence. For example, the nopaline synthetase (NOS) polyadenylation region or the octopine synthetase (OCS) polyadenylation region may be used.

Using an *Agrobacterium* binary vector system for transformation, the selected coding region, under control of a constitutive or inducible promoter as described above, may be linked to a nuclear drug resistance marker, such as kanamycin resistance. Other useful selectable marker systems include, but are not limited to: other genes that confer antibiotic resistances (e.g., resistance to hygromycin or bialaphos) or herbicide resistance (e.g., resistance to sulfonylurea, phosphinothricin, or glyphosate).

Any of the methods of the present invention, as discussed above or below, can be used to transform any plant cell. In this manner, genetically modified plants, plant cells, plant tissue, seed, and the like can be obtained. The plant cell(s) to be transformed may be a plant cell from any plant selected from angiosperms or gymnosperms. Non-exhaustive examples of angiosperms for treatment or transformation by a method of the invention may include any member of the Aceraceae, Anacardiaceae, Apiaceae, Asteraceae, Betulaceae, Brassicaceae, Buxaceae, Chenopodiaceae/Amaranthaceae, Compositae, Cucurbitaceae, Fabaceae, Fagaceae, Gramineae, Juglandaceae, Lamiaceae, Lauraceae, Leguminosae, Moraceae, Myrtaceae, Oleaceae, Platanaceae, Poaceae, Polygonaceae, Rosaceae, Rutaceae, Salicaceae, Solanaceae, Ulmaceae or Vitaceae. Examples of gymnosperms for treatment or transformation by a method of the invention may include any member of the Cuppressaceae, Pinaceae, Taxaceae or Taxodiaceae.

Cells which have been transformed may be grown into plants in accordance with conventional methods as are known in the art (See, for example, McCormick, S. et al (1986), Plant Cell Reports 5:81-84). The resulting plants may be self-pollinated, pollinated with the same transformed strain or different strains or hybridised, and the resulting plant(s) having modulated root architecture compared to wild-type plants identified. Two or more generations may be grown to ensure that this phenotypic characteristic is stably maintained. Alternatively, in vegetatively propagated crops, mature mutant/transgenic plants may be propagated by cutting or by tissue culture techniques to produce identical plants. Selection of mutant/transgenic plants can be carried out and new varieties may be obtained and propagated vegetatively for commercial use. For a general description of plant transformation and regeneration see, for example, Walbot et al. (1983) in "Genetic Engineering of Plants", Kosuge et al. (eds.) Plenum Publishing Corporation, 1983 and "Plant Cell, Tissue and Organ Culture: Fundamental Methods", Gamborg and Phillips (Eds.), Springer-Verlag, Berlin (1995). See also Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000).

Plants transformed/mutated by the methods of the invention may be screened based on expression of a marker gene, by detecting root architecture modulation by the introduced nucleotide sequence, molecular analysis using specific oligonucleotide probes and/or amplification of the target gene.

Modulation of root architecture may also be achieved through increasing or decreasing RAR signaling by modulation of the affinity of one or more RARs for the corresponding RAR receptor(s) through mutation of the RAR(s) or RAR receptor(s), or introducing exogenous sequences coding for one or more RAR(s) or RAR receptor(s) with desirable signaling interaction attributes. Methods for introducing mutations into target nucleotide sequences, and screening thereof, are described further below.

Furthermore, it is also known that microRNAs (small post-transcriptional regulators that bind to complementary sequences on target mRNAs, resulting in translational repression or target degradation and gene silencing) are expressed by plants, and that these play a significant role in control of most, if not all, plant development regulatory mechanisms. See, for example, Voinnet O (2009) Cell 136(4): 669-687; Jones-Rhoades MW et al (2006) Annual Review of Plant Biology 57:19-53. It is therefore contemplated that RARs and RAR receptor(s) would be subject to such regulation, the amount of mRNA encoding these species present in plant cells being regulated by expression of such microRNAs. Control or inhibition of expression of such microRNAs, or control of their interaction with targeted RAR or RAR receptor mRNAs or their inactivation (such as by use of microRNA decoys - see, for example, Ivashuta S et al (2011) PLoS ONE 6(6): e21330) is therefore contemplated as a further, or as a complementary means for modulating plant root architecture. Identification of endogenous plant microRNAs which target RAR-encoding or RAR receptor-encoding mRNAs may be achieved using the nucleotide sequence encoding the subject RAR or RAR receptor, or homologues thereof by methods well known in the art. Alternatively an artificial microRNA approach could be adopted as disclosed in Schwab R et al. (2006), "Highly Specific Gene Silencing by Artificial MicroRNAs in Arabidopsis", Plant Cell 18:1121-1133. RNAi gene silencing is another approach to silence plant or nematode genes *in planta,* as disclosed in Rosso MN et al. (2009) "RNAi and Functional Genomics in Plant Parasitic Nematodes" Annual Review of Phytopathology 47: 207-232, and Plant Biotechnol J.(2011) 10:1467-7652. "Biotechnological application of functional genomics towards plant-parasitic nematode control".

Alternatively, avoidance of microRNA suppression of RAR or RAR receptor expression may be achieved by introducing into a subject plant, as described above, an exogenous RAR-encoding or RAR receptor-encoding sequence sufficiently different to any endogenous homologue sequences such that the microRNA is insufficiently homologous to the introduced sequence to achieve suppression. RKN RAR-encoding sequences may be advantageous in this regard.

The methods described above, may result in plants having greater primary root development, at the expense of lateral root development, and may exhibit greater density of root hairs at periodic root bumps. The methods may also result in an increase in symbiotic nitrogen-fixing nodule numbers on the root(s) of plants, such as members of the Fabaceae or Leguminoseae, or even nitrogen-fixing nodules on the roots of plants which normally do not have nitrogen-fixing nodules (such as, for example, cereals).

Methods of the present invention for modulating root architecture, based on the herein disclosed understanding of RARs and their effects, may also include methods for promoting lateral root development in plants compared to wild-type plants. The studies leading to the present invention found that overexpression of RARs leads to suppression of lateral root development. It is contemplated that suppression of RAR expression will, conversely, promote lateral root growth and development.

Methods of the invention as defined in the claims for promoting lateral root growth and development, relative to an untreated or wild-type plant, may comprise contacting the root zone of said plant with a RAR antagonist or introducing at least one mutation or at least one exogenous nucleic acid into one or more plant cells which at least one mutation or nucleic acid results in:
(i) decreased expression of one or more RARs, decreased expression of one or more RAR receptors, or decreased expression of one or more RARs and one or more RAR receptors by root cells of a plant regenerated from or comprising said one or more plant cells, wherein said decreased expression of said RAR(s) or RAR receptor(s) occurs under conditions which would otherwise promote expression of said RAR(s) or RAR receptor(s); or
(ii) reduced affinity of one or more RARs for their respective RAR receptors, which reduced affinity arises through modifications in the RAR(s), RAR receptor(s) or in both expressed RAR(s) and RAR receptor(s) expressed by root cells of a plant regenerated from or comprising said one or more plant cells.

A RAR signaling antagonist for use in a method of the present invention or disclosure, respectively, may be any substance that interferes with the physiological response usually triggered by an RAR when it binds to its receptor, or which interferes with binding of the RAR to its receptor. For example, a RAR antagonist may be a substance that binds to a RAR or a RAR receptor to inhibit interaction between the RAR and the RAR receptor, which interaction would trigger a root architectural response. RAR antagonists may include antibodies to RARs or RAR receptors.

Decreased expression of one or more RARs, one or more RAR receptors, or both, may be achieved by any suitable technique, many being known in the art, including, antisense technology, interfering RNA technology, ribozyme technology, mutation of the gene(s) to create null mutants, and replacement or mutation of regulatory regions to reduce or obviate gene expression.

For example, a method of the invention may comprise inserting into said one or more plant cells exogenous nucleic acid which inhibits expression of the activity of an endogenous RAR (for example, via regulatory regions controlling expression of a RAR, via the RAR-encoding sequence, or via mRNA translated from the RAR-encoding sequence), or which replaces expression of an endogenous RAR or homologue thereof with expression of an exogenous protein. The exogenous protein may be an exogenous mutant RAR or homologue thereof, or any other suitable protein, such as a protein providing a screenable phenotype.

A plant with promoted lateral root growth or development, relative to a wild-type plant, may also be created by inhibiting translation of an *RAR* mRNA by RNA interference (RNAi), antisense or post-transcriptional gene silencing techniques. The *RAR* gene targeted for down-regulation, or a fragment thereof, may be utilized to control the production of the encoded protein. Full-length antisense molecules can be used for this purpose. Alternatively, double stranded oligonucleotides, sense and/or antisense oligonucleotides, or a combination thereof targeted to specific regions of the RAR-encoded RNA may be utilized. The use of oligonucleotide molecules to decrease expression levels of a pre-determined gene is known in the art (see, for example, Hamilton, A.J. and Baulcombe, D.C. (1999), Science 286:950-952; Waterhouse P.M. et al (1998), Proc. Natl. Acad. Sci. USA 95:13959-13964; Fire et al. (1998) Nature 391: 806-811; Hammond, et al. (2001) Nature Rev, Genet. 2: 110-1119; Hammond et al. (2000) Nature 404: 293-296; Bernstein et al. (2001) Nature 409: 363-366; Elbashir et al (2001) Nature 411: 494-498; and International patent publications WO 99/53050, WO 99/49029, WO 99/32619). RNA interference (RNAi) refers to a means of selective post-transcriptional gene silencing by destruction of specific mRNA by small interfering RNA molecules (siRNA). The siRNA is typically generated *in vivo* by cleavage of double stranded RNA, where one strand is identical to the message to be inactivated. Double-stranded RNA molecules may be synthesised in which one strand is identical to a specific region of the mRNA transcript and introduced directly. Alternatively corresponding dsDNA can be employed, which, once presented intracellularly is converted into dsRNA. Methods for the synthesis of suitable single or double-stranded oligonucleotides, or constructs capable of expressing them *in planta* for use in antisense or RNAi and for achieving suppression of gene expression are known to those of skill in the art. The skilled addressee will appreciate that a range of suitable single- or double-stranded oligonucleotides capable of inhibiting the expression of the disclosed polynucleotides, or constructs capable of expressing them *in planta* can be identified and generated based on knowledge of the sequence of the gene in question using routine procedures known to those skilled in the art without undue experimentation. Oligonucleotide molecules may be provided *in situ* by transforming plant cells with a DNA construct which, upon transcription, produces double stranded and/or antisense RNA sequences, which may be full-length or partial sequences. The gene silencing effect may be enhanced by overproducing both sense and/or antisense sequences (which may be full-length or partial) so that a high amount of dsRNA is produced.

Suitable molecules can be manufactured by chemical synthesis, recombinant DNA procedures or by transcription *in vitro* or *in vivo* when linked to a promoter, by methods known to those skilled in the art, and may be modified by chemistries well known in the art for stabilising the molecules *in vivo* and/or enhancing or stabilising their interaction with target complexes or molecules.

Those skilled in the art will appreciate that there need not necessarily be 100% nucleotide sequence match between the target sequence and the RNAi sequence. The capacity for mismatch is dependent largely on the location of the mismatch within the sequences. In some instances, mismatches of 2 or 3 nucleotides may be acceptable but in other instances a single nucleotide mismatch is enough to negate the effectiveness of the siRNA. The suitability of a particular siRNA molecule may be determined using routine procedures known to those skilled in the art without undue experimentation.

Sequences of/for antisense constructs may be derived from various regions of the target gene(s). Antisense constructs may be designed to target and bind to regulatory regions of the nucleotide sequence, such as the promoter, or to coding (exon) or non-coding (intron) sequences. Antisense constructs may be generated which are at least substantially complementary across their length to the region of the gene in question. Binding of an antisense construct to its complementary cellular sequence may interfere with transcription, RNA processing, transport, translation and/or mRNA stability.

In particular aspects of the invention or disclosure, respectively, suitable sequences encoding inhibitory nucleic acid molecules may be administered in a vector. The vector may be a plasmid vector, a viral vector, or any other suitable vehicle adapted for the insertion of foreign sequences and introduction into eukaryotic cells. Preferably the vector is an expression vector capable of directing the transcription of the DNA sequence of an inhibitory nucleic acid molecule in context with the invention into RNA.

Transgenic plants expressing a sense and/or antisense RAR-encoding sequence, or a portion thereof under an inducible promoter are also contemplated in context with the present invention or disclosure, respectively. Promoters inducible by nutrient conditions, such as low nitrogen are especially contemplated by the present invention. Promoters which may be used according to the invention may include, for example, the root-specific glutamine synthetase gene promoters, or promoters such as the *M. truncatula* promoter disclosed herein as SEQ ID NO: 337, for expression in the transformed plant.

Suitable constructs and vectors and transformation techniques for introducing inhibitory nucleic acids or constructs encoding them into plants, as well as methods for regenerating plants from transformed cells have already been discussed above.

As mentioned above, a further means of inhibiting gene expression may be achieved by introducing catalytic antisense nucleic acid constructs, such as ribozymes, which are capable of cleaving RNA transcripts and thereby preventing the production of the native protein. Ribozymes are targeted to and anneal with a particular sequence by virtue of two regions of sequence complementarity to the target flanking the ribozyme catalytic site. After binding, the ribozyme cleaves the target in a site-specific manner. The design and testing of ribozymes which specifically recognize and cleave sequences of interest, such as RAR-encoding sequences and RAR receptor-encoding sequences, can be achieved by techniques well known to those in the art (for example Lieber and Strauss, (1995) Mol. Cell. Biol. 15:540-551, and de Feyter R and Gaudron J (1998) "Expressing Ribozymes in Plants", Methods in Molecular Biology 74: 403-415).

Suitable constructs and vectors and transformation techniques for introducing ribozymes or constructs encoding them into plants, as well as methods for regenerating plants from transformed cells have already been discussed above.

Similar to the situation described above, where RAR or RAR receptor expression or overexpression is promoted to modulate root architecture, microRNA manipulation may also be employed to suppress RAR or RAR receptor expression. It is contemplated that overexpression, or constitutive expression of microRNAs specifically targeting subject RAR-encoding or RAR receptor-encoding sequences may be employed to suppress expression of those sequences to promote lateral root growth or development. Alternatively, exogenous nucleotide construct(s) encoding microRNAs specific for subject RAR-encoding or RAR receptor-encoding sequences, under the control of desired regulatory sequences may be introduced into plant cells, as disclosed in Schwab R et al. (2006), "Highly Specific Gene Silencing by Artificial MicroRNAs in Arabidopsis", Plant Cell 18:1121-1133. Modulation of root architecture by a method of the present invention may also be achieved by modulating the affinity of RAR(s) for respective RAR receptor(s). Reduced affinity or reduced expression of one or more RARs for their respective RAR receptors, or *vice versa,* so as to promote lateral root growth in plants, rather than primary root growth, may be effected by a number of means, such as through modifications in endogenous sequences coding for the RAR(s) or RAR receptor(s), or by replacing the RAR-encoding sequence(s) or RAR receptor-encoding sequence(s) with sequences coding for an RAR or RAR receptor having less binding affinity for the corresponding molecule.

Modifications in endogenous sequences coding for RAR(s) or RAR receptor(s) may be achieved by *in situ* mutation, either by physical or chemical mutagenesis or by introduction of exogenous nucleic acid which introduces mutations into the target nucleotide sequence(s).

In one embodiment the exogenous nucleic acid may comprise an oligonucleotide or polynucleotide which introduces a mutation comprising single or multiple nucleotide insertions, deletions or substitutions into the endogenous nucleotide sequence encoding an RAR or an RAR receptor, or a homologue(s) thereof via homologous recombination.

Single or multiple nucleotide insertions, deletions or substitutions may be introduced via recombination of the target mutation site with an introduced targeting nucleotide sequence. Such an introduced nucleotide sequence may, for example, comprise a nucleotide sequence to be introduced into the genome flanked either side by nucleotide sequences homologous to target sequences contiguous in or located either side of a desired mutation insertion point. In accordance with the methods of the present invention, a nucleotide sequence to be introduced into the genome may also include a selectable marker operably linked to desired regulatory regions (which may include, for example, a root-specific promoter).

The nucleotide sequences homologous to the target sequences may be isogenic with the target sequences to thereby promote the frequency of homologous recombination.

Homologous nucleotide sequences that are not strictly isogenic to the target sequences can also be used. Although mismatches between the homologous nucleotide sequences and the target sequences can adversely affect the frequency of homologous recombination, isogenicity is not strictly required and substantial homology may be sufficient. For the purposes of the present invention, the level of homology between the homologous sequences and the target sequences may be at least about 90% identity, at least about 95% identity, at least about 99% identity or 100% identity.

A targeting nucleotide sequence can be comprised in a vector. Representative vectors include plasmids, cosmids, and viral vectors. Vectors can also comprise nucleic acids including expression control elements, such as transcription/translation control signals, origins of replication, polyadenylation signals, internal ribosome entry sites, promoters, enhancers, etc., wherein the control elements are operatively associated with a nucleic acid encoding a gene product. Selection of these and other common vector elements are conventional and many such sequences can be derived from commercially available vectors. See, for example, Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000).

A targeting vector can be introduced into targeting cells using any suitable method known in the art for introducing DNA into cells, including but not limited to microinjection, electroporation, calcium phosphate precipitation, liposome-mediated delivery, viral infection, protoplast fusion, and particle-mediated uptake.

Optionally, a targeting DNA is co-administered with a recombinase, for example recA, to a target cell to thereby enhance the rate of homologous recombination. The target cell(s) may already comprise, or have been transformed to comprise suitable recombinase target sequences, if required. For example, a recombinase protein(s) can be loaded onto a targeting DNA as described in U.S. Pat. No. 6,255,113. To enhance the loading process, a targeting DNA can contain one or more recombinogenic nucleation sequences. A targeting DNA can also be coated with a recombinase protein by pre-incubating the targeting polynucleotide with a recombinase, whereby the recombinase is non-covalently bound to the polynucleotide. See, for example, A. Vergunst et al (1998), Nucleic Acids Res. 26:2729 and A. Vergunst and P. Hooykaas (1998), Plant Molec. Biol. 38:393 406, International patent publications WO 99/25821, WO 99/25840, WO 99/25855, and WO 99/25854 and U.S. Pat. Nos. 5,780,296, 6,255,113, and 6,686,515.

Suitable constructs and vectors and transformation techniques for introducing targeting sequences as discussed above, as well as methods for regenerating plants from transformed cells have already been discussed above.

Plants transformed/mutated by the methods of the invention may be screened based on the lack of or reduced expression, or of overexpression of a RAR or RAR receptor protein, or homologues thereof, or of their activity or by observation of modulated root growth compared to wild-type plants, molecular analysis using specific oligonucleotide probes and/or amplification of the target gene.

A mutation which results in reduced expression of RAR(s) or RAR receptor(s), or homologues thereof in plant cells may be introduced into the one or more plant cells by any appropriate methods as are known in the art. For example, suitable methods may comprise exposing the one or more plant cells (which may be plant seed cells, or cells of a part of a plant, as well as isolated plant cells) to chemical or physical mutagenic means, or insertional mutagenic means such as transposons, retrotransposons, retroviruses, or T-DNA. Suitable materials and methods for introducing mutations into a plant genome are also described in, for example, International patent publication WO 98/26082, "Arabidopsis Protocols" (2nd Edition, Salinas, J. and Sanchez-Serrano, J., eds, Methods in Molecular Biology 323 (2006), Humana Press), and "Current Protocols in Molecular Biology" (Ausubel et al. (eds), John Wiley & Sons (2000)).

The mutation may also be introduced into the one or more plant cells by crossing a wild-type plant with a plant comprising a desirable mutation (as determined previously by genetic screening and/or analysis - plants comprising a desired mutation may already exist in available plant germplasm/culture/seed collections/varieties), and plants may be generated from the resulting seed and then screened for inheritance of the mutation.

The mutation(s) may be introduced into one or more sequence(s) encoding RAR(s) or RAR receptor(s), or may be introduced into other sequences affecting expression of those proteins (such as upstream sequences, including promoters).

According to an embodiment of the invention, a mutation is introduced into a nucleotide sequence encoding a RAR or RAR receptor or a homologue thereof in one or more plant cells, and may comprise an insertion, deletion or substitution of one or more nucleotides in the nucleotide sequence encoding the RAR or RAR receptor or homologue thereof. In one embodiment the mutation is a RAR or RAR receptor null mutation. Alternatively, the mutation may result in an expressed product which, however, has at least reduced affinity for its binding partner.

The methods of the present invention can employ any mutagenic agent known in the art (employing methods also known in the art) including, but not limited to ultraviolet light, X-ray radiation, gamma radiation or fast neutron mutagenesis, N-ethyl-N-nitrosourea (ENU), methylnitrosourea (MNU), procarbazine (PRC), triethylene melamine (TEM), acrylamide monomer (AA), chlorambucil (CHL), melphalan (MLP), cyclophosphamide (CPP), diethyl sulfate (DES), ethyl methane sulfonate (EMS), methyl methane sulfonate (MMS), 6-mercaptopurine (6-MP), mitomycin-C (MMC), N-methyl-N'-nitro-N-nitrosoguanidine (MNNG), ³H₂O, and urethane (UR).

The frequency of genetic modification upon exposure to one or more mutagenic agents can be modulated by varying dose and/or repetition of treatment, and can be tailored for a particular application. The treatment dose and regimen may not induce substantial cytotoxicity to the one or more cells.

Mutations in RAR(s) or RAR receptor(s) or homologues thereof can be detected and followed (through generations) by probing with known RAR-encoding sequence(s), such as are disclosed herein (see SEQ ID NOs: 3, 5, 7, 9, 11, 13, 15, 17, 19, 21, 23, 25, 27, 29, 31, 33, 35, 364, 366, 368, 370, 372, 374, 376, 378, 380, 382, 384 or 386) or RAR receptor-encoding sequence(s), which may be identified as described above, using techniques well known in the art and suitable probes or primers based on the gene(s) or nucleotide sequence(s) encoding RAR(s), RAR receptor(s) or homologues thereof.

If the mutation is in a sequence other than RAR-encoding sequence(s) or RAR receptor-encoding sequence(s), the mutation may need to be identified, located and/or characterised before it can be traced/followed through plant generations. Suitable methods for identifying, locating and characterising unknown mutations are known to those in the art and are described in a number of well-known standard texts, such as Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000). See also Rossel, J.B., Cuttriss, A. and Pogson, B.J. "Identifying Photoprotection Mutants in Arabidopsis thaliana" in Methods in Molecular Biology 274: 287-299 (Carpentier, R. ed, Humana Press). More recent methods for identifying mutant alleles include 'Tilling' and high resolution melts (HRMs).

TILLING (Targeting Induced Local Lesions in Genomes) is a method in molecular biology that allows directed identification of mutations in a specific gene. The method combines a standard technique (for example, mutagenesis with a chemical mutagen such as Ethyl methanesulfonate (EMS)) with a sensitive DNA screening-technique that identifies single base mutations (also called point mutations) in a target gene. The first paper describing TILLING in *Arabidopsis* (McCallum CM, Comai L, Greene EA, Henikoff S, "Targeted screening for induced mutations", Nat Biotechnol. (2000) Apr;18(4):455-7) used dHPLC HPLC to identify mutations. The method was made more high throughput by using the restriction enzyme Cel-I combined with a gel based system to identify mutations (Colbert T, Till BJ, Tompa R, Reynolds S, Steine MN, Yeung AT, McCallum CM, Comai L, Henikoff S, "High-throughput screening for induced point mutations", Plant Physiol. (2001) Jun;126(2):480-4). Other methods of mutation detection, such as resequencing DNA, have been combined for TILLING. TILLING has since been used as a reverse genetics method in other organisms such as zebrafish, corn, wheat, rice, soybean, tomato and lettuce. See also: McCallum CM, Comai L, Greene EA, Henikoff S. "Targeting induced local lesions in genomes (TILLING) for plant functional genomics" Plant Physiol. (2000) Jun;123(2):439-42; Colbert T, Till BJ, Tompa R, Reynolds S, Steine MN, Yeung AT, McCallum CM, Comai L, Henikoff S. High-throughput screening for induced point mutations", Plant Physiol. (2001) Jun;126(2):480-4; Draper BW, McCallum CM, Stout JL, Slade AJ, Moens CB, "A high-throughput method for identifying N-ethyl-N-nitrosourea (ENU)-induced point mutations in zebrafish", Methods Cell Biol. (2004);77:91-112; and Slade AJ, Fuerstenberg SI, Loeffler D, Steine MN, Facciotti D, "A reverse genetic, nontransgenic approach to wheat crop improvement by TILLING", Nat Biotechnol. (2005) Jan;23(1):75-81.

HRM (High Resolution Melt) is a recent development that can greatly extend the utility of traditional DNA melting analysis by taking advantage of recent improvements in high resolution melt instrumentation and the development of double strand specific DNA (dsDNA) binding dyes that can be used at high enough concentrations to saturate all double stranded sites produced during PCR amplifications (see http://www.corbettlifescience.com/control.cfm?page=Introduction_4&bhcp=1), as well as: Dufresne SD, Belloni DR, Wells WA, Tsongalis GJ, "BRCA1 and BRCA2 Mutation Screening using SmartCyclerII high-resolution melt curve analysis", Arch Pathol Lab Med (2006) 130: 185-187; Graham R, Liew M, Meadows C, Lyon E, Wittwer CT, "Distinguishing different DNA heterozygotes by high resolution melting", Clinical Chemistry (2005) 51: 1295-1298; Hermann MG, Durtschl JD, Bromley K, Wittwer CT, Voelkerding KV, "Amplicon DNA melting analysis for mutation scanning and genotyping: cross-platform comparison of instruments and dyes", Clinical Chemistry (2006) 52: 494-503; Liew M, Pryor R, Palais R, Meadows C, Erali M, Lyon E, Wittwer C, "Genotyping of single nucleotide polymorphisms by high resolution melting of small amplicons", Clinical Chemistry (2004) 50: 1156-1164; Margraf RL, Mao R, Highsmith WE, Holtegaard LM, Wittwer CT, "Mutation Scanning of the RET protooncogene using high resolution melting analysis", Clinical Chemistry (2006) 52: 138-141; NGRL (Wessex) Reference Reagent Report January 2006, "Plasmid based generic mutation detection reference reagents; production and performance indicator field trial" (www.ngrl.org.uk/Wessex/downloads.htm); NGRL (Wessex) Reference Reagent Report January 2006. "Production and field trial evaluation of reference reagents for mutation screening of BRCA1, BRCA2, hMLH1 and MHS2" (www.ngrl.org.uk/Wessex/downloads.htm); NGRL (Wessex) Reference Reagent Report June 2006, "Mutation Scanning by High Resolution Melts: Evaluation of Rotor-Gene™ 6000 (Corbett Life Science), HR-1™ and 384 well LightScanner™ (Idaho Technology)" (www.ngrl.org.uk/Wessex/downloads.htm); Reed GH, Wittwer CT, "Sensitivity and specificity of single-nucleotide polymorphism scanning by high resolution melting analysis", Clinical Chemistry (2004) 50: 1748-1754; Willmore-Payne C, Holden JA, Tripp S, Layfield LJ, "Human malignant melanoma: detection of BRAF- and c-kit-activating mutations by high-resolution amplicon melting analysis", Human Pathology (2005) 36: 486-493; Wittwer CT, Reed GH, Gundry CN, Vandersteen JG, Pryor RJ, "High-resolution genotyping by amplicon melting analysis using LCGreen" Clinical Chemistry (2003) 49: 853-860; Worm J, Aggerholm A, Guldberg P, "In-tube DNA methylation profiling by fluorescence melting curve analysis" Clinical Chemistry (2001) 47: 1183-1189; Zhou L, Myers AN, Vandersteen JG, Wang L, Wittwer CT, "Closed-tube genotyping with unlabeled oligonucleotide probes and a saturating DNA dye", Clinical Chemistry (2004) 50: 1328-1335; and Zhou L, Wang L, Palais R, Pryor R, Wittwer CT, "High-resolution DNA melting analysis for simultaneous mutation scanning and genotyping in solution", Clinical Chemistry (2005) 51: 1770-1777.

Oligonucleotide primers can be designed or other techniques can be applied to screen lines for mutations/insertions in RAR-encoding sequence(s) or RAR receptor-encoding sequence(s). Through breeding, a plant line may then be developed that is homozygous for the mutated copy of the RAR-encoding sequence(s) or RAR receptor-encoding sequence(s). PCR primers for this purpose may be designed so that a large portion of the coding sequence of the desired sequence is specifically amplified using the sequence of the sequence from the species to be probed (see, for example, Baumann, E. et al. (1998), "Successful PCR-based reverse genetic screens using an En-1-mutagenised Arabidopsis thaliana population generated via single-seed descent", Theor. Appl. Genet. 97:729 734).

Other RAR or RAR receptor mutants may be isolated from mutant populations or existing germplasm using the distinctive phenotypes characterized in accordance with the present invention (such as modulated root architecture compared to the wild-type plants). That the phenotype is caused by a mutation in RAR-encoding sequence(s) or RAR receptor-encoding sequence(s) or a homologue thereof may then be established by molecular means well known in the art.

RAR or RAR receptor mutants, including mutants heterozygous for the allele, and which may not express the modulated phenotype, may also be screened for, as described herein, and the mutants used for breeding programs to introgress the mutation into homozygous line, or the mutant gene isolated and used in recombinant techniques for generating mutant plants.

While mutants of the present invention may be generated by random mutagenesis (or may already exist), any plant may be recombinantly engineered to display a similar phenotype, for example once the genetic basis of the mutation, such as a mutated RAR-encoding gene, has been determined. For a general description of plant transformation and regeneration see, for example, Walbot et al. (1983) in "Genetic Engineering of Plants", Kosuge et al. (eds.) Plenum Publishing Corporation, 1983 and "Plant Cell, Tissue and Organ Culture: Fundamental Methods", Gamborg and Phillips (Eds.), Springer-Verlag, Berlin (1995). See also Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000).

Screening a plant for the presence of at least one mutant allele of a nucleotide sequence encoding a RAR, RAR receptor, or homologue thereof, may comprise analysing DNA of the plant using at least one nucleic acid molecule suitable as a probe or primer which is capable of hybridising to a *RAR* gene, RAR receptor gene, or homologue thereof under stringent conditions. In a more specific method, the screening method may comprise the use of at least one oligonucleotide primer pair suitable for amplification of a region of the *RAR* gene, RAR receptor gene, or homologue thereof, comprising a forward primer and a reverse primer to detect the presence or absence of a mutation in said region. The region may comprise the whole *RAR* gene, RAR receptor gene, or homologue thereof, or may comprise only a portion thereof.

DNA from the plant to be assessed may be extracted by a number of suitable methods known to those skilled in the art, such as are described in a wide range of well known texts, including (but not limited to) Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000). See also the methods described in Lukowitz, W., Gillmor, C.S. and Scheble, W-R. (2000) "Positional Cloning in Arabidopsis: Why It Feels Good to Have a Genome Initiative Working for You" Plant Physiology 123, 795-805, and references cited therein.

Once suitable DNA has been isolated, this may be analysed for the presence or absence of a mutation by any suitable method as known in the art, and which method/strategy is employed may depend on the specificity desired, and the availability of suitable sequences and/or enzymes for restriction fragment length polymorphism (RFLP) analysis. Suitable methods may involve detection of labelled hybridisation product(s) between a mutation-specific probe and at least a portion of the *RAR* gene, RAR receptor gene, or homologue thereof or, more typically, by amplification of at least a portion of the *RAR* gene, RAR receptor gene, or homologue thereof using either a primer and suitable probe, or using a pair of primers (forward and reverse primers) for amplification of a specific portion of the *RAR* gene, RAR receptor gene, or homologue thereof, followed by either direct partial and/or complete sequencing of the amplified DNA, or RFLP analysis thereof. Suitable primer pairs for amplifying portions of *RAR* genes from *Medicago truncatula* are provided in Table 1 - other suitable primers or primer pairs for analysing *RAR* genes or homologues thereof may be designed based on anyone of SEQ ID NOs: 3, 5, 7, 9, 11, 13, 15, 17, 19, 21, 23, 25, 27, 29, 31, 33, 35, 364, 366, 368, 370, 372, 374, 376, 378, 380, 382, 384 or 386.

The methods and reagents for use in a PCR amplification reaction are well known to those skilled in the art. Suitable protocols and reagents will largely depend on individual circumstances. Guidance may be obtained from a variety of sources, such as for example Sambrook, J. et al., Molecular Cloning, Cold Spring Harbor Laboratory (1989), Sambrook, J. and Russell, D.W. (2001), "Molecular Cloning: A Laboratory Manual", 3rd edition, Cold Spring Harbor Laboratory Press, and references cited therein and Ausubel et al. (eds) Current Protocols in Molecular Biology, John Wiley & Sons (2000).

A person skilled in the art would readily appreciate that various parameters of the PCR reaction may be altered without affecting the ability to amplify the desired product. For example the Mg²⁺ concentration and temperatures employed may be varied. Similarly, the amount of genomic DNA used as a template may also be varied depending on the amount of DNA available.

Other methods of analysis of the amplified DNA to determine the presence or absence of a mutation are well known to those skilled in the art. For instance, following digestion of the amplified DNA with a suitable restriction enzyme to detect a mutation in a *RAR* gene, RAR receptor gene, or homologue thereof, the DNA may be analysed by a range of suitable methods, including electrophoresis. Of particular use is agarose or polyacrylamide gel electrophoresis, a technique commonly used by those skilled in the art for separation of DNA fragments on the basis of size. The concentration of agarose or polyacrylamide in the gel in large part determines the resolution ability of the gel and the appropriate concentration of agarose or polyacrylamide will therefore depend on the size of the DNA fragments to be distinguished.

Detection and/or determination of the existence of a mutation in a *RAR* gene, RAR receptor gene, or homologue thereof may be aided by computer analysis using any appropriate software. Suitable software packages for comparison of determined nucleotide sequences are well known in the art and are readily available.

Methods for modulating root architecture in plants, as compared to wild-type plants, may also be developed along any of the lines described above based on RKN RGFs (see SEQ ID NOs: 416 or 417) and their encoding sequences, and/or RKN IDAs (see SEQ ID NO: 418) and their encoding sequences.

### Inhibition of RKN-modulation of root architecture

Further contemplated herein are methods for inhibiting root architecture modulation by root knot nematodes (RKNs). As mentioned previously, RKNs are obligate parasites infecting a broad spectrum of vascular plants, causing crop yield losses estimated at more than USD50 billion per annum, yet effective RKN control strategies are limited. Similarities observed during the present studies between the bumps formed on developing roots in plants, overexpressing RARs, development of nodules in legumes, and development of galls formed on plant roots as a result of RKN infestation led to our discovery that RKNs, but not other nematodes possess RAR sequences. The 'knots' induced by RKN appear to incorporate two developmental responses: gall formation, which involves limited proliferation of mainly cortical cells in a circumferential manner around the root and the formation of RKN feeding sites in the plant vascular tissue, which include giant cells. RKN and *Medicago* RAR peptides only induce the circumferential cell proliferation (CCP sites), not RKN feeding sites. The apparent unique distribution of RAR only in root knot, but not other nematode genomes (including cyst nematodes and other plant associating nematodes), and their expression during RKN infection, suggests that RAR may be required as a virulence determinant.

Thus, it is contemplated that modulation of plant root architecture by RKNs may be inhibited by inhibiting expression of RKN RARs, and/or by interfering with RKN RAR interaction with plant RAR receptors or by mimicking endogenous plant RAR ligands and thereby disrupting normal RAR function. This may be achieved by a number of means, including:
(a) contacting the root zone of said plant with a root knot nematode RAR signaling antagonist;
(b) introducing at least one exogenous nucleic acid into one or more plant cells which results in modulated RGF expression in a root knot nematode;
(c) introducing at least one mutation or exogenous nucleic acid into one or more plant cells which results in modulated affinity of one or more root knot nematode RARs for plant RAR receptors, which modulated affinity arises through modifications in the plant RAR receptor(s); or
(d) introducing at least one exogenous nucleic acid into one or more plant cells which results in expression of one or more root knot nematode RGF binding agents or antagonists by said cell(s).

An RKN RAR signaling antagonist for use herein may be any substance that interferes with the physiological response usually triggered by an RKN RAR when it binds to a plant RAR receptor, or which interferes with binding of the RKN RAR to a plant RAR receptor. For example, a RKN RAR antagonist may be a substance that binds to a RKN RAR to inhibit interaction between the RKN RAR and the plant RAR receptor, which interaction would trigger a root architectural response. RKN RAR antagonists may include antibodies to RKN RARs.

An antibody (or fragment thereof) may be raised against a RKN RAR or immunogenic portion thereof using any suitable method known in the art. For instance, a monoclonal antibody, typically containing Fab portions, may be prepared using the hybridoma technology described in Antibodies-A Laboratory Manual, Harlow and Lane, eds., Cold Spring Harbor Laboratory, N.Y. (1988). Any technique that provides for the production of antibody molecules by continuous cell lines in culture may be used. Suitable techniques include the hybridoma technique originally developed by Kohler et al., Nature, 256:495-497 (1975), the trioma technique, the human B-cell hybridoma technique [Kozbor et al., Immunology Today, 4:72 (1983)], and the EBV-hybridoma technique to produce human monoclonal antibodies [Cole et al., in Monoclonal Antibodies and Cancer Therapy, pp. 77-96, Alan R. Liss, Inc., (1985)]. Immortal, antibody-producing cell lines can be created by techniques other than fusion, such as direct transformation of B lymphocytes with oncogenic DNA, or transfection with Epstein-Barr virus. See, e.g., M. Schreier et al., "Hybridoma Techniques" (1980); Hammerling et al., "Monoclonal Antibodies and T-cell Hybridomas" (1981); Kennett et al., "Monoclonal Antibodies" (1980).

For convenient production of adequate amounts of antibody(ies), these may be manufactured by batch fermentation with serum free medium, and then purified via a multistep procedure incorporating chromatography and viral inactivation/removal steps. For example, the antibody may be first separated by Protein A affinity chromatography and then treated with solvent/detergent to inactivate any lipid enveloped viruses. Further purification, typically by size-exclusion, reverse-phase, anion and/or cation exchange chromatographies, may be used to remove residual undesired contaminants such as proteins, solvents/detergents and nucleic acids. The antibody(ies) thus obtained may be further purified and formulated as desired.

These antibodies can include but are not limited to polyclonal, monoclonal, chimeric, single chain, Fab fragments, and a Fab expression library.

A monoclonal antibody refers to an antibody secreted by a single clone of antibody-producing cells and which is monospecific for a particular antigen or epitope. Therefore, a monoclonal antibody displays a single binding affinity for any antigen with which it immunoreacts.

There are also various procedures known in the art which may be used for the production of polyclonal antibodies to a RKN RAR or immunogenic portion thereof. For example, one or more host animals can be immunised by injection with the relevant polypeptide, or a derivative (e.g., fragment or fusion protein) thereof. Suitable hosts include, for example, rabbits, mice, rats, sheep, goats, etc.

The antibody (or fragment thereof) will have a binding affinity or avidity. This binding affinity or avidity may be greater than about 10⁵ M⁻¹, such as greater than about 10⁶ M⁻¹, greater than about 10⁷ M⁻¹, or greater than about 10⁸ M⁻¹.

Rather than apply antibodies to the root zone of a plant, it may be advantageous to transform a plant to produce antibodies specific for RKN RARs. Once monoclonal antibodies have been raised against a RKN RAR, by methods referred to above, sequences encoding the desired antibody component(s) may be cloned from cDNAs, assembled into plant expression vectors, and transfected into plant cells. Methods for expressing antibodies in plant cells have been well described in the art. See, for example, Stoger E et al (2004), "Antibody production in transgenic plants", in Lo BKC (ed) "Methods in Molecular Biology. Antibody Engineering: Protocols and Methods" (2nd edn). Humana Press Inc., NJ, pp 301-318; Fischer R et al (2003), "Production of antibodies in plants and their use for global health", Vaccine 21: 820-825; Wycoff KL (2005), "Secretory IgA antibodies from plants", Curr Pharm Des. 11(19): 2429-37; and Prins M et al (2008), "Strategies for antiviral resistance in transgenic plants", Molecular Plant Pathology 9(1): 73-83.

The antibody(ies) may be secreted by the transformed plant cells.

Other methods for inhibiting root architecture modulation by RKNs may include introducing into plant cells exogenous nucleotide sequences coding for nucleotide molecules which, once taken up or incorporated by RKNs, suppress expression of RKN RARs. Such nucleotide sequences may encode antisense, RNAi or microRNA molecules, as described previously. Suitable constructs and vectors and transformation techniques for introducing targeting sequences, or nucleotide constructs for expressing them, as well as methods for regenerating plants from transformed cells have already been discussed above.

Yet further methods for modulation of root architecture may comprise decreasing RKN RAR signaling by modulation of the affinity of one or more RKN RARs for plant RAR receptor(s) through mutation of the plant RAR receptor(s), or introducing exogenous sequences coding for one or more plant RAR receptor(s) with less affinity for RKN RAR(s). Methods for introducing mutations into target nucleotide sequences, and screening thereof, have been described further above.

Methods as described above for inhibiting root architecture modulation by RKNs may also be developed based on the RKN root growth factors (RGFs) and IDA peptides disclosed herein. Exemplary RKN RGF peptides are disclosed herein as SEQ ID NOs: 416 and 417, having RGF domains comprising amino acid sequences as shown in SEQ ID NOs: 460 and 461 (*Meloidogyne hapla* and *M. incognita* respectively). An exemplary RKN IDA peptide is disclosed herein as SEQ ID NOs: 418, having an IDA domain comprising the amino acid sequence as shown in SEQ ID NO: 496 (*Meloidogyne hapla*).

Another route for inhibiting root architecture modulation by RKNs may be based on the fact that RKNs have been found to secrete a number of analogs of plant signaling peptides, including CLEs, as well as RARs, RGFs and IDAs, as shown by the present studies. All of these peptides are predicted to be secreted, after post-translational modifications of the product translated from the encoding sequence, and after cleavage of signal peptides from the nascent peptides.

A component of SPC (Signal peptidase complex), signal peptidase I exists in RKN genomes and is a contemplated as a target for in nematodes to disrupt the nematode secretory pathway that is critical for their parasitism. Being an early component of the protein secretory pathway in the endoplasmic reticulum (ER), the SPC cleaves signal peptides off nascent polypeptides that are destined for intracellular compartments and the extracellular matrix (Paetzel M et al. 2002 Chem Rev. 102(12):4549-80. Signal peptidases). Engineered disruption of signal peptidase I (SPI) are contemplated to disable proper secretion of extracellular proteins and peptide including RARs, RGFs, CLEs and IDAs, and thus suppress plant parasitise nematodes.

Thus, it is further disclosed that modulation of plant root architecture by RKNs may be inhibited by inhibiting a component of a root knot nematode signal peptidase complex. This may be achieved by introducing at least one exogenous nucleic acid into one or more plant cells which suppresses, or the product of which suppresses expression of a component of a root knot nematode signal peptidase complex. The component may be signal peptidase I, which may comprise a nucleotide sequence as shown in SEQ ID NO: 419, or an amino acid sequence as shown in SEQ ID NO. 420.

Thus, and as described above in the context of RARs and RGFs, methods for inhibiting root architecture modulation by RKNs may include introducing into plant cells exogenous nucleotide sequences coding for nucleotide molecules which, once ingested by RKNs, suppress expression of such components of the RKN SPC. Such nucleotide sequences may encode antisense, RNAi or microRNA molecules, as described previously. Suitable constructs and vectors and transformation techniques for introducing targeting sequences, or nucleotide constructs for expressing them, as well as methods for regenerating plants from transformed cells have already been discussed above.

### Plants with modulated root architecture or which resist root architecture modulation by RKNs

Plants having modulated root architecture as defined in the claims, or which resist root architecture modulation by RKNs, obtained by any of the methods described above, are also encompassed within the ambit of the present invention or disclosure, respectively.

Also encompassed by the present invention or disclosure, respectively, are plant parts, including but not restricted to leaves, stems, roots, tubers, flowers, fruits and seeds obtained from such plants.

Preferred forms of the present invention will now be described, by way of example only, with reference to the following examples, including comparative data, and which are not to be taken to be limiting in any way.

### Examples

### Example 1 - Materials and Methods

### Plant Materials and Growth Conditions

Seeds of *M. truncatula* cv Jemalong genotype A17 wild-type and M. *truncatula* 2HA line carrying either *GH3* promoter-*GUS* reporter fusion gene (*GH3:GUS*) were grown under standard conditions (Holmes P, Goffard N, Weiller GF, Rolfe BG, & Imin N (2008), "Transcriptional profiling of Medicago truncatula meristematic root cells", BMC Plant Biol 8:21).

### Identification of RARs in Nematodes

All available genome sequence for the plant parasitic nematodes *Meloidogyne hapla, M. incognita,* and *M. chitwoodi, Globodera rostochiensis, Heterodera glycines, Pratylenchus coffeae, Radopholus similis* as well as the free-living nematode *C. elegans* were processed to discover open reading frames between 30 and 150 amino acids long, from ATG to stop, using the program getorf. SignalP was used to search for signal sequences in all resulting ORFs, using both neural network (NN) and Hidden Markov Model (HMM) modes. A custom-made database of ORFs with an identifiable signal sequence was created and searched for the pattern "xfrPTxpGxSPGxGx" (SEQ ID NO: 421) using a double-affine Smith-Waterman algorithm from TimeLogic (TimeLogic DeCypher systems). Resulting matches were hand-curated for conservation of RAR domains as compared to RAR domains found in *A. thaliana* and *M. truncatula.*

### Identification of RARs in plants

The pervasiveness of genes with RAR domains in plant genomes was examined using the conserved 15-amino-acid *M. truncatula* RAR sequences as queries for BLAST searches (http://blast.ncbi.nlm.nih.gov/Blast.cgi).

### RNA Extraction, cDNA Synthesis and qRT-PCR Analysis.

RNA extraction, cDNA synthesis and qRT-PCR analysis was performed as described in L. Kusumawati, N. Imin, M. A. Djordjevic, (2008), "Characterization of the secretome of suspension cultures of Medicago species reveals proteins important for defense and development" J. Proteome Res. 7: 4508. The primers used are listed in Table 1 (see over). Normalization was conducted by calculating the differences between the C_{T} of the target gene and the C_{T} of *MtUBQ10* (MtGI accession number TC161574). Normalization for relative quantification for the transcript level of each gene was carried out according to 'delta-delta method (2). According to the method, the average C_{T} values of the gene of interest from the technical triplicate of a sample is subtracted with the average C_{T} values of the housekeeper gene (*MtUBQ10*) from the same sample as shown in the formula below: ΔCt = C_{T}^{gene of interest} - C_{T}^{housekeeper gene (MtUBQ10)}. The same calculation was carried out for both the control sample and the sample of study. The ΔC_{T} value obtained from the above calculation was then used to calculate the 'delta-delta' Ct value

**Table 1. Primers used for cloning of M. truncatula RAR gene and the real-time qRT-PCR analysis. Accession numbers are from either Affymetrix probe IDs or M. truncatula gene index IDs (compbio.dfci.harvard.edu) or from International Medicago Genome Annotation (www.medicago.org/genome/IMGAG/) IDs. MtRAR6-9 sequences are from unannotated sequences. *: Annotated as LOB domain-containing protein 38 (ID, Medtr4g095600.1) by IMGAG.**

| **Name** | **Accession number** | **Forward primer (5'-3')** | **Reverse primer (5'-3')** |
|---|---|---|---|
| **Gateway cloning** | | | |
| *MtRAR1* | Mtr.7265.1.S1_at | | *TCAATTTCCAATTTTGTTTTGGT* (SEQ ID NO: 423) |

| **qRT-PCR analysis** | | | |
|---|---|---|---|
| *MtRAR1* | Mtr.7265.1.S1_at | *CCGATGAAGATATCGACGTGAA* (SEQ ID NO: 424) | |
| *MtRAR2* | *META519TF* | *TAGCTCGCATTTGCTTGTTC* (SEQ ID NO: 426) | *GGCTGAATGCTTTGTCTCAA* (SEQ ID NO: 427) |
| *MtRAR3* | *TC125059* | *ACGTTGAGCTCCACCATTTT* (SEQ ID NO: 428) | *GAGCGCTCCACCTCCTATTA* (SEQ ID NO: 429) |
| *MtRAR4* | *Medtr5g025790.1* | *CATGGAGGTGGTGTTTGATG* (SEQ ID NO: 430) | *TTTTCGCCCTACAAGTCCAG* (SEQ ID NO: 431) |
| *MtRAR5* | *Medtr5g017710.1* | *GTGTTGTTTTGAGCCCAAGG* (SEQ ID NO: 432) | *TGTTGGTCGAAAAGCTTCAA* (SEQ ID NO: 433) |
| *MtRAR6* | *AC233112_1004.1* | *GCTCATCATGGAGGGAAGTC* (SEQ ID NO: 434) | *TATGCCCTGGAGATGTAGGC* (SEQ ID NO: 435) |
| *MtRAR7* | *AC233112_1013.1* | *CCGGATGTTGAGGTTTTTGT* (SEQ ID NO: 436) | *GGCCAACTCCAGGACTATGA* (SEQ ID NO: 437) |
| *MtRAR8* | *AC233112_1014.1* | *TCCAACAATATTGCCACCAA* (SEQ ID NO: 438) | *GGGTTGTGGGTCTAAAAGCA* (SEQ ID NO: 439) |
| *MtRAR9* | *AC233112_1014.1* | *TGATGCCAAATCATGGTGTC* (SEQ ID NO: 440) | *GGACTGCTTCCTGGTGTTGT* (SEQ ID NO: 441) |
| *MtRAR10* | *Medtr5g030490.1* | *TCAATGGAAGCATCAAGGTTT* (SEQ ID NO: 442) | *TATATGTCCCACCCCAAGAC* (SEQ ID NO: 443) |
| *MtRAR11* | *Medtr8g086660.1* | *AGCTCCTTCCATTGGCTTTT* (SEQ ID NO: 444) | *CCCCACCAGGACTATGACC* (SEQ ID NO: 445) |
| *MtNRT2.5* | Mtr.35456.1.S1_at | *GGAGAAGGAGAAAGGGTCTCA* (SEQ ID NO: 446) | *TCAGAAGGCCTAGTTGAAATG* (SEQ ID NO: 447) |
| *MtAGL1* | *Mtr. 15656. 1. S1_at* | *GAACCGAAGGGAAGCATAA* (SEQ ID NO: 448) | *TGTCGTGCCATACACCTTTT* (SEQ ID NO: 449) |
| *MtLBD38** | Mtr.22734.1.S1_at | *GCCACGCTACTGTTTTCGTA* (SEQ ID NO: 450) | *GAGCTGGTCTCTGTGGTTCA* (SEQ ID NO: 451) |
| *MhRAR10* | Mh_Contig368 | *GCACCTCAACCTCCTTTCTGCA* (SEQ ID NO: 452) | |
| *MtUBQ10* | TC100142 | *AACTTGTTGCATGGGTCTTGA* (SEQ ID NO: 454) | |

according to the formula below: ΔΔC_{T} = AC_{T}^{sample of study} - ΔC_{T}^{control}. These values were then used to calculate for the fold differences of each sample by using the following formula: Fold difference = 2^{-ΔΔC}_{T}. From the calculation, the control samples were valued close to 1 and all the other samples had a relative value to the controls. These values were then calculated in Excel for their standard error and their P-values using Student's t-test. Three biological (independent root samples), two experimental (independent cDNA synthesis) and three technical repeats (independent real-time PCR) were done for each sample.

### Agrobacterium rhizogenes-Mediated Hairy Root Transformation

A PCR fragment corresponding to the full-length open reading frames of *MtRAR1* was amplified from *M. truncatula* cDNA and cloned into the pK7WG2D vector by methods as as described in Karimi M, Inze D, & Depicker A (2002) "GATEWAY vectors for Agrobacterium-mediated plant transformation", Trends Plant Sci 7(5):193-195. The respective constructs were transformed into *A. rhizogenes* strain *Arqua1* as described in Saur IM, Oakes M, Djordjevic MA, & Imin N (2011), "Crosstalk between the nodulation signaling pathway and the autoregulation of nodulation in Medicago truncatula", New Phytol, 190(4):865-874. Transgenic roots were identified by the presence of green fluorescent protein (GFP) with an Olympus SZX16 stereomicroscope equipped with a GFP filter unit (Model SZX2-FGFPA, Shinjuku-ku, Tokyo, Japan).

### Nodulation with Sinorhizobium meliloti and Assessment of Nodule Numbers

The 3-weeks old transformed hairy-roots plants were first transferred to a modified Fahraeus media without NH₃NO₄ and kanamycin to starve the plants of nitrogen for 4 days. Inoculation with *Sinorhizobium meliloti* was done as described in Saur IM, Oakes M, Djordjevic MA, & Imin N (2011), "Crosstalk between the nodulation signaling pathway and the autoregulation of nodulation in Medicago truncatula", New Phytol 190(4):865-874.

### Exogenous Application of Synthetic Peptides

The RAR peptides were synthesized at the Biomolecular Resource Facility, The Australian National University. The 15 amino acid (aa) peptides corresponding to the conserved domains of MtRAR1 (AFQHypTTPGNSHypGVGH and EFQKTNPGHSHypGVGH, where Hyp indicates hydroxy proline residue) and *M. hapla* MhRAR2 (AFRHypTAPGHSHypGVGH) were synthesized and validated as previously described in Djordjevic MA, et al. (2011), "Border sequences of Medicago truncatula CLE36 are specifically cleaved by endoproteases common to the extracellular fluids of Medicago and soybean", J Exp Bot 62(13):4649-4659. The root length of wild-type plants was measured four days after transfer to Fåhraeus-medium containing the synthetic peptide. For the hormone assays, A17 plants were grown on Fåhraeus-medium for 10 days before transferring to Fåhraeus-medium containing 10⁻⁶ M of the respective phytohormones; 1-aminocyclopropane-1-carboxylic acid (ACC), 6-benzylaminopurine (BAP), gibberellic acid (GA), synthetic analog of strigolactone (GR24), methyl jasmonate (MeJA) and 1-naphthaleneacetic acid (NAA).

### β-glucuronidase (GUS) Staining and Sectioning

GUS activity was localized in transgenic hairy roots carrying *GH3:GUS* or *MtRAR1:GUS* constructs. For the promoter analysis of *MtRAR1,* the upstream 2.2-kb promoter region of *MtRAR1* was amplified by genomic PCR, then cloned into the binary vector pKGWFS7. *M. truncatula* (A17) roots was transformed with these constructs via *Agrobacterium rhizogenes* by hairy root transformation method as described in Saur IM, Oakes M, Djordjevic MA, & Imin N (2011), "Crosstalk between the nodulation signaling pathway and the autoregulation of nodulation in Medicago truncatula", New Phytol 190(4):865-874. Histochemical analysis of GUS gene expression in the transformed plant roots was performed as described in Vitha S, Benes K, Phillips JP, & Gartland KM (1995), "Histochemical GUS analysis", Methods Mol Biol 44:185-193. Staining and sectioning was performed three times, each time taking roots of four plants, and similar results were obtained each time. Staining was examined with a Nikon SMZ1500 stereomicroscope and photographed with a mounted Digital Sight DS-Ril camera (Nikon Inc., Melville, New York, USA). Sectioning of the roots was done as described in Saur IM, Oakes M, Djordjevic MA, & Imin N (2011), "Crosstalk between the nodulation signaling pathway and the autoregulation of nodulation in Medicago truncatula", New Phytol 190(4):865-874.

### Confocal microscopy

Root samples were fixed in fixative (50% methanol and 10% acetic acid) at 4°C for overnight, rinsed with water and stained with 10 µg /ml propidium iodide in water at room temperature (avoiding light) until plants were visibly stained (less than 3 h). Then the roots were examined by multiphoton imaging using a LSM 780 confocal microscopy (Carl Zeiss, Jena, Germany).

### Example 2 - Plant RARs

We examined the distribution and function of a multigene family we call RARs (Root Architecture Regulators). Phylogenetic analyses indicate that RAR genes are unique to the genomes of higher plants and RKN, and encode a conserved 15 amino acid RAR domain that is predicted to be secreted. Using expression analysis we show that in the model legume *Medicago truncatula,* RARs are regulated by lowered N-status and elevated CO₂ and they play an important role in controlling root development and the expression of genes integral to the control of N-status and uptake including ANR1, NRT2.1, NRT2.5 and LBD38. Due to the technological difficulties experienced with knockdown strategies for large multigene families we used over-expression studies to demonstrate that the RAR domain encoding gene, *MtRAR1,* profoundly affect multiple aspects of root architecture and development including lateral root and nodule formation and root hair development. Superficially, the periodic bumps induced resemble galls, and this is corroborated by confocal imaging.

Ohyama et al (2008) had previously shown that an Arabidopsis gene *AtCEP1* (C-terminal encoded peptide) produces a 14 or 15 amino acid secreted ligand that affects primary root growth only (Ohyama K, Ogawa M, & Matsubayashi Y (2008), "Identification of a biologically active, small, secreted peptide in Arabidopsis by in silico gene screening, followed by LC-MS-based structure analysis", Plant J 55:152-160). *AtCEP1* corresponds to *AtRAR1,* and is thus the historical canonical member of this family. However, since our results place *M. truncatula* RARs at the crossroads of root development and responses to nutritional cues, we renamed the CEPs to reflect this key function. In addition, our results also point to RAR ligand mimicry by RKNs suggesting a role for these nematode peptides in gall formation.

Conserved RAR domains are widely dispersed in angiosperms as multigene families (Fig. 1A, Fig. 5A and SEQ ID NOs: 2, 4, 6, 8, 10, 12, 14, 16, 18, 20, 22, 24, 26, 28, 30, 32, 34 and 36-147 for peptide sequences, and SEQ ID NOs: 1, 3, 5, 7, 9, 11, and 13 for *Arabidopsis thaliana* RAR-encoding sequences, and SEQ ID NOs: 15, 17, 19, 21, 23, 25, 27, 29, 31, 33 and 35 for *Medicago truncatula* RAR-encoding sequences).

*M. truncatula* was found to encode eleven RAR loci (see SEQ ID NOs: 15, 17, 19, 21, 23, 25, 27, 29, 31, 33 and 35 for *Medicago truncatula* RAR-encoding sequences and SEQ ID NOs: 16, 18, 20, 22, 24, 26, 28, 30, 32, 34 and 36 for *Medicago truncatula* RAR translated peptide sequences). Except for the peptide domains, little sequence conservation exits amongst RAR genes (see Figures 1A and 6). However, RAR genes encode an amino-terminal signal peptide or a non-classical secretion signal (see, for example, Figures 1B to 1D, 5D and 6), which is a feature of secreted regulatory plant peptide families. A strongly conserved functional RAR sub-domain occurs at the *C-*terminus of the RAR domain (Fig. 1A).

RAR genes can encode single or multiple RAR peptides. For example, *MtRAR1* (SEQ ID NO: 15) encodes two peptides (see Figure 1B and SEQ ID NOs: 166 and 167; see also SEQ ID NO: 16 for fully translated sequence), *MtRAR10* (SEQ ID NO: 33) encodes four peptides (see SEQ ID NOs: 178 to 181; see also SEQ ID NO: 34 for fully translated sequence) and the poplar gene, *PtRAR2,* encodes seven (see SEQ ID NOs: 231-237 and SEQ ID NO: 72 for fully translated sequence).

RAR domains in monocots are distinctive to those in dicots (Fig. 1A and Fig. 5A, and see also SEQ ID NOs: 301 to 336 - monocot RAR peptide domain sequences - vs SEQ ID NOs: 148 to 300 - dicot RAR peptide domain sequences). Monocot RAR peptides universally lack the conserved phenylalanine residue common to dicot RAR peptides, and all dicot RAR domains terminated with histidine whereas monocot RAR domains terminated with histidine or asparagine (Figs. 1A and 5A). We also found genes encoding RAR-like domains in gymnosperms (white spruce and lodgepole pine - peptide sequences: SEQ ID NOs: 338-350; domain sequences: SEQ ID NOs: 351-363) but not in the evolutionary more primitive plants, *Selaginella* or mosses (Fig. 1A, Table 2), unlike CLEs (found in *Selaginella* and moss) or RGFs (found in *Selaginella*). Angiosperm RAR genes encoded an amino-terminal secretion signal, lacked introns, and consisted of one to seven, 15 amino acid, RAR Apart from the secretion signals and the RAR domains themselves, plant RAR genes had little other sequence conservation (See, for example, Figure 1A). The gymnosperm RAR-like domains are different from angiosperm RAR in that they exhibit divergence at the first 6 amino acids and have a highly conserved leucine, instead of proline, at position 7. However, the remaining eight carboxyl amino acids of gymnosperm RAR-like domains are strongly conserved with those of angiosperm RAR domains (Fig 1 A).

### Example 3 - RAR Genes Are Found Exclusively in Higher Plants and Root-Knot Nematodes.

Apart from higher plants (angiosperms and gymnosperms) only the obligate plant parasitic animals, root knot nematodes (RKNs), were found to encode RAR genes.

**Table 2. Existence of growth regulatory peptide coding genes in plants and nematodes. A representative domain sequence is given for species in each clade.**

| | **RAR (or RAR-like*)** | **RGF** |
|---|---|---|
| Moss (*Physcomitrella patens*) | X | X |
| Pteridophyte (*Selaginella moellendorffii*) | X | DYTPVHRKPPINN (SEQ ID NO: 462) |
| Gymnosperms (*Pinus contorta & Picea sitchensis*) | ISPFKPLGHSPGIGH* | DYSTPGDEPDRQKN |
| | (SEQ ID NO: 359) | (SEQ ID NO: 463) |
| Angiosperms (*Arabidopsis*) | DFRPTNPGNSPGVGH (SEQ ID NO: 148) | DYSNPGHHPPRHN (SEQ ID NO: 475) |
| Root-Knot Nematodes (*M. hapla*) | DFRPTNPGHSPGIGH (SEQ ID NO: 396) | DYTGPKHHGPRNN (SEQ ID NO: 460) |
| Cyst Nematodes (*Heterodera glycines*) | X | X |

Eight and twelve RAR genes occur in the genomes of *Meloidogyne incognita* and *M. hapla,* respectively (Fig. 5D, Table 2 and SEQ ID NOs: 364, 366, 368, 370, 372, 374, 376, 378, 380, 382, 384 and 386 for *M. hapla* RAR-encoding sequences, SEQ ID NOs: 365, 367, 369, 371, 373, 375, 377, 379, 381, 383, 385 and 387 for *M. hapla* RAR peptide sequences, and SEQ ID NOs: 388-395 for *M. incognita* RAR peptides), but none are found in non-root-knot nematodes including *C. elegans* or the plant parasitic cyst nematodes (see Table 2).

Like plant *RAR* genes, each RKN *RAR* identified encoded a putative amino terminal secretion signal but only a single RAR domain peptide, and some RKN RAR genes (e.g. *MiRAR* 1, 3 and 4) encode only an amino terminal signal sequence and a RAR domain (Fig 5D).

There are two types of RKN RARs: type one has flanking sequences between the signal sequence and RAR domain and at the C-terminus end of the RAR domain; type two has no flanking sequences between the signal sequence and RAR domain or at the C-terminus end of the RAR domain (Fig. 1C&D). The juxtaposition of a signal sequence to a RKN RAR domain would obviate the need for processing of several of the RAR domains by additional protease cleavage. Conservation exists between RKN RAR domains and the RAR domains of plant hosts (Fig. 1A; Fig. 5C to 5D and SEQ ID NOs: 148-336, 351-363 and 396-415).

Because the precise evolutionary history of the RAR genes is not known, it is not possible to ascribe orthology, either within the genus, or with plant RARs. Thus, we named the RKN RARs according to their genome assembly coordinates.

RAR loci are absent from the available non-RKN plant parasitic nematode genomes including those of the soybean cyst nematode (*Heterodera glycines*), potato cyst nematode (*Globodera rostochiensis*) and the migratory plant parasitic nematodes (*Radopholus similis* and *Pratylenchus coffeae*) as well as *C. elegans.*

Phylogenetic analysis has shown that different MhRAR are more similar to dicot RAR than to each other (Fig. 5c). For instance, the RAR domain sequence (AFRPTAPGHSPGVGH) of *M. hapla* MhRAR2 and MhRAR11 was identical to RAR domains of *Euphorbia esula* (Green spurge; EeRAR2.1, 2.2, 2.3 and 2.5), and the RAR domain sequence (AFRPTNPGHSPGVGH) of *M. incognita* MiRAR3 was identical to the RAR domains in *Ricinus communis* (castor oil plant; RcRAR3 and RcRAR7) and *Jatropha curcas* (physic nut; JcRARl). This result may point to RKN and plant RARs sharing an overlapping functional space.

RKN RAR peptides exhibit remarkable similarity to plant RAR peptides (Figs. 1A and 5), and an overall consensus sequence based on the RAR domains (plant and RKN) may be represented as a 14 to 15 amino acid peptide represented as (X₁)X₂X₃X₄X₅X₆PGX₉SPGX₁₃GX₁₅ (SEQ ID NO:459). Typically, the peptide will comprise 15 amino acids. Aspartic acid, glycine, proline and alanine are typically present at position X₁, although serine and valine, and to a lesser extent other amino acids may be present at this position. Phenylalanine or valine are typically present at position X₂, although threonine, serine, alanine, lysine and tyrosine are often also found at this position, with other amino acids occasionally being observed. Arginine is the predominant amino acid found at position X₃, especially in monocots and RKNs, proline is the predominant amino acid found at position X₄, threonine, serine or glycine predominantly at position X₅, threonine, alanine or asparagine at position X₆, asparagine or histidine is predominant at position X₉, isoleucine, alanine or valine predominant at position X₁₃ and asparagine or histidine is predominant at position X₁₅. While amino acid substitutions have been observed at positions 7, 8, these are infrequent (and only proline observed at position 7 in monocots, and isoleucine, serine, asparagine and glutamine observed at position 8 in dicots and RKNs). The SPG motif at positions 10-12 is particularly strongly conserved, especially in monocots and RKNs, with very few substitutions being observed in dicots, and only rare substitutions (arginine or threonine) have been observed at position 14, in monocots and dicots.

Surprisingly, RKNs were also found to have genes encoding root growth factors (RGFs; see Fig. 11 and SEQ ID NOs: 416 and 417 for full peptide sequences for *M. hapla* and *M. incognita,* respectively, as well as Table 2 and SEQ ID NO:460 and 461 for the RKN RGF peptide domain sequences) and Inflorescence Deficient in Abscission peptides (IDAs; see Fig. 12 and SEQ ID NO: 418 for full peptide sequence for *M. hapla* and SEQ ID NO:496 for the RKN IDA peptide domain sequence), and were known to secrete CLEs. Unlike nematode CLE and RGF genes, RAR gene copy number expanded in RKNs. RNA sequence analysis of galls at three weeks post-infection revealed differential transcriptional activity of each of the *Meloidogyne hapla* RARs. This observation was confirmed for MhRAR4 (SEQ ID NO: 371) and MhRAR10 (SEQ ID NO: 383) by real-time quantitative reverse transcription PCR, showing them to be expressed at 3-5% relative to the control MhGADPH.

Our studies show that RAR or RAR-like genes occur only in higher plants (angiosperms and gymnosperms) and RKNs. Central to the obligate parasitism of diverse higher plants by RKNs is their ability to subvert intrinsic developmental pathways to enable gall formation. The periodic bumps induced by over-expressing *MtRAR1* or the ligands of MtRAR1 or *M. hapla* MhRAR2, outwardly resemble galls and this supports RAR peptides being bioactive. RKN RAR ligands most likely mimic plant RARs and co-opt plant RAR-dependent pathways during infection and gall formation. RKN RAR expression during gall formation and the tight distribution of RAR loci in the RKN genomes supports this.

*M. truncatula* bumps initiate in the zone of elongation. Bumps that lack arrested lateral roots arise due to periodic induction of cell divisions in the pericycle, cortical and epidermal cell layers and take ∼10 hours to form. Although the periodic bumps resemble galls and plant cell division is induced during gall formation, bumps do not contain the giant cells induced at RKN feeding sites in the stele. Hence, secreted RKN RARs could play an important role in establishing galls but they play no obvious role in giant cell formation. The known RKN RARs more closely resemble dicot RARs and this may in part explain why in monocots RKN fail to elicit large galls surrounding the obligatory giant cells.

We found that RKN genomes also encode RGF, IDA (inflorescence deficient in abscission) and CLE peptides. Thus, RKNs are unique in that they encode at least four distinct plant-like regulatory peptides that may work in concert during gall formation. Adding to this potential for RKN to produce diverse regulatory ligands is the role of proteolytic modification by the host. Those RKN RARs with the signal sequence directly juxtaposed to the RAR motif are presumably introduced into the apoplast as mature peptides, thereby avoiding any regulatory control by the host and any additional cleavage (beyond removal of the signal sequence) by the RKN itself. By contrast, plant RARs require proteolytic cleavage as an additional regulatory step. Analogous processing is presumably required for those RKN RARs with sequences flanking the RAR peptide. The recent finding that plant proteolytic processing machinery is active on cyst nematode CLEs (Guo Y, Ni J, Denver R, Wang X, & Clark SE (2011), "Mechanisms of molecular mimicry of plant CLE peptide ligands by the parasitic nematode Globodera rostochiensis", Plant Physiol 157:476-84) provides a precedent.

All twelve *M. hapla* RAR genes lie in two clusters approximately 100kb apart. Correlating sequence similarity of the deduced MhRAR proteins with the genomic location and orientation of their genes points to an inverted duplication as an early event in expansion of the RAR family. Such events have been implicated as nucleation events in gene family amplification (Ruiz JC & Wahl GM (1988), "Formation of an inverted duplication can be an initial step in gene amplification", Mol Cell Biol 8:4302-4313). Analysis of three representative MhRARs in relation to plant RARs clearly places the MhRARs with dicot RARs, including MhRAR5 which does not have the conserved phenylalanine residue in the second position of the RAR peptide (Figs. 5C, 5D). The three *M. hapla* RARs used in the phylogenetic reconstruction do not cluster together, suggesting similar degrees of diversity within the species as is found amongst the plant RARs and this implies functional significance. Collectively, the evidence points to an ancient horizontal gene transfer (HGT) from dicot to the nematode as a mode of acquisition of RARs in RKN. To our knowledge, this is the first example of a plant-parasitic nematode having acquired a dicot gene.

### Example 4 - M. truncatula RAR Genes Are Regulated by N-levels, Elevated CO₂ and Show Differential Expression in Lateral Organs.

We examined RAR transcript levels in *M. truncatula* by real-time quantitative reverse transcription PCR (qRT-PCR). Eight of eleven MtRAR genes (*MtRAR1, MtRAR2, MtRAR4, MtRAR5, MtRAR6, MtRAR8, MtRAR9* and *MtRAR11*) were significantly induced between 4 and 36-fold by shifting plants to nitrogen starvation conditions for four days (Fig. 2A). The available microarray data for *MtRAR1* is consistent with our findings; *MtRAR1* is up-regulated in *M. truncatula* roots after the imposition of a nitrogen limitation (Fig. 2D).

Recently, elevated CO₂ was shown to inhibit nitrate assimilation in wheat and Arabidopsis, which may lead to a less-than-expected stimulation of plant growth (Bloom AJ, Burger M, Rubio Asensio JS, & Cousins AB (2010), "Carbon dioxide enrichment inhibits nitrate assimilation in wheat and Arabidopsis", Science 328:899-903). An examination of RAR gene expression in *M. truncatula* grown with 800 ppm CO₂ under different nitrate regimes showed that *MtRAR1, MtRAR2, MtRAR5, MtRAR6* and *MtRAR8* were highly up-regulated by a combination of elevated CO₂ and low nitrate (0.25 mM KNO₃) (Fig. 2B). *MtRAR1* and *MtRAR2* were up-regulated by elevated CO₂ even in the presence of 5 mM nitrate. *MtRAR1* and *MtRAR11* were also up-regulated under low nitrate. Although *MtRAR1* was highly responsive to alterations to N and C levels it did not respond to phytohormones (Fig. 2C).

Given the responsiveness of *MtRAR1* expression to environmental conditions, this gene was explored further by fusing the 2 kb *MtRAR1* promoter sequence to *GUS,* yielding *proMtRAR1:GUS.* N-starved roots showed strong GUS staining near root tips and in the vascular tissue (Fig. 2E) but subdued staining occurred in high-N grown roots (Fig. 21, 2M). Serial sections through the root tip region (Fig. 2J to 2L), showed GUS staining localised in the lateral root cap and apical meristem region (Fig. 2 K) and, in the basal meristem/root elongation region, in cells destined to be vasculature (Fig. 2K). *MtRAR1* did not express in columella cells (Fig. 2E, 21, 2F, 2M). In mature roots staining occurred in the procambium and pericycle (Fig. 2L) but phloem, cortical or epidermal cells were not stained (Fig. 2L).

Subdued GUS staining occurred in roots grown with 5 mM nitrate after a 24-hour incubation in GUS substrate whereas GUS staining in low N-grown plants was already apparent after 2 hours (Fig. 21 and 2M). Although *ProMtRAR1:GUS* staining was visible during lateral root initiation and emergence under N-starved conditions (Fig. 2F) it became progressively weaker during nodule development induced by the nitrogen fixing *Sinorhizobium meliloti* strain WSM1022, (Fig. 2G then Fig. 2N, then Fig. 2H). In roots containing one week old WSM1022 nodules, where leghemoglobin was already apparent, GUS expression occurred in zone II of the developing nodules (Fig 2 H). However, localised suppression of *ProMtRAR1:GUS* expression was evident in proximal vascular tissue (Fig. 2G, 2H). As the pink nodules approached two weeks of age, *ProMtRAR1:GUS* expression was further suppressed in WSM1022 nodules (Figs. 2G, compared to 2H and 2N) and the surrounding and supporting root vasculature near nodules but notably, not in nearby emerging lateral roots (Fig. 2N). Microarray data also shows negligible *MtRAR1* expression during nodule initiation and formation (Fig. 2D).

In contrast to the results obtained with plant inoculated with WSM1022, *ProMtRAR1:GUS* staining was strongly retained in nodules induced by the model S. *meliloti* strain Sm1021. At day 7, there was no observable leghemoglobin production indicating that little or no nitrogen fixation occurred under the conditions used (no AVG added to roots) at this time. In two week old Sm1021 nodules, *ProMtRAR1:GUS* expression was reduced concurrently with the appearance of leghemoglobin. The N-starvation induction of *MtRAR1* and the gradual decrease in *MtRAR1* expression in one and two week old Sm 1021 induced nodules was reflected in *M. truncatula* gene atlas microarray data which showed that *MtRAR1* was induced predominantly by nitrogen starvation in the roots but decreased in the developing nodules induced by Sm1021.

We hypothesised that the gradual reduction in *MtRAR1* expression in nodules induced by WSM1022 compared to Sm1021 was due in part to the earlier *induction nitrogen* fixation in WSM1022 nodules. To examine this and to investigate the effect of different levels of nitrogen sources on *MtRAR1* expression, we measured the relative expression of *MtRAR1* after exposure of plants to two forms of inorganic (nitrate or ammonium) and organic (asparagine or glutamine) nitrogen. Given the results in Fig. 2B, and genevestigator analyses indicated that high CO₂ was an abiotic factor that altered RAR gene expression in *Arabidopsis,* the experiment was also conducted under ambient and high CO₂ conditions.

*MtRAR1* was significantly up-regulated at low N levels at ambient CO₂ when nitrate, asparagine or glutamine was supplied as the sole nitrogen source. However, elevated N levels suppressed expression, with the strongest suppression by asparagine and glutamine at ambient CO₂ level. This is consistent with the suppression of *MtRAR1* expression in more mature nodules where assimilation of N resulting from nitrogen fixation is known to result in asparagine and glutamine which are the main products of nitrogen fixation. In addition, higher CO₂ further up-regulated *MtRAR1,* even at high nitrogen levels, when asparagine was supplied the sole nitrogen source. Consistent with the results in Fig. 2B, the highest up-regulation of *MtRAR1* resulted from the combination of high CO₂ and low nitrogen for all nitrogen sources. Statistical analysis showed significant interactions between CO₂ levels and nitrogen levels for nitrate and asparagine, although a similar trend was observed for the other two nitrogen sources, which indicated that low N and high CO₂ independently regulated *MtRAR1* expression.

The studies described herein show that *M. truncatula* RAR genes *respond* to changed N and CO₂ levels, suggesting that RARs may be involved in regulating responses to changes in C:N. Low nitrate and high CO₂ significantly elevates five of nine MtRAR genes and shifting plants to N-starvation conditions significantly induced eight MtRAR genes. *MtRAR1* is unique in responding to low nitrate or N-starvation under ambient CO₂ and to elevated CO₂ in high nitrate. These results point to RARs playing an important regulatory role in responses to environmental cues. *MtRAR1* may mediate at least some of its effects by regulating key transcription factors (*MtAGL1* and *MtLBD38*), and/or through the important transporter *MtNRT2.5.* In Arabidopsis, ANR1 (which is very similar to *MtAGL1*) and LBD38, regulate N-limitation responses and lateral root formation by controlling the transcription of key nitrate transporters (Gan Y, Filleur S, Rahman A, Gotensparre S, & Forde BG (2005), "Nutritional regulation of ANR1 and other root-expressed MADS-box genes in Arabidopsis thaliana", Planta 222:730-742; Okamoto M, Vidmar JJ, & Glass AD (2003), "Regulation of NRT1 and NRT2 gene families of Arabidopsis thaliana: responses to nitrate provision", Plant Cell Physiol 44:304-317). MADS box transcription factors are implicated in forming lateral root primordia (Burgeff C, Liljegren SJ, Tapia-Lopez R, Yanofsky MF, & Alvarez-Buylla ER (2002), "MADS-box gene expression in lateral primordia, meristems and differentiated tissues of Arabidopsis thaliana roots", Planta 214:365-372), the promotion of lateral root growth under N-limiting conditions (Gan *et al* (2005), *supra*), and have an involvement in tuberous root initiation (Ku AT, Huang YS, Wang YS, Ma D, & Yeh KW (2008), "IbMADS1 (Ipomoea batatas MADS-box 1 gene) is involved in tuberous root initiation in sweet potato (Ipomoea batatas)", Ann Bot 102:57-67). Recently an ABA-regulated LBD gene was implicated in lateral root formation in *M. truncatula* (Ariel FD, Diet A, Crespi M, & Chan RL (2010), "The LOB-like transcription factor Mt LBD1 controls Medicago truncatula root architecture under salt stress", Plant Signal Behav 5:1666-1668). The unresponsiveness of *MtRAR1* to phytohormones, especially ABA, and the ability of MtRAR1 to control important MADS box and LBD transcription factors suggests that MtRAR1 is pivotal to the integration of environmental cues and root architecture in higher plants. The altered distribution of auxin at bump sites suggests this is downstream of RAR regulation.

### Example 5 - Ectopic Expression of MtRAR1 Profoundly Alters Root Architecture.

*MtRAR1* was over-expressed in *M. truncatula* roots using the CaMV 35S promoter and confirmed by qRT-PCR (Fig. 7A). The *MtRAR1ox* roots exhibited five root architecture phenotypes. First, lateral root numbers were significantly reduced 4-fold compared to controls (Figs. 31, 7D and 7E) but the growth of the earliest forming hairy roots was not noticeably affected. Second, approximately 60% of the transformed roots bearing *MtRAR1ox* formed multiple periodic "bumps", typified by circumferential cell proliferations (CCP), at the expense of lateral roots (Figs. 3B, 3C and 3H, 4C, 4D, 7B and 7E). The CCP sites exhibited increased root hair density (Fig. 3B, 3D, 3E). The periodicity was determined by scoring the number of CCP sites over time in several independent measurements under different conditions and it was found to be approximately 24 hours. Maximum CCP site generation was induced by the application of the 15 AA MtRAR1 and MhRAR2 peptides with their expected post translational modifications: all RAR peptide structural variants tested imparted significantly lower, or no, CCP sites. This included a RAR1 peptide with a *C*-terminal deletion of Histidine; it did not impart any detectible phenotype and was indistinguishable from untreated wild type plants. The timing of the appearance of the youngest CCP sites and their proximity to the root tip showed that they initiated near the basal meristem (Fig. 3C, 3E). Third, approximately 20% of bump sites sectioned contained arrested lateral roots (Figs. 3H, L-M and 4D) that lacked connection to the root vasculature (Fig. 4D). Fourth, increased root hair development, which is an environmentally-sensitive development pathway (see Schiefelbein J, Kwak SH, Wieckowski Y, Barron C, & Bruex A (2009), "The gene regulatory network for root epidermal cell-type pattern formation in Arabidopsis", J Exp Bot 60:1515-1521), occurred on bump surfaces but was reduced between bumps (Figs. 3B-C and 7E). Interestingly, *MtRAR1ox* roots growing through the agar support medium had fewer and shortened root hairs (Fig. 7F). Finally, *MtRAR1ox* roots formed 6-fold more nodules (Figs. 3G and 7C) compared to controls.

Confocal microscopy (Fig. 4) indicated that CCP sites induced by *MtRAR1* over-expression were 80 µm wider than the normal root diameter (Fig 4B to 4E), and showed that the increased root girth at CCP sites was attributable to limited cortical, epidermal and pericycle cell division (Figures 4E and 4F) and the formation of an extra cortical cell layer. The nucleus was centrally located in many CCP cells (Fig. 4C). Counting cells over a 200 µm² area at several CCP sites indicated a more than two-fold increase in cortical cell number (Fig. 4F). Serial vibratome sectioning of over 60 plants containing an average of 6 CCP sites each showed no evidence of lateral root initiations at the majority of CCP sites (80%) (e.g. Fig 4B, 4C). Serial sectioning and confocal microscopy showed that the intervening root segments between the CCP sites showed normal cell patterning and root girth (Fig. 4A). We tested the possibility that CCP sites represented sites where lateral roots would have emerged in the absence of RAR treatment. To do this CCP sites were marked with a black dye and then the root tip was excised (3 mm of tissue was removed only) to release apical dominance. This resulted, within 24-48 h, in lateral roots emerging from CCP sites, but never between them. On average, approximately 24% of the CCP sites formed lateral roots when the root tip was cut. Although a small number of CCP sites generated lateral roots, lateral root number was still not fully restored to the number occurring on plants not exposed to RAR Therefore, lateral root initiation and/or emergence was promoted by removal of the RAM in RAR treated plants but not restored fully to wild type levels. This rapid emergence of lateral roots from CCP sites contrasts strongly with the persistent inhibition of lateral root initiation and emergence seen on plants on RAR treated plants or where *MtRAR1* was over-expressed. Plants exposed to non-functional RAR peptide variants, in which non-conservative substitutions were made to highly conserved amino acids, did not show the persistent inhibition of lateral root emergence.

*MtRAR1* over-expression in *M. truncatula* plants containing *GH3:GUS,* an auxin responsive reporter construct (Mathesius U, et al. (1998), "Auxin transport inhibition precedes root nodule formation in white clover roots and is regulated by flavonoids and derivatives of chitin oligosaccharides", Plant J 14:23-34), induces periodic bumps that co-localize with elevated *GH3:GUS* activity (Fig. 3H) suggesting an alteration to auxin distribution at bump sites. Sectioning revealed intense *GH3:GUS* expression in bump vascular tissue. The histology of bumps housing arrested lateral roots confirmed their poor connection to root vasculature (Fig 3L, 3M); the arrested lateral roots lacked *GH3:GUS* expression, although a patch of *GH3:GUS* expression was observed ahead of some arrested lateral roots (Fig. 3M).

Biological activity of RAR ligands was confirmed using two synthetic *M. truncatula* MtRAR1 ligands and a *M. hapla* RAR2 ligand (Figs. 1 and 6 vs 5D), and SEQ ID NOs 166 and 167 - MtRAR1 domains - and 398 - MhRAR2 domain) each made with the expected post-translational modifications. Wild-type plant roots exposed to these peptides at or above 10⁻⁷ M phenocopied *MtRAR1ox* roots by showing periodic bump (CCP site) formation, perturbation of root hair development (Figs. 3D-E) and an inhibition of lateral root formation (Fig. 8A). In some instances, primary root growth was also inhibited (Fig. 8B). Confocal microscopy showed that CCP sites were 80µm wider than a non-bump (CCP) site (Fig. 4E) which was attributable to cortical, epidermal and pericycle cell divisions. A more than two-fold increase in cortical cell number occurred at the bump sites and these cells possessed central nuclei (P<0.0001; Figs. 4C and F). Arrested lateral roots contained small undifferentiated cells (Fig. 4D).

Inhibition of lateral root emergence was sustained over a two month period of growth by the addition to roots of the regulatory peptides. The inhibition of lateral root emergence by RAR1 peptide occurred in a dose-dependent manner at greater than 10⁻⁷ M, and was not affected by inoculation by *S. meliloti.* Because several residues in the 15 amino acid RAR domain were highly conserved in dicots, peptide variants were synthesised and tested for biological activity. These included peptides which were otherwise identical to MtRAR1 but which included deletion of the highly conserved C-terminal histidine, or substitution of the highly conserved glycine at position 8, or removal of the *N*-terminal residue or sequential removal of the proline post-translational modifications at positions 4 and 11. The PGN sequence of the MtRAR1 peptide is indicative of a beta turn which would be an important structural feature of the mature peptide *in vivo,* and substitution of Gly-8 would affect this structural feature. Structure activity relationship studies conducted with these MtRAR1 peptide variants showed that inhibition of lateral root emergence was abolished by removing the conserved *C*-terminal histidine residue or greatly attenuated by substituting the strongly conserved glycine at position eight to alanine, removing the *N*-terminal residue, or by not hydroxylating the proline residues.

The studies disclosed herein indicate that *MtRAR1* regulates the developmental pathways of lateral roots, nodules and root hairs. Lateral root emergence is significantly reduced by over-expressing *MtRAR1* and instead periodic bumps form, some of which house arrested lateral roots. The initiation of periodic bumps occurs in the zone of elongation and their rapid formation over ten hours suggests that they may coincide with the lateral root 'pre-branch sites' identified in Arabidopsis (Moreno-Risueno MA, et al. (2010), "Oscillating Gene Expression Determines Competence for Periodic Arabidopsis Root Branching", Science 329:1306-1311). Alternatively RAR may inhibit lateral root formation after pre branch site formation. When arrested lateral roots occur at CCP sites (about 20% of CCP sites) they lack cellular differentiation and fail to attract vascular connections, indicating that these organs are locked at a developmental checkpoint. CCP sites that lack arrested lateral roots exhibit anticlinal cell divisions of cortical, epidermal and pericycle cells, and an extra cortical cell layer is observed which presumably arises by periclinal division. The increased number of epidermal cells at bumps could account for the increased root hair development.

Increased root nodule formation occurred on plants over-expressing *MtRAR1.* This may indicate that bumps are more susceptible to rhizobial infection. Bumps partially resemble an early root nodule primordium, which initially comprises a focus of anticlinal cell divisions in the cortex, endodermis and pericycle. Rhizobia are known to 'hijack' sites of endogenous cell division sites in legumes, and this observation strengthens the developmental link between nodule ontogeny and gall formation.

### Example 6 - Nodulation is enhanced by ectopic expression of MtRAR1 or by peptide application

The effect of over-expressing *MtRAR1* on nodule organogenesis under different nitrogen regimes was studied.

The data shown in Figure 31 relates to studies that show that *MtRAR1* over-expression increases nodulation and promotes nodule development at different nitrate concentrations: **(a)** Comparison of nodulation between *MtRAR1* over-expressing plants and control plants at different temperatures when inoculated with *S. meliloti* strain 1021. **(b)** Partial nitrate tolerance of nodule development induced by WSM1022 on *MtRAR1* over-expressing plants. Three weeks old composite plants were inoculated and scored two weeks post inoculation. N ≥ 30. student's t-test; *: *P* < 0.05; **: *P* < 0.01; ***: *P* < 0.001. **(c)** Acetylene reduction assay. MtRAR1 treated (1 µM) and non treated wild-type plants grown in Fåhraeus medium were harvested at 2 week post inoculation by *S. meliloti* WSM1022. Nitrogenase activity was calculated from peak areas of samples relative to acetylene and ethylene standards to obtain nmoles ethylene/min. N ≥ 3. student's t-test; *: *P* < 0.05; **: *P* < 0.01. Error bars indicate SE. **(d)** Inoculation of plants with preformed CCPs led to root nodule formation at CCP sites at 25 mM KNO₃. Arrow head: CCP sites; Arrows: nodules. **(e)** Nodule sections showing accelerated nodule development in RAR1 peptide-treated compared to untreated plants grown at either 0, 5 mM or 25 mM KNO₃ for three weeks. Scale bar: 100 µm. Plants were grown at 20 °C.

Figure 32: *M. truncatula* plants were grown in 0.8% agar-containing plates supplemented with F media with or without MtRAR1 peptide. Five-day old seedlings were inoculated with *S. meliloti* strain WSM1022 and further grown for two more weeks. MtRAR1 Domain 1: AFQ(P)TTPGNS(P)GVGH; MtRAR1 Domain 1 with G->A transition: AFQ(P)TTPANS(P)GVGH. (P) is hydroxylproline and the altered amino acid is underlined.

The data shown/illustrated in Figures 31 and 32 indicates that more nodules formed on composite plants over-expressing *MtRAR1* compared to controls when they were infected by *S. meliloti* and grown on either 20°C or 25°C in the absence of nitrogen (Fig. 31a). This extent of increased nodulation was more evident when the plants were inoculated with the model *S. meliloti* strain 1021 which induces poor nodule numbers with poorly symbiotic performance on the control plants compared to the plants inoculated with the highly effective strain WSM1022 which nodulates and fixes nitrogen more vigorously (Fig. 31a-b).

Because MtRAR1 peptide and *MtRAR1* over-expression raised nodule number, the effect of *MtRAR1* on symbiotic capacity, as measured by the frequency of pink nodules formed at 5 mM and 25 mM KNO₃ was also scored, as leghemoglobin production is an important indicator of symbiotic capacity. There were significantly higher numbers of pink functional nodules on plants over-expressing *MtRAR1* or exposed to 1 µM MtRAR1 peptide compared to controls (Figure 32). At 5 mM and 25 mM KNO₃, the nodule numbers formed on roots over-expressing *MtRAR1* increased significantly (e.g. 10-fold higher at 25 mM nitrate; Fig. 31b). A similar trend was observed when 1 µM MtRAR1 peptide was applied to the plants but not when the non-functional MtRAR1 peptide where the central glycine residue was replaced with alanine was applied (Figure 32). All across the nitrate regimen, the nodules were much larger on MtRAR1 peptide treated roots than on plants treated with no peptide or with the MtRAR1 peptide where the central glycine residue was replaced with alanine (Figure 31e and 32). After three weeks post inoculation with *S. meliloti,* a clear bacteroid like zone was apparent in nodule sections on MtRAR1 peptide-treated plants and nodules often spanned across two xylem poles; nodules at 25 mM nitrate possessed leghemoglobin (Fig. 31e). At 25 mM nitrate, nodules were induced at, but not between, preexisting CCP sites on plants over-expressing *MtRAR1* or plants treated with peptides (Fig. 31d). The results showed that for plants exposed to elevated MtRAR1 by over-expression or ectopic application, the susceptibility to infection by *S. meliloti* and nodulation ability was more tolerant to nitrate and overall nodulation development was enhanced. To further confirm the enhancement of nodulation by RAR addition, we measured nitrogenase activity using acetylene reduction assay. The results showed significant increase in the nitrogenease activity from 35% at 0 mM KNO₃ to 200% at 5 mM KNO₃ when 1 µM MtRAR1 was applied (Fig. 31c).

### Example 7 - MtRAR1 Over-expression Regulates Key Genes Involved in N-Uptake Pathways.

As its expression responded to N-status, we investigated the role of *MtRAR1* in controlling *M. truncatula* homologs of key genes that regulate the N-signaling network in *Arabidopsis. Arabidopsis* MADS box transcription factors ANR1 and AGL21 (AGAMOUS-LIKE 21) are known to regulate lateral root growth in response to external nitrogen levels (Gan Y, Filleur S, Rahman A, Gotensparre S, & Forde BG (2005), "Nutritional regulation of ANR1 and other root-expressed MADS-box genes in Arabidopsis thaliana", Planta 222:730-742). Over-expression of *MtRAR1* up-regulates the ANR1/AGL21 homolog *MtAGL1* (Figs. 3J and 9) and down-regulates *MtLBD38,* a LATERAL ORGAN BOUNDARIES-domain containing transcription factor that negatively regulates N-availability signals (Rubin G, Tohge T, Matsuda F, Saito K, & Scheible WR (2009), "Members of the LBD family of transcription factors repress anthocyanin synthesis and affect additional nitrogen responses in Arabidopsis", Plant Cell 21:3567-3584) and the high affinity nitrate transporter *MtNRT2.5* (Figs. 3J and 10).

### Example 8 - Exemplifications of RAR peptide effect in lucerne, subterranean clover, white clover, tomato, soybean and rice plants

To further validate and exemplify the RAR technology, we have examined RAR peptide addition of roots of various plant species. Lateral root emergence was significantly inhibited (students' t-test *P*<0.05) by the addition to roots of the regulatory peptide 1 µM MtRAR1 in *M. truncatula,* Lucerne (*M. sativa*), Subterranean Clover (Cultivar *Woogenellup*) and white clover (*Trifolium repens*) as shown in Figures 14-17. These peptide treated legume plants not only showed altered lateral root phenotype similar to *M. truncatula,* they also formed CCP (circumferential cell proliferations; "bump") sites similar to the ones in *M. truncatula.*

Using plate assays, we also have tested effect of 1 µM MtRAR1 peptides on microtom tomato seedlings and data showed significant (students' t-test *P*<0.05) reduction in number of lateral roots when 1 µM MtRAR1 was applied into the liquid medium (Figures 18 and 19). Primary root length was not affected (Figure 6).

Using liquid culture system, we also have tested effect of dicot RAR peptides on soybean seedlings and data showed significant (students' t-test *P*<0.05) reduction in number of roots when 1 µM dicot RAR was applied into the liquid medium (Figures 20 and 21). Primary root length was also affected (Figure 21).

Using the same liquid culture system but supplemented with half stress MS medium, we have tested effect of monocot RAR peptides on rice seedlings and data showed significant (students' t-test *P*<0.05) reduction in number of roots when 1 µM dicot RAR was applied into the liquid medium (Figures 22 and 23). Primary root length was also affected (Figure 23).

### Example 9 - Phenotypic analysis: RAR peptide addition in Arabidopsis.

To test the effect or RAR peptide addition on *Arabidopsis thaliana* roots, wild type (WT) ecotype Columbia (Col) plants were grown vertically on ½ MS with 1% phytagel and 1 µM of RAR peptide (see Table 3, overleaf) or no peptide, with 16 hr days at 22°C. Plates were imaged at 12 days and analysed with the SmartRoot plugin in ImageJ. Several different peptides with varied modifications were assayed. Modifications: hyP = 2 hydroxylated proline residues, no hyP = no hydroxylated prolinate residues, phos = phosphorylation on residue 6 plus 2 hydroxylated proline residues.

As can be seen from the results, all RAR peptides tested have the same effect in *Arabidopsis:* primary root length is reduced and the total number of lateral roots per plant is reduced; however the extent of the phenotype differs.

**Table 3 - List of RAR peptides assayed and sequence. hyP indicates hydroxyproline residues. (p-) indicates a phosphorylated residue.**

| **Peptide** | **Sequence** |
|---|---|
| RAR3 hyP | D T F R hyP T E P G H S hyP G I G H |
| RAR5 hyP (domain 1) | D F V hyP T S P G N S hyP G V G H |
| RAR5 no HyP(domain 1) | DFVPTSPGNSPGVGH |
| RAR5 phos(domain 1) | D F V hyP T (p-S) P G N S hyP G V G H |
| RAR6 hyP | D F R hyP T T P GH S hyP G I G H |
| RAR6 phos | D F R hyP T (p-T) P G H S hyP G I G H |

The results are shown in Figures 24 to 26. In Figure 24, scale bar = 1 cm. In Figures 25 and 26, error bars are +/- standard error. Significance:* p <0.05; ** p <0.005; ***p <0.001.

### Example 10 - RAR Bioactivity in Arabidopsis.

To investigate the effect of different modifications on peptide bioactivity, several peptides were assayed for activity at different concentrations (Table 4). WT Col plants were grown vertically on ½ MS plates with 1% phytagel and containing varying concentration of RAR peptide ranging from 10⁻⁶ M to 10⁻¹² M or no peptide. Plates were imaged at 12days.

**Table 4 - Bioactivity of different peptides at varied concentrations. Modifications: hyP = 2 hydroxylated proline residues, no hyP = no hydroxylated prolinate residues, phos = phosphorylation on residue 6 plus 2 hydroxylated proline residues. light shading indicates biological activity (ie. primary root length and number of lateral roots are significantly lower than untreated samples, p<0.05). Dark shading indicates no biological activity (treated plants not significantly different to untreated samples, p>0.05). No shading indicates the concentration was not assayed.**

| | **RAR3 hyP** | **RAR5 hyP** | **RAR5 no hyP** | **RAR5 phos** | **RAR6 hyP** | **RAR6 phos** |
|---|---|---|---|---|---|---|
| **10⁻⁶ M** | | | | | | |
| **10⁻⁷ M** | | | | | | |
| **10⁻⁸ M** | | | | | | |
| **10⁻⁹ M** | | | | | | |
| **10⁻¹⁰ M** | | | | | | |
| **10⁻¹¹ M** | | | | | | |
| **10⁻¹² M** | | | | | | |

The most bioactive peptide is phosphorylated RAR6, which is active at picomolar concentrations. It is 1000x more active than the non-phosphorylated RAR6 peptide. The RAR5 peptide appears to be more active when it is not hydroxyprolinated compared to a hydroxylated sample. It is active at a 10⁻⁹ M compared to the hydroxyprolinated peptide, which is active at 10⁻⁸ M. However phosphorylation of Arabidopsis RAR5 enhances activity.

In conclusion, different modifications, including hydroxylation of two proline residues and phosphorylation affect peptide bioactivity in *Arabidopsis.*

### Example 11 - Time Course: RAR action in Arabidopsis over time.

To see how RAR was exerting its effect on PR growth and lateral root emergence and growth, a time course was performed over 12 days. WT Col plants were grown vertically on ½ MS with 1% phytagel and 1µM RAR6 hyP or no peptide, with 16 hr days at 22°C. Plates were scanned every day for 12 days. The results are shown in Figures 27 and 28.

In Figure 27 it can be seen that the primary root length was significantly (p <0.05) different on all days except day 2 (marked ns = not significant). Error bars are +/standard error.

In Figure 28 it can be seen that the number of lateral roots was not significantly different until day 8. Error bars are +/- standard error. * p <0.05; ** p <0.005; ***p <0.001.

Overall the data show that germination and root establishment are not affected by RAR treatment as there was no significant difference in PR length between treated and untreated samples at 2 days post imbibing. However, from day 3 onwards, the PR length in RAR treated plants was significantly shorter than in untreated plants. The difference in lateral root number was not significant between treated and untreated samples until day 8. This was 2 days after the first lateral roots appeared in both treatments. Therefore, the emergence of the first lateral roots are not affected by RAR, however subsequent lateral root emergence is affected.

### Example 12 - Rescue: Removing RAR releases its effect.

To investigate whether the effect of RAR peptide addition could be released, WT Col plants were grown vertically for 6 days on ½ MS plates with 1% phytagel. Plants were transferred from plates containing no RAR or 1µM RAR6 hyP peptide (pre-treatment) to plates containing no RAR or 1µM RAR6 hyP peptide as specified where they were left to grow for a further 6 days before being analysed.

The results are illustrated in Figures 29 and 30. Figure 29 shows that the number of lateral roots is the same in plants transferred to the no RAR treatment regardless of pre-treatment (p<0.05), and Figure 30 shows that the primary root length of plants pre-treated with RAR and transferred to no RAR is significantly higher (p<0.05) than plants exposed to RAR continuously. Error bars are +/- standard error. Letters indicate samples that are significantly different from each other (p<0.05).

The data show that the number of lateral roots is the same in plants transferred to the no RAR treatment regardless of pre-treatment. It can therefore be concluded that the effect of RAR peptide addition on the total number of lateral roots can be released by removing RAR exposure. This is further supported by the lack of significant difference in the number of lateral roots in plants transferred to RAR6 plates from either RAR6 or no RAR pre-treatments. Additionally, primary root length of plants pre-treated with RAR and transferred to no RAR is significantly higher than plants exposed to RAR continuously, indicating that the plants begin to recover upon removal of RAR.

The results described herein indicate that RARs mediate root development in response to nutritional cues, a process that evolved most intricately in the plants with true roots. These observations may be used to affect plant root architecture to, for example, allow plants to more efficiently utilise nutrient resources, or to grow in environments that wild-type counterparts typically do not.

The present studies also show that RARs are unique regulators in higher plants and the obligate parasites that subvert their innate developmental processes. This suggests that RKNs have targeted an indispensible system in higher plants required for true root development. These observations not only provide insight into how RKNs interact with plants, but also point to mechanisms for interfering with this interaction and to inhibit or lessen the significant damage to crop plants often caused by RKNs.
It will be appreciated that, although specific embodiments of the invention have been described herein for the purpose of illustration, various modifications may be made without deviating from the invention as defined in the following claims.

## Claims

1. A method for reducing lateral root development by a plant relative to an untreated or wild-type plant, comprising contacting the root zone of said plant with at least one RAR or introducing at least one exogenous RAR-encoding nucleic acid into one or more plant cells which results in increased RAR expression by cells of a plant regenerated from or comprising said one or more plant cells,
wherein said RAR is not an *Arabidopsis thaliana* CEP1 peptide and said RAR-encoding nucleic acid is not an *Arabidopsis* CEP1-encoding sequence, and
wherein said RAR comprises an RAR domain comprising an amino acid sequence (X₁)ₙX₂X₃X₄X₅X₆PGX₉SPGX₁₃GX₁₅ (SEQ ID NO: 459), wherein n may be 1 or 0.

2. The method of claim 1, wherein said RAR-encoding nucleic acid is operably linked to a promoter, optionally wherein said promoter is root-specific, optionally wherein said promoter is the *Medicago truncatula RAR1* promoter (SEQ ID NO: 337).

3. The method of claim 2, wherein said promoter is constitutive or inducible by nutrient status, optionally wherein said promoter is inducible by nitrogen starvation or high carbon dioxide.

4. The method of any one of claims 1 to 3, wherein said plant comprises a greater density of root hairs at periodic root bumps.

5. The method of any one of claims 1 to 4, wherein said RAR comprises an amino acid sequence selected from SEQ ID NOs: 4, 6, 8, 10, 12, 14, 16, 18, 20, 22, 24, 26, 28, 30, 32, 34, 36 to 147, 338-350, 365, 367, 369, 371, 373, 375, 377, 379, 381, 383, 385, 387-395 or comprises an RAR domain having an amino acid sequence selected from SEQ ID Nos: 149-336, 351-363 or 396-415, or wherein said RAR-encoding nucleic acid comprises a nucleotide sequence selected from SEQ ID NOs 3, 5, 7, 9, 11, 13, 15, 17, 19, 21, 23, 25, 27, 29, 31, 33, 35, 364, 366, 368, 370, 372, 374, 376, 378, 380, 382, 384 or 386.

6. A method for promoting lateral root growth and development of a plant relative to an untreated or wild-type plant, comprising introducing at least one mutation in (a) one or more RAR-encoding genes or upstream sequences thereof; or (b) one or more RAR receptor-encoding genes or upstream sequences; into one or more plant cells which at least one mutation results in:
(i) decreased expression of one or more RARs, decreased expression of one or more RAR receptors, or decreased expression of one or more RARs and one or more RAR receptors by cells of a plant regenerated from or comprising said one or more plant cells, wherein said decreased expression of said RAR(s) or RAR receptor(s) occurs under conditions which would otherwise promote expression of said RAR(s) or RAR receptor(s); or
(ii) reduced affinity of one or more RARs for their respective RAR receptors, which reduced affinity arises through modifications in the RAR(s), RAR receptor(s) or in both expressed RAR(s) and RAR receptor(s)expressed by cells of a plant regenerated from or comprising said one or more plant cells, and
wherein said RAR comprises an RAR domain comprising an amino acid sequence (X₁)ₙX₂X₃X₄X₅X₆PGX₉SPGX₁₃GX₁₅ (SEQ ID NO: 459), wherein n may be 1 or 0.

7. The method of claim 6, wherein said RAR comprises an amino acid sequence selected from SEQ ID NOs: 4, 6, 8, 10, 12, 14, 16, 18, 20, 22, 24, 26, 28, 30, 32, 34, 36 to 147, 338-350, 365, 367, 369, 371, 373, 375, 377, 379, 381, 383, 385, 387-395 or comprises an RAR domain having an amino acid sequence selected from SEQ ID Nos: 149-336, 351-363 or 396-415, or wherein said RAR-encoding nucleic acid comprises a nucleotide sequence selected from SEQ ID NOs 3, 5, 7, 9, 11, 13, 15, 17, 19, 21, 23, 25, 27, 29, 31, 33, 35, 364, 366, 368, 370, 372, 374, 376, 378, 380, 382, 384 or 386.

8. The method of any one of claims 1 to 7, wherein said RAR comprises an RAR domain comprising an amino acid sequence (X₁)ₙX₂X₃X₄X₅X₆PGX₉SPGX₁₃GX₁₅ (SEQ ID NO: 459), wherein:
n may be 0 or 1
X₁ is selected from D, G, P, A, S, E and V;
X₂ is selected from F, V, R, T, S, A, K and Y;
X₃ is selected from R, K, E, H, Q, S, P, D, V, G, and A;
X₄ is selected from P and G;
X₅ is selected from T, S and G;
X₆ is selected from N, A, T, G, P, D, K and S;
X₉ is selected from N, H, Y and S;
X₁₃ is selected from I, A and V; and
X₁₅ is selected from N and H;
wherein
the amino acid at position 6, if threonine or serine, may be phosphorylated;
the P at position 11, a P at position 4, or both such prolines may be hydroxylated; and
tyrosine residues may be sulphonated.

9. A plant with altered horizontal or vertical root system growth pattern relative to a wild-type plant, or a part of said plant, wherein said plant or part thereof comprises at least one exogenous RAR-encoding nucleic acid, and wherein said plant or part thereof is obtainable by the method of any one of claims 1 to 5.

## Patentansprüche

1. Verfahren zur Verringerung von Seitenwurzelentwicklung einer Pflanze relativ zu einer unbehandelten oder Wildtyp-Pflanze, umfassend In-Kontakt-Bringen der Wurzelzone der Pflanze mit mindestens einem RAR oder Einführen mindestens einer exogenen RAR-kodierenden Nukleinsäure in eine oder mehrere Pflanzenzellen, was in einer erhöhten RAR-Expression durch Zellen einer Pflanze, die aus der einen oder den mehreren Pflanzenzellen regeneriert wurde, oder diese umfasst, resultiert,
wobei das RAR kein *Arabidopsis thaliana* CEP1-Peptid ist, und die RAR-kodierende Nucleinsäure keine *Arabidopsis* CEP1-kodierende Sequenz ist, und
wobei das RAR eine RAR-Domäne, umfassend eine Aminosäuresequenz (X₁)ₙX₂X₃X₄X₅X₆PGX₉SPGX₁₃GX₁₅ (SEQ ID NO: 459) umfasst, wobei n 1 oder 0 sein kann.

2. Verfahren nach Anspruch 1, wobei die RAR-kodierende Nucleinsäure funktional verknüpft ist mit einem Promotor, optional wobei der Promotor Wurzelspezifisch ist, optional wobei der Promotor der *Medicago truncatula RAR1*-Promotor (SEQ ID NO: 337) ist.

3. Verfahren nach Anspruch 2, wobei der Promotor konstitutiv ist oder durch Nährstoffstatus induzierbar ist, optional wobei der Promotor durch Stickstoffmangel oder hohes Kohlenstoffdioxid induzierbar ist.

4. Verfahren nach einem beliebigen der Ansprüche 1 bis 3, wobei die Pflanze eine größere Dichte an Wurzelhaaren an periodischen Wurzelstößen umfasst.

5. Verfahren nach einem beliebigen der Ansprüche 1 bis 4, wobei das RAR eine Aminosäuresequenz, ausgewählt aus SEQ ID NOs: 4, 6, 8, 10, 12, 14, 16, 18, 20, 22, 24, 26, 28, 30, 32, 34, 36 bis 147, 338-350, 365, 367, 369, 371, 373, 375, 377, 379, 381, 383, 385, 387-395 umfasst, oder eine RAR-Domäne mit einer Aminosäuresequenz, ausgewählt aus SEQ ID NOs: 149-336, 351-363 oder 396-415, umfasst, oder wobei die RAR-kodierende Nucleinsäure eine Nucleotidsequenz, ausgewählt aus SEQ ID NOs 3, 5, 7, 9, 11, 13, 15, 17, 19, 21, 23, 25, 27, 29, 31, 33, 35, 364, 366, 368, 370, 372, 374, 376, 378, 380, 382, 384 oder 386, umfasst.

6. Verfahren zum Fördern von Seitenwurzelwachstum und (-)Entwicklung einer Pflanze im Vergleich zu einer unbehandelten oder Wildtyp-Pflanze, umfassend Einführen mindestens einer Mutation in (a) ein oder mehrere RAR-kodierende Gene oder Stromaufwärts-Sequenzen davon; oder (b) ein oder mehrere RAR-Rezeptor-kodierende Gene oder Stromaufwärts-Sequenzen; in einer oder mehreren Pflanzenzellen, wobei die mindestens eine Mutation resultiert in:
(i) verringerter Expression von einem oder mehreren RARs, verringerter Expression von einem oder mehreren RAR-Rezeptoren, oder verringerter Expression von einem oder mehreren RARs und einem oder mehreren RAR-Rezeptoren, durch Zellen einer Pflanze, regeneriert aus oder umfassend eine(r) oder mehrere(n) der Pflanzenzellen, wobei die verringerte Expression des/der RAR(s) oder RAR-Rezeptors/en unter Bedingungen auftritt, die ansonsten die Expression des/der RAR(s) oder des/der RAR-Rezeptors/en fördern würden; oder
(ii) reduzierter Affinität von einem oder mehreren RARs für seine/ihre entsprechenden RAR-Rezeptoren, wobei diese reduzierte Affinität entsteht durch Modifikationen in dem/den RAR(s), dem/den RAR-Rezeptor(en) oder in sowohl exprimierten RAR(s) als auch RAR-Rezeptor(en), exprimiert von Zellen einer Pflanze, regeneriert aus oder umfassend eine(r) oder mehrere(n) Pflanzenzellen, und
wobei das RAR eine RAR-Domäne, umfassend eine Aminosäuresequenz (X₁)ₙX₂X₃X₄X₅X₆PGX₉SPGX₁₃GX₁₅ (SEQ ID NO: 459), umfasst, wobei n 1 oder 0 sein kann.

7. Verfahren nach Anspruch 6, wobei das RAR eine Aminosäuresequenz, ausgewählt aus SEQ ID NOs: 4, 6, 8, 10, 12, 14, 16, 18, 20, 22, 24, 26, 28, 30, 32, 34, 36 bis 147, 338-350, 365, 367, 369, 371, 373, 375, 377, 379, 381, 383, 385, 387-395, umfasst, oder eine RAR-Domäne mit einer Aminosäuresequenz, ausgewählt aus SEQ ID NOs: 149-336, 351-363 oder 396-415, umfasst, oder wobei die RAR-kodierende Nucleinsäure eine Nucleotidsequenz, ausgewählt aus SEQ ID NOs 3, 5, 7, 9, 11, 13, 15, 17, 19, 21, 23, 25, 27, 29, 31, 33, 35, 364, 366, 368, 370, 372, 374, 376, 378, 380, 382, 384 oder 386, umfasst.

8. Verfahren nach einem beliebigen der Ansprüche 1 bis 7, wobei das RAR eine RAR-Domäne, umfassend eine Aminosäuresequenz (X₁)ₙX₂X₃X₄X₅X₆PGX₉SPGX₁₃GX₁₅ (SEQ ID NO: 459), umfasst, wobei:
n 0 oder 1 sein kann
X₁ ausgewählt ist aus D, G, P, A, S, E und V;
X₂ ausgewählt ist aus F, V, R, T, S, A, K und Y;
X₃ ausgewählt ist aus R, K, E, H, Q, S, P, D, V, G, und A;
X₄ ausgewählt ist aus P und G;
X₅ ausgewählt ist aus T, S und G;
X₆ ausgewählt ist aus N, A, T, G, P, D, K und S;
X₉ ausgewählt ist aus N, H, Y und S;
X₁₃ ausgewählt ist aus I, A und V; und
X₁₅ ausgewählt ist aus N und H;
wobei
die Aminosäure an Position 6, falls Threonin oder Serin, phosphoryliert sein kann;
das P an Position 11, ein P an Position 4, oder beide solche Proline hydroxyliert sein kann/ können; und
Tyrosinreste sulphoniert sein können.

9. Pflanze mit verändertem Wachstumsmuster des horizontalen oder vertikalen Wurzelsystems relativ zu einer Wildtyp-Pflanze, oder Teil der Pflanze, wobei die Pflanze oder der Teil davon mindestens eine exogene RAR-kodierende Nukleinsäure umfasst, und wobei die Pflanze oder der Teil davon durch das Verfahren gemäß einem beliebigen der Ansprüche 1 bis 5 erhältlich ist.

## Revendications

1. Procédé pour réduire le développement racinaire latéral d'une plante par rapport à une plante non traitée ou de type sauvage, comprenant la mise en contact de la zone racinaire de ladite plante avec au moins un RAR ou l'introduction d'au moins un acide nucléique codant pour un RAR exogène dans une ou plusieurs cellules végétales, ce qui entraîne une expression accrue du RAR par les cellules d'une plante régénérée à partir de ou comprenant lesdites une ou plusieurs cellules végétales,
dans lequel ledit RAR n'est pas un peptide CEP1 d*'Arabidopsis thaliana* et ledit acide nucléique codant pour un RAR n'est pas une séquence codant pour le CEP1 d*'Arabidopsis,* et
dans lequel ledit RAR comprend un domaine de RAR comprenant une séquence d'acides aminés (X₁)ₙX₂X₃X₄X₅X₆PGX₉SPGX₁₃GX₁₅ (SEQ ID NO: 459), dans laquelle n peut être 1 ou 0.

2. Procédé selon la revendication 1, dans lequel ledit acide nucléique codant pour un RAR est en liaison fonctionnelle avec un promoteur, facultativement dans lequel ledit promoteur est spécifique aux racines, facultativement dans lequel ledit promoteur est le promoteur *RAR1 de Medicago truncatula* (SEQ ID NO: 337).

3. Procédé selon la revendication 2, dans lequel ledit promoteur est constitutif ou inductible en fonction du statut des nutriments, facultativement dans lequel ledit promoteur est inductible par une privation d'azote ou une teneur élevée en dioxyde de carbone.

4. Procédé selon l'une quelconque des revendications 1 à 3, dans lequel ladite plante comprend une plus grande densité de poils racinaires lors de poussées racinaires périodiques.

5. Procédé selon l'une quelconque des revendications 1 à 4, dans lequel ledit RAR comprend une séquence d'acides aminés sélectionnée parmi SEQ ID NO: 4, 6, 8, 10, 12, 14, 16, 18, 20, 22, 24, 26, 28, 30, 32, 34, 36 à 147, 338-350, 365, 367, 369, 371, 373, 375, 377, 379, 381, 383, 385, 387-395 ou comprend un domaine de RAR possédant une séquence d'acides aminés sélectionnée parmi SEQ ID NO: 149-336, 351-363 ou 396-415, ou dans lequel ledit acide nucléique codant pour un RAR comprend une séquence de nucléotides sélectionnée parmi SEQ ID NO: 3, 5, 7, 9, 11, 13, 15, 17, 19, 21, 23, 25, 27, 29, 31, 33, 35, 364, 366, 368, 370, 372, 374, 376, 378, 380, 382, 384 ou 386.

6. Procédé pour favoriser la croissance et le développement racinaire latéral d'une plante par rapport à une plante non traitée ou de type sauvage, comprenant l'introduction d'au moins une mutation dans (a) un ou plusieurs gènes codant pour un RAR ou des séquences en amont de ceux-ci ; ou (b) un ou plusieurs gènes codant pour un récepteur RAR ou des séquences en amont ; dans une ou plusieurs cellules végétales dont au moins une mutation entraîne :
(i) une expression réduite d'un ou de plusieurs RAR, une expression réduite d'un ou de plusieurs récepteurs RAR, ou une expression réduite d'un ou de plusieurs RAR et d'un ou de plusieurs récepteurs RAR par les cellules d'une plante régénérée à partir de ou comprenant lesdites une ou plusieurs cellules végétales, dans lequel ladite expression réduite dudit/desdits RAR ou récepteur(s) RAR se produit dans des conditions qui autrement favoriseraient l'expression dudit/desdits RAR ou récepteur(s) RAR ; ou
(ii) une affinité réduite d'un ou de plusieurs RAR pour leurs récepteurs RAR respectifs, laquelle affinité réduite survient par des modifications dans le(s) RAR, le(s) récepteur (s) RAR, ou à la fois dans le(s) RAR et le (s) récepteur(s) RAR exprimé(s) par les cellules d'une plante régénérée à partir de ou comprenant lesdites une ou plusieurs cellules végétales, et
dans lequel ledit RAR comprend un domaine de RAR comprenant une séquence d'acides aminés (X₁) ₙX₂X₃X₄X₅X₆PGX₉SPGX₁₃GX₁₅ (SEQ ID NO: 459), dans laquelle n peut être 1 ou 0.

7. Procédé selon la revendication 6, dans lequel ledit RAR comprend une séquence d'acides aminés sélectionnée parmi SEQ ID NO: 4, 6, 8, 10, 12, 14, 16, 18, 20, 22, 24, 26, 28, 30, 32, 34, 36 à 147, 338-350, 365, 367, 369, 371, 373, 375, 377, 379, 381, 383, 385, 387-395 ou comprend un domaine de RAR possédant une séquence d'acides aminés sélectionnée parmi SEQ ID NO: 149-336, 351-363 ou 396-415, ou dans lequel ledit acide nucléique codant pour un RAR comprend une séquence de nucléotides sélectionnée parmi SEQ ID NO: 3, 5, 7, 9, 11, 13, 15, 17, 19, 21, 23, 25, 27, 29, 31, 33, 35, 364, 366, 368, 370, 372, 374, 376, 378, 380, 382, 384 ou 386.

8. Procédé selon l'une quelconque des revendications 1 à 7, dans lequel ledit RAR comprend un domaine de RAR comprenant une séquence d'acides aminés (X₁) ₙX₂X₃X₄X₅X₆PGX₉SPGX₁₃GX₁₅ (SEQ ID NO: 459), dans laquelle :
n peut être 0 ou 1
X₁ est sélectionné parmi D, G, P, A, S, E et V ;
X₂ est sélectionné parmi F, V, R, T, S, A, K et Y ;
X₃ est sélectionné parmi R, K, E, H, Q, S, P, D, V, G, et A ;
X₄ est sélectionné parmi P et G ;
X₅ est sélectionné parmi T, S et G ;
X₆ est sélectionné parmi N, A, T, G, P, D, K et S ;
X₉ est sélectionné parmi N, H, Y et S ;
X₁₃ est sélectionné parmi I, A et V ; et
X₁₅ est sélectionné parmi N et H ;
dans laquelle
l'acide aminé à la position 6, s'il est la thréonine ou la sérine, peut être phosphorylé ; la P à la position 11, une P à la position 4, ou ces deux prolines peuvent être hydroxylées ; et
les résidus tyrosine peuvent être sulfonés.

9. Plante présentant un profil de croissance du système racinaire horizontal ou vertical altéré par rapport à une plante de type sauvage, ou partie de ladite plante, où ladite plante ou partie de celle-ci comprend au moins un acide nucléique codant pour un RAR exogène, et où ladite plante ou partie de celle-ci peut être obtenue par le procédé selon l'une quelconque des revendications 1 à 5.
